# EUROPEAN PATENT APPLICATION

(11) **EP 4 186 507 A1**
(43) Date of publication of application: **31.05.2023**
(21) Application number: 23150594.2
(22) Date of filing: 15.11.2018
(51) Int. Cl.: A61K 31/4745, A61K 31/519, A61K 45/06, A61P 1/16, A61P 11/00, A61P 43/00, A61K 9/00

(54) **COMPOUNDS AND PHARMACEUTICAL COMPOSITIONS THEREOF FOR USE IN THE TREATMENT OF FIBROTIC DISEASES**

(30) Priority: 15.11.2017 GB 201718838; 25.10.2018 GB 201817346
(62) Divisional of application: 18822268.1
(71) Applicant: Galapagos N.V., 2800 Mechelen (BE)
(72) Inventor: BRYS, Reginald, Christophe, Xavier, 2800 Mechelen (BE); CLÉMENT-LACROIX, Philippe, 93230 Romainville (FR); DE VOS, Steve, Irma, Joel, 2800 Mechelen (BE); SANIERE, Laurent, Raymond, Maurice, 93230 Romainville (FR)
(74) Representative: Geney, Raphaël Jean Joël

(57) **Abstract**

The present invention relates to compounds useful in the prophylaxis and/or treatment of one or more fibrotic diseases. In particular, the compounds of the invention antagonize GPR84, a G-protein-coupled receptor. The present invention also provides pharmaceutical compositions comprising the compounds for use and methods for the prophylaxis and/or treatment of one or more fibrotic diseases by administering said compound.

## Description

### FIELD OF THE INVENTION

The present invention relates to compounds useful in the prophylaxis and/or treatment of one or more fibrotic diseases. In particular, it has been identified that the compounds of the invention which antagonize GPR84, a G-protein-coupled receptor may useful in the prophylaxis and/or treatment of one or more fibrotic diseases. The present invention also provides pharmaceutical compositions comprising the compounds for use and methods for the prophylaxis and/or treatment of one or more fibrotic diseases by administering said compound.

### BACKGROUND OF THE INVENTION

Fibrosis is a process that can be triggered by chronic tissue damage because of toxic substances, viral infection, inflammation, or mechanical stress (Nanthakumar et al., 2015); and may be defined as the abnormal or excessive production and accumulation of extracellular matrix (ECM).

In particular, fibrosis is a key driver of progressive organ dysfunction in many inflammatory and metabolic diseases, including idiopathic pulmonary fibrosis (IPF), advanced liver disease (e.g. non-alcoholic steatohepatitis (NASH)) and advanced kidney disease. These conditions remain poorly treated despite advances in the understanding of the disease mechanism and, more recently, an increase in the number of clinical trials reflecting the need to identify new treatments, particularly in IPF (Nanthakumar et al., 2015).

Non-alcoholic fatty liver disease (NAFLD) is initially characterized by pure steatosis with progression to non-alcoholic steatohepatitis (NASH), mainly caused by excess energy intake and physical inactivity apart from genetic defects, and closely associated with obesity, insulin resistance, and other related metabolic complications. (Neuschwander-Tetri and Caldwell, 2003). If untreated, NASH leads to lethal liver failure.

The mechanisms that promote the progression from NAFLD to NASH and end-stage liver diseases are complex and may be triggered by an acute inflammatory insult and oxidative stress. (Day and James, 1998).

GPR84 (also known as EX33) has been isolated and characterized from human B cells (Wittenberger et al., 2001) and also using a degenerate primer reverse transcriptase-polymerase chain reaction (RT-PCR) approach (Yousefi et al., 2001). It remained an orphan GPCR until the identification of medium-chain Free Fatty Acids (FFAs) with carbon chain lengths of 9-14 as ligands for this receptor (Wang et al., 2006).

GPR84 is activated by medium-chain FFAs, such as capric acid (C10:0), undecanoic acid (C11:0) and lauric acid (12:0) which amplify lipopolysaccharide stimulated production of pro-inflammatory cytokines/chemokines (TNFa, IL-6, IL-8, CCL2 and others), and is highly expressed in neutrophils and monocytes (macrophages).(Miyamoto et al., 2016)

In contrast, GPR84-ligand mediated chemotaxis of neutrophils and monocytes/macrophages is inhibited by GPR84 antagonists. (Suzuki et al., 2013)

Although the recruitment of monocytes/macrophages to livers may appear to occur concomitantly with fibrogenesis in patients with chronic liver diseases (Marra et al., 1998; Zimmermann et al., 2010), this has not resulted in novel therapies.

No approved drug for the treatment of NASH is available at present, and consequently liver transplant remains the last option for end stage disease status. In the case of IPF for example, only two drugs have been approved despite their undesirable side effects (Brunnemer et al., 2018; Lancaster et al., 2017; Richeldi et al., 2014), and therefore there is clear need for improved therapies (Raghu, 2015).

Therefore, the identification and development of novel compounds for use in the preparation of a medicament for the prophylaxis and/or treatment of one or more fibrotic diseases, and more particularly NASH and/or IPF remains highly desirable.

### SUMMARY OF THE INVENTION

The present invention relates to compounds useful in the prophylaxis and/or treatment of one or more fibrotic diseases. In particular, it has been identified that the compounds of the invention which antagonize GPR84, a G-protein-coupled receptor may be useful in the prophylaxis and/or treatment of one or more fibrotic diseases. The present invention also provides pharmaceutical compositions comprising the compounds for use and methods for the prophylaxis and/or treatment of one or more fibrotic diseases, by administering said compound.

Accordingly, in a first aspect of the invention, are provided compounds having GPR84 antagonist activity for use in the prophylaxis and/or treatment of one or more fibrotic diseases.

In a particular aspect, are provided the compounds of Formula I, II, III, and IV, respectively disclosed in WO 2013/092791, WO 2014/095798, WO 2015/197550, and WO 2016/169911 the entire disclosure being incorporated herein by reference for use in the prophylaxis and/or treatment of one or more fibrotic diseases.

The compounds of Formula I are shown below: 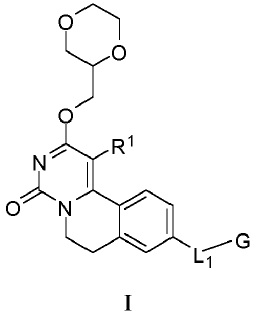 wherein
R¹ is H, Me, or halo;
L₁ is absent or is -O-, -S-, or -NR^{4a}-;
Gis
   - R²,
   - -W-L₂-R², or
   - -W-L₃-R³;
W is C₁₋₄ alkylene, C₂₋₄ alkenylene having one double bond, or C₂₋₄ alkynylene having one triple bond; L₂ is absent or is -O-;
R² is
   - H,
   - C₁₋₈ alkyl, optionally substituted with one to three groups independently selected from
      ∘ OH,
      ∘ halo,
      ∘ CN,
      ∘ C₁₋₆ alkoxy,
      ∘ C₃₋₇ cycloalkyl,
      ∘ 4-6 membered heterocycloalkyl comprising one to three heteroatoms independently selected from S, and O,
      ∘ 5-6 membered heteroaryl comprising one to three heteroatoms independently selected from N, S, and O, and
      ∘ phenyl,
   - C₄₋₇ cycloalkenyl comprising one double bond,
   - 5-7 membered heterocycloalkenyl comprising one double bond, and one to three heteroatoms independently selected from N, O, and S,
   - C₃₋₇ cycloalkyl optionally substituted with one or more independently selected R⁵ groups,
   - 4-10 membered heterocycloalkyl comprising one to two heteroatoms independently selected from S, and O, optionally substituted with one to three independently selected R⁵ groups,
   - 5-10 membered heteroaryl comprising one to three heteroatoms independently selected from N, S, and O, optionally substituted with one to three independently selected R⁶ groups, or
   - C₆₋₁₀ aryl optionally substituted with one or more independently selected R⁶ groups;
L₃ is -NR^{4b}-;
R³ is
   - C₁₋₄ alkyl substituted with
      ∘ C₆₋₁₀ aryl optionally substituted with one or more independently selected R⁷ groups, or
      ∘ 5-10 membered heteroaryl comprising one to three heteroatoms independently selected from N, S, and O, optionally substituted with one or more independently selected R⁷ groups,
   - 5-10 membered heteroaryl comprising one to three heteroatoms independently selected from N, S, and O, optionally substituted with one or more independently selected R⁷ groups, or
   - C₆₋₁₀ aryl optionally substituted with one or more independently selected R⁷ groups;
Each R^{4a} and R^{4b} is independently selected from H, C₁₋₄ alkyl, and C₃₋₇ cycloalkyl;
R⁵ is oxo or R⁶;
R⁶ is
   - OH,
   - halo,
   - -NOz,
   - C₁₋₆ alkyl optionally substituted with one to three groups independently selected from halo, and OH,
   - C₁₋₆ alkoxy optionally substituted with one to three groups independently selected from halo, and OH,
   - C₃₋₇ cycloalkyl,
   - -C(=O)OR⁸,
   - -C(=O)NR⁹R¹⁰,
   - -NHC(=O)-C₁₋₄ alkyl,
   - -CN,
   - phenyl,
   - -O-phenyl,
   - 4-7 membered heterocycloalkyl comprising one to three heteroatoms independently selected from N, O, and S, or
   - 5-6 membered heteroaryl comprising one to three heteroatoms independently selected from N, O, and S; optionally substituted with one or more independently selected C₁₋₄ alkyl, C₁₋₄ alkoxy, CN, halo, and -C(=O)OR¹¹;
R⁷ is C₁₋₄ alkyl, or halo, and
each of R⁸, R⁹, R¹⁰ and R¹¹ is independently selected from H and C₁₋₄ alkyl.

The compounds of Formula II are shown below: 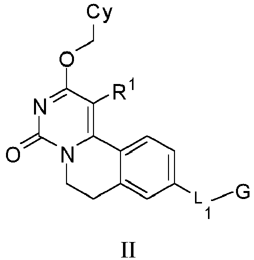 wherein
Cy is 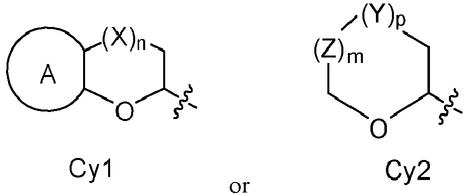
wherein
X is O or S;
Y is -CH₂-, or S;
Z is -CH₂-;
each of the subscript n, m, or p is independently selected from 0, and 1; and A is phenyl, or 5-6-membered heteroaryl comprising one or two N-atoms; optionally substituted with one or more independently selected
R⁵ groups;
any one of Cy1 and Cy2 is optionally substituted by one or more independently selected C₁₋₄ alkyl groups;
R¹ is H, Me, or halo;
L₁ is absent or is -O-, -S-, or -NR^{4a}-;
G is
   - R²,
   - -W-L₂-R², or
   - -W-L₃-R³;
W is C₁₋₄ alkylene, C₂₋₄ alkenylene having one double bond, or C₂₋₄ alkynylene having one triple bond;
L₂ is absent or is -O-;
R² is
   - H,
   - C₁₋₈ alkyl optionally substituted with one to three groups independently selected from
      ∘ OH,
      ∘ halo,
      ∘ CN,
      ∘ C₁₋₆ alkoxy,
      ∘ C₃₋₇ cycloalkyl,
      ∘ 4-6 membered heterocycloalkyl (comprising one to three heteroatoms independently selected from S, and O),
      ∘ 5-6 membered heteroaryl (comprising one to three heteroatoms independently selected from N, S, and O), and
      ∘ phenyl,
   - C₄₋₇ cycloalkenyl comprising one double bond,
   - 5-7 membered heterocycloalkenyl comprising one double bond, and one to three heteroatoms independently selected from O, and S,
   - C₃₋₇ cycloalkyl (optionally substituted with one or more independently selected R⁶ groups),
   - 4-10 membered heterocycloalkyl comprising one to two heteroatoms independently selected from S, and O, (optionally substituted with one to three independently selected R⁶ groups),
   - 5-10 membered heteroaryl comprising one to three heteroatoms independently selected from N, S, and O (optionally substituted with one to three independently selected R⁷ groups), or
   - C₆₋₁₀ aryl (optionally substituted with one or more independently selected R⁷ groups);
L₃ is -NR^{4b}-;
R³ is
   - C₁₋₄ alkyl substituted with C₆₋₁₀ aryl (optionally substituted with one or more independently selected R⁸ groups), or 5-10 membered heteroaryl comprising one to three heteroatoms independently selected from N, S, and O, (optionally substituted with one or more independently selected R⁸ groups),
   - 5-10 membered heteroaryl comprising one to three heteroatoms independently selected from N, S, and O, (optionally substituted with one or more independently selected R⁸ groups), or
   - C₆₋₁₀ aryl (optionally substituted with one or more independently selected R⁸ groups);
each R^{4a} and R^{4b} is independently selected from H, C₁₋₄ alkyl, and C₃₋₇ cycloalkyl;
R⁵ is halo, C₁₋₄ alkyl or C₁₋₄ alkoxy;
R⁶ is oxo or R⁷;
R⁷ is
   - OH,
   - halo,
   - -NOz,
   - C₁₋₆ alkyl (optionally substituted with one to three groups independently selected from halo, and OH),
   - C₁₋₆ alkoxy (optionally substituted with one to three groups independently selected from halo, and OH),
   - C₃₋₇ cycloalkyl,
   - -C(=O)OR⁹,
   - -C(=O)NR¹⁰R¹¹,
   - -NHC(=O)-C₁₋₄ alkyl,
   - -CN,
   - phenyl,
   - -O-phenyl,
   - 4-7 membered heterocycloalkyl comprising one to three heteroatoms independently selected from N, O, and S, or
   - 5-6 membered heteroaryl comprising one to three heteroatoms independently selected from N, O, and S; (optionally substituted with one or more independently selected C₁₋₄ alkyl, C₁₋₄ alkoxy, CN, halo, and -C(=O)OR¹²);
R⁸ is C₁₋₄ alkyl, or halo; and
each of R⁹, R¹⁰, R¹¹ and R¹², is independently selected from H and C₁₋₄ alkyl.

The compounds of Formula III are shown below: 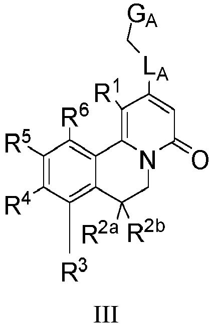 wherein
R¹ is H, C₁₋₄ alkyl, or cyclopropyl;
L_{A} is O or NH;
Ga is:
   - 4-6 membered monocyclic heterocycloalkyl containing one or two O,
   - C₃₋₇ monocyclic cycloalkyl, or
   - a bicyclic group of formula Cy: 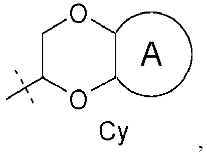
wherein A is phenyl or 5-6 membered heteroaryl containing one or two heteroatoms independently selected from N, O and S;
each R^{2a} and R^{2b} are independently H or -CH₃;
R³ is H, -OH or -OCH₃;
R⁴ is -CN or -L₁-W₁-G₁,
wherein
   - L₁ is absent or O,
   - W₁ is absent, or is C₁₋₆ alkylene, C₂₋₄ alkenylene having one double bond or C₂₋₄ alkynylene having one triple bond, each of which is optionally substituted with one or more independently selected halo, -CN or C₁₋₄ alkoxy,
   - G₁ is
      ∘ H,
      ∘ -CF₃,
      ∘ -C(=O)-C₁₋₄ alkyl,
      ∘ -S(=O)₂-C₁₋₄ alkyl, optionally substituted with one or more independently selected halo,
      ∘ 4-6 membered monocyclic heterocycloalkyl containing one or two O (which heterocycloalkyl is optionally substituted with one or more independently selected R⁷ groups),
      ∘ 6 membered monocyclic heterocycloalkenyl containing one or two O (which heterocycloalkenyl is optionally substituted with one or more independently selected R⁷ groups),
      ∘ C₃₋₇ monocyclic cycloalkyl optionally substituted with one or more independently selected R⁷ groups,
      ∘ phenyl optionally substituted with one or more independently selected R⁷ groups,
      ∘ or 5-6 membered heteroaryl containing one to four heteroatoms independently selected from N, O and S (which heteroaryl is optionally substituted with one or more independently selected R⁷ groups),
each R⁷ is:
   - halo,
   - -OH,
   - C₁₋₄ alkyl, C₃₋₄ monocyclic cycloalkyl, or C₁₋₄ alkoxy, each of which is optionally substituted with one or more independently selected halo;
R⁵ is -CN or -L₂-W₂-G₂,
wherein
   - L₂ is absent, O or S,
   - W₂ is absent or C₁₋₄ alkylene, optionally substituted with one or more independently selected halo,
   - Gz is
      ∘ H,
      ∘ -CF₃,
      ∘ C₃₋₇ monocyclic cycloalkyl (which cycloalkyl is optionally substituted with one or more independently selected halo),
      ∘ phenyl,
      ∘ or 5-6 membered heteroaryl containing one to three heteroatoms independently selected from N, O and S; and
R⁶ is H, -OH or -OCH₃.

The compounds of Formula IV are shown below: 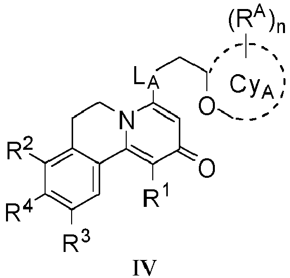 wherein
L_{A} is O, or NH;
Cy_{A} is monocyclic 4-6 membered heterocycloalkyl, comprising one or two O atoms;
each R^{A} is independently selected from halo, and C₁₋₃ alkyl;
the subscript n is 0, 1 or 2;
R¹ is H or C₁₋₃ alkyl;
R² is H, -OH, or C₁₋₃ alkoxy;
R³ is H or C₁₋₃ alkoxy;
R⁴ is
   - -CN,
   - -OH,
   - -O-S(=O)₂-C₁₋₄ alkyl optionally substituted with one or more independently selected halo, or
   - -L₁-W₁-G₁;
L₁ is a direct bond, -O-, -S-, -SO₂-, -C(=O)NR^{5a}-, -NR^{5b}C(=O)-, or -NR^{5c}-;
R^{5a}, R^{5b} and R^{5c} are independently H or C₁₋₄ alkyl;
W₁ is a direct bond or C₁₋₂ alkylene optionally substituted with one or more independently selected halo;
G₁ is
   - C₃₋₆ cycloalkyl optionally substituted with one or more independently selected halo,
   - 5-6 membered heteroaryl comprising one to four heteroatoms independently selected from N, O, and S, which heteroaryl is optionally substituted with one or more independently selected C₁₋₄ alkyl,
   - 5-7 membered heterocycloalkenyl comprising one double bond, and one to three heteroatoms independently selected from N, O, and S, which heterocycloalkenyl is optionally substituted with one or more independently selected R⁶,
   - monocyclic or spiro bicyclic 4-8 membered heterocycloalkyl comprising one to three heteroatoms independently selected from N, O, and S, which heterocycloalkyl is optionally substituted with one or more independently selected R⁶,
   - monocyclic 4-6 membered heterocycloalkyl comprising one to three heteroatoms independently selected from N, O, and S, fused to one or two phenyls,
   - C₁₋₄ alkyl optionally substituted with one or more independently selected halo, -NR^{7a}R^{7b}, or C₁₋₄ alkoxy, which alkoxy is optionally substituted with one or more independently selected halo,
   - phenyl optionally substituted with one or more independently selected halo or C₁₋₄ alkoxy, which alkoxy is optionally substituted with one or more independently selected halo;
R⁶ is
   - halo,
   - =O,
   - -CN,
   - -OH,
   - -C(=O)-C₁₋₄ alkoxy optionally substituted with one or more independently selected halo,
   - -C(=O)-C₃₋₄ cycloalkyl,
   - -S(=O)₂-C₁₋₄ alkyl,
   - C₁₋₄ alkyl optionally substituted by one or more independently selected C1-3 alkoxy, halo, or -OH,
   - C₁₋₄ alkoxy,
   - phenyl optionally substituted by one or more independently selected halo,
   - -C(=O)-monocyclic 4-6 membered heterocycloalkyl comprising one to three heteroatoms independently selected from N, O, and S,
   - -C(=O)NR^{8a}R^{8b}, or
   - 5-7 membered heteroaryl comprising one to four heteroatoms independently selected from N, O, and S, which heteroaryl is optionally substituted with one or more independently selected C₁₋₄ alkyl;
R^{7a} and R^{7b} are independently H or C₁₋₄ alkyl, and
R^{8a} and R^{8b} are independently H or C₁₋₃ alkyl.

In a particular aspect, the compounds of the invention are provided for use in the prophylaxis and / or treatment of one or more fibrotic diseases.in a more particular aspect the fibrotic disease is NASH.

In particular, it has now been identified that the compounds of the invention, may induce a reduction of the NAS score in mice, a clinical NASH diagnostic severity index, in particular the NAS steatosis component diagnostic. In a further embodiment, the compounds of the invention induce a reduction of the NAS score by at least 1, at least 2, at least 3, or at least 4.

In a further embodiment, the present invention disclosed a method for treating NASH comprising the steps of
a. Measuring the NAS score in a subject,
b. Administering a therapeutically effective amount of a compound according to Formula I, II, III, or IV;
c. Repeating step a above,
d. Calculating the response to the treatment of said subject,
e. Determining whether the dose of the compound according to Formula I, II, III, or IV should be decreased, maintained or increased based on the response of step d.

In a further embodiment, the present invention disclosed a method for treating NASH comprising the steps of
a. Measuring one or more NASH associated biomarkers levels in a subject, wherein said biomarker is selected from CCL2, Col1a1, TNFα, ALT or AST.
b. Administering a therapeutically effective amount of a compound according to Formula I, II, III, or IV;
c. Repeating step a above,
d. Calculating the response to the treatment of said subject,
e. Determining whether the dose of the compound according to Formula I, II, III, or IV should be decreased, maintained or increased based on the response of step d.

In a further aspect, the present invention provides pharmaceutical compositions comprising a compound of the invention, and a pharmaceutical carrier, excipient or diluent for use in treatment of one or more fibrotic diseases. In a particular aspect, the pharmaceutical composition may additionally comprise further therapeutically active ingredients suitable for use in combination with the compounds of the invention. In a further particular aspect, said further therapeutic agent is for the treatment and/or prophyslaxis of fibrotic disease. In a more particular aspect, the further therapeutically active ingredient is an agent for the treatment of NASH. Also in a more particular aspect, the further therapeutically active ingredient is an agent for the treatment of IPF.

Moreover, the compounds of the invention, useful in the pharmaceutical compositions and treatment methods disclosed herein, are pharmaceutically acceptable as prepared and used.

In a further aspect of the invention, this invention provides a method of treating a mammal, in particular humans, afflicted with a condition selected from among those listed herein, and particularly NASH, which method comprises administering an effective amount of the pharmaceutical composition or compounds of the invention as described herein.

The present invention also provides pharmaceutical compositions comprising a compound of the invention, and a suitable pharmaceutical carrier, excipient or diluent for use fibrotic diseases, and more particularly NASH.

In a further aspect of the invention, this invention provides a method of treating a mammal, in particular humans, suffering IPF, which method comprises administering an effective amount of the pharmaceutical composition or compounds of the invention as described herein.

The present invention also provides pharmaceutical compositions comprising a compound of the invention, and a suitable pharmaceutical carrier, excipient or diluent for use in IPF.

In additional aspects, this invention provides methods for synthesizing the compounds of the invention, with representative synthetic protocols and pathways disclosed later on herein.

Other objects and advantages will become apparent to those skilled in the art from a consideration of the ensuing detailed description.

It will be appreciated that compounds of the invention may be metabolized to yield biologically active metabolites.

### DESCRIPTION OF THE FIGURES

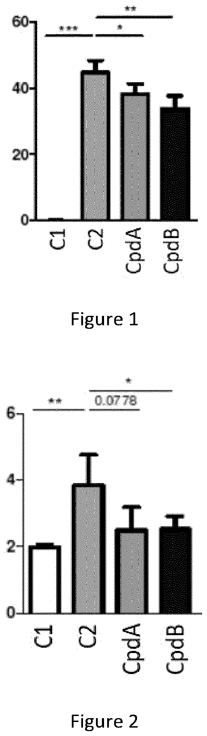

**Figure 1** relates to example 3.2 and shows the necrotic area fraction [%] observed in liver samples obtained from animals in control group 1 (C1), control group 2 (C2), test group dosed with Compound A (CpdA) and test group dosed with Compound B (CpdB); * means p<0.05, ** means p<0.01, *** means p<0.001.
**Figure 2** relates to example 3.2 and shows the F4/80 stained area fraction [%] observed in liver samples obtained from animals in control group 1 (C1), control group 2 (C2), test group dosed with Compound A (CpdA) and test group dosed with Compound B (CpdB); * means p<0.05, ** means p<0.01, *** means p<0.001.

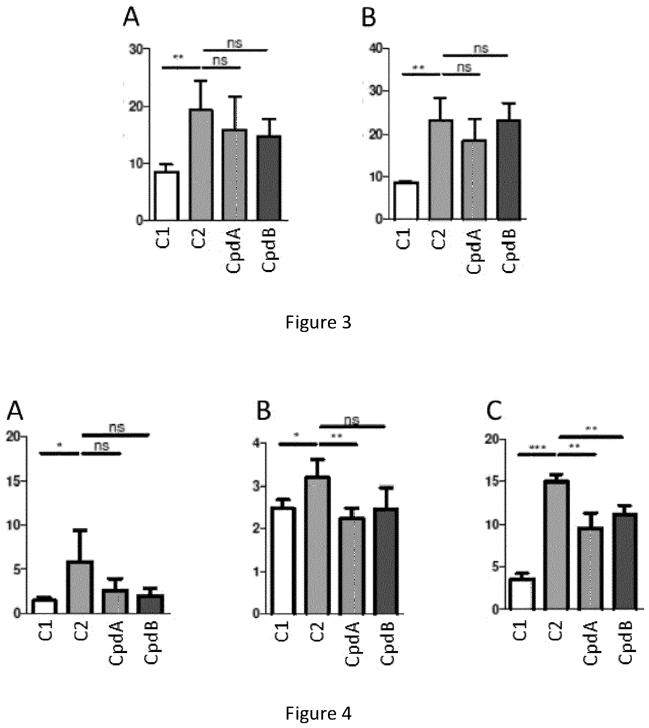

**Figure 3** relates to example 3.2 and shows the percentage of neutrophils (panel A) and of monocytes (panel B) in total leucocyte cells of blood samples obtained from animals in control group 1 (C1), control group 2 (C2), test group dosed with Compound A (CpdA) and test group dosed with Compound B (CpdB); ** means p<0.01, ns means not statistically significant.
**Figure 4** relates to example 3.2 and shows the percentage of neutrophils (panel A), of monocytes (panel B) and of MoMF in total leucocyte cells of liver samples obtained from animals in control group 1 (C1), control group 2 (C2), test group dosed with Compound A (CpdA) and test group dosed with Compound B (CpdB); * means p<0.05, ** means p<0.01, *** means p<0.001, ns means not statistically significant.

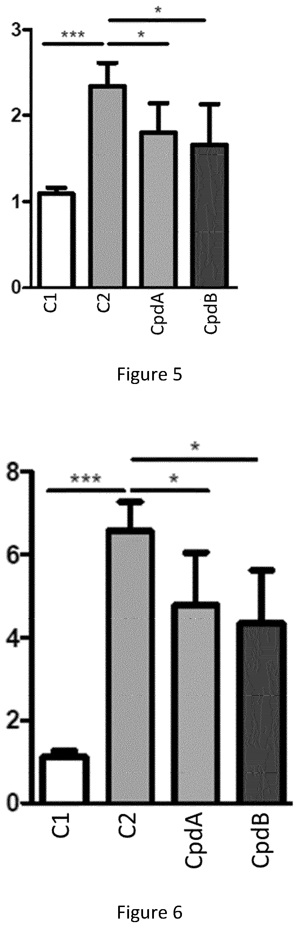

**Figure 5** relates to example 3.3 and shows the necrotic area fraction [%] observed in liver samples obtained from animals in control group 1 (C1), control group 2 (C2), test group dosed with Compound A (CpdA) and test group dosed with Compound B (CpdB); * means p<0.05, *** means p<0.001.
**Figure 6** relates to example 3.3 and shows the F4/80 stained area fraction [%] observed in liver samples obtained from animals in control group 1 (C1), control group 2 (C2), test group dosed with Compound A (CpdA) and test group dosed with Compound B (CpdB); * means p<0.05, *** means p<0.001.

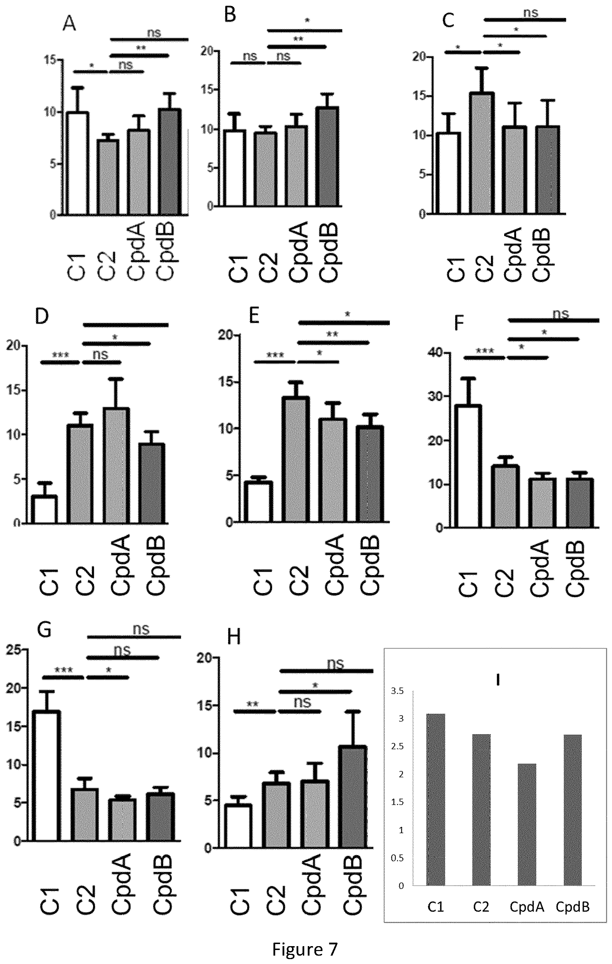

**Figure 7** relates to example 3.3 and shows Fluorescence-activated cell sorting (FACS) results measured in blood and liver samples obtained from animals in control group 1 (C1), control group 2 (C2), test group dosed with Compound A (CpdA) and test group dosed with Compound B (CpdB) for CD4 cells (Panel A), CD8 cells (Panel B), Blood monocyte (Panel C), Liver neutrophils cells (Panel D), Liver infiltrating monocyte-derived macrophage (MoMF) cells (Panel E), Liver CD19 cells (Panel F), Liver Natural Killer (NK) cells (Panel G), Liver Natural Killer T (NKT) cells (Panel H) and liver Kupfer cells (Panel I); * means p<0.05, ** means p<0.01, *** means p<0.001, ns means not statistically significant.

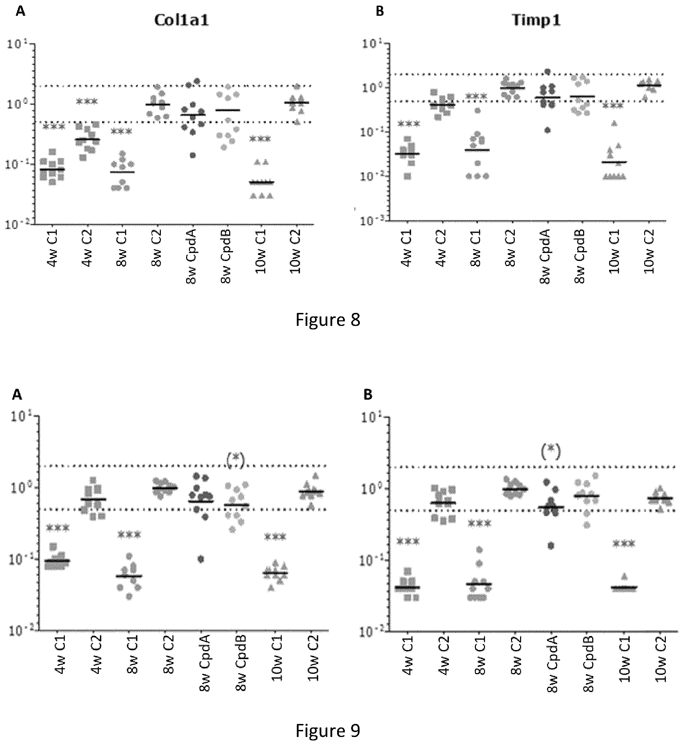

**Figure 8** relates to example 3.3 and shows gene expression of Col1a1 (panel A) and Timp1 (panel B) measured in blood samples obtained from animals in control group 1 (C1), control group 2 (C2), test group dosed with Compound A (CpdA) and test group dosed with Compound B (CpdB), expressed as Normalised Relative Quantity scaled versus disease; *** means p<0.001.
**Figure 9** relates to example 3.3 and shows gene expression of TNFα (panel A) and CCL2 (panel B) measured in liver samples obtained from animals in control group 1 (C1), control group 2 (C2), test group dosed with Compound A (CpdA) and test group dosed with Compound B (CpdB), expressed as Normalised Relative Quantity scaled versus disease; *** means p<0.001, (*) means p<0.05 and biological trend (i.e., 0.5<NRQ-scaled<0.7 or 1.4<NRQ-scaled<2).

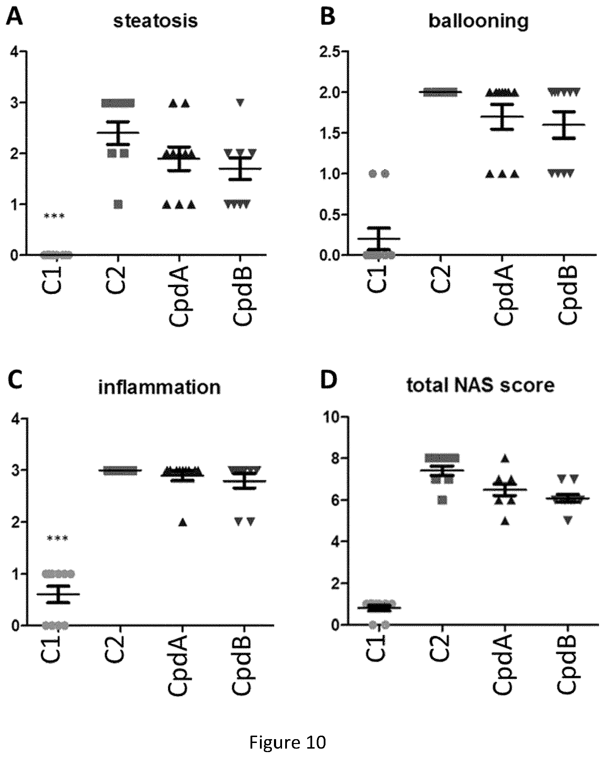

**Figure 10** relates to example 3.3 and shows non-alcoholic fatty liver disease activity scoring (NAS) data from animals in control group 1 (C1), control group 2 (C2), test group dosed with Compound A (CpdA) and test group dosed with Compound B (CpdB); panel A - steatosis score, panel B - ballooning score, panel C - inflammation score, panel D - total NAS score; *** means p<0.001 versus control group 2.

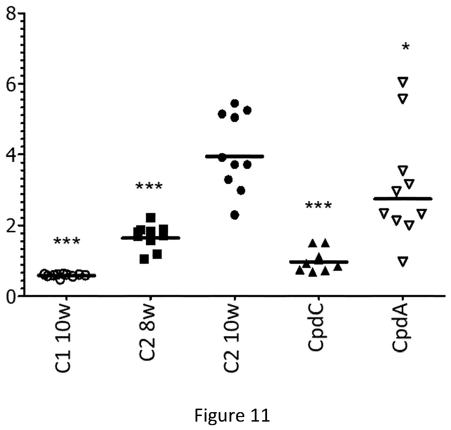

**Figure 11** relates to example 3.4 and shows the fibrotic area fraction [%] observed in liver samples obtained from animals in control group 1 (C1), control group 2 (C2; after 8 weeks and after 10 weeks), test group dosed with Compound A (CpdA) and test group dosed with elafibranor (CpdC); * means p<0.05, *** means p<0.001.

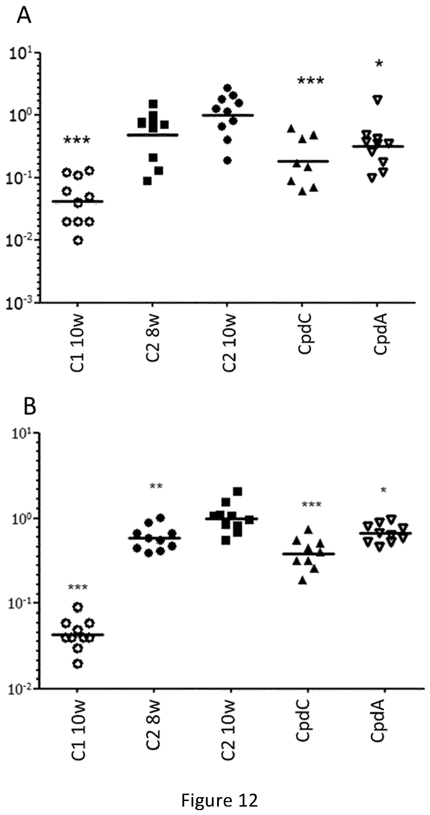

**Figure 12** relates to example 3.4 and shows data on gene expression of Col1A1 (panel A) and TNFα (panel B) as measured in samples obtained from animals in control group 1 (C1), control group 2 (C2; after 8 weeks and after 10 weeks), test group dosed with Compound A (CpdA) and test group dosed with elafibranor (CpdC), expressed as Normalised Relative Quantity scaled versus disease; * means p<0.05, ** means p<0.01, *** means p<0.001.

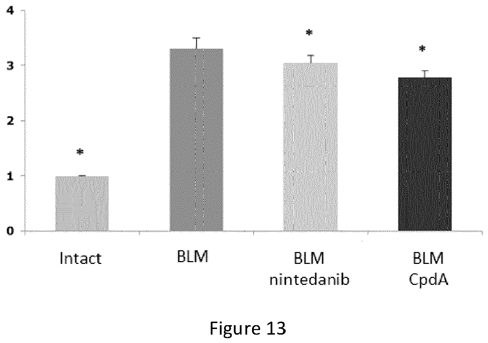

**Figure 13** relates to example 3.5 and shows average ± s.e.m. Ashcroft scores with Matsuse's modification after 2 week dosing period initiated 1 week after bleomycin instillation for animals in control group 1 (Intact; sham), control group 2 (BLM; vehicle), control group 3 dosed with nintedanib at 60 mg/kg q.d. (BLM nintedanib) and test group dosed with Compound A at 30 mg/kg b.i.d. (BLM CpdA); * means p<0.05 versus BLM vehicle group.

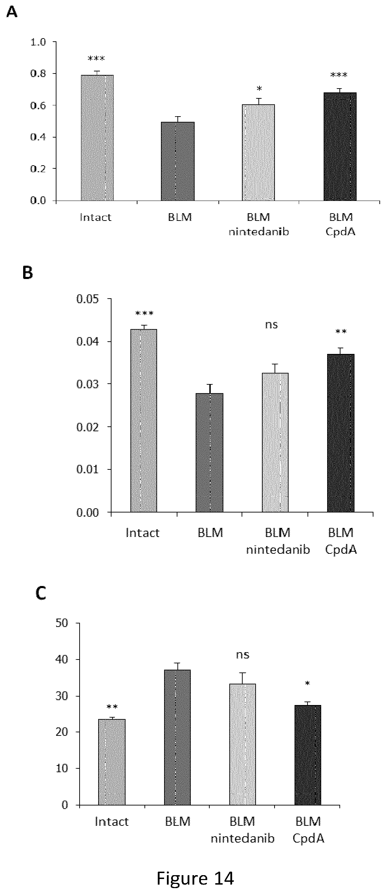

**Figure 14** relates to example 3.5 and shows Pressure-Volume loop perturbation after 2 week dosing period initiated 1 week after bleomycin instillation for animals in control group 1 (Intact; sham), control group 2 (BLM; vehicle), control group 3 dosed with nintedanib at 60 mg/kg q.d. (BLM nintedanib) and test group dosed with Compound A at 30 mg/kg b.i.d. (BLM CpdA); panel A - average ± s.e.m. Inspiratory Capacity (mL), panel B - average ± s.e.m. Compliance of the Respiratory System (mL/cmHzO), panel C - average ± s.e.m. Elastance of the Respiratory System (cmH₂O/mL); ^{∗} means p<0.05, ** means p<0.01, *** means p<0.001, ns means not statistically significant versus BLM vehicle group.

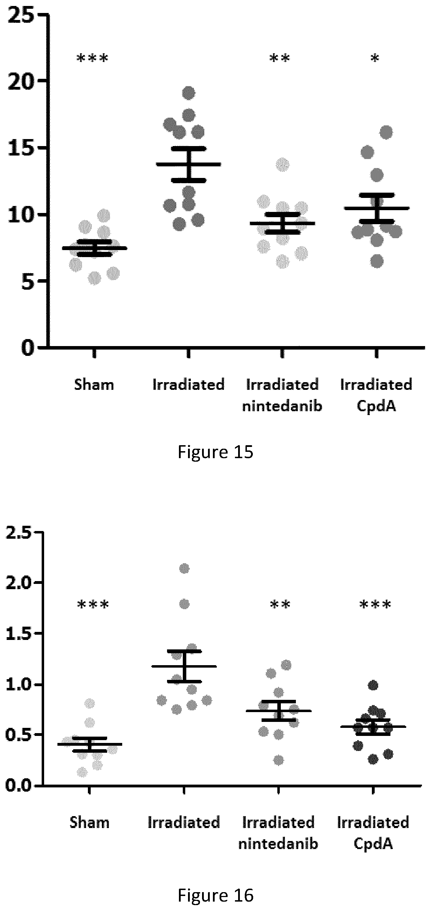

**Figure 15** relates to example 3.6 and shows collagen deposition induced by irradiation as type I collagen stained area fraction [%] in microscopical images of irradiated mouse lung post immunostaining for animals in control group 1 (Sham), control group 2 (Irradiated; vehicle), control group 3 dosed with nintedanib at 60 mg/kg q.d. (Irradiated nintedanib) and test group dosed with Compound A at 30 mg/kg q.d. (Irradiated CpdA); * means p<0.05, ** means p<0.01, *** means p<0.001 versus the Irradiated vehicle group.
**Figure 16** relates to example 3.6 and shows MnSOD stained area fraction [%] in microscopical images of irradiated mouse lung post immunostaining for animals in control group 1 (Sham), control group 2 (Irradiated; vehicle), control group 3 dosed with nintedanib at 60 mg/kg q.d. (Irradiated nintedanib) and test group dosed with Compound A at 30 mg/kg q.d. (Irradiated CpdA); ** means p<0.01, *** means p<0.001 versus the Irradiated vehicle group.

### DETAILED DESCRIPTION OF THE INVENTION

### Definitions

The following terms are intended to have the meanings presented therewith below and are useful in understanding the description and intended scope of the present invention.

When describing the invention, which may include compounds, pharmaceutical compositions containing such compounds and methods of using such compounds and compositions, the following terms, if present, have the following meanings unless otherwise indicated. It should also be understood that when described herein any of the moieties defined forth below may be substituted with a variety of substituents, and that the respective definitions are intended to include such substituted moieties within their scope as set out below. Unless otherwise stated, the term "substituted" is to be defined as set out below. It should be further understood that the terms "groups" and "radicals" can be considered interchangeable when used herein.

The articles 'a' and 'an' may be used herein to refer to one or to more than one (*i.e.* at least one) of the grammatical objects of the article. By way of example `an analogue' means one analogue or more than one analogue.

'Alkyl' means straight or branched aliphatic hydrocarbon having the specified number of carbon atoms. Particular alkyl groups have 1 to 6 carbon atoms or 1 to 4 carbon atoms. Branched means that one or more alkyl groups such as methyl, ethyl or propyl is attached to a linear alkyl chain. Particular alkyl groups are methyl (-CH₃), ethyl (-CH₂-CH₃), n-propyl (-CH₂-CH₂-CH₃), isopropyl (-CH(CH₃)₂), n-butyl (-CH₂-CH₂-CH₂-CH₃), tert-butyl (-CH₂-C(CH₃)₃), sec-butyl (-CH₂-CH(CH₃)₂), n-pentyl (-CH₂-CH₂-CH₂-CH₂-CH₃), n-hexyl (-CH₂-CH₂-CH₂-CH₂-CH₂-CH₃), and 1,2-dimethylbutyl (-CHCH₃)-C(CH₃)H₂-CH₂-CH₃). Particular alkyl groups have between 1 and 4 carbon atoms.

'Alkenyl' refers to monovalent olefinically (unsaturated) hydrocarbon groups with the number of carbon atoms specified. Particular alkenyl has 2 to 8 carbon atoms, and more particularly, from 2 to 6 carbon atoms, which can be straight-chained or branched and having at least 1 and particularly from 1 to 2 sites of olefinic unsaturation. Particular alkenyl groups include ethenyl (-CH=CH₂), n-propenyl (-CH₂CH=CH₂), isopropenyl (-C(CH₃)=CH₂) and the like.

`Alkylene' refers to divalent alkene radical groups having the number of carbon atoms specified, in particular having 1 to 6 carbon atoms and more particularly 1 to 4 carbon atoms which can be straight-chained or branched. This term is exemplified by groups such as methylene (-CH₂-), ethylene (-CH₂-CH₂-), or -CH(CH₃)- and the like.

'Alkynylene' refers to divalent alkyne radical groups having the number of carbon atoms and the number of triple bonds specified, in particular 2 to 6 carbon atoms and more particularly 2 to 4 carbon atoms which can be straight-chained or branched. This term is exemplified by groups such as -C≡C-, -CH₂-C≡C-, and -C(CH₃)H-C≡CH-.

'Alkoxy' refers to the group O-alkyl, where the alkyl group has the number of carbon atoms specified. In particular the term refers to the group -O-C₁₋₆ alkyl. Particular alkoxy groups are methoxy, ethoxy, n-propoxy, isopropoxy, n-butoxy, tert-butoxy, sec-butoxy, n-pentoxy, n-hexoxy, and 1,2-dimethylbutoxy. Particular alkoxy groups are lower alkoxy, i.e. with between 1 and 6 carbon atoms. Further particular alkoxy groups have between 1 and 4 carbon atoms.

`Amino' refers to the radical -NH₂.

'Aryl' refers to a monovalent aromatic hydrocarbon group derived by the removal of one hydrogen atom from a single carbon atom of a parent aromatic ring system. In particular aryl refers to an aromatic ring structure, monocyclic or fused polycyclic, with the number of ring atoms specified. Specifically, the term includes groups that include from 6 to 10 ring members. Particular aryl groups include phenyl, and naphthyl.

`Cycloalkyl'refers to a non-aromatic hydrocarbyl ring structure, monocyclic, fused polycyclic, bridged polycyclic, or spirocyclic, with the number of ring atoms specified. A cycloalkyl may have from 3 to 12 carbon atoms, in particular from 3 to 10, and more particularly from 3 to 7 carbon atoms. Such cycloalkyl groups include, by way of example, single ring structures such as cyclopropyl, cyclobutyl, cyclopentyl, cyclohexyl, and cycloheptyl.

'Cyano' refers to the radical -CN.

`Halo' or `halogen' refers to fluoro (F), chloro (Cl), bromo (Br) and iodo (I). Particular halo groups are either fluoro or chloro.

'Hetero' when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, *e.g.* heteroalkyl, cycloalkyl, *e.g.* heterocycloalkyl, aryl, *e.g.* heteroaryl, and the like having from 1 to 4, and particularly from 1 to 3 heteroatoms, more typically 1 or 2 heteroatoms, for example a single heteroatom.

`Heteroaryl' means an aromatic ring structure, monocyclic or fused polycyclic, that includes one or more heteroatoms independently selected from O, N and S and the number of ring atoms specified. In particular, the aromatic ring structure may have from 5 to 9 ring members. The heteroaryl group can be, for example, a five membered or six membered monocyclic ring or a fused bicyclic structure formed from fused five and six membered rings or two fused six membered rings or, by way of a further example, two fused five membered rings. Each ring may contain up to four heteroatoms typically selected from nitrogen, sulphur and oxygen. Typically the heteroaryl ring will contain up to 4 heteroatoms, more typically up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general the number of basic nitrogen atoms present in the heteroaryl group, including any amino group substituents of the ring, will be less than five.

Examples of five membered monocyclic heteroaryl groups include but are not limited to pyrrolyl, furanyl, thiophenyl, imidazolyl, furazanyl, oxazolyl, oxadiazolyl, oxatriazolyl, isoxazolyl, thiazolyl, isothiazolyl, pyrazolyl, triazolyl and tetrazolyl groups.

Examples of six membered monocyclic heteroaryl groups include but are not limited to pyridinyl, pyrazinyl, pyridazinyl, pyrimidinyl and triazinyl.

Particular examples of bicyclic heteroaryl groups containing a five membered ring fused to another five-membered ring include but are not limited to imidazothiazolyl and imidazoimidazolyl.

Particular examples of bicyclic heteroaryl groups containing a six membered ring fused to a five membered ring include but are not limited to benzofuranyl, benzothiophenyl, benzoimidazolyl, benzoxazolyl, isobenzoxazolyl, benzisoxazolyl, benzothiazolyl, benzoisothiazolyl, isobenzofuranyl, indolyl, isoindolyl, indolizinyl, purinyl (e.g. adenine, guanine), indazolyl, pyrazolopyrimidinyl, triazolopyrimidinyl, and pyrazolopyridinyl groups.

Particular examples of bicyclic heteroaryl groups containing two fused six membered rings include but are not limited to quinolinyl, isoquinolinyl, pyridopyridinyl, quinoxalinyl, quinazolinyl, cinnolinyl, phthalazinyl, naphthyridinyl, and pteridinyl groups. Particular heteroaryl groups are those derived from thiophenyl, pyrrolyl, benzothiophenyl, benzofuranyl, indolyl, pyridinyl, quinolinyl, imidazolyl, oxazolyl and pyrazinyl.

Examples of representative heteroaryls include the following: 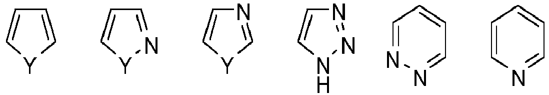 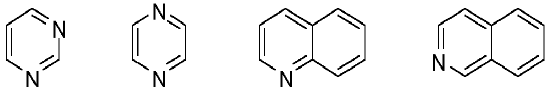 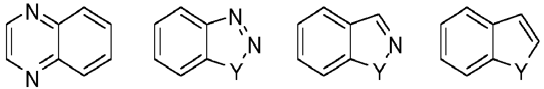 wherein each Y is selected from >C=O, NH, O and S.

`Heterocycloalkyl' means a non-aromatic fully saturated ring structure, monocyclic, fused polycyclic, spirocyclic, or bridged polycyclic, that includes one or more heteroatoms independently selected from O, N and S and the number of ring atoms specified. The heterocycloalkyl ring structure may have from 4 to 12 ring members, in particular from 4 to 10 ring members and more particularly from 4 to 7 ring members. Each ring may contain up to four heteroatoms typically selected from nitrogen, sulphur and oxygen. Typically the heterocycloalkyl ring will contain up to 4 heteroatoms, more typically up to 3 heteroatoms, more usually up to 2, for example a single heteroatom. Examples of heterocyclic rings include, but are not limited to azetidinyl, oxetanyl, thietanyl, pyrrolidinyl (e.g. 1-pyrrolidinyl, 2-pyrrolidinyl and 3-pyrrolidinyl), tetrahydrofuranyl (e.g. 1-tetrahydrofuranyl, 2-tetrahydrofuranyl and 3-tetrahydrofuranyl), tetrahydrothiophenyl (e.g. 1-tetrahydrothiophenyl, 2-tetrahydrothiophenyl and 3-tetrahydrothiophenyl), piperidinyl (e.g. 1-piperidinyl, 2-piperidinyl, 3-piperidinyl and 4-piperidinyl), tetrahydropyranyl (e.g. 4-tetrahydropyranyl), tetrahydrothiopyranyl (*e.g.* 4-tetrahydrothiopyranyl), morpholinyl, thiomorpholinyl, dioxanyl, or piperazinyl.

As used herein, the term 'heterocycloalkenyl' means a 'heterocycloalkyl', which comprises at least one double bond. Particular examples of heterocycloalkenyl groups are shown in the following illustrative examples: 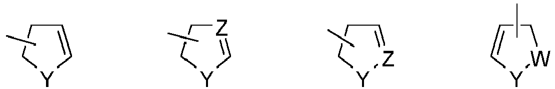 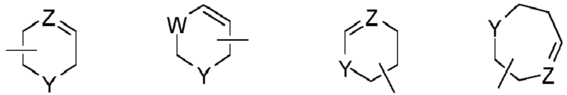 wherein each W is selected from CH₂, NH, O and S; each Y is selected from NH, O, C(=O), SOz, and S; and each Z is selected from N or CH.

Particular examples of monocyclic rings are shown in the following illustrative examples: 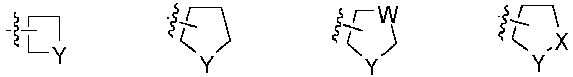 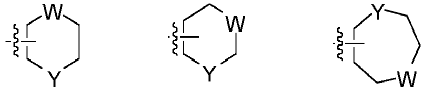 wherein each W and Y is independently selected from -CH₂-, -NH-, -O- and -S-.

Particular examples of fused bicyclic rings are shown in the following illustrative examples: 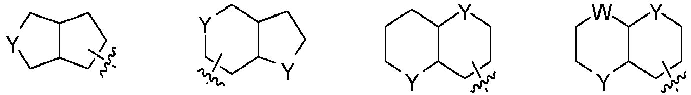 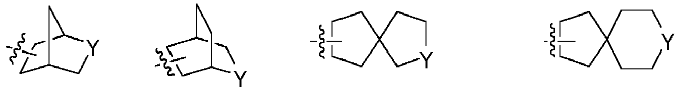 wherein each W and Y is independently selected from -CH₂-, -NH-, -O- and -S-.

Particular examples of bridged bicyclic rings are shown in the following illustrative examples: 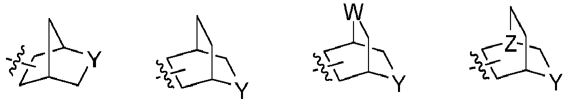 wherein each W and Y is independently selected from -CH₂-, -NH-, -O- and -S- and each Z is selected from N or CH.

Particular examples of spirocyclic rings are shown in the following illustrative examples: 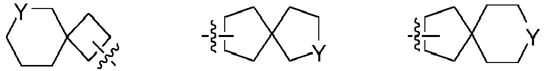 wherein each Y is selected from -CH₂-, -NH-, -O- and -S-.

`Hydroxyl' refers to the radical -OH.

'Oxo' refers to the radical =O.

'Substituted' refers to a group in which one or more hydrogen atoms are each independently replaced with the same or different substituent(s).

'Sulfo' or `sulfonic acid' refers to a radical such as -SO₃H.

'Thiol' refers to the group -SH.

As used herein, term 'substituted with one or more' refers to one to four substituents. In one embodiment it refers to one to three substituents. In further embodiments it refers to one or two substituents. In a yet further embodiment it refers to one substituent.

'Thioalkoxy' refers to the group -S-alkyl where the alkyl group has the number of carbon atoms specified. In particular the term refers to the group -S-C₁₋₆ alkyl. Particular thioalkoxy groups are thiomethoxy, thioethoxy, n-thiopropoxy, isothiopropoxy, n-thiobutoxy, tert-thiobutoxy, sec-thiobutoxy, n-thiopentoxy, n-thiohexoxy, and 1,2-dimethylthiobutoxy. Particular thioalkoxy groups are lower thioalkoxy, *i.e.* with between 1 and 6 carbon atoms. Further particular alkoxy groups have between 1 and 4 carbon atoms.

One having ordinary skill in the art of organic synthesis will recognize that the maximum number of heteroatoms in a stable, chemically feasible heterocyclic ring, whether it is aromatic or non-aromatic, is determined by the size of the ring, the degree of unsaturation and the valence of the heteroatoms. In general, a heterocyclic ring may have one to four heteroatoms so long as the heteroaromatic ring is chemically feasible and stable.

'Pharmaceutically acceptable' means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

'Pharmaceutically acceptable salt' refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethane sulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.* an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like. The term 'pharmaceutically acceptable cation' refers to an acceptable cationic counter-ion of an acidic functional group. Such cations are exemplified by sodium, potassium, calcium, magnesium, ammonium, tetraalkylammonium cations, and the like.

'Pharmaceutically acceptable vehicle' refers to a diluent, adjuvant, excipient or carrier with which a compound of the invention is administered.

'Prodrugs' refers to compounds, including derivatives of the compounds of the invention, which have cleavable groups and become by solvolysis or under physiological conditions the compounds of the invention which are pharmaceutically active *in vivo.* Such examples include, but are not limited to, choline ester derivatives and the like, N-alkylmorpholine esters and the like.

`Solvate' refers to forms of the compound that are associated with a solvent, usually by a solvolysis reaction. This physical association includes hydrogen bonding. Conventional solvents include water, EtOH, acetic acid and the like. The compounds of the invention may be prepared *e*.*g*. in crystalline form and may be solvated or hydrated. Suitable solvates include pharmaceutically acceptable solvates, such as hydrates, and further include both stoichiometric solvates and non-stoichiometric solvates. In certain instances the solvate will be capable of isolation, for example when one or more solvent molecules are incorporated in the crystal lattice of the crystalline solid. 'Solvate' encompasses both solution-phase and isolable solvates. Representative solvates include hydrates, ethanolates and methanolates.

'Subject' includes humans. The terms 'human', 'patient' and 'subject' are used interchangeably herein.

`Effective amount' means the amount of a compound of the invention that, when administered to a subject for treating a disease, is sufficient to effect such treatment for the disease. The "effective amount" can vary depending on the compound, the disease and its severity, and the age, weight, etc., of the subject to be treated.

'Preventing' or 'prevention' refers to a reduction in risk of acquiring or developing a disease or disorder (*i*.*e*. causing at least one of the clinical symptoms of the disease not to develop in a subject that may be exposed to a disease-causing agent, or predisposed to the disease in advance of disease onset.

The term 'prophylaxis' is related to 'prevention', and refers to a measure or procedure the purpose of which is to prevent, rather than to treat or cure a disease. Non-limiting examples of prophylactic measures may include the administration of vaccines; the administration of low molecular weight heparin to hospital patients at risk for thrombosis due, for example, to immobilization; and the administration of an anti-malarial agent such as chloroquine, in advance of a visit to a geographical region where malaria is endemic or the risk of contracting malaria is high.

'Treating' or 'treatment' of any disease or disorder refers, in one embodiment, to ameliorating the disease or disorder (*i.e.* arresting the disease or reducing the manifestation, extent or severity of at least one of the clinical symptoms thereof). In another embodiment 'treating' or 'treatment' refers to ameliorating at least one physical parameter, which may not be discernible by the subject. In yet another embodiment, 'treating' or 'treatment' refers to modulating the disease or disorder, either physically, (*e.g.* stabilization of a discernible symptom), physiologically, (*e.g.* stabilization of a physical parameter), or both. In a further embodiment, "treating" or "treatment" relates to slowing the progression of the disease.

As used herein the term `fibrotic diseases' refers to diseases characterized by excessive scarring due to excessive production, deposition, and contraction of extracellular matrix, and are that are associated with the abnormal accumulation of cells and/or fibronectin and/or collagen and/or increased fibroblast recruitment and include but are not limited to fibrosis of individual organs or tissues such as the heart, kidney, liver, joints, lung, pleural tissue, peritoneal tissue, skin, cornea, retina, musculoskeletal and digestive tract. In particular, the term fibrotic diseases refers to idiopathic pulmonary fibrosis (IPF); cystic fibrosis, other diffuse parenchymal lung diseases of different etiologies including iatrogenic drug-induced fibrosis, occupational and/or environmental induced fibrosis, granulomatous diseases (sarcoidosis, hypersensitivity pneumonia), collagen vascular disease, alveolar proteinosis, Langerhans cell granulomatosis, lymphangioleiomyomatosis, inherited diseases (Hermansky-Pudlak Syndrome, tuberous sclerosis, neurofibromatosis, metabolic storage diseases, familial interstitial lung disease); radiation induced fibrosis; chronic obstructive pulmonary disease; scleroderma; bleomycin induced pulmonary fibrosis; chronic asthma; silicosis; asbestos induced pulmonary fibrosis; acute respiratory distress syndrome (ARDS); kidney fibrosis; tubulointerstitium fibrosis; glomerular nephritis; diabetic nephropathy, focal segmental glomerular sclerosis; IgA nephropathy; hypertension; Alport; gut fibrosis; liver fibrosis; cirrhosis; alcohol induced liver fibrosis; toxic/drug induced liver fibrosis; hemochromatosis; alcoholic steato hepatitis (ASH), nonalcoholic steatohepatitis (NASH), nonalcoholic fatty liver disease (NAFLD); cholestasis, biliary duct injury; primary sclerosing cholangitis (PSC), primary biliary cirrhosis (PBC); infection induced liver fibrosis; viral induced liver fibrosis; and autoimmune hepatitis; corneal scarring; hypertrophic scarring; Dupuytren disease, keloids, cutaneous fibrosis; cutaneous scleroderma; systemic sclerosis, spinal cord injury/fibrosis; myelofibrosis; Duchenne muscular dystrophy (DMD) associated musculoskeletal fibrosis, vascular restenosis; atherosclerosis; arteriosclerosis; Wegener's granulomatosis; Peyronie's disease, or chronic lymphocytic. More particularly, the term "fibrotic diseases" refers to idiopathic pulmonary fibrosis (IPF), Dupuytren disease, nonalcoholic steatohepatitis (NASH), nonalcoholic fatty liver disease (NAFLD), Alcoholic steato hepatitis, (ASH), portal hypertension, systemic sclerosis, renal fibrosis, and cutaneous fibrosis. Most particularly, the term "fibrotic diseases" refers to nonalcoholic steatohepatitis (NASH), and/or nonalcoholic fatty liver disease (NAFLD). Alternatively, most particularly, the term "fibrotic diseases" refers to IPF.

`Compound(s) of the invention', and equivalent expressions, are meant to embrace compounds of the Formula(e) as herein described, which expression includes the pharmaceutically acceptable salts, and the solvates, *e*.*g*. hydrates, and the solvates of the pharmaceutically acceptable salts where the context so permits. Similarly, reference to intermediates, whether or not they themselves are claimed, is meant to embrace their salts, and solvates, where the context so permits.

When ranges are referred to herein, for example but without limitation, C₁₋₈ alkyl, the citation of a range should be considered a representation of each member of said range.

Other derivatives of the compounds of this invention have activity in both their acid and acid derivative forms, but in the acid sensitive form often offers advantages of solubility, tissue compatibility, or delayed release in the mammalian organism (Bundgard, H, 1985). Prodrugs include acid derivatives well know to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are particularly useful prodrugs. In some cases it is desirable to prepare double ester type prodrugs such as (acyloxy)alkyl esters or ((alkoxycarbonyl)oxy)alkylesters. Particular such prodrugs are the C₁₋₈ alkyl, C₂₋₈ alkenyl, C₆₋₁₀ optionally substituted aryl, and (C₆₋₁₀ aryl)-(C₁₋₄ alkyl) esters of the compounds of the invention.

The present disclosure includes all isotopic forms of the compounds of the invention provided herein, whether in a form (i) wherein all atoms of a given atomic number have a mass number (or mixture of mass numbers) which predominates in nature (referred to herein as the "natural isotopic form") or (ii) wherein one or more atoms are replaced by atoms having the same atomic number, but a mass number different from the mass number of atoms which predominates in nature (referred to herein as an "unnatural variant isotopic form"). It is understood that an atom may naturally exists as a mixture of mass numbers. The term "unnatural variant isotopic form" also includes embodiments in which the proportion of an atom of given atomic number having a mass number found less commonly in nature (referred to herein as an "uncommon isotope") has been increased relative to that which is naturally occurring e.g. to the level of >20%, >50%, >75%, >90%, >95% or> 99% by number of the atoms of that atomic number (the latter embodiment referred to as an "isotopically enriched variant form"). The term "unnatural variant isotopic form" also includes embodiments in which the proportion of an uncommon isotope has been reduced relative to that which is naturally occurring. Isotopic forms may include radioactive forms (i.e. they incorporate radioisotopes) and non-radioactive forms. Radioactive forms will typically be isotopically enriched variant forms.

An unnatural variant isotopic form of a compound may thus contain one or more artificial or uncommon isotopes such as deuterium (²H or D), carbon-11 (¹¹C), carbon-13 (¹³C), carbon-14 (¹⁴C), nitrogen-13 (¹³N), nitrogen-15 (¹⁵N), oxygen-15 (¹⁵O), oxygen-17 (¹⁷O), oxygen-18 (¹⁸O), phosphorus-32 (³²P), sulphur-35 (³⁵S), chlorine-36 (³⁶Cl), chlorine-37 (³⁷Cl), fluorine-18 (¹⁸F) iodine-123 (¹²³I), iodine-125 (¹²⁵I) in one or more atoms or may contain an increased proportion of said isotopes as compared with the proportion that predominates in nature in one or more atoms.

Unnatural variant isotopic forms comprising radioisotopes may, for example, be used for drug and/or substrate tissue distribution studies. The radioactive isotopes tritium, i.e. ³H, and carbon-14, i.e. ¹⁴C, are particularly useful for this purpose in view of their ease of incorporation and ready means of detection. Unnatural variant isotopic forms which incorporate deuterium i.e ²H or D may afford certain therapeutic advantages resulting from greater metabolic stability, for example, increased *in vivo* halflife or reduced dosage requirements, and hence may be preferred in some circumstances. Further, unnatural variant isotopic forms may be prepared which incorporate positron emitting isotopes, such as ¹¹C, ¹⁸F, ¹⁵0 and ¹³N, and would be useful in Positron Emission Topography (PET) studies for examining substrate receptor occupancy.

It is also to be understood that compounds that have the same molecular formula but differ in the nature or sequence of bonding of their atoms or the arrangement of their atoms in space are termed `isomers'. Isomers that differ in the arrangement of their atoms in space are termed `stereoisomers'.

Stereoisomers that are not mirror images of one another are termed 'diastereomers' and those that are non-superimposable mirror images of each other are termed 'enantiomers'. When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e. as (+) or (-)-isomers respectively). A chiral compound can exist as either individual enantiomer or as a mixture thereof. A mixture containing equal proportions of the enantiomers is called a `racemic mixture' .

'Tautomers' refer to compounds that are interchangeable forms of a particular compound structure, and that vary in the displacement of hydrogen atoms and electrons. Thus, two structures may be in equilibrium through the movement of *π* electrons and an atom (usually H). For example, enols and ketones are tautomers because they are rapidly interconverted by treatment with either acid or base. Another example of tautomerism is the aci- and nitro- forms of phenyl nitro methane that are likewise formed by treatment with acid or base.

Tautomeric forms may be relevant to the attainment of the optimal chemical reactivity and biological activity of a compound of interest.

The compounds of the invention may possess one or more asymmetric centers; such compounds can therefore be produced as individual (R)- or (S)- stereoisomers or as mixtures thereof.

Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art.

It will be appreciated that compounds of the invention may be metabolized to yield biologically active metabolites.

### The invention

The present invention relates to compounds useful in the prophylaxis and/or treatment of one or more fibrotic diseases. In particular, it has been identified that the compounds of the invention which antagonize GPR84, a G-protein-coupled receptor may useful in the prophylaxis and/or treatment of one or more fibrotic diseases. The present invention also provides pharmaceutical compositions comprising the compounds for use and methods for the prophylaxis and/or treatment of one or more fibrotic diseases by administering said compound.

Accordingly, in a first aspect of the invention, are provided compounds having GPR84 antagonist activity for use in the prophylaxis and/or treatment of one or more fibrotic diseases.

In a particular aspect, are provided the compounds of Formula I, II, III, and IV, respectively disclosed in WO 2013/092791, WO 2014/095798, WO 2015/197550, and WO 2016/169911 the entire disclosure being incorporated herein by reference for use in the prophylaxis and/or treatment of one or more fibrotic diseases.

The compounds of Formula I are shown below: 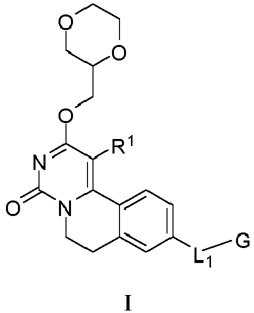 wherein
R¹ is H, Me, or halo;
L₁ is absent or is -O-, -S-, or -NR^{4a}-;
G is
   - R²,
   - -W-L₂-R², or
   - -W-L₃-R³;
W is C₁₋₄ alkylene, C₂₋₄ alkenylene having one double bond, or C₂₋₄ alkynylene having one triple bond;
L₂ is absent or is -O-;
R² is
   - H,
   - C₁₋₈ alkyl, optionally substituted with one to three groups independently selected from
      β OH,
      ∘ halo,
      ∘ CN,
      ∘ C₁₋₆ alkoxy,
      ∘ C₃₋₇ cycloalkyl,
      ∘ 4-6 membered heterocycloalkyl comprising one to three heteroatoms independently selected from S, and O,
      ∘ 5-6 membered heteroaryl comprising one to three heteroatoms independently selected from N, S, and O, and
      ∘ phenyl,
   - C₄₋₇ cycloalkenyl comprising one double bond,
   - 5-7 membered heterocycloalkenyl comprising one double bond, and one to three heteroatoms independently selected from N, O, and S,
   - C₃₋₇ cycloalkyl optionally substituted with one or more independently selected R⁵ groups,
   - 4-10 membered heterocycloalkyl comprising one to two heteroatoms independently selected from S, and O, optionally substituted with one to three independently selected R⁵ groups,
   - 5-10 membered heteroaryl comprising one to three heteroatoms independently selected from N, S, and O, optionally substituted with one to three independently selected R⁶ groups, or
   - C₆₋₁₀ aryl optionally substituted with one or more independently selected R⁶ groups;
L₃ is -NR^{4b}-;
R³ is
   - C₁₋₄ alkyl substituted with
      ∘ C₆₋₁₀ aryl optionally substituted with one or more independently selected R⁷ groups, or
      ∘ 5-10 membered heteroaryl comprising one to three heteroatoms independently selected from N, S, and O, optionally substituted with one or more independently selected R⁷ groups,
   - 5-10 membered heteroaryl comprising one to three heteroatoms independently selected from N, S, and O, optionally substituted with one or more independently selected R⁷ groups, or
   - C₆₋₁₀ aryl optionally substituted with one or more independently selected R⁷ groups;
Each R^{4a} and R^{4b} is independently selected from H, C₁₋₄ alkyl, and C₃₋₇ cycloalkyl;
R⁵ is oxo or R⁶;
R⁶ is
   - OH,
   - halo,
   - -NOz,
   - C₁₋₆ alkyl optionally substituted with one to three groups independently selected from halo, and OH,
   - C₁₋₆ alkoxy optionally substituted with one to three groups independently selected from halo, and OH,
   - C₃₋₇ cycloalkyl,
   - -C(=O)OR⁸,
   - -C(=O)NR⁹R¹⁰,
   - -NHC(=O)-C₁₋₄ alkyl,
   - -CN,
   - phenyl,
   - -O-phenyl,
   - 4-7 membered heterocycloalkyl comprising one to three heteroatoms independently selected from N, O, and S, or
   - 5-6 membered heteroaryl comprising one to three heteroatoms independently selected from N, O, and S; optionally substituted with one or more independently selected C₁₋₄ alkyl, C₁₋₄ alkoxy, CN, halo, and -C(=O)OR¹¹;
R⁷ is C₁₋₄ alkyl, or halo, and
each of R⁸, R⁹, R¹⁰ and R¹¹ is independently selected from H and C₁₋₄ alkyl.

In one embodiment, the compound according to Formula I is according to anyone of Formula I.Ia
- I.Id: 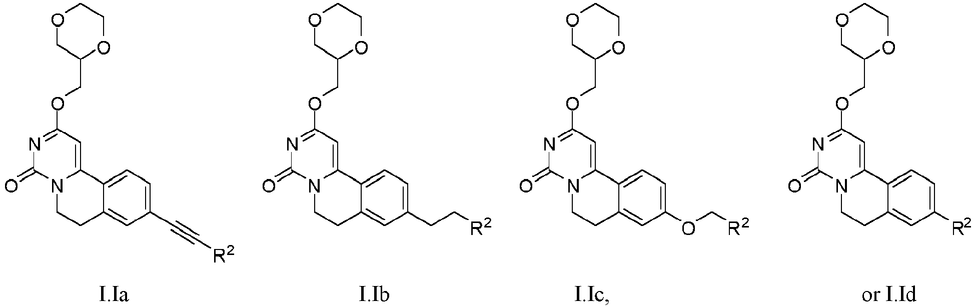 wherein R² is as described previously.

In one embodiment, the compound according to Formula I is according to any one of Formula I.IIa
- I.IId: 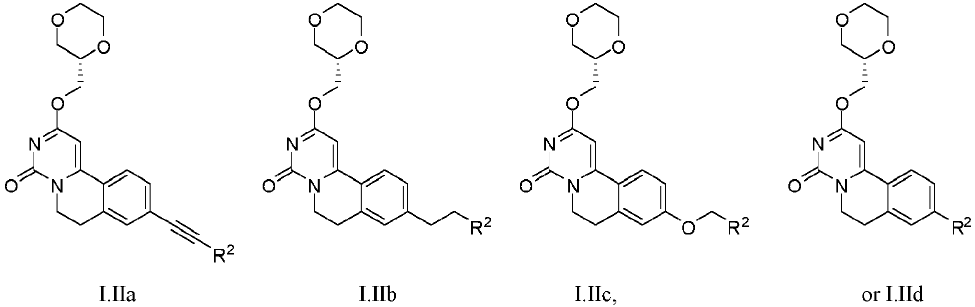 wherein R² is as described previously.

In one embodiment, the compound according to Formula I is according to any one of Formula I.IIIa
- I.IIId: 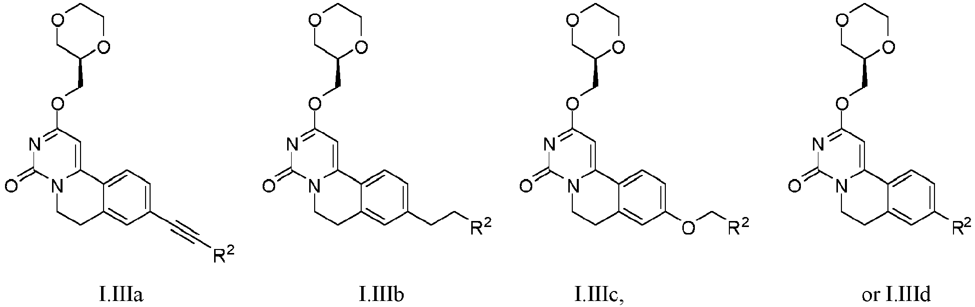 wherein R² is as described previously.

In one embodiment, the compound of the invention is according to anyone of Formula I.Ia, I.Ib, I.IIa, I.IIb, I.IIIa, or I.IIIb, wherein R² is C₃₋₇ cycloalkyl. In a preferred embodiment, R² is cyclopropyl, cyclobutyl, cyclopentyl, or cyclohexyl. In a more preferred embodiment, R² is cyclopropyl.

In one embodiment, the compound of the invention is according to anyone of Formula I.Ia, I.Ib, I.IIa, I.IIb, I.IIIa, or I.IIIb, wherein R² is not C₃₋₇ cycloalkyl. In a preferred embodiment, R² is not cyclopropyl, cyclobutyl, cyclopentyl, or cyclohexyl. In a more preferred embodiment, R² is not cyclopropyl.

In one embodiment, the compound of the invention is according to anyone of Formula I.Ia, I.Ib, I.IIa, I.IIb, I.IIIa, or I.IIIb, wherein R² is C₃₋₇ cycloalkyl substituted with one to three independently selected R⁵ groups. In a preferred embodiment, R² is C₃₋₇ cycloalkyl substituted with one R⁵ group. In a more preferred embodiment, R² is cyclopropyl, cyclobutyl, cyclopentyl or cyclohexyl, each of which is substituted with one R⁵ group. In another more prefered embodiment, R² is C₃₋₇ cycloalkyl substituted with one R⁵ group, wherein R⁵ is oxo, or R⁶ wherein R⁶ is selected from OH, or C₁₋₆ alkyl. In a most prefered embodiment, R² is cyclopropyl, cyclobutyl, cyclopentyl or cyclohexyl, each of which is substituted with one R⁵ group, wherein R⁵ is oxo, or R⁶ wherein R⁶ is selected from OH, and C₁₋₆ alkyl. In a further most prefered embodiment, R² is cyclopropyl, cyclobutyl, cyclopentyl or cyclohexyl, each of which is substituted with one R⁵ group, wherein R⁵ is OH.

In one embodiment, the compound of the invention is according to anyone of Formula I.Ia, I.Ib, I.IIa, I.IIb, I.IIIa, or I.IIIb, wherein R² is not C₃₋₇ cycloalkyl substituted with one to three independently selected R⁵ groups. In a preferred embodiment, R² is not C₃₋₇ cycloalkyl substituted with one R⁵ group. In a more preferred embodiment, R² is not cyclopropyl, cyclobutyl, cyclopentyl or cyclohexyl, each of which is substituted with one R⁵ group. In another more prefered embodiment, R² is not C₃₋₇ cycloalkyl substituted with one R⁵ group, wherein R⁵ is oxo, or R⁶ wherein R⁶ is selected from OH, and C₁₋₆ alkyl. In a most prefered embodiment, R² is not cyclopropyl, cyclobutyl, cyclopentyl or cyclohexyl, each of which is substituted with one R⁵ group, wherein R⁵ is oxo, or R⁶ wherein R⁶ is selected from OH, and C₁₋₆ alkyl. In a further most prefered embodiment, R² is not cyclopropyl, cyclobutyl, cyclopentyl or cyclohexyl, each of which is substituted with one R⁵ group, wherein R⁵ is OH.

In one embodiment, the compound of the invention is according to Formula I.Ic, I.Id, I.IIc, I.IId, I.IIIc, or I.IIId, wherein R² is 5-10 membered heteroaryl comprising one to three heteroatoms independently selected from N, S, and O. In a preferred embodiment, R² is furanyl, thienyl, oxazolyl, thiazolyl, oxadiazolyl, thiadiazolyl, imidazolyl, triazolyl, pyridinyl, pyrazinyl, pyrimidinyl, indanyl, or indazolyl.

In one embodiment, the compound of the invention is according to Formula I.Ic, I.Id, I.IIc, I.IId, I.IIIc, or I.IIId, wherein R² is not 5-10 membered heteroaryl comprising one to three heteroatoms independently selected from N, S, and O. In a preferred embodiment, R² is not furanyl, thienyl, oxazolyl, thiazolyl, oxadiazolyl, thiadiazolyl, imidazolyl, triazolyl, pyridinyl, pyrazinyl, pyrimidinyl, indanyl, or indazolyl.

In another embodiment, the compound of the invention is according to Formula I.Ic, I.Id, I.IIc, I.IId, I.IIIc, or I.IIId, wherein R² is 5-10 membered heteroaryl comprising one to three heteroatoms independently selected from N, S, and O, substituted with one to three independently selected R⁶ groups. In a prefered embodiment, R² is 5-10 membered heteroaryl comprising one to three heteroatoms independently selected from N, S, and O, substituted with one or two independently selected R⁶ groups. In a more preferred embodiment, R² is furanyl, thienyl, oxazolyl, thiazolyl, oxadiazolyl, thiadiazolyl, imidazolyl, triazolyl, pyridinyl, pyrazinyl, pyrimidinyl, indanyl, or indazolyl, substituted with one or two independently selected R⁶ groups. In another more preferred embodiment, R² is 5-10 membered heteroaryl comprising one to three heteroatoms independently selected from N, S, and O, substituted with one or two independently selected R⁶ groups, wherein each R⁶ is independently selected from OH, halo, C₁₋₆ alkyl, C₁₋₆ alkyl substituted with one or more halo, C₁₋₆ alkoxy, -CN, C₃₋₇ cycloalkyl , 4-7 membered heterocycloalkyl comprising one to three heteroatoms independently selected from N, O, and S, and phenyl. In a most preferred embodiment, R² is furanyl, thienyl, oxazolyl, thiazolyl, oxadiazolyl, thiadiazolyl, imidazolyl, triazolyl, pyridinyl, pyrazinyl, pyrimidinyl, indanyl, or indazolyl, each of which is substituted with one or two independently selected R⁶ groups, wherein each R⁶ is independently selected from OH, halo, C₁₋₆ alkyl, C₁₋₆ alkyl substituted with one or more halo, C₁₋₆ alkoxy, -CN, C₃₋₇ cycloalkyl, 4-7 membered heterocycloalkyl comprising one to three heteroatoms independently selected from N, O, and S, and phenyl. In another most preferred embodiment, R² is 5-10 membered heteroaryl comprising one to three heteroatoms independently selected from N, S, and O, substituted with one or two independently selected R⁶ groups, wherein each R⁶ is independently selected from OH, F, Cl, Me, Et, Pr, i-Pr, t-Bu, -CF₃, -OMe, -OEt, Oi-Pr, -CN, cyclopropyl, pyrrolidinyl, morpholinyl, piperidinyl, and phenyl. In a further most preferred embodiment, R² is furanyl, thienyl, oxazolyl, thiazolyl, oxadiazolyl, thiadiazolyl, imidazolyl, triazolyl, pyridinyl, pyrazinyl, pyrimidinyl, indanyl, or indazolyl, each of which is substituted with one or two independently selected R⁶ groups, wherein each R⁶ is independently selected from OH, F, Cl, Me, Et, Pr, i-Pr, t-Bu, -CF₃, -OMe, -OEt, -Oi-Pr, -CN, cyclopropyl, pyrrolidinyl, morpholinyl, piperidinyl, and phenyl.

In another embodiment, the compound of the invention is according to Formula I.Ic, I.Id, I.IIc, I.IId, I.IIIc, or I.IIId, wherein R² is not 5-10 membered heteroaryl comprising one to three heteroatoms independently selected from N, S, and O, substituted with one to three independently selected R⁶ groups. In a prefered embodiment, R² is not 5-10 membered heteroaryl comprising one to three heteroatoms independently selected from N, S, and O, substituted with one or two independently selected R⁶ groups. In a more preferred embodiment, R² is not furanyl, thienyl, oxazolyl, thiazolyl, oxadiazolyl, thiadiazolyl, imidazolyl, triazolyl, pyridinyl, pyrazinyl, pyrimidinyl, indanyl, or indazolyl, substituted with one or two independently selected R⁶ groups. In another more preferred embodiment, R² is not 5-10 membered heteroaryl comprising one to three heteroatoms independently selected from N, S, and O, substituted with one or two independently selected R⁶ groups, wherein each R⁶ is independently selected from OH, halo, C₁₋₆ alkyl, C₁₋₆ alkyl substituted with one or more halo, C₁₋₆ alkoxy, -CN, C₃₋₇ cycloalkyl , 4-7 membered heterocycloalkyl comprising one to three heteroatoms independently selected from N, O, and S, and phenyl. In a most preferred embodiment, R² is not furanyl, thienyl, oxazolyl, thiazolyl, oxadiazolyl, thiadiazolyl, imidazolyl, triazolyl, pyridinyl, pyrazinyl, pyrimidinyl, indanyl, or indazolyl, each of which is substituted with one or two independently selected R⁶ groups, wherein each R⁶ is independently selected from OH, halo, C₁₋₆ alkyl, C₁₋₆ alkyl substituted with one or more halo, C₁₋₆ alkoxy, -CN, C₃₋₇ cycloalkyl , 4-7 membered heterocycloalkyl comprising one to three heteroatoms independently selected from N, O, and S, and phenyl. In another most preferred embodiment, R² is not 5-10 membered heteroaryl comprising one to three heteroatoms independently selected from N, S, and O, substituted with one or two independently selected R⁶ groups, wherein each R⁶ is independently selected from OH, F, Cl, Me, Et, Pr, i-Pr, t-Bu, -CF₃, -OMe, -OEt, Oi-Pr, -CN, cyclopropyl, pyrrolidinyl, morpholinyl, piperidinyl, and phenyl. In a further most preferred embodiment, R² is not furanyl, thienyl, oxazolyl, thiazolyl, oxadiazolyl, thiadiazolyl, imidazolyl, triazolyl, pyridinyl, pyrazinyl, pyrimidinyl, indanyl, or indazolyl, each of which is substituted with one or two independently selected R⁶ groups, wherein each R⁶ is independently selected from OH, F, Cl, Me, Et, Pr, i-Pr, t-Bu, -CF₃, -OMe, -OEt, -Oi-Pr, -CN, cyclopropyl, pyrrolidinyl, morpholinyl, piperidinyl, and phenyl.

In another embodiment, the compound of the invention is according to Formula I.Ic, I.Id, I.IIc, I.IId, I.IIIc, or I.IIId, wherein R² is C₆₋₁₀ aryl. In a preferred embodiment, R² is phenyl.

In another embodiment, the compound of the invention is according to Formula I.Ic, I.Id, I.IIc, I.IId, I.IIIc, or I.IIId, wherein R² is not C₆₋₁₀ aryl. In a preferred embodiment, R² is not phenyl.

In another embodiment, the compound of the invention is according to Formula I.Ic, I.Id, I.IIc, I.IId, I.IIIc, or I.IIId, wherein R² is C₆₋₁₀ aryl substituted with one or more independently selected R⁶ groups. In a prefered embodiment, R² is C₆₋₁₀ aryl substituted with one or two independently selected R⁶ groups. In a more preferred embodiment, R² is C₆₋₁₀ aryl substituted with one or two independently selected R⁶ groups, wherein each R⁶ group is selected from halo, CN, C₁₋₆ alkyl, C₁₋₆ alkoxy, and - NHC(=O)-C₁₋₄ alkyl. In another more preferred embodiment, R² is C₆₋₁₀ aryl substituted with one or two independently selected R⁶ groups, wherein each R⁶ group is selected from -C(=O)NR⁹R¹⁰, and each R⁹ and R¹⁰ is independently selected from from H and C₁₋₄ alkyl. In another more preferred embodiment, R² is phenyl substituted with one or two independently selected R⁶ groups. In a most preferred embodiment, R² is phenyl substituted with one or two independently selected R⁶ groups, wherein each R⁶ group is selected from halo, CN, C₁₋₆ alkyl, C₁₋₆ alkoxy, and -NHC(=O)-C₁₋₄ alkyl. In another most preferred embodiment, R² is phenyl substituted with one or two independently selected R⁶ groups, wherein each R⁶ group is selected from -C(=O)NR⁹R¹⁰, and each R⁹ and R¹⁰ is independently selected from from H and C₁₋₄ alkyl. In a further most preferred embodiment R² is phenyl substituted with one or two independently selected R⁶ groups, wherein each R⁶ group is selected from F, Cl, CN, Me, -OMe, -OEt, - and -NHC(=O)Me. In a further most preferred embodiment R² is phenyl substituted with one or two independently selected R⁶ groups, wherein each R⁶ group is selected from C(=O)NH₂, and -C(=O)NHMe.

In another embodiment, the compound of the invention is according to Formula I.Ic, I.Id, I.IIc, I.IId, I.IIIc, or I.IIId, wherein R² is not C₆₋₁₀ aryl substituted with one or more independently selected R⁶ groups. In a prefered embodiment, R² is not C₆₋₁₀ aryl substituted with one or two independently selected R⁶ groups. In a more preferred embodiment, R² is not C₆₋₁₀ aryl substituted with one or two independently selected R⁶ groups, wherein each R⁶ group is selected from halo, CN, C₁₋₆ alkyl, C₁₋₆ alkoxy, and -NHC(=O)-C₁₋₄ alkyl. In another more preferred embodiment, R² is not C₆₋₁₀ aryl substituted with one or two independently selected R⁶ groups, wherein each R⁶ group is selected from -C(=O)NR⁹R¹⁰, and each R⁹ and R¹⁰ is independently selected from from H and C₁₋₄ alkyl. In another more preferred embodiment, R² is not phenyl substituted with one or two independently selected R⁶ groups. In a most preferred embodiment, R² is not phenyl substituted with one or two independently selected R⁶ groups, wherein each R⁶ group is selected from halo, CN, C₁₋₆ alkyl, C₁₋₆ alkoxy, and - NHC(=O)-C₁₋₄ alkyl. In another most preferred embodiment, R² is not phenyl substituted with one or two independently selected R⁶ groups, wherein each R⁶ group is selected from -C(=O)NR⁹R¹⁰, and each R⁹ and R¹⁰ is independently selected from from H and C₁₋₄ alkyl. In a further most preferred embodiment R² is not phenyl substituted with one or two independently selected R⁶ groups, wherein each R⁶ group is selected from F, Cl, CN, Me, -OMe, -OEt, - and -NHC(=O)Me. In a further most preferred embodiment R² is not phenyl substituted with one or two independently selected R⁶ groups, wherein each R⁶ group is selected from C(=O)NHz, and -C(=O)NHMe.

The compounds of Formula II are shown below: 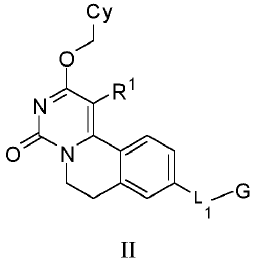 wherein
Cy is 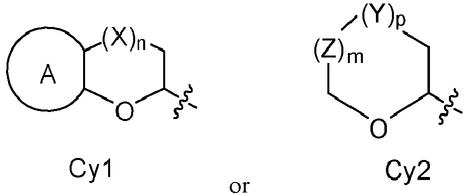
wherein
X is O or S;
Y is -CH₂-, or S;
Z is -CH₂-;
each of the subscript n, m, or p is independently selected from 0, and 1; and A is phenyl, or 5-6-membered heteroaryl comprising one or two N-atoms; optionally substituted with one or more independently selected R⁵ groups;
any one of Cy1 and Cy2 is optionally substituted by one or more independently selected C₁₋₄ alkyl groups;
R¹ is H, Me, or halo;
L₁ is absent or is -O-, -S-, or -NR^{4a}-;
G is
   - R²,
   - -W-L₂-R², or
   - -W-L₃-R³;
W is C₁₋₄ alkylene, C₂₋₄ alkenylene having one double bond, or C₂₋₄ alkynylene having one triple bond;
L₂ is absent or is -O-;
R² is
   - H,
   - C₁₋₈ alkyl optionally substituted with one to three groups independently selected from
      ∘ OH,
      ∘ halo,
      ∘ CN,
      ∘ C₁₋₆ alkoxy,
      ∘ C₃₋₇ cycloalkyl,
      ∘ 4-6 membered heterocycloalkyl (comprising one to three heteroatoms independently selected from S, and O),
      ∘ 5-6 membered heteroaryl (comprising one to three heteroatoms independently selected from N, S, and O), and
      ∘ phenyl,
   - C₄₋₇ cycloalkenyl comprising one double bond,
   - 5-7 membered heterocycloalkenyl comprising one double bond, and one to three heteroatoms independently selected from O, and S,
   - C₃₋₇ cycloalkyl (optionally substituted with one or more independently selected R⁶ groups),
   - 4-10 membered heterocycloalkyl comprising one to two heteroatoms independently selected from S, and O, (optionally substituted with one to three independently selected R⁶ groups),
   - 5-10 membered heteroaryl comprising one to three heteroatoms independently selected from N, S, and O (optionally substituted with one to three independently selected R⁷ groups), or
   - C₆₋₁₀ aryl (optionally substituted with one or more independently selected R⁷ groups);
L₃ is -NR^{4b}-;
R³ is
   - C₁₋₄ alkyl substituted with C₆₋₁₀ aryl (optionally substituted with one or more independently selected R⁸ groups), or 5-10 membered heteroaryl comprising one to three heteroatoms independently selected from N, S, and O, (optionally substituted with one or more independently selected R⁸ groups),
   - 5-10 membered heteroaryl comprising one to three heteroatoms independently selected from N, S, and O, (optionally substituted with one or more independently selected R⁸ groups), or
   - C₆₋₁₀ aryl (optionally substituted with one or more independently selected R⁸ groups);
each R^{4a} and R^{4b} is independently selected from H, C₁₋₄ alkyl, and C₃₋₇ cycloalkyl;
R⁵ is halo, C₁₋₄ alkyl or C₁₋₄ alkoxy;
R⁶ is oxo or R⁷;
R⁷ is
   - OH,
   - halo,
   - -NO₂,
   - C₁₋₆ alkyl (optionally substituted with one to three groups independently selected from halo, and OH),
   - C₁₋₆ alkoxy (optionally substituted with one to three groups independently selected from halo, and OH),
   - C₃₋₇ cycloalkyl,
   - -C(=O)OR⁹,
   - -C(=O)NR¹⁰R¹¹,
   - -NHC(=O)-C₁₋₄ alkyl,
   - -CN,
   - phenyl,
   - -O-phenyl,
   - 4-7 membered heterocycloalkyl comprising one to three heteroatoms independently selected from N, O, and S, or
   - 5-6 membered heteroaryl comprising one to three heteroatoms independently selected from N, O, and S; (optionally substituted with one or more independently selected C₁₋₄ alkyl, C₁₋₄ alkoxy, CN, halo, and -C(=O)OR¹²);
R⁸ is C₁₋₄ alkyl, or halo; and
each of R⁹, R¹⁰, R¹¹and R¹², is independently selected from H and C₁₋₄ alkyl.

The compounds of Formula III are shown below: 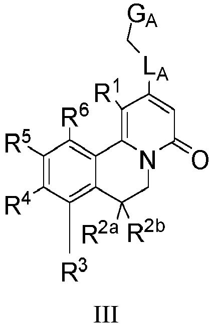 wherein
R¹ is H, C₁₋₄ alkyl, or cyclopropyl;
L_{A} is O or NH;
G_{A} is:
   - 4-6 membered monocyclic heterocycloalkyl containing one or two O,
   - C₃₋₇ monocyclic cycloalkyl, or
   - a bicyclic group of formula Cy: 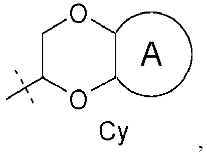
wherein A is phenyl or 5-6 membered heteroaryl containing one or two heteroatoms independently selected from N, O and S;
each R^{2a} and R^{2b} are independently H or -CH₃;
R³ is H, -OH or -OCH₃;
R⁴ is -CN or -L₁-W₁-G₁,
wherein
   - L₁ is absent or O,
   - W₁ is absent, or is C₁₋₆ alkylene, C₂₋₄ alkenylene having one double bond or C₂₋₄ alkynylene having one triple bond, each of which is optionally substituted with one or more independently selected halo, -CN or C₁₋₄ alkoxy,
   - G₁ is
      ∘ H,
      ∘ -CF₃,
      ∘ -C(=O)-C₁₋₄ alkyl,
      ∘ -S(=O)₂-C₁₋₄ alkyl, optionally substituted with one or more independently selected halo,
      ∘ 4-6 membered monocyclic heterocycloalkyl containing one or two O (which heterocycloalkyl is optionally substituted with one or more independently selected R⁷ groups),
      ∘ 6 membered monocyclic heterocycloalkenyl containing one or two O (which heterocycloalkenyl is optionally substituted with one or more independently selected R⁷ groups),
      ∘ C₃₋₇ monocyclic cycloalkyl optionally substituted with one or more independently selected R⁷ groups,
      ∘ phenyl optionally substituted with one or more independently selected R⁷ groups,
      ∘ or 5-6 membered heteroaryl containing one to four heteroatoms independently selected from N, O and S (which heteroaryl is optionally substituted with one or more independently selected R⁷ groups),
each R⁷ is:
   - halo,
   - -OH,
   - C₁₋₄ alkyl, C₃₋₄ monocyclic cycloalkyl, or C₁₋₄ alkoxy, each of which is optionally substituted with one or more independently selected halo;
R⁵ is -CN or -L₂-W₂-G₂,
wherein
   - L₂ is absent, O or S,
   - Wz is absent or C₁₋₄ alkylene, optionally substituted with one or more independently selected halo,
   - G₂ is
      ∘ H,
      ∘ -CF₃,
      ∘ C₃₋₇ monocyclic cycloalkyl (which cycloalkyl is optionally substituted with one or more independently selected halo),
      ∘ phenyl,
      ∘ or 5-6 membered heteroaryl containing one to three heteroatoms independently selected from N, O and S; and
R⁶ is H, -OH or -OCH₃.

The compounds of Formula IV are shown below: 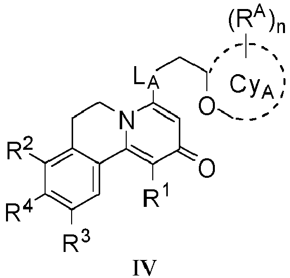 wherein
L_{A} is O, or NH;
Cy_{A} is monocyclic 4-6 membered heterocycloalkyl, comprising one or two O atoms;
each R^{A} is independently selected from halo, and C₁₋₃ alkyl;
the subscript n is 0, 1 or 2;
R¹ is H or C₁₋₃ alkyl;
R² is H, -OH, or C₁₋₃ alkoxy;
R³ is H or C₁₋₃ alkoxy;
R⁴ is
   - -CN,
   - -OH,
   - -O-S(=O)₂-C₁₋₄ alkyl optionally substituted with one or more independently selected halo, or
   - -L₁-W₁-G₁;
L₁ is a direct bond, -O-, -S-, -SO₂-, -C(=O)NR^{5a}-, -NR^{5b}C(=O)-, or -NR^{5c}-;
R^{5a}, R^{5b} and R^{5c} are independently H or C₁₋₄ alkyl;
W₁ is a direct bond or C₁₋₂ alkylene optionally substituted with one or more independently selected halo;
G₁ is
   - C₃₋₆ cycloalkyl optionally substituted with one or more independently selected halo,
   - 5-6 membered heteroaryl comprising one to four heteroatoms independently selected from N, O, and S, which heteroaryl is optionally substituted with one or more independently selected C₁₋₄ alkyl,
   - 5-7 membered heterocycloalkenyl comprising one double bond, and one to three heteroatoms independently selected from N, O, and S, which heterocycloalkenyl is optionally substituted with one or more independently selected R⁶,
   - monocyclic or spiro bicyclic 4-8 membered heterocycloalkyl comprising one to three heteroatoms independently selected from N, O, and S, which heterocycloalkyl is optionally substituted with one or more independently selected R⁶,
   - monocyclic 4-6 membered heterocycloalkyl comprising one to three heteroatoms independently selected from N, O, and S, fused to one or two phenyls,
   - C₁₋₄ alkyl optionally substituted with one or more independently selected halo, -NR^{7a}R^{7b}, or C₁₋₄ alkoxy, which alkoxy is optionally substituted with one or more independently selected halo,
   - phenyl optionally substituted with one or more independently selected halo or C₁₋₄ alkoxy, which alkoxy is optionally substituted with one or more independently selected halo;
R⁶ is
   - halo,
   - =O,
   - -CN,
   - -OH,
   - -C(=O)-C₁₋₄ alkoxy optionally substituted with one or more independently selected halo,
   - -C(=O)-C3-4 cycloalkyl,
   - -S(=O)2-C₁₋₄ alkyl,
   - C₁₋₄ alkyl optionally substituted by one or more independently selected C1-3 alkoxy, halo, or -OH,
   - C₁₋₄ alkoxy,
   - phenyl optionally substituted by one or more independently selected halo,
   - -C(=O)-monocyclic 4-6 membered heterocycloalkyl comprising one to three heteroatoms independently selected from N, O, and S,
   - -C(=O)NR^{8a}R^{8b}, or
   - 5-7 membered heteroaryl comprising one to four heteroatoms independently selected from N, O, and S, which heteroaryl is optionally substituted with one or more independently selected C₁₋₄ alkyl;
R^{7a} and R^{7b} are independently H or C₁₋₄ alkyl, and
R^{8a} and R^{8b} are independently H or C₁₋₃ alkyl.

In one embodiment, the compound according to Formula IV is according to Formula IV.Ia: 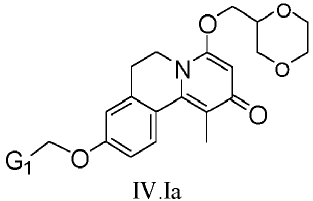 wherein G₁ is as defined above.

In one embodiment, the compound according to Formula IV is according to Formula IV.Ib: 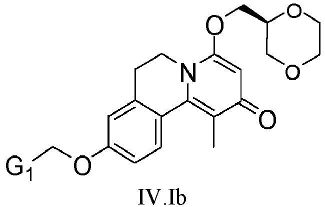 wherein G₁ is as defined above.

In one embodiment, the compound according to Formula IV is according to Formula IV.Ia, or IV.Ib, wherein G₁ is C₃₋₆ cycloalkyl. In a particular embodiment, G₁ is cyclopropyl, cyclobutyl, cyclopentyl or cyclohexyl. In a particular embodiment, G₁ is cyclopropyl, cyclobutyl, or cyclopentyl. In a more particular embodiment, G₁ is cyclopropyl.

In one embodiment, the compound according to Formula IV is according to Formula IV.Ia, or IV.Ib, wherein G₁ is C₃₋₆ cycloalkyl substituted with one or more halo. In a particular embodiment, G₁ is cyclopropyl, cyclobutyl, cyclopentyl or cyclohexyl, each of which is substituted with one or more halo. In a particular embodiment, G₁ is cyclopropyl, cyclobutyl, or cyclopentyl, each of which is substituted with one or more halo. In another particular embodiment, G₁ is C₃₋₆ cycloalkyl substituted with one or more F. In a more particular embodiment, G₁ is cyclopropyl, cyclobutyl, cyclopentyl or cyclohexyl, each of which is substituted with one or more F. In a more particular embodiment, G₁ is cyclopropyl, cyclobutyl, or cyclopentyl, each of which is substituted with one or more F. In a most particular embodiment, G₁ is cyclopropyl substituted with one or more F.

In one embodiment, the compound according to Formula IV is according to Formula IV.Ia, or IV.Ib, wherein G₁ is C₁₋₄ alkyl, substituted with one or more independently selected halo, -NR^{7a}R^{7b}, or C₁₋₄ alkoxy, which alkoxy is optionally substituted with one or more independently selected halo, and wherein R^{7a} and R^{7b} are independently H or C₁₋₄ alkyl. In a particular embodiment, G₁ is -CH₃, or - CH₂CH₃, each of which substituted with one or more independently selected halo, -NR^{7a}R^{7b}, or C₁₋₄ alkoxy, which alkoxy is optionally substituted with one or more independently selected halo, and wherein R^{7a} and R^{7b} are independently H or C₁₋₄ alkyl. In another particular embodiment, G₁ is C₁₋₄ alkyl, substituted with one or more independently selected F, -NHCH₃, -NHCH₂CH₃, -N(CH₃)₂, -OCH₃, -OCH₂CH₃, -OCF₃, or -OCH₂CF₃. In a more particular embodiment, G₁ is C₁₋₄ alkyl, substituted with one -NHCH₃, -NHCH₂CH₃, -N(CH₃)₂, -OCH₃, -OCH₂CH₃, -OCF₃, or -OCH₂CF₃. In a most particular embodiment, G₁ is -CF₃, -CHF₂, -CH₂-CHF₂, -CH₂-CF₃, -CH₂-CH₂-N(CH₃)₂, or -CH₂-CH₂-OCF₃. In a further most particular embodiment, G₁ is -CF₃,

In one embodiment, the compound for use is selected from:
9-Allyloxy-2-([1,4]dioxan-2-ylmethoxy)-6,7-dihydro-pyrimido[6,1-a]isoquinolin-4-one,
2-([1,4]Dioxan-2-ylmethoxy)-9-pyridin-3-yl-6,7-dihydro-pyrimido[6,1-a]isoquinolin-4-one,
2-([1,4]Dioxan-2-ylmethoxy)-9-pyridin-4-yl-6,7-dihydro-pyrimido[6,1-a]isoquinolin-4-one,
2-[2-([1,4]Dioxan-2-ylmethoxy)-4-oxo-6,7-dihydro-4H-pyrimido[6,1-a]isoquinolin-9-yl]-benzonitrile,
3-[2-([1,4]Dioxan-2-ylmethoxy)-4-oxo-6,7-dihydro-4H-pyrimido[6,1-a]isoquinolin-9-yl]-benzonitrile,
4-[2-([1,4]Dioxan-2-ylmethoxy)-4-oxo-6,7-dihydro-4H-pyrimido[6,1-a]isoquinolin-9-yl]-benzonitrile,
[2-([1,4]Dioxan-2-ylmethoxy)-4-oxo-6,7-dihydro-4H-pyrimido[6,1-a]isoquinolin-9-yloxy]-acetonitrile,
2-([1,4]Dioxan-2-ylmethoxy)-9-(oxazol-2-ylmethoxy)-6,7-dihydro-pyrimido[6,1-a]isoquinolin-4-one,
2-([1,4]Dioxan-2-ylmethoxy)-9-(pyridin-2-ylmethoxy)-6,7-dihydro-pyrimido[6,1-a]isoquinolin-4-one,
9-(3,5-Dichloro-phenyl)-2-([1,4]dioxan-2-ylmethoxy)-6,7-dihydro-pyrimido[6,1-a]isoquinolin-4-one,
9-Benzofuran-2-yl-2-([1,4]dioxan-2-ylmethoxy)-6,7-dihydro-pyrimido[6,l-a]isoquinolin-4-one,
2-[2-([1,4]Dioxan-2-ylmethoxy)-4-oxo-6,7-dihydro-4H-pyrimido[6,1-a]isoquinolin-9-yl]-indole-1-carboxylic acid tert-butyl ester,
2-([1,4]Dioxan-2-ylmethoxy)-9-(1H-indol-2-yl)-6,7-dihydro-pyrimido[6,1-a]isoquinolin-4-one,
2-([1,4]Dioxan-2-ylmethoxy)-9-(6-methoxy-pyridin-3-yl)-6,7-dihydro-pyrimido[6,1-a]isoquinolin-4-one,
2-([1,4]Dioxan-2-ylmethoxy)-9-(6-trifluoromethyl-pyridin-3-yl)-6,7-dihydro-pyrimido[6,1-a]isoquinolin-4-one,
2-([1,4]Dioxan-2-ylmethoxy)-9-(3-methyl-3H-imidazol-4-ylethynyl)-6,7-dihydro-pyrimido[6,1-a]isoquinolin-4-one,
9-(5-tert-Butyl-[1,2,4]oxadiazol-3-ylmethoxy)-2-([1,4]dioxan-2-ylmethoxy)-6,7-dihydro-pyrimido[6,1-a]isoquinolin-4-one,
5-[2-([1,4]Dioxan-2-ylmethoxy)-4-oxo-6,7-dihydro-4H-pyrimido[6,1-a]isoquinolin-9-yl]-pyridine-2-carboxylic acid methylamide,
2-([1,4]Dioxan-2-ylmethoxy)-9-pent-1-ynyl-6,7-dihydro-pyrimido[6,1-a]isoquinolin-4-one,
2-([1,4]Dioxan-2-ylmethoxy)-9-(2-pyridin-2-yl-ethyl)-6,7-dihydro-pyrimido[6,1-a]isoquinolin-4-one,
2-([1,4]Dioxan-2-ylmethoxy)-9-(2-pyrazin-2-yl-ethyl)-6,7-dihydro-pyrimido[6,1-a]isoquinolin-4-one,
2-([1,4]Dioxan-2-ylmethoxy)-9-(1H-indol-5-yl)-6,7-dihydro-pyrimido[6,1-a]isoquinolin-4-one,
2-([1,4]Dioxan-2-ylmethoxy)-9-(2-methoxy-phenyl)-6,7-dihydro-pyrimido[6,1-a]isoquinolin-4-one,
2-([1,4]Dioxan-2-ylmethoxy)-9-(5-methoxy-pyridin-3-yl)-6,7-dihydro-pyrimido[6,1-a]isoquinolin-4-one,
2-([1,4]Dioxan-2-ylmethoxy)-9-(1H-indazol-5-yl)-6,7-dihydro-pyrimido[6,1-a]isoquinolin-4-one,
2-([1,4]Dioxan-2-ylmethoxy)-9-(4-methoxy-phenyl)-6,7-dihydro-pyrimido[6,1-a]isoquinolin-4-one,
3-[2-([1,4]Dioxan-2-ylmethoxy)-4-oxo-6,7-dihydro-4H-pyrimido[6,1-a]isoquinolin-9-yl]-benzamide,
5-[2-([1,4]Dioxan-2-ylmethoxy)-4-oxo-6,7-dihydro-4H-pyrimido[6,1-a]isoquinolin-9-yl]-2-fluoro-benzamide,
N-{3-[2-([1,4]Dioxan-2-ylmethoxy)-4-oxo-6,7-dihydro-4H-pyrimido[6,1-a]isoquinolin-9-yl]-phenyl}-acetamide,
9-Cyclopropylethynyl-2-([1,4]dioxan-2-ylmethoxy)-6,7-dihydro-pyrimido[6,1-a]isoquinolin-4-one,
2-([1,4]Dioxan-2-ylmethoxy)-9-(1-hydroxy-cyclopentylethynyl)-6,7-dihydro-pyrimido[6,1-a]isoquinolin-4-one,
2-([1,4]Dioxan-2-ylmethoxy)-9-pyrimidin-5-yl-6,7-dihydro-pyrimido[6,1-a]isoquinolin-4-one,
9-Cyclohex-1-enyl-2-([1,4]dioxan-2-ylmethoxy)-6,7-dihydro-pyrimido[6,1-a]isoquinolin-4-one,
2-([1,4]Dioxan-2-ylmethoxy)-9-(1-methyl-1H-indol-5-yl)-6,7-dihydro-pyrimido[6,1-a]isoquinolin-4-one,
2-([1,4]Dioxan-2-ylmethoxy)-9-(6-methyl-pyridin-3-yl)-6,7-dihydro-pyrimido[6,1-a]isoquinolin-4-one,
2-([1,4]Dioxan-2-ylmethoxy)-9-pyridin-2-ylethynyl-6,7-dihydro-pyrimido[6,1-a]isoquinolin-4-one,
2-([1,4]Dioxan-2-ylmethoxy)-9-(3-methoxy-prop-1-ynyl)-6,7-dihydro-pyrimido[6,1-a]isoquinolin-4-one,
5-[2-([1,4]Dioxan-2-ylmethoxy)-4-oxo-6,7-dihydro-4H-pyrimido[6,1-a]isoquinolin-9-yl]-pent-4-ynenitrile,
2-([1,4]Dioxan-2-ylmethoxy)-9-(3-hydroxy-prop-1-ynyl)-6,7-dihydro-pyrimido[6,1-a]isoquinolin-4-one,
2-([1,4]Dioxan-2-ylmethoxy)-9-(4-methoxy-phenylethynyl)-6,7-dihydro-pyrimido[6,1-a]isoquinolin-4-one,
2-([1,4]Dioxan-2-ylmethoxy)-9-pyridin-3-ylethynyl-6,7-dihydro-pyrimido[6,1-a]isoquinolin-4-one,
4-[2-([1,4]Dioxan-2-ylmethoxy)-4-oxo-6,7-dihydro-4H-pyrimido[6,1-a]isoquinolin-9-yl]-N-methyl-benzamide,
2-([1,4]Dioxan-2-ylmethoxy)-9-(3-methoxy-phenyl)-6,7-dihydro-pyrimido[6,1-a]isoquinolin-4-one,
9-(2-Chloro-phenyl)-2-([1,4]dioxan-2-ylmethoxy)-6,7-dihydro-pyrimido[6,1-a]isoquinolin-4-one,
2-([1,4]Dioxan-2-ylmethoxy)-9-(4-hydroxy-but-1-ynyl)-6,7-dihydro-pyrimido[6,1-a]isoquinolin-4-one,
9-(1,5-Dimethyl-1H-pyrazol-3-ylmethoxy)-2-([1,4]dioxan-2-ylmethoxy)-6,7-dihydro-pyrimido[6,1-a] isoquinolin-4-one,
2-([1,4]Dioxan-2-ylmethoxy)-9-(1-methyl-1H-pyrazol-3-ylmethoxy)-6,7-dihydro-pyrimido[6,1-a]isoquinolin-4-one,
2-([1,4]Dioxan-2-ylmethoxy)-9-(3-methyl-[1,2,4]oxadiazol-5-ylmethoxy)-6,7-dihydro-pyrimido[6,1-a]isoquinolin-4-one,
2-([1,4]Dioxan-2-ylmethoxy)-9-(4-morpholin-4-yl-phenyl)-6,7-dihydro-pyrimido[6,1-a]isoquinolin-4-one,
3-[2-([1,4]Dioxan-2-ylmethoxy)-4-oxo-6,7-dihydro-4H-pyrimido[6,1-a]isoquinolin-9-yl]-4-fluoro-benzamide,
3-[2-([1,4]Dioxan-2-ylmethoxy)-4-oxo-6,7-dihydro-4H-pyrimido[6,1-a]isoquinolin-9-yl]-5-fluoro-benzamide,
9-(3,3-Dimethyl-but-1-ynyl)-2-([1,4]dioxan-2-ylmethoxy)-6,7-dihydro-pyrimido[6,1-a]isoquinolin-4-one,
2-([1,4]Dioxan-2-ylmethoxy)-9-pyridin-4-ylethynyl-6,7-dihydro-pyrimido[6,1-a]isoquinolin-4-one,
2-([1,4]Dioxan-2-ylmethoxy)-9-(3-methyl-isoxazol-5-ylmethoxy)-6,7-dihydro-pyrimido[6,1-a]isoquinolin-4-one,
2-([1,4]Dioxan-2-ylmethoxy)-9-(3-hydroxy-3-methyl-but-1-ynyl)-6,7-dihydro-pyrimido[6,1-a]isoquinolin-4-one,
2-([1,4]Dioxan-2-ylmethoxy)-9-(2-methoxy-pyridin-3-yl)-6,7-dihydro-pyrimido[6,1-a]isoquinolin-4-one,
2-([1,4]Dioxan-2-ylmethoxy)-6,7-dihydro-pyrimido[6,1-a]isoquinolin-4-one,
9-(3,6-Dihydro-2H-pyran-4-yl)-2-([1,4]dioxan-2-ylmethoxy)-6,7-dihydro-pyrimido[6,1-a]isoquinolin-4-one,
5-[2-([1,4]Dioxan-2-ylmethoxy)-4-oxo-6,7-dihydro-4H-pyrimido[6,1-a]isoquinolin-9-yl]-pyridine-2-carbonitrile,
2-([1,4]Dioxan-2-ylmethoxy)-9-(6-isopropoxy-pyridin-3-yl)-6,7-dihydro-pyrimido[6,1-a]isoquinolin-4-one,
2-([1,4]Dioxan-2-ylmethoxy)-9-(6-ethoxy-pyridin-3-yl)-6,7-dihydro-pyrimido[6,1-a]isoquinolin-4-one,
2-([1,4]Dioxan-2-ylmethoxy)-9-(6-morpholin-4-yl-pyridin-3-yl)-6,7-dihydro-pyrimido[6,1-a]isoquinolin-4-one,
9-(2,3-Dimethoxy-phenyl)-2-([1,4]dioxan-2-ylmethoxy)-6,7-dihydro-pyrimido[6,1-a]isoquinolin-4-one,
9-(3-Chloro-2-methoxy-pyridin-4-yl)-2-([1,4]dioxan-2-ylmethoxy)-6,7-dihydro-pyrimido[6,1-a]isoquinolin-4-one,
2-([1,4]Dioxan-2-ylmethoxy)-9-(2-methyl-pyridin-4-yl)-6,7-dihydro-pyrimido[6,1-a]isoquinolin-4-one,
3-[2-([1,4]Dioxan-2-ylmethoxy)-4-oxo-6,7-dihydro-4H-pyrimido[6,1-a]isoquinolin-9-yl]-isonicotinonitrile,
9-(2,5-Dimethoxy-phenyl)-2-([1,4]dioxan-2-ylmethoxy)-6,7-dihydro-pyrimido[6,1-a]isoquinolin-4-one,
2-([1,4]Dioxan-2-ylmethoxy)-9-(3,4,5,6-tetrahydro-2H-[1,2']bipyridinyl-5'-yl)-6,7-dihydro-pyrimido[6,1-a]isoquinolin-4-one,
2-([1,4]Dioxan-2-ylmethoxy)-9-(2-ethoxy-pyridin-3-yl)-6,7-dihydro-pyrimido[6,1-a]isoquinolin-4-one,
9-(2,6-Dimethoxy-pyridin-3-yl)-2-([1,4]dioxan-2-ylmethoxy)-6,7-dihydro-pyrimido[6,1-a]isoquinolin-4-one,
4-[2-([1,4]Dioxan-2-ylmethoxy)-4-oxo-6,7-dihydro-4H-pyrimido[6,1-a]isoquinolin-9-yl]-nicotinonitrile,
9-tert-Butoxymethyl-2-([1,4]dioxan-2-ylmethoxy)-6,7-dihydro-pyrimido[6,1-a]isoquinolin-4-one,
2-([1,4]Dioxan-2-ylmethoxy)-9-(2-pyrrolidin-1-yl-pyridin-3-yl)-6,7-dihydro-pyrimido[6,1-a]isoquinolin-4-one,
2-([1,4]Dioxan-2-ylmethoxy)-9-(6-pyrrolidin-1-yl-pyridin-3-yl)-6,7-dihydro-pyrimido[6,1-a]isoquinolin-4-one,
2-([1,4]Dioxan-2-ylmethoxy)-9-(5-phenyl-oxazol-2-ylmethoxy)-6,7-dihydro-pyrimido[6,1-a]isoquinolin-4-one,
9-(5-tert-Butyl-oxazol-2-ylmethoxy)-2-([1,4]dioxan-2-ylmethoxy)-6,7-dihydro-pyrimido[6,1-a]isoquinolin-4-one,
9-(5-Cyclopropyl-[1,2,4]oxadiazol-3-ylmethoxy)-2-([1,4]dioxan-2-ylmethoxy)-6,7-dihydro-pyrimido[6,1-a]isoquinolin-4-one,
2-([1,4]Dioxan-2-ylmethoxy)-9-(5-ethyl-[1,2,4]oxadiazol-3-ylmethoxy)-6,7-dihydro-pyrimido[6,1-a]isoquinolin-4-one,
2-([1,4]Dioxan-2-ylmethoxy)-9-(5-methyl-[1,2,4]oxadiazol-3-ylmethoxy)-6,7-dihydro-pyrimido[6,1-a]isoquinolin-4-one,
2-([1,4]Dioxan-2-ylmethoxy)-9-(5-isopropyl-[1,2,4]oxadiazol-3-ylmethoxy)-6,7-dihydro-pyrimido[6,1-a]isoquinolin-4-one,
9-Cyclopentylethynyl-2-([1,4]dioxan-2-ylmethoxy)-6,7-dihydro-pyrimido[6,1-a]isoquinolin-4-one,
9-Cyclohexylethynyl-2-([1,4]dioxan-2-ylmethoxy)-6,7-dihydro-pyrimido[6,1-a]isoquinolin-4-one,
2-([1,4]Dioxan-2-ylmethoxy)-9-(3-methyl-but-1-ynyl)-6,7-dihydro-pyrimido[6,1-a]isoquinolin-4-one,
2-([1,4]Dioxan-2-ylmethoxy)-9-hex-1-ynyl-6,7-dihydro-pyrimido[6,1-a]isoquinolin-4-one,
9-[3-(Benzyl-methyl-amino)-prop-1-ynyl]-2-([1,4]dioxan-2-ylmethoxy)-6,7-dihydro-pyrimido[6,1-a]isoquinolin-4-one,
2-([1,4]Dioxan-2-ylmethoxy)-9-(3-hydroxy-5-methyl-hex-1-ynyl)-6,7-dihydro-pyrimido[6,1-a]isoquinolin-4-one,
2-([1,4]Dioxan-2-ylmethoxy)-9-(3-hydroxy-but-1-ynyl)-6,7-dihydro-pyrimido[6,1-a]isoquinolin-4-one,
9-Cyclopropyl-2-([1,4]dioxan-2-ylmethoxy)-6,7-dihydro-pyrimido[6,1-a]isoquinolin-4-one,
2-([1,4]Dioxan-2-ylmethoxy)-9-(3-hydroxy-pent-1-ynyl)-6,7-dihydro-pyrimido[6,1-a]isoquinolin-4-one,
2-([1,4]Dioxan-2-ylmethoxy)-9-(3-hydroxy-4-methyl-pent-1-ynyl)-6,7-dihydro-pyrimido[6,1-a]isoquinolin-4-one,
2-([1,4]Dioxan-2-ylmethoxy)-9-(3-ethyl-3-hydroxy-pent-1-ynyl)-6,7-dihydro-pyrimido[6,1-a]isoquinolin-4-one,
2-([1,4]Dioxan-2-ylmethoxy)-9-(3-hydroxy-3-phenyl-but-1-ynyl)-6,7-dihydro-pyrimido[6,1-a]isoquinolin-4-one,
9-(3-Benzylamino-prop-1-ynyl)-2-([1,4]dioxan-2-ylmethoxy)-6,7-dihydro-pyrimido[6,1-a]isoquinolin-4-one,
2-([1,4]Dioxan-2-ylmethoxy)-9-[(furan-2-ylmethyl)-amino]-6,7-dihydro-pyrimido[6,1-a]isoquinolin-4-one,
2-([1,4]Dioxan-2-ylmethoxy)-9-(1-ethyl-1H-pyrazol-4-yl)-6,7-dihydro-pyrimido[6,1-a]isoquinolin-4-one,
2-([1,4]Dioxan-2-ylmethoxy)-9-[1-(3-methyl-butyl)-1H-pyrazol-4-yl]-6,7-dihydro-pyrimido[6,1-a]isoquinolin-4-one,
2-([1,4]Dioxan-2-ylmethoxy)-9-(5-methyl-furan-2-yl)-6,7-dihydro-pyrimido[6,1-a]isoquinolin-4-one,
2-([1,4]Dioxan-2-ylmethoxy)-9-(3-hydroxy-hex-1-ynyl)-6,7-dihydro-pyrimido[6,1-a]isoquinolin-4-one,
9-(3,5-Dimethyl-1H-pyrazol-4-yl)-2-([1,4]dioxan-2-ylmethoxy)-6,7-dihydro-pyrimido[6,1-a]isoquinolin-4-one,
2-([1,4]Dioxan-2-ylmethoxy)-9-(1H-pyrazol-4-yl)-6,7-dihydro-pyrimido[6,1-a]isoquinolin-4-one,
2-([1,4]Dioxan-2-ylmethoxy)-9-(1-propyl-1H-pyrazol-4-yl)-6,7-dihydro-pyrimido[6,1-a]isoquinolin-4-one,
2-[2-((R)-1-[1,4]Dioxan-2-ylmethoxy)-4-oxo-6,7-dihydro-4H-pyrimido[6,1-a]isoquinolin-9-yl]-benzonitrile,
2-[2-((S)-1-[1,4]Dioxan-2-ylmethoxy)-4-oxo-6,7-dihydro-4H-pyrimido[6,1-a]isoquinolin-9-yl]-benzonitrile,
9-(5-Cyclopropyl-[1,2,4]oxadiazol-3-ylmethoxy)-2-((R)-1-[1,4]dioxan-2-ylmethoxy)-6,7-dihydro-pyrimido[6,1-a]isoquinolin-4-one,
2-([1,4]Dioxan-2-ylmethoxy)-9-ethynyl-6,7-dihydro-pyrimido[6,1-a]isoquinolin-4-one,
2-([1,4]Dioxan-2-ylmethoxy)-9-pyrimidin-2-ylethynyl-6,7-dihydro-pyrimido[6,1-a]isoquinolin-4-one,
2-([1,4]Dioxan-2-ylmethoxy)-9-(3-phenylamino-prop-1-ynyl)-6,7-dihydro-pyrimido[6,1-a]isoquinolin-4-one,
2-([1,4]Dioxan-2-ylmethoxy)-9-(3-hydroxy-3-pyridin-3-yl-prop-1-ynyl)-6,7-dihydro-pyrimido[6,1-a] isoquinolin-4-one,
9-Cyclopentyloxymethyl-2-([1,4]dioxan-2-ylmethoxy)-6,7-dihydro-pyrimido[6,1-a]isoquinolin-4-one,
2-([1,4]Dioxan-2-ylmethoxy)-9-(3-methoxy-4-methyl-pent-1-ynyl)-6,7-dihydro-pyrimido[6,1-a]isoquinolin-4-one,
9-Cyclopropylethynyl-2-((R)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(3-methyl-but-1-ynyl)-6,7-dihydro-pyrimido[6,1-a]isoquinolin-4-one,
2-([1,4]Dioxan-2-ylmethoxy)-9-(3-imidazol-1-yl-prop-1-ynyl)-6,7-dihydro-pyrimido[6,1-a]isoquinolin-4-one,
9-(2-Cyclopropyl-ethyl)-2-((R)-1-[1,4]dioxan-2-ylmethoxy)-6,7-dihydro-pyrimido[6,1-a]isoquinolin-4-one,
9-Cyclopentyloxymethyl-2-((R)-1-[1,4]dioxan-2-ylmethoxy)-6,7-dihydro-pyrimido[6,1-a]isoquinolin-4-one,
2-([1,4]Dioxan-2-ylmethoxy)-9-(3-hydroxy-3-pyridin-3-yl-propyl)-6,7-dihydro-pyrimido[6,1-a]isoquinolin-4-one,
9-Allyloxy-2-((R)-1-[1,4]dioxan-2-ylmethoxy)-6,7-dihydro-pyrimido[6,1-a]isoquinolin-4-one,
9-Allyloxy-2-((S)-1-[1,4]dioxan-2-ylmethoxy)-6,7-dihydro-pyrimido[6,1-a]isoquinolin-4-one,
2-((R)-1-[1,4]Dioxan-2-ylmethoxy)-9-(tetrahydro-pyran-4-yloxymethyl)-6,7-dihydro-pyrimido[6,1-a]isoquinolin-4-one,
2-([1,4]Dioxan-2-ylmethoxy)-9-{3-[(pyridin-3-ylmethyl)-amino]-prop-1-ynyl}-6,7-dihydro-pyrimido[6,1-a]isoquinolin-4-one,
2-((R)-1-[1,4]Dioxan-2-ylmethoxy)-9-pentyl-6,7-dihydro-pyrimido[6,1-a]isoquinolin-4-one,
9-Cyclopropylethynyl-2-((S)-1-[1,4]dioxan-2-ylmethoxy)-6,7-dihydro-pyrimido[6,1-a]isoquinolin-4-one,
9-(2-Cyclopropyl-ethyl)-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(oxetan-3-yloxymethyl)-6,7-dihydro-pyrimido[6,1-a]isoquinolin-4-one,
2-((S)-1-[1,4]Dioxan-2-ylmethoxy)-9-(3-methyl-oxetan-3-ylmethoxymethyl)-6,7-dihydro-pyrimido[6,1-a]isoquinolin-4-one,
9-(2,2-Dimethyl-butylamino)-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(3-hydroxy-4-methyl-pentyl)-6,7-dihydro-pyrimido[6,1-a]isoquinolin-4-one,
2-((S)-1-[1,4]Dioxan-2-ylmethoxy)-9-(2-ethyl-hexylamino)-6,7-dihydro-pyrimido[6,1-a]isoquinolin-4-one,
2-((S)-1-[1,4]Dioxan-2-ylmethoxy)-9-(2-methoxy-ethoxy)-6,7-dihydro-pyrimido[6,1-a]isoquinolin-4-one,
2-((S)-1-[1,4]Dioxan-2-ylmethoxy)-9-(2-ethoxy-ethoxy)-6,7-dihydro-pyrimido[6,1-a]isoquinolin-4-one,
9-Cyclopropylmethoxy-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(2-fluoro-ethoxy)-6,7-dihydro-pyrimido[6,1-a]isoquinolin-4-one,
2-((S)-1-[1,4]Dioxan-2-ylmethoxy)-9-[3-(2-methoxy-ethoxy)-prop-1-ynyl]-6,7-dihydro-pyrimido[6,1-a]isoquinolin-4-one,
2-((S)-1-[1,4]Dioxan-2-ylmethoxy)-9-[3-(2-ethoxy-ethoxy)-prop-1-ynyl]-6,7-dihydro-pyrimido[6,1-a]isoquinolin-4-one,
2-((S)-1-[1,4]Dioxan-2-ylmethoxy)-9-[3-(2-fluoro-ethoxy)-prop-1-ynyl]-6,7-dihydro-pyrimido[6,1-a]isoquinolin-4-one,
9-(2,2-Dimethyl-propoxymethyl)-2-((S)-1-[1,4]dioxan-2-ylmethoxy)-6,7-dihydro-pyrimido[6,1-a]isoquinolin-4-one,
9-Cyclohexyloxymethyl-2-((S)-1-[1,4]dioxan-2-ylmethoxy)-6,7-dihydro-pyrimido[6,1-a]isoquinolin-4-one,
9-Cyclopropylmethoxymethyl-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(tetrahydro-pyran-2-ylmethoxy)-6,7-dihydro-pyrimido [6,1 - a]isoquinolin-4-one,
2-((S)-1-[1,4]Dioxan-2-ylmethoxy)-9-(3-hydroxy-butyl)-6,7-dihydro-pyrimido[6,1-a]isoquinolin-4-one,
9-(4,4-Dimethyl-pentyloxy)-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(3-methoxy-4-methyl-pentyl)-6,7-dihydro-pyrimido[6,1-a]isoquinolin-4-one,
9-(3-Cyclopropyl-propoxy)-2-((S)-1-[1,4]dioxan-2-ylmethoxy)-6,7-dihydro-pyrimido[6,1-a]isoquinolin-4-one,
9-Cyclohexylamino-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(3-hydroxy-4,4-dimethyl-pentyl)-6,7-dihydro-pyrimido[6,1-a]isoquinolin-4-one,
9-Cyclopentylmethoxymethyl-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(3-methoxy-butyl)-6,7-dihydro-pyrimido[6,1-a]isoquinolin-4-one,
2-((S)-1-[1,4]Dioxan-2-ylmethoxy)-9-(3-phenylamino-propyl)-6,7-dihydro-pyrimido[6,1-a]isoquinolin-4-one,
2-((S)-1-[1,4]Dioxan-2-ylmethoxy)-9-(4-hydroxy-pentyl)-6,7-dihydro-pyrimido[6,1-a]isoquinolin-4-one,
2-((S)-1-[1,4]Dioxan-2-ylmethoxy)-9-(4-hydroxy-butyl)-6,7-dihydro-pyrimido[6,1-a]isoquinolin-4-one,
9-(Cyclohexyl-methyl-amino)-2-((S)-1-[1,4]dioxan-2-ylmethoxy)-6,7-dihydro-pyrimido[6,1-a]isoquinolin-4-one,
9-(Cyclohexylmethyl-amino)-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-[(tetrahydro-pyran-4-ylmethyl)-amino]-6,7-dihydro-pyrimido[6,1-a]isoquinolin-4-one,
2-((S)-1-[1,4]Dioxan-2-ylmethoxy)-9-(3-ethyl-3-hydroxy-pentyl)-6,7-dihydro-pyrimido[6,1-a]isoquinolin-4-one,
2-((S)-1-[1,4]Dioxan-2-ylmethoxy)-9-(3-hydroxy-3-methyl-butyl)-6,7-dihydro-pyrimido[6,1-a]isoquinolin-4-one,
2-((S)-1-[1,4]Dioxan-2-ylmethoxy)-9-(3-hydroxy-pentyl)-6,7-dihydro-pyrimido[6,1-a]isoquinolin-4-one,
9-(2,2-Dimethyl-propoxy)-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(tetrahydro-pyran-4-ylmethoxy)-6,7-dihydro-pyrimido [6,1 - a]isoquinolin-4-one,
2-((S)-1-[1,4]Dioxan-2-ylmethoxy)-9-(4-hydroxy-4-methyl-pentyl)-6,7-dihydro-pyrimido[6,1-a]isoquinolin-4-one,
2-((S)-1-[1,4]Dioxan-2-ylmethoxy)-9-(tetrahydro-pyran-4-ylmethoxymethyl)-6,7-dihydro-pyrimido[6,1-a]isoquinolin-4-one,
2-([1,4]Dioxan-2-ylmethoxy)-9-methoxy-6,7-dihydro-pyrimido[6,1-a]isoquinolin-4-one,
2-((S)-1-[1,4]Dioxan-2-ylmethoxy)-9-(oxetan-3-ylmethoxy)-6,7-dihydro-pyrimido[6,1-a]isoquinolin-4-one,
9-(3-Cyclopropyl-propoxy)-2-((R)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(3-methoxy-propyl)-6,7-dihydro-pyrimido[6,1-a]isoquinolin-4-one,
2-((S)-1-[1,4]Dioxan-2-ylmethoxy)-9-[2-(1-hydroxy-cyclopentyl)-ethyl]-6,7-dihydro-pyrimido[6,1-a]isoquinolin-4-one,
2-((R)-1-[1,4]Dioxan-2-ylmethoxy)-9-(4-hydroxy-tetrahydro-pyran-4-ylethynyl)-6,7-dihydro-pyrimido[6,1-a]isoquinolin-4-one.
2-((R)-1-[1,4]Dioxan-2-ylmethoxy)-9-(3-methoxy-propyl)-6,7-dihydro-pyrimido[6,1-a]isoquinolin-4-one,
2-((R)-1-[1,4]Dioxan-2-ylmethoxy)-9-[2-(1-hydroxy-cyclopentyl)-ethyl]-6,7-dihydro-pyrimido[6,1-a]isoquinolin-4-one,
2-((S)-1-[1,4]Dioxan-2-ylmethoxy)-9-(2-propoxy-ethoxy)-6,7-dihydro-pyrimido[6,1-a]isoquinolin-4-one,
2-((S)-1-[1,4]Dioxan-2-ylmethoxy)-9-(2-isopropoxy-ethoxy)-6,7-dihydro-pyrimido[6,1-a]isoquinolin-4-one,
2-((R)-1-[1,4]Dioxan-2-ylmethoxy)-9-(2-propoxy-ethoxy)-6,7-dihydro-pyrimido[6,1-a]isoquinolin-4-one,
2-((R)-1-[1,4]Dioxan-2-ylmethoxy)-9-(2-isopropoxy-ethoxy)-6,7-dihydro-pyrimido[6,1-a]isoquinolin-4-one, and
2-((S)-1-[1,4]Dioxan-2-ylmethoxy)-9-(4-methoxy-butyl)-6,7-dihydro-pyrimido[6,1-a]isoquinolin-4-one.

In another embodiment, the compound for use is selected from
9-Methoxy-2-(tetrahydro-furan-2-ylmethoxy)-6,7-dihydro-pyrimido[6,1-a]isoquinolin-4-one,
2-(Chroman-2-ylmethoxy)-9-methoxy-6,7-dihydro-pyrimido[6,1-a]isoquinolin-4-one,
2-(2,3-Dihydro-benzo[1,4]dioxin-2-ylmethoxy)-9-methoxy-6,7-dihydro-pyrimido[6,1-a]isoquinolin-4-one,
9-Allyloxy-2-(tetrahydro-furan-2-ylmethoxy)-6,7-dihydro-pyrimido[6,1-a]isoquinolin-4-one,
2-(2,3-Dihydro-benzofuran-2-ylmethoxy)-9-methoxy-6,7-dihydro-pyrimido[6,1-a]isoquinolin-4-one,
9-Hydroxy-2-(tetrahydro-furan-2-ylmethoxy)-6,7-dihydro-pyrimido[6,1-a]isoquinolin-4-one,
9-Benzyloxy-2-(tetrahydro-furan-2-ylmethoxy)-6,7-dihydro-pyrimido[6,1-a]isoquinolin-4-one,
9-(Pyridin-3-ylmethoxy)-2-(tetrahydro-furan-2-ylmethoxy)-6,7-dihydro-pyrimido [6,1-a] isoquinolin-4-one,
[4-Oxo-2-(tetrahydro-furan-2-ylmethoxy)-6,7-dihydro-4H-pyrimido[6,1-a]isoquinolin-9-yloxy]-acetonitrile,
9-Butoxy-2-(tetrahydro-furan-2-ylmethoxy)-6,7-dihydro-pyrimido[6,1-a]isoquinolin-4-one,
9-Cyclopropylmethoxy-2-(tetrahydro-furan-2-ylmethoxy)-6,7-dihydro-pyrimido[6,1-a]isoquinolin-4-one,
9-Phenethyloxy-2-(tetrahydro-furan-2-ylmethoxy)-6,7-dihydro-pyrimido[6,1-a]isoquinolin-4-one,
9-(Pyridin-4-ylmethoxy)-2-(tetrahydro-furan-2-ylmethoxy)-6,7-dihydro-pyrimido[6,1-a]isoquinolin-4-one,
9-(Pyridin-2-ylmethoxy)-2-(tetrahydro-furan-2-ylmethoxy)-6,7-dihydro-pyrimido[6,1-a]isoquinolin-4-one,
9-(Pyridin-2-ylmethoxy)-2-(tetrahydro-furan-2-ylmethoxy)-6,7-dihydro-pyrimido[6,1-a]isoquinolin-4-one,
9-(2-Phenoxy-ethoxy)-2-(tetrahydro-furan-2-ylmethoxy)-6,7-dihydro-pyrimido[6,1-a]isoquinolin-4-one,
9-Methoxy-2-(tetrahydro-pyran-4-ylmethoxy)-6,7-dihydro-pyrimido[6,1-a]isoquinolin-4-one,
9-Methoxy-2-(tetrahydro-pyran-2-ylmethoxy)-6,7-dihydro-pyrimido[6,1-a]isoquinolin-4-one,
9-Methoxy-2-(tetrahydro-pyran-2-ylmethoxy)-6,7-dihydro-pyrimido[6,1-a]isoquinolin-4-one,
4-[4-Oxo-2-(tetrahydro-furan-2-ylmethoxy)-6,7-dihydro-4H-pyrimido[6,1-a]isoquinolin-9-yloxymethyl]-benzonitrile,
4-[4-Oxo-2-(tetrahydro-furan-2-ylmethoxy)-6,7-dihydro-4H-pyrimido[6,1-a]isoquinolin-9-yloxymethyl]-benzonitrile,
9-(2,3-Dihydro-benzo[1,4]dioxin-2-ylmethoxy)-2-(tetrahydro-furan-2-ylmethoxy)-6,7-dihydro-pyrimido[6,1-a]isoquinolin-4-one,
9-(2,3-Dihydro-benzo[1,4]dioxin-2-ylmethoxy)-2-(tetrahydro-furan-2-ylmethoxy)-6,7-dihydro-pyrimido[6,1-a]isoquinolin-4-one,
3-[4-Oxo-2-(tetrahydro-furan-2-ylmethoxy)-6,7-dihydro-4H-pyrimido[6,1-a]isoquinolin-9-yloxymethyl]-benzonitrile,
2-[4-Oxo-2-(tetrahydro-furan-2-ylmethoxy)-6,7-dihydro-4H-pyrimido[6,1-a]isoquinolin-9-yloxymethyl]-benzonitrile,
9-(4-Chloro-benzyloxy)-2-(tetrahydro-furan-2-ylmethoxy)-6,7-dihydro-pyrimido[6,1-a]isoquinolin-4-one,
9-(3-Chloro-benzyloxy)-2-(tetrahydro-furan-2-ylmethoxy)-6,7-dihydro-pyrimido[6,1-a]isoquinolin-4-one,
9-(2-Chloro-benzyloxy)-2-(tetrahydro-furan-2-ylmethoxy)-6,7-dihydro-pyrimido[6,1-a]isoquinolin-4-one,
9-(4-Fluoro-benzyloxy)-2-(tetrahydro-furan-2-ylmethoxy)-6,7-dihydro-pyrimido[6,1-a]isoquinolin-4-one,
9-(2-Nitro-benzyloxy)-2-(tetrahydro-furan-2-ylmethoxy)-6,7-dihydro-pyrimido[6,1-a]isoquinolin-4-one,
4-[4-Oxo-2-(tetrahydro-furan-2-ylmethoxy)-6,7-dihydro-4H-pyrimido[6,1-a]isoquinolin-9-yloxymethyl]-benzoic acid methyl ester,
3-[4-Oxo-2-(tetrahydro-furan-2-ylmethoxy)-6,7-dihydro-4H-pyrimido [6,1-a] isoquinolin-9-yloxymethyl] - benzoic acid methyl ester,
3-[4-Oxo-2-(tetrahydro-furan-2-ylmethoxy)-6,7-dihydro-4H-pyrimido[6,1-a]isoquinolin-9-yloxymethyl]-benzoic acid methyl ester,
2-(2,3-Dihydro-benzo[1,4]dioxin-2-ylmethoxy)-9-(pyridin-2-ylmethoxy)-6,7-dihydro-pyrimido[6,1-a]isoquinolin-4-one,
[2-(2,3-Dihydro-benzo[1,4]dioxin-2-ylmethoxy)-4-oxo-6,7-dihydro-4H-pyrimido[6,1-a]isoquinolin-9-yloxy]-acetic acid tert-butyl ester,
2,9-Bis-(2,3-dihydro-benzo[1,4]dioxin-2-ylmethoxy)-6,7-dihydro-pyrimido[6,1-a]isoquinolin-4-one,
[2-(2,3-Dihydro-benzo[1,4] dioxin-2-ylmethoxy)-4-oxo-6,7-dihydro-4H-pyrimido [6,1-a] isoquinolin-9-yloxy]-acetic acid,
9-(3-Nitro-benzyloxy)-2-(tetrahydro-furan-2-ylmethoxy)-6,7-dihydro-pyrimido[6,1-a]isoquinolin-4-one,
9-(3-Nitro-benzyloxy)-2-(tetrahydro-furan-2-ylmethoxy)-6,7-dihydro-pyrimido[6,1-a]isoquinolin-4-one,
9-(4-Nitro-benzyloxy)-2-(tetrahydro-furan-2-ylmethoxy)-6,7-dihydro-pyrimido[6,1-a]isoquinolin-4-one,
9-(3-Methyl-benzyloxy)-2-(tetrahydro-furan-2-ylmethoxy)-6,7-dihydro-pyrimido[6,1-a]isoquinolin-4-one,
9-(4-Methyl-benzyloxy)-2-(tetrahydro-furan-2-ylmethoxy)-6,7-dihydro-pyrimido[6,1-a]isoquinolin-4-one,
9-(4-Methoxy-benzyloxy)-2-(tetrahydro-furan-2-ylmethoxy)-6,7-dihydro-pyrimido[6,1-a]isoquinolin-4-one,
9-(Naphthalen-2-ylmethoxy)-2-(tetrahydro-furan-2-ylmethoxy)-6,7-dihydro-pyrimido[6,1-a]isoquinolin-4-one,
9-(Naphthalen-1-ylmethoxy)-2-(tetrahydro-furan-2-ylmethoxy)-6,7-dihydro-pyrimido[6,1-a]isoquinolin-4-one,
9-(2-Methyl-benzyloxy)-2-(tetrahydro-furan-2-ylmethoxy)-6,7-dihydro-pyrimido[6,1-a]isoquinolin-4-one,
9-[2-(2-Methoxy-phenoxy)-ethoxy]-2-(tetrahydro-furan-2-ylmethoxy)-6,7-dihydro-pyrimido[6,1-a]isoquinolin-4-one,
9-[2-(3-Methoxy-phenoxy)-ethoxy]-2-(tetrahydro-furan-2-ylmethoxy)-6,7-dihydro-pyrimido[6,1-a]isoquinolin-4-one,
9-[2-(4-Methoxy-phenoxy)-ethoxy]-2-(tetrahydro-furan-2-ylmethoxy)-6,7-dihydro-pyrimido[6,1-a]isoquinolin-4-one,
9-[2-(2-Chloro-phenoxy)-ethoxy]-2-(tetrahydro-furan-2-ylmethoxy)-6,7-dihydro-pyrimido[6,1-a]isoquinolin-4-one,
9-[2-(3-Chloro-phenoxy)-ethoxy]-2-(tetrahydro-furan-2-ylmethoxy)-6,7-dihydro-pyrimido[6,1-a]isoquinolin-4-one,
9-[2-(4-Chloro-phenoxy)-ethoxy]-2-(tetrahydro-furan-2-ylmethoxy)-6,7-dihydro-pyrimido[6,1-a] isoquinolin-4-one,
2-(2,3-Dihydro-benzo[1,4]dioxin-2-ylmethoxy)-9-(2-morpholin-4-yl-2-oxo-ethoxy)-6,7-dihydro-pyrimido[6,1-a]isoquinolin-4-one,
2-(2,3-Dihydro-benzo[1,4]dioxin-2-ylmethoxy)-9-(2-morpholin-4-yl-2-oxo-ethoxy)-6,7-dihydro-pyrimido[6,1-a]isoquinolin-4-one,
2-(2,3-Dihydro-benzo[1,4]dioxin-2-ylmethoxy)-9-(2-morpholin-4-yl-2-oxo-ethoxy)-6,7-dihydro-pyrimido[6,1-a]isoquinolin-4-one,
2-(2,3-Dihydro-benzo[1,4]dioxin-2-ylmethoxy)-9-(2-morpholin-4-yl-2-oxo-ethoxy)-6,7-dihydro-pyrimido[6,1-a]isoquinolin-4-one,
2-[2-(2,3-Dihydro-benzo[1,4]dioxin-2-ylmethoxy)-4-oxo-6,7-dihydro-4H-pyrimido[6,1-a]isoquinolin-9-yloxy]-acetamide,
2-[2-(2,3-Dihydro-benzo[1,4]dioxin-2-ylmethoxy)-4-oxo-6,7-dihydro-4H-pyrimido[6,1-a]isoquinolin-9-yloxy]-N,N-dimethyl-acetamide,
9-(2,2-Dimethoxy-ethoxy)-2-(tetrahydro-furan-2-ylmethoxy)-6,7-dihydro-pyrimido[6,1-a]isoquinolin-4-one,
2-[2-(2,3-Dihydro-benzo[1,4]dioxin-2-ylmethoxy)-4-oxo-6,7-dihydro-4H-pyrimido[6,1-a]isoquinolin-9-yloxy]-2-methyl-propionamide,
2-(2,3-Dihydro-benzo[1,4]dioxin-2-ylmethoxy)-9-(1,1-dimethyl-2-morpholin-4-yl-2-oxo-ethoxy)-6,7-dihydro-pyrimido [6, 1-a] isoquinolin-4-one,
9-(2-Benzyloxy-ethoxy)-2-(tetrahydro-furan-2-ylmethoxy)-6,7-dihydro-pyrimido[6,1-a]isoquinolin-4-one,
2,9-Bis-(tetrahydro-furan-2-ylmethoxy)-6,7-dihydro-pyrimido[6,1-a]isoquinolin-4-one,
9-(6-Phenyl-pyridin-2-ylmethoxy)-2-(tetrahydro-furan-2-ylmethoxy)-6,7-dihydro-pyrimido[6,1-a]isoquinolin-4-one,
2-(2,3-Dihydro-benzo[1,4]dioxin-2-ylmethoxy)-9-(tetrahydro-furan-2-ylmethoxy)-6,7-dihydro-pyrimido[6,1-a]isoquinolin-4-one,
9-[6-(1-Methyl-1H-pyrazol-4-yl)-pyridin-2-ylmethoxy]-2-(tetrahydro-furan-2-ylmethoxy)-6,7-dihydro-pyrimido[6,1-a]isoquinolin-4-one,
9-(6-Furan-3-yl-pyridin-2-ylmethoxy)-2-(tetrahydro-furan-2-ylmethoxy)-6,7-dihydro-pyrimido[6,1-a]isoquinolin-4-one,
9-(6-Pyrimidin-5-yl-pyridin-2-ylmethoxy)-2-(tetrahydro-furan-2-ylmethoxy)-6,7-dihydro-pyrimido[6,1-a]isoquinolin-4-one,
9-(1-Cyclopropyl-1H-tetrazol-5-ylmethoxy)-2-(tetrahydro-furan-2-ylmethoxy)-6,7-dihydro-pyrimido[6,1-a]isoquinolin-4-one,
2-[4-Oxo-2-(tetrahydro-furan-2-ylmethoxy)-6,7-dihydro-4H-pyrimido[6,1-a]isoquinolin-9-yloxy]-acetamide,
N,N-Diethyl-2-[4-oxo-2-(tetrahydro-furan-2-ylmethoxy)-6,7-dihydro-4H-pyrimido[6,1-a]isoquinolin-9-yloxy]-acetamide,
N,N-Dimethyl-2-[4-oxo-2-(tetrahydro-furan-2-ylmethoxy)-6,7-dihydro-4H-pyrimido[6,1-a]isoquinolin-9-yloxy]-acetamide,
N-Isopropyl-N-methyl-2-[4-oxo-2-(tetrahydro-furan-2-ylmethoxy)-6,7-dihydro-4H-pyrimido[6,1-a]isoquinolin-9-yloxy]-acetamide,
2-[4-Oxo-2-(tetrahydro-furan-2-ylmethoxy)-6,7-dihydro-4H-pyrimido[6,1-a]isoquinolin-9-yloxy]-N-phenyl-acetamide,
9-(1-Propyl-1H-tetrazol-5-ylmethoxy)-2-(tetrahydro-furan-2-ylmethoxy)-6,7-dihydro-pyrimido[6,1-a]isoquinolin-4-one,
9-(Oxazol-2-ylmethoxy)-2-(tetrahydro-furan-2-ylmethoxy)-6,7-dihydro-pyrimido[6,1-a]isoquinolin-4-one,
2-[2-(2,3-Dihydro-benzo[1,4]dioxin-2-ylmethoxy)-4-oxo-6,7-dihydro-4H-pyrimido[6,1-a]isoquinolin-9-yloxy]-N,N-diethyl-acetamide,
2-[2-(2,3-Dihydro-benzo[1,4]dioxin-2-ylmethoxy)-4-oxo-6,7-dihydro-4H-pyrimido[6,1-a]isoquinolin-9-yloxy]-N-isopropyl-N-methyl-acetamide,
2-[2-(2,3-Dihydro-benzo[1,4]dioxin-2-ylmethoxy)-4-oxo-6,7-dihydro-4H-pyrimido[6,1-a]isoquinolin-9-yloxy]-N-phenyl-acetamide,
2-(2,3-Dihydro-benzo[1,4]dioxin-2-ylmethoxy)-9-(1-propyl-1H-tetrazol-5-ylmethoxy)-6,7-dihydro-pyrimido[6,1-a]isoquinolin-4-one,
9-(1-Butyl-1H-tetrazol-5-ylmethoxy)-2-(2,3-dihydro-benzo[1,4]dioxin-2-ylmethoxy)-6,7-dihydro-pyrimido[6,1-a]isoquinolin-4-one,
2-(2,3-Dihydro-benzo[1,4]dioxin-2-ylmethoxy)-9-(2-morpholin-4-yl-ethoxy)-6,7-dihydro-pyrimido[6,1-a]isoquinolin-4-one,
[2-(2,3-Dihydro-benzo[1,4] dioxin-2-ylmethoxy)-4-oxo-6,7-dihydro-4H-pyrimido [6,1-a] isoquinolin-9-yloxy]-acetonitrile,
2-(2,3-Dihydro-benzo[1,4]dioxin-2-ylmethoxy)-9-(oxazol-2-ylmethoxy)-6,7-dihydro-pyrimido[6,1-a]isoquinolin-4-one,
2-(2,3-Dihydro-benzo[1,4]dioxin-2-ylmethoxy)-9-(2-oxo-2-pyrrolidin-1-yl-ethoxy)-6,7-dihydro-pyrimido[6,1-a]isoquinolin-4-one,
9-(1-Cyclopropyl-1H-tetrazol-5-ylmethoxy)-2-(2,3-dihydro-benzo[1,4]dioxin-2-ylmethoxy)-6,7-dihydro-pyrimido[6,1-a]isoquinolin-4-one,
2-(2,3-Dihydro-benzo[1,4]dioxin-2-ylmethoxy)-9-(1H-tetrazol-5-ylmethoxy)-6,7-dihydro-pyrimido[6,1-a]isoquinolin-4-one,
9-Allyloxy-2-(2,3-dihydro-[1,4]dioxino[2,3-b]pyridin-2-ylmethoxy)-6,7-dihydro-pyrimido[6,1-a]isoquinolin-4-one,
N-Benzyl-2-[2-(2,3-dihydro-benzo[1,4]dioxin-2-ylmethoxy)-4-oxo-6,7-dihydro-4H-pyrimido[6,1-a]isoquinolin-9-yloxy]-acetamide,
2-(2,3-Dihydro-benzo[1,4]dioxin-2-ylmethoxy)-9-(2-pyrrolidin-1-yl-ethoxy)-6,7-dihydro-pyrimido[6,1-a]isoquinolin-4-one,
2-(2,3-Dihydro-benzo[1,4]dioxin-2-ylmethoxy)-9-(2-piperidin-1-yl-ethoxy)-6,7-dihydro-pyrimido[6,1-a]isoquinolin-4-one,
2-(2,3 -Dihydro-benzo[1,4] dioxin-2-ylmethoxy)-9-(3 -piperidin-1-yl-propoxy)-6,7-dihydro-pyrimido[6,1-a]isoquinolin-4-one,
2-(2,3-Dihydro-benzo[1,4]dioxin-2-ylmethoxy)-9-(3-dimethylamino-propoxy)-6,7-dihydro-pyrimido[6,1-a]isoquinolin-4-one,
2-(2,3-Dihydro-benzo[1,4]dioxin-2-ylmethoxy)-9-[2-(1-methyl-pyrrolidin-2-yl)-ethoxy]-6,7-dihydro-pyrimido[6,1-a]isoquinolin-4-one,
2-(2,3-Dihydro-benzo[1,4]dioxin-2-ylmethoxy)-9-[3-(4-methyl-piperazin-1-yl)-propoxy]-6,7-dihydro-pyrimido[6,1-a]isoquinolin-4-one,
5-[2-(2,3-Dihydro-benzo[1,4]dioxin-2-ylmethoxy)-4-oxo-6,7-dihydro-4H-pyrimido[6,1-a]isoquinolin-9-yloxymethyl]-furan-2-carboxylic acid ethyl ester,
2-(2,3-Dihydro-[1,4]dioxino[2,3-b]pyridin-2-ylmethoxy)-9-(oxazol-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(oxazol-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(oxazol-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(oxazol-2-ylmethoxy)-6,7-dihydro-pyrimido[6,1-a]isoquinolin-4-one,
9-(5-tert-Butyl-[1,2,4]oxadiazol-3-ylmethoxy)-2-(tetrahydro-furan-2-ylmethoxy)-6,7-dihydro-pyrimido[6,1-a]isoquinolin-4-one,
9-(5-Phenyl-[1,2,4]oxadiazol-3-ylmethoxy)-2-(tetrahydro-furan-2-ylmethoxy)-6,7-dihydro-pyrimido[6,1-a]isoquinolin-4-one,
[2-(2,3-Dihydro-[1,4]dioxino[2,3-b]pyridin-2-ylmethoxy)-4-oxo-6,7-dihydro-4H-pyrimido[6,1-a] isoquinolin-9-yloxy]-acetonitrile,
[2-(2,3-Dihydro-[1,4]dioxino[2,3-b]pyridin-2-ylmethoxy)-4-oxo-6,7-dihydro-4H-pyrimido[6,1-a] isoquinolin-9-yloxy]-acetonitrile,
[2-(2,3-Dihydro-[1,4]dioxino[2,3-b]pyridin-2-ylmethoxy)-4-oxo-6,7-dihydro-4H-pyrimido[6,1-a] isoquinolin-9-yloxy]-acetonitrile,
2-(2,3-Dihydro-[1,4]dioxino[2,3-b]pyridin-2-ylmethoxy)-9-(2-morpholin-4-yl-ethoxy)-6,7-dihydro-pyrimido[6,1-a]isoquinolin-4-one,
2-(2,3-Dihydro-[1,4]dioxino[2,3-b]pyridin-2-ylmethoxy)-9-(pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-[2-(1-methyl-pyrrolidin-2-yl)-ethoxy]-6,7-dihydro-pyrimido [6, 1-a] isoquinolin-4-one,
2-(2,3-Dihydro-[1,4]dioxino[2,3-b]pyridin-2-ylmethoxy)-9-(1H-tetrazol-5-ylmethoxy)-6,7-dihydro-pyrimido[6,1-a]isoquinolin-4-one,
9-Pyridin-3-yl-2-(tetrahydro-furan-2-ylmethoxy)-6,7-dihydro-pyrimido[6,1-a]isoquinolin-4-one,
3-[4-Oxo-2-(tetrahydro-furan-2-ylmethoxy)-6,7-dihydro-4H-pyrimido[6,1-a]isoquinolin-9-yl]-benzonitrile,
9-Phenyl-2-(tetrahydro-furan-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-pyridin-4-yl-6,7-dihydro-pyrimido[6,1-a]isoquinolin-4-one,
3-[2-(2,3-Dihydro-[1,4]dioxino[2,3-b]pyridin-2-ylmethoxy)-4-oxo-6,7-dihydro-4H-pyrimido[6,1-a] isoquinolin-9-yl] -benzonitrile,
9-(2-Methoxy-phenyl)-2-(tetrahydro-furan-2-ylmethoxy)-6,7-dihydro-pyrimido[6,1-a]isoquinolin-4-one,
9-(3-Methoxy-phenyl)-2-(tetrahydro-furan-2-ylmethoxy)-6,7-dihydro-pyrimido[6,1-a]isoquinolin-4-one,
9-(4-Methoxy-phenyl)-2-(tetrahydro-furan-2-ylmethoxy)-6,7-dihydro-pyrimido[6,1-a]isoquinolin-4-one,
4-[4-Oxo-2-(tetrahydro-furan-2-ylmethoxy)-6,7-dihydro-4H-pyrimido[6,1-a]isoquinolin-9-yl]-benzonitrile,
3-[4-Oxo-2-(tetrahydro-furan-2-ylmethoxy)-6,7-dihydro-4H-pyrimido[6,1-a]isoquinolin-9-yl]-benzoic acid,
4-[4-Oxo-2-(tetrahydro-furan-2-ylmethoxy)-6,7-dihydro-4H-pyrimido[6,1-a]isoquinolin-9-yl]-benzoic acid,
9-(4-Dimethylamino-phenyl)-2-(tetrahydro-furan-2-ylmethoxy)-6,7-dihydro-pyrimido[6,1-a]isoquinolin-4-one,
4-[4-Oxo-2-(tetrahydro-furan-2-ylmethoxy)-6,7-dihydro-4H-pyrimido[6,1-a]isoquinolin-9-yl]-benzamide,
2-[4-Oxo-2-(tetrahydro-furan-2-ylmethoxy)-6,7-dihydro-4H-pyrimido[6,1-a]isoquinolin-9-yl]-benzonitrile,
9-(1-Methyl-1H-pyrazol-4-yl)-2-(tetrahydro-furan-2-ylmethoxy)-6,7-dihydro-pyrimido[6,1-a]isoquinolin-4-one,
N,N-Dimethyl-3-[4-oxo-2-(tetrahydro-furan-2-ylmethoxy)-6,7-dihydro-4H-pyrimido[6,1-a]isoquinolin-9-yl]-benzamide,
9-(6-Methoxy-pyridin-3-yl)-2-(tetrahydro-furan-2-ylmethoxy)-6,7-dihydro-pyrimido[6,1-a]isoquinolin-4-one,
9-(2,6-Dimethyl-phenyl)-2-(tetrahydro-furan-2-ylmethoxy)-6,7-dihydro-pyrimido[6,1-a]isoquinolin-4-one,
9-(3,5-Dimethyl-isoxazol-4-yl)-2-(tetrahydro-furan-2-ylmethoxy)-6,7-dihydro-pyrimido[6,1-a]isoquinolin-4-one,
9-Naphthalen-2-yl-2-(tetrahydro-furan-2-ylmethoxy)-6,7-dihydro-pyrimido[6,1-a]isoquinolin-4-one,
9-Naphthalen-1-yl-2-(tetrahydro-furan-2-ylmethoxy)-6,7-dihydro-pyrimido[6,1-a]isoquinolin-4-one,
9-Pyrimidin-5-yl-2-(tetrahydro-furan-2-ylmethoxy)-6,7-dihydro-pyrimido[6,1-a]isoquinolin-4-one,
9-(5-Chloro-thiophen-2-yl)-2-(tetrahydro-furan-2-ylmethoxy)-6,7-dihydro-pyrimido[6,1-a]isoquinolin-4-one,
2-[4-Oxo-2-(tetrahydro-furan-2-ylmethoxy)-6,7-dihydro-4H-pyrimido[6,1-a]isoquinolin-9-yl]-pyrrole-1-carboxylic acid tert-butyl ester,
Trifluoro-methanesulfonic acid 4-oxo-2-(tetrahydro-furan-2-ylmethoxy)-6,7-dihydro-4H-pyrimido[6,1-a]isoquinolin-9-yl ester,
2-(2,3-Dihydro-[1,4]dioxino[2,3-b]pyridin-2-ylmethoxy)-9-(6-methoxy-pyridin-3-yl)-6,7-dihydro-pyrimido[6,1-a]isoquinolin-4-one,
9-Cyclopropylethynyl-2-(tetrahydro-furan-2-ylmethoxy)-6,7-dihydro-pyrimido[6,1-a]isoquinolin-4-one,
9-(3,3-Dimethyl-but-1-ynyl)-2-(tetrahydro-furan-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(pyridin-4-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-pyridin-3-yl-6,7-dihydro-pyrimido[6,1-a]isoquinolin-4-one,
2-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-benzonitrile,
2-(2,3-Dihydro-[1,4]dioxino[2,3-b]pyridin-2-ylmethoxy)-9-(2-methoxy-phenyl)-6,7-dihydro-pyrimido[6,1-a]isoquinolin-4-one,
2-(2,3-Dihydro-[1,4]dioxino[2,3-b]pyridin-2-ylmethoxy)-9-(1H-indazol-5-yl)-6,7-dihydro-pyrimido[6,1-a]isoquinolin-4-one,
2-(2,3-Dihydro-[1,4]dioxino[2,3-b]pyridin-2-ylmethoxy)-9-pyrimidin-5-yl-6,7-dihydro-pyrimido[6,1-a]isoquinolin-4-one,
3-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-benzamide,
2-(2,3-Dihydro-[1,4]dioxino[2,3-b]pyridin-2-ylmethoxy)-9-(2-dimethylamino-phenyl)-6,7-dihydro-pyrimido[6,1-a]isoquinolin-4-one,
2-(2,3-Dihydro-[1,4]dioxino[2,3-b]pyridin-2-ylmethoxy)-9-pyridin-3-ylethynyl-6,7-dihydro-pyrimido[6,1-a]isoquinolin-4-one,
2-(2,3-Dihydro-[1,4]dioxino[2,3-b]pyridin-2-ylmethoxy)-9-(3-methoxy-prop-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(4-hydroxy-but-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(1,5-dimethyl-1H-pyrazol-3-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(3-methyl-[1,2,4]oxadiazol-5-ylmethoxy)-6,7-dihydro-pyrimido [6,1-a] isoquinolin-4-one,
2-(2,3-Dihydro-[1,4]dioxino[2,3-b]pyridin-2-ylmethoxy)-9-(3-hydroxy-3-methyl-but-1-ynyl)-6,7-dihydro-pyrimido [6,1-a] isoquinolin-4-one,
2-(2,3-Dihydro-[1,4]dioxino[2,3-b]pyridin-2-ylmethoxy)-9-pyridin-4-ylethynyl-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-prop-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(6-methyl-pyridin-3-yl)-6,7-dihydro-pyrimido[6,1-a]isoquinolin-4-one,
2-(2,3-Dihydro-[1,4]dioxino[2,3-b]pyridin-2-ylmethoxy)-9-(5-methoxy-pyridin-3-yl)-6,7-dihydro-pyrimido[6,1-a]isoquinolin-4-one,
9-Cyclopropylethynyl-2-(2,3-dihydro-[1,4]dioxino[2,3-b]pyridin-2-ylmethoxy)-6,7-dihydro-pyrimido[6,1-a]isoquinolin-4-one,
9-Cyclopropylethynyl-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(1-hydroxy-cyclopentylethynyl)-6,7-dihydro-pyrimido[6,1-a]isoquinolin-4-one,
5-[2-(2,3-Dihydro-[1,4]dioxino[2,3-b]pyridin-2-ylmethoxy)-4-oxo-6,7-dihydro-4H-pyrimido[6,1-a] isoquinolin-9-yl] -pent-4-ynenitrile,
2-(2,3-Dihydro-[1,4]dioxino[2,3-b]pyridin-2-ylmethoxy)-9-(3,3-dimethyl-but-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(2-methoxy-pyridin-3-yl)-6,7-dihydro-pyrimido[6,1-a]isoquinolin-4-one,
2-(2,3-Dihydro-[1,4]dioxino[2,3-b]pyridin-2-ylmethoxy)-6,7-dihydro-pyrimido[6,1-a]isoquinolin-4-one,
5-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-pyridine-2-carboxylic acid methylamide,
2-(2,3-Dihydro-[1,4]dioxino[2,3-b]pyridin-2-ylmethoxy)-9-furan-3-yl-6,7-dihydro-pyrimido[6,1-a]isoquinolin-4-one,
2-(2,3-Dihydro-[1,4]dioxino[2,3-b]pyridin-2-ylmethoxy)-9-(1-methyl-1H-pyrazol-4-yl)-6,7-dihydro-pyrimido[6,1-a]isoquinolin-4-one,
2-(2,3-Dihydro-[1,4]dioxino[2,3-b]pyridin-2-ylmethoxy)-9-morpholin-4-ylmethyl-6,7-dihydro-pyrimido[6,1-a]isoquinolin-4-one,
[2-(2,3-Dihydro-[1,4]dioxino[2,3-b]pyridin-2-ylmethoxy)-4-oxo-6,7-dihydro-4H-pyrimido[6,1-a]isoquinolin-9-ylamino]-acetonitrile,
2-(2,3-Dihydro-[1,4]dioxino[2,3-b]pyridin-2-ylmethoxy)-4-oxo-6,7-dihydro-4H-pyrimido[6,1-a]isoquinoline-9-carbonitrile,
2-(2,3-Dihydro-[1,4]dioxino[2,3-b]pyridin-2-ylmethoxy)-9-[(oxazol-2-ylmethyl)-amino]-6,7-dihydro-pyrimido[6,1-a]isoquinolin-4-one,
2-(2,3-Dihydro-[1,4]dioxino[2,3-b]pyridin-2-ylmethoxy)-9-(5-methyl-[1,2,4]oxadiazol-3-ylmethoxy)-6,7-dihydro-pyrimido [6, 1-a] isoquinolin-4-one,
2-(2,3-Dihydro-[1,4]dioxino[2,3-b]pyridin-2-ylmethoxy)-9-(5-ethyl-[1,2,4]oxadiazol-3-ylmethoxy)-6,7-dihydro-pyrimido [6, 1-a] isoquinolin-4-one,
9-(5-Cyclopropyl-[1,2,4]oxadiazol-3-ylmethoxy)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(5-isopropyl-[1,2,4]oxadiazol-3-ylmethoxy)-6,7-dihydro-pyrimido[6,1-a]isoquinolin-4-one,
9-(5-tert-Butyl-[1,2,4]oxadiazol-3-ylmethoxy)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(3-methyl-isoxazol-5-ylmethoxy)-6,7-dihydro-pyrimido [6, 1-a] isoquinolin-4-one,
9-(3-Chloro-2-methoxy-pyridin-4-yl)-2-(2,3-dihydro-[1,4]dioxino[2,3-b]pyridin-2-ylmethoxy)-6,7-dihydro-pyrimido [6, 1-a] isoquinolin-4-one,
2-(2,3-Dihydro-[1,4]dioxino[2,3-b]pyridin-2-ylmethoxy)-9-(3,6-dihydro-2H-pyran-4-yl)-6,7-dihydro-pyrimido[6,1-a]isoquinolin-4-one,
5-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-pyridine-2-carbonitrile,
2-(2,3-Dihydro-[1,4]dioxino[2,3-b]pyridin-2-ylmethoxy)-9-(2-ethoxy-pyridin-3-yl)-6,7-dihydro-pyrimido[6,1-a]isoquinolin-4-one,
2-(2,3-Dihydro-[1,4]dioxino[2,3-b]pyridin-2-ylmethoxy)-9-(6-ethoxy-pyridin-3-yl)-6,7-dihydro-pyrimido[6,1-a]isoquinolin-4-one,
2-(2,3-Dihydro-[1,4]dioxino[2,3-b]pyridin-2-ylmethoxy)-9-(6-morpholin-4-yl-pyridin-3-yl)-6,7-dihydro-pyrimido[6,1-a]isoquinolin-4-one,
2-(2,3-Dihydro-[1,4]dioxino[2,3-b]pyridin-2-ylmethoxy)-9-(2-methoxy-pyrimidin-5-yl)-6,7-dihydro-pyrimido[6,1-a]isoquinolin-4-one,
2-(2,3-Dihydro-[1,4]dioxino[2,3-b]pyridin-2-ylmethoxy)-9-(2,3-dimethoxy-phenyl)-6,7-dihydro-pyrimido[6,1-a]isoquinolin-4-one,
2-(2,3-Dihydro-[1,4]dioxino[2,3-b]pyridin-2-ylmethoxy)-9-(2,5-dimethoxy-phenyl)-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-3-phenyl-prop-1-ynyl)-6,7-dihydro-pyrimido [6, 1-a] isoquinolin-4-one,
3-{3-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-prop-2-ynyloxy}-propionitrile,
2-(2,3-Dihydro-[1,4]dioxino[2,3-b]pyridin-2-ylmethoxy)-9-(3-methylamino-prop-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(3-dimethylamino-prop-1-ynyl)-6,7-dihydro-pyrimido[6,1-a]isoquinolin-4-one,
9-[3-(Benzyl-methyl-amino)-prop-1-ynyl]-2-(2,3-diohydro-[1,4]dioxino[2,3-b]pyridin-2-ylmethoxy)-6,7-dihydro-pyrimido [6, 1-a] isoquinolin-4-one,
2-(2,3-Dihydro-[1,4]dioxino[2,3-b]pyridin-2-ylmethoxy)-9-[3-(1,1-dioxo-thiomorpholin-4-yl)-prop-1-ynyl] -6,7-dihydro-pyrimido[6,1-a]isoquinolin-4-one,
{3-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-prop-2-ynyl}-urea,
1-{3-[2-(2,3-Dihydro-[1,4]dioxino[2,3-b]pyridin-2-ylmethoxy)-4-oxo-6,7-dihydro-4H-pyrimido[6,1-a] isoquinolin-9-yl] -prop-2-ynyl} -imidazolidine-2,4-dione,
9-(3-Diethylamino-prop-1-ynyl)-2-(2,3-dihydro-[1,4]dioxino[2,3-b]pyridin-2-ylmethoxy)-6,7-dihydro-pyrimido[6,1-a]isoquinolin-4-one,
9-(3-Amino-3-methyl-but-1-ynyl)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(tetrahydro-pyran-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(1H-pyrazol-4-yl)-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-but-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-pent-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-hex-1-ynyl)-6,7-dihydro-pyrimido[6,1-a]isoquinolin-4-one,
9-(1-Amino-cyclohexylethynyl)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-5-methyl-hex-1-ynyl)-6,7-dihydro-pyrimido [6, 1-a] isoquinolin-4-one,
2-(2,3-Dihydro-[1,4]dioxino[2,3-b]pyridin-2-ylmethoxy)-9-(3-ethyl-3-hydroxy-pent-1-ynyl)-6,7-dihydro-pyrimido[6,1-a]isoquinolin-4-one,
2-(2,3-Dihydro-[1,4]dioxino [2,3 -b]pyridin-2-ylmethoxy)-9-(3-hydroxy-3-phenyl-but-1-ynyl)-6,7-dihydro-pyrimido [6, 1-a] isoquinolin-4-one,
2-(2,3-Dihydro-[1,4]dioxino[2,3-b]pyridin-2-ylmethoxy)-9-(3-hydroxy-4-methyl-pent-1-ynyl)-6,7-dihydro-pyrimido [6, 1-a] isoquinolin-4-one,
2-[(R)-1-(2,3-Dihydro-[1,4]dioxino[2,3-b]pyridin-2-yl)methoxy]-9-(oxazol-2-ylmethoxy)-6,7-dihydro-pyrimido[6,1-a]isoquinolin-4-one,
2-[(R)-1-(2,3-Dihydro-[1,4]dioxino[2,3-b]pyridin-2-yl)methoxy]-9-(oxazol-2-ylmethoxy)-6,7-dihydro-pyrimido[6,1-a]isoquinolin-4-one,
2-[(S)-1-(2,3-Dihydro-[1,4]dioxino[2,3-b]pyridin-2-yl)methoxy]-9-(oxazol-2-ylmethoxy)-6,7-dihydro-pyrimido[6,1-a]isoquinolin-4-one,
9-(3-Butylamino-prop-1-ynyl)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(2-morpholin-4-yl-ethoxymethyl)-6,7-dihydro-pyrimido[6,1-a]isoquinolin-4-one,
9-(3-Benzylamino-prop-1-ynyl)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(3-phenylamino-prop-1-ynyl)-6,7-dihydro-pyrimido[6,1-a]isoquinolin-4-one,
2-(2,3-Dihydro-[1,4]dioxino[2,3-b]pyridin-2-ylmethoxy)-4-oxo-6,7-dihydro-4H-pyrimido[6,1-a]isoquinoline-9-carboxylic acid (tetrahydro-pyran-4-yl)-amide,
2-(2,3-Dihydro-[1,4]dioxino[2,3-b]pyridin-2-ylmethoxy)-4-oxo-6,7-dihydro-4H-pyrimido[6,1-a]isoquinoline-9-carboxylic acid (oxetan-3-ylmethyl)-amide,
2-(2,3-Dihydro-[1,4]dioxino[2,3-b]pyridin-2-ylmethoxy)-9-pyrrolidin-1-ylmethyl-6,7-dihydro-pyrimido[6,1-a]isoquinolin-4-one,
9-(tert-Butylamino-methyl)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-piperidin-1-ylmethyl-6,7-dihydro-pyrimido[6,1-a]isoquinolin-4-one,
2-(2,3-Dlhydro-[1,4]dioxino[2,3-b]pyridin-2-ylmethoxy)-9-(3-methyl-oxetan-3-ylmethoxymethyl)-6,7-dihydro-pyrimido[6,1-a] isoquinolin-4-one,
2-(2,3-Dlhydro-[1,4]dioxino[2,3-b]pyridin-2-ylmethoxy)-9-(3-methyl-oxetan-3-ylmethoxymethyl)-6,7-dihydro-pyrimido[6,1-a] isoquinolin-4-one,
2-(2,3-Dlhydro-[1,4]dioxino[2,3-b]pyridin-2-ylmethoxy)-9-(oxetan-3-yloxymethyl)-6,7-dihydro-pyrimido[6,1-a]isoquinolin-4-one,
9-Cyclopropylethynyl-2-(4-isopropyl-oxetan-2-ylmethoxy)-6,7-dihydro-pyrimido[6,1-a]isoquinolin-4-one, and
9-Cyclopropylethynyl-2-((R)-4-isopropyl-oxetan-2-ylmethoxy)-6,7-dihydro-pyrimido[6,1-a]isoquinolin-4-one.

In yet another embodiment, the compound for use is selected from:
9-methoxy-1-methyl-2-[(tetrahydro-furan-2-ylmethyl)-amino]-10-(2,2,2-trifluoro-ethoxy)-6,7-dihydro-pyrido[2,1-a]isoquinolin-4-one,
9-cyclopropylethynyl-2-((S)-1-[1,4]dioxan-2-ylmethoxy)-10-methoxy-1-methyl-6,7-dihydro-pyrido[2,1-a]isoquinolin-4-one,
9-(2,2-difluoro-cyclopropylmethoxy)-2-((S)-1-[1,4]dioxan-2-ylmethoxy)-10-methoxy-1-methyl-6,7-dihydro-pyrido[2,1-a]isoquinolin-4-one,
10-(2,2-difluoro-ethoxy)-2-[([1,4]dioxan-2-ylmethyl)-amino]-9-methoxy-1-methyl-6,7-dihydro-pyrido[2,1-a]isoquinolin-4-one,
9-(2,2-difluoro-ethoxy)-10-methoxy-1-methyl-2-[(tetrahydro-furan-2-ylmethyl)-amino]-6,7-dihydro-pyrido[2,1-a]isoquinolin-4-one,
10-methoxy-1-methyl-2-[(tetrahydro-furan-2-ylmethyl)-amino]-9-(2,2,2-trifluoro-ethoxy)-6,7-dihydro-pyrido[2,1-a]isoquinolin-4-one,
9-(6-cyclopropyl-pyridin-3-yl)-2-((S)-1-[1,4]dioxan-2-ylmethoxy)-10-methoxy-1-methyl-6,7-dihydro-pyrido[2,1-a]isoquinolin-4-one,
2-(2,3-dihydro-thieno[3,4-b][1,4]dioxin-2-ylmethoxy)-9,10-dimethoxy-6,7-dihydro-pyrido[2,1-a]isoquinolin-4-one,
trifluoro-methanesulfonic acid 2-((S)-1-[1,4]dioxan-2-ylmethoxy)-10-methoxy-1-methyl-4-oxo-6,7-dihydro-4H-pyrido[2,1-a]isoquinolin-9-yl ester,
2-[([1,4]dioxan-2-ylmethyl)-amino]-9-methoxy-1-methyl-10-(2,2,2-trifluoro-ethoxy)-6,7-dihydro-pyrido[2,1-a]isoquinolin-4-one,
10-(2,2-difluoro-ethoxy)-9-methoxy-1-methyl-2-[(tetrahydro-furan-2-ylmethyl)-amino]-6,7-dihydro-pyrido[2,1-a]isoquinolin-4-one,
2-((S)-1-[1,4]dioxan-2-ylmethoxy)-10-methoxy-1-methyl-9-[6-(2,2,2-trifluoro-ethoxy)-pyridin-3-yl]-6,7-dihydro-pyrido [2, 1-a] isoquinolin-4-one,
2-((S)-1-[1,4]dioxan-2-ylmethoxy)-9-(6-fluoro-pyridin-3-yl)-10-methoxy-1-methyl-6,7-dihydro-pyrido[2,1-a]isoquinolin-4-one,
9,10-bis-(2,2-difluoro-cyclopropylmethoxy)-2-((S)-1-[1,4]dioxan-2-ylmethoxy)-1-methyl-6,7-dihydro-pyrido[2,1-a]isoquinolin-4-one,
9-(3,3-difluoro-cyclobutylmethoxy)-2-((S)-1-[1,4]dioxan-2-ylmethoxy)-10-methoxy-1-methyl-6,7-dihydro-pyrido [2, 1-a] isoquinolin-4-one,
2-((S)-1-[1,4]dioxan-2-ylmethoxy)-10-methoxy-1-methyl-9-(2,2,2-trifluoro-ethoxy)-6,7-dihydro-pyrido[2,1-a]isoquinolin-4-one,
10-difluoromethoxy-9-methoxy-1-methyl-2-[(tetrahydro-furan-2-ylmethyl)-amino]-6,7-dihydro-pyrido[2,1-a]isoquinolin-4-one,
10-(2,2-difluoro-ethoxy)-9-methoxy-1-methyl-2-(tetrahydro-pyran-2-ylmethyl)-amino]-6,7-dihydro-pyrido[2,1-a]isoquinolin-4-one,
2-((S)-1-[1,4]dioxan-2-ylmethoxy)-10-methoxy-1-methyl-9-(6-oxo-1,6-dihydro-pyridin-3-yl)-6,7-dihydro-pyrido [2, 1-a] isoquinolin-4-one,
2-((S)-1-[1,4]dioxan-2-ylmethoxy)-10-methoxy-1 -methyl-9-(3 -methyl-oxetan-3 -ylmethoxy)-6,7-dihydro-pyrido[2,1-a]isoquinolin-4-one,
9-methoxy-1-methyl-2-[(tetrahydro-pyran-2-ylmethyl)-amino]-10-(2,2,2-trifluoro-ethoxy)-6,7-dihydro-pyrido[2,1-a]isoquinolin-4-one,
9-(2,2-difluoro-ethoxy)-2-((S)-1-[1,4]dioxan-2-ylmethoxy)-10-methoxy-1-methyl-6,7-dihydro-pyrido[2,1-a]isoquinolin-4-one,
2-[([1,4]dioxan-2-ylmethyl)-amino]-9,10-dimethoxy-1-methyl-6,7-dihydro-pyrido[2,1-a]isoquinolin-4-one,
9-cyclopropylethynyl-2-((S)-1-[1,4]dioxan-2-ylmethoxy)-1-methyl-6,7-dihydro-pyrido[2,1-a] isoquinolin-4-one,
2-(2,3-dihydro-benzo[1,4]dioxin-2-ylmethoxy)-9-hydroxy-10-methoxy-6,7-dihydro-pyrido[2,1-a]isoquinolin-4-one,
9-difluoromethoxy-2-((S)-1-[1,4]dioxan-2-ylmethoxy)-10-methoxy-1 -methyl-6,7-dihydro-pyrido[2,1-a]isoquinolin-4-one,
9-cyclopropylmethoxy-2-((S)-1-[1,4]dioxan-2-ylmethoxy)-10-methoxy-1 -methyl-6,7-dihydro-pyrido[2,1-a]isoquinolin-4-one,
2-((S)-1-[1,4]dioxan-2-ylmethoxy)-9-(4-ethoxy-3-trifluoromethyl-phenyl)-10-methoxy-1-methyl-6,7-dihydro-pyrido[2,1-a]isoquinolin-4-one,
9,10-dimethoxy-1-methyl-2-[(tetrahydro-pyran-2-ylmethyl)-amino]-6,7-dihydro-pyrido[2,1-a]isoquinolin-4-one,
8,9-dimethoxy-2-[(tetrahydro-pyran-2-ylmethyl)-amino]-6,7-dihydro-pyrido[2,1-a]isoquinolin-4-one,
9,10-dimethoxy-1-methyl-2-[(tetrahydro-furan-2-ylmethyl)-amino]-6,7-dihydro-pyrido[2,1-a]isoquinolin-4-one,
10-difluoromethylsulfanyl-2-((S)-1-[1,4]dioxan-2-ylmethoxy)-9-methoxy-1-methyl-6,7-dihydro-pyrido[2,1-a]isoquinolin-4-one,
9-methoxy-1-methyl-2-[(tetrahydro-furan-2-ylmethyl)-amino]-6,7-dihydro-pyrido[2,1-a]isoquinolin-4-one,
2-((S)-1-[1,4]dioxan-2-ylmethoxy)-10-methoxy-1-methyl-9-(4-trifluoromethoxy-phenyl)-6,7-dihydro-pyrido[2,1-a]isoquinolin-4-one,
9-(2,2-difluoro-cyclopropylmethoxy)-1-methyl-2-[(tetrahydro-furan-2-ylmethyl)-amino]-6,7-dihydro-pyrido[2,1-a]isoquinolin-4-one,
8,9-dimethoxy-1-methyl-2-[(tetrahydro-furan-2-ylmethyl)-amino]-6,7-dihydro-pyrido[2,1-a]isoquinolin-4-one,
2-(2,3-dihydro-benzo[1,4]dioxin-2-ylmethoxy)-9,10-dimethoxy-1-methyl-6,7-dihydro-pyrido[2,1-a]isoquinolin-4-one,
2-((S)-1-[1,4]dioxan-2-ylmethoxy)-9-methoxy-1,10-dimethyl-6,7-dihydro-pyrido[2,1-a]isoquinolin-4-one,
8.11-dimethoxy-2-[(tetrahydro-pyran-2-ylmethyl)-amino]-6,7-dihydro-pyrido[2,1-a]isoquinolin-4-one,
2-[([1,4]dioxan-2-ylmethyl)-amino]-8,11-dimethoxy-6,7-dihydro-pyrido[2,1-a]isoquinolin-4-one,
8,11-dimethoxy-2-[(tetrahydro-furan-2-ylmethyl)-amino]-6,7-dihydro-pyrido[2,1-a]isoquinolin-4-one,
2-[(R)-1-(2,3-dihydro-[1,4]dioxino[2,3-b]pyridin-2-yl)methoxy]-9,10-dimethoxy-1-methyl-6,7-dihydro-pyrido[2,1-a]isoquinolin-4-one,
2-[([1,4]dioxan-2-ylmethyl)-amino]-8,9-dimethoxy-1-methyl-6,7-dihydro-pyrido[2,1-a]isoquinolin-4-one,
10- (2,2-difluoro-ethoxy)-9-methoxy-1 -methyl-2-(tetrahydro-pyran-2-ylmethoxy)-6,7-dihydro-pyrido [2,1 - a]isoquinolin-4-one,
8,9-dimethoxy-2-[(tetrahydro-furan-2-ylmethyl)-amino]-6,7-dihydro-pyrido[2,1-a]isoquinolin-4-one,
2-((S)-1-[1,4]dioxan-2-ylmethoxy)-8,9-dimethoxy-6,7-dihydro-pyrido[2,1-a]isoquinolin-4-one,
2-[(S)-1-(2,3-dihydro-[1,4]dioxino[2,3-b]pyridin-2-yl)methoxy]-9,10-dimethoxy-1-methyl-6,7-dihydro-pyrido[2,1-a]isoquinolin-4-one,
2-((S)-1-[1,4]dioxan-2-ylmethoxy)-1-methyl-9-(pyridin-2-ylmethoxy)-6,7-dihydro-pyrido[2,1-a]isoquinolin-4-one,
2-[([1,4]dioxan-2-ylmethyl)-amino]-8,9-dimethoxy-6,7-dihydro-pyrido[2,1-a]isoquinolin-4-one,
2-((S)-1-[1,4]dioxan-2-ylmethoxy)-10-methoxy-1 -methyl-4-oxo-6,7-dihydro-4H-pyrido[2,1-a]isoquinoline-9-carbonitrile,
9-methoxy-1-methyl-2-(tetrahydro-pyran-2-ylmethoxy)-10-(2,2,2-trifluoro-ethoxy)-6,7-dihydro-pyrido[2,1-a]isoquinolin-4-one,
9-(2-cyclopropyl-ethyl)-2-((S)-1-[1,4]dioxan-2-ylmethoxy)-1-methyl-6,7-dihydro-pyrido[2,1-a]isoquinolin-4-one,
9-(2-cyclopropyl-ethyl)-2-((S)-1-[1,4]dioxan-2-ylmethoxy)-1,7,7-trimethyl-6,7-dihydro-pyrido[2,1-a]isoquinolin-4-one,
2-((S)-1-[1,4]dioxan-2-ylmethoxy)-1,7,7-trimethyl-9-(pyridin-2-ylmethoxy)-6,7-dihydro-pyrido[2,1-a]isoquinolin-4-one,
9,11-dimethoxy-2-[(tetrahydro-furan-2-ylmethyl)-amino]-6,7-dihydro-pyrido[2,1-a]isoquinolin-4-one,
2-(2,3 -dihydro-benzo[1,4]dioxin-2-ylmethoxy)-9,10-dimethoxy-6,7-dihydro-pyrido[2,1-a] isoquinolin-4-one,
2-((S)-1-[1,4]dioxan-2-ylmethoxy)-9,10-dimethoxy-1-methyl-6,7-dihydro-pyrido[2,1-a]isoquinolin-4-one,
methanesulfonic acid 2-((S)-1-[1,4]dioxan-2-ylmethoxy)-10-methoxy-1-methyl-4-oxo-6,7-dihydro-4H-pyrido[2,1-a]isoquinolin-9-yl ester,
2-((S)-1-[1,4]dioxan-2-ylmethoxy)-1,7,7-trimethyl-9-pentyl-6,7-dihydro-pyrido[2,1-a]isoquinolin-4-one,
2-((S)-1-[1,4]dioxan-2-ylmethoxy)-1-methyl-9-(3-methyl-butyl)-6,7-dihydro-pyrido[2,1-a] isoquinolin-4-one,
2-((S)-1-[1,4]dioxan-2-ylmethoxy)-10-methoxy-1-methyl-6,7-dihydro-pyrido[2,1-a]isoquinolin-4-one, 2-([1,4]dioxan-2-ylmethoxy)-9,10-dimethoxy-6,7-dihydro-pyrido[2,1-a]isoquinolin-4-one,
8,11-dimethoxy-2-(tetrahydro-pyran-2-ylmethoxy)-6,7-dihydro-pyrido[2,1-a]isoquinolin-4-one,
2-((S)-1-[1,4]dioxan-2-ylmethoxy)-9-(5-ethyl-[1,2,4]oxadiazol-3-ylmethoxy)-1-methyl-6,7-dihydro-pyrido[2,1-a]isoquinolin-4-one,
2-[([1,4]dioxan-2-ylmethyl)-amino]-9,11-dimethoxy-6,7-dihydro-pyrido[2,1-a]isoquinolin-4-one,
8,9-dimethoxy-2-(tetrahydro-pyran-2-ylmethoxy)-6,7-dihydro-pyrido[2,1-a]isoquinolin-4-one,
2-(2,3-dihydro-benzo[1,4]dioxin-2-ylmethoxy)-9-methoxy-6,7-dihydro-pyrido[2,1-a]isoquinolin-4-one,
9-methoxy-1-methyl-10-(pyridin-2-ylmethoxy)-2-[(tetrahydro-furan-2-ylmethyl)-amino]-6,7-dihydro-pyrido[2,1-a]isoquinolin-4-one,
9-(3,5-dimethyl-isoxazol-4-yl)-2-((S)-1-[1,4]dioxan-2-ylmethoxy)-1-methyl-6,7-dihydro-pyrido[2,1-a]isoquinolin-4-one,
9-benzyloxy-2-((S)-1-[1,4]dioxan-2-ylmethoxy)-1-methyl-6,7-dihydro-pyrido [2,1-a] isoquinolin-4-one,
9,11-dimethoxy-2-[(tetrahydro-pyran-2-ylmethyl)-amino]-6,7-dihydro-pyrido[2,1-a]isoquinolin-4-one,
2-((S)-1-[1,4]dioxan-2-ylmethoxy)-9-hydroxy-10-methoxy-1 -methyl-6,7-dihydro-pyrido[2,1-a]isoquinolin-4-one,
8,9-dimethoxy-1-methyl-2-[(tetrahydro-pyran-2-ylmethyl)-amino]-6,7-dihydro-pyrido[2,1-a]isoquinolin-4-one,
9-benzyloxy-1-cyclopropyl-2-[(tetrahydro-furan-2-ylmethyl)-amino]-6,7-dihydro-pyrido[2,1-a]isoquinolin-4-one,
2-((S)-1-[1,4]dioxan-2-ylmethoxy)-8,11-dimethoxy-6,7-dihydro-pyrido[2,1-a]isoquinolin-4-one,
9-(3,6-dihydro-2H-pyran-4-yl)-2-((S)-1-[1,4]dioxan-2-ylmethoxy)-1-methyl-6,7-dihydro-pyrido[2,1-a]isoquinolin-4-one,
2-((S)-1-[1,4]dioxan-2-ylmethoxy)-1,7,7-trimethyl-9-(oxazol-2-ylmethoxy)-6,7-dihydro-pyrido[2,1-a]isoquinolin-4-one,
9,11-dimethoxy-2-(tetrahydro-pyran-2-ylmethoxy)-6,7-dihydro-pyrido[2,1-a]isoquinolin-4-one,
2-((S)-1-[1,4]dioxan-2-ylmethoxy)-1-methyl-9-(tetrahydro-pyran-4-yl)-6,7-dihydro-pyrido[2,1-a]isoquinolin-4-one,
9-benzyloxy-2-([1,4]dioxan-2-ylmethoxy)-1,7,7-trimethyl-6,7-dihydro-pyrido[2,1-a]isoquinolin-4-one, [2-((S)-1-[1,4]dioxan-2-ylmethoxy)-1 -methyl-4-oxo-6,7-dihydro-4H-pyrido [2,1-a] isoquinolin-9-yloxy] - acetonitrile,
9-cyclopropylethynyl-2-((S)-1-[1,4]dioxan-2-ylmethoxy)-1,7,7-trimethyl-6,7-dihydro-pyrido[2,1-a]isoquinolin-4-one,
9,10-dimethoxy-1-methyl-2-(tetrahydro-furan-2-ylmethoxy)-6,7-dihydro-pyrido[2,1-a]isoquinolin-4-one,
2-((S)-1-[1,4]dioxan-2-ylmethoxy)-1-methyl-9-(3-methyl-but-1-ynyl)-6,7-dihydro-pyrido[2,1-a]isoquinolin-4-one,
9-(5,6-dihydro-[1,4]dioxin-2-yl)-2-((S)-1-[1,4]dioxan-2-ylmethoxy)-1-methyl-6,7-dihydro-pyrido[2,1-a]isoquinolin-4-one,
2-((S)-1-[1,4]dioxan-2-ylmethoxy)-9-ethoxy-1-methyl-6,7-dihydro-pyrido [2,1-a] isoquinolin-4-one,
2-((S)-1-[1,4]dioxan-2-ylmethoxy)-1-methyl-9-(1-methyl-1H-pyrazol-4-yl)-6,7-dihydro-pyrido[2,1-a]isoquinolin-4-one,
[2-((S)-1-[1,4]dioxan-2-ylmethoxy)-1 -ethyl-4-oxo-6,7-dihydro-4H-pyrido [2,1-a] isoquinolin-9-yloxy] - acetonitrile,
2-((S)-1-[1,4]dioxan-2-ylmethoxy)-1-methyl-9-(1-propyl-1H-tetrazol-5-ylmethoxy)-6,7-dihydro-pyrido[2,1-a]isoquinolin-4-one,
2-((S)-1-[1,4]dioxan-2-ylmethoxy)-8,9-dimethoxy-1-methyl-6,7-dihydro-pyrido[2,1-a]isoquinolin-4-one,
8,11-dimethoxy-2-(tetrahydro-furan-2-ylmethoxy)-6,7-dihydro-pyrido[2,1-a]isoquinolin-4-one,
9-[1,4]dioxan-2-yl-2-((S)-1-[1,4]dioxan-2-ylmethoxy)-1-methyl-6,7-dihydro-pyrido 2,1-a]isoquinolin-4-one,
2-((S)-1-[1,4]dioxan-2-ylmethoxy)-9-methoxy-1-methyl-6,7-dihydro-pyrido [2,1-a] isoquinolin-4-one,
2-((S)-1-[1,4]dioxan-2-ylmethoxy)-8-hydroxy-9-methoxy-1-methyl-6,7-dihydro-pyrido[2,1-a]isoquinolin-4-one,
9-(1-cyclopropyl-1H-tetrazol-5-ylmethoxy)-2-((S)-1-[1,4]dioxan-2-ylmethoxy)-1-methyl-6,7-dihydro-pyrido[2,1-a]isoquinolin-4-one,
2-((S)-1-[1,4]dioxan-2-ylmethoxy)-1-methyl-4-oxo-9-(2,2,2-trifluoro-ethoxy)-6,7-dihydro-4H-pyrido[2, 1-a] isoquinoline-10-carbonitrile,
2,9-bis-((S)-1-[1,4]dioxan-2-ylmethoxy)-1-methyl-6,7-dihydro-pyrido[2,1-a]isoquinolin-4-one,
2-((S)-1-[1,4]dioxan-2-ylmethoxy)-9,11-dimethoxy-6,7-dihydro-pyrido[2,1-a]isoquinolin-4-one,
8,9-dimethoxy-1-methyl-2-(tetrahydro-furan-2-ylmethoxy)-6,7-dihydro-pyrido[2,1-a]isoquinolin-4-one,
2-((S)-1-[1,4]dioxan-2-ylmethoxy)-1-methyl-9-(3-methyl-oxetan-3-ylmethoxy)-6,7-dihydro-pyrido[2,1-a]isoquinolin-4-one,
9-cyclopropylethynyl-2-((S)-1-[1,4]dioxan-2-ylmethoxy)-1-ethyl-6,7-dihydro-pyrido[2,1-a] isoquinolin-4-one,
9,10-dimethoxy-2-(tetrahydro-furan-2-ylmethoxy)-6,7-dihydro-pyrido[2,1-a]isoquinolin-4-one,
2-((S)-1-[1,4]dioxan-2-ylmethoxy)-1-ethyl-9-(pyridin-2-ylmethoxy)-6,7-dihydro-pyrido[2,1-a]isoquinolin-4-one,
2-((S)-1-[1,4]dioxan-2-ylmethoxy)-1-ethyl-9-(1H-tetrazol-5ylmethoxy)-6,7-dihydro-pyrido[2,1-a]isoquinolin-4-one,
2-cyclohexylmethoxy-9,10-dimethoxy-1-methyl-6,7-dihydro-pyrido[2,1-a]isoquinolin-4-one,
9,10-dimethoxy-1-methyl-2-[(tetrahydro-pyran-3-ylmethyl)-amino]-6,7-dihydro-pyrido[2,1-a]isoquinolin-4-one,
2-((S)-1-[1,4]dioxan-2-ylmethoxy)-1-ethyl-9-(1-methyl-1H-pyrazol-4-yl)-6,7-dihydro-pyrido[2,1-a]isoquinolin-4-one,
9,10-dimethoxy-1-methyl-2-(tetrahydro-pyran-4-ylmethoxy)-6,7-dihydro-pyrido[2,1-a]isoquinolin-4-one,
9-cyclopropylmethoxy-2-((S)-1-[1,4]dioxan-2-ylmethoxy)-1,7,7-trimethyl-6,7-dihydro-pyrido[2,1-a]isoquinolin-4-one,
8,9-dimethoxy-2-(tetrahydro-furan-2-ylmethoxy)-6,7-dihydro-pyrido[2,1-a]isoquinolin-4-one,
2-((S)-1-[1,4]dioxan-2-ylmethoxy)-1-ethyl-9-(3-methoxy-but-1-ynyl)-6,7-dihydro-pyrido[2,1-a]isoquinolin-4-one,
8,9-dimethoxy-1-methyl-2-(tetrahydro-pyran-2-ylmethoxy)-6,7-dihydro-pyrido[2,1-a]isoquinolin-4-one,
9-benzyloxy-1-cyclopropyl-2-((S)-1-[1,4]dioxan-2-ylmethoxy)-6,7-dihydro-pyrido[2,1-a]isoquinolin-4-one,
2-((S)-1-[1,4]dioxan-2-ylmethoxy)-1-ethyl-9-(3-methyl-but-1-ynyl)-6,7-dihydro-pyrido[2,1-a]isoquinolin-4-one,
trifluoro-methanesulfonic acid 2-((S)-1-[1,4]dioxan-2-ylmethoxy)-1-ethyl-4-oxo-6,7-dihydro-4H-pyrido[2,1-a]isoquinolin-9-yl ester,
9-(2-cyclopropyl-ethyl)-2-((S)-1-[1,4]dioxan-2-ylmethoxy)-1-ethyl-6,7-dihydro-pyrido [2,1-a] isoquinolin-4-one,
9-cyclopropylmethoxy-2-([1,4]dioxan-2-ylmethoxy)-1-methyl-6,7-dihydro-pyrido[2,1-a]isoquinolin-4-one,
2-((S)-1-[1,4]dioxan-2-ylmethoxy)-1-ethyl-9-(3-methyl-butyl)-6,7-dihydro-pyrido[2,1-a]isoquinolin-4-one,
9,11-dimethoxy-2-(tetrahydro-furan-2-ylmethoxy)-6,7-dihydro-pyrido[2,1-a]isoquinolin-4-one,
2-((S)-1-[1,4]dioxan-2-ylmethoxy)-9-hydroxy-1,7,7-trimethyl-6,7-dihydro-pyrido[2,1-a]isoquinolin-4-one,
2-((S)-1-[1,4]dioxan-2-ylmethoxy)-9-methoxy-1-methyl-4-oxo-6,7-dihydro-4H-pyrido[2,1-a] isoquinoline-10-carbonitrile,
2-((S)-1-[1,4]dioxan-2-ylmethoxy)-1-ethyl-9-pentyl-6,7-dihydro-pyrido [2,1-a] isoquinolin-4-one,
1-cyclopropyl-2-((S)-1-[1,4]dioxan-2-ylmethoxy)-9-pentyl-6,7-dihydro-pyrido[2,1-a]isoquinolin-4-one,
9-benzyloxy-2-((S)-1-[1,4]dioxan-2-ylmethoxy)-1-isopropyl-6,7-dihydro-pyrido[2,1-a] isoquinolin-4-one,
2-((S)-1-[1,4]dioxan-2-ylmethoxy)-1-ethyl-9-(3-methoxy-butyl)-6,7-dihydro-pyrido[2,1-a]isoquinolin-4-one,
1-cyclopropyl-2-((S)-1-[1,4]dioxan-2-ylmethoxy)-9-(3-methyl-butyl)-6,7-dihydro-pyrido[2,1-a]isoquinolin-4-one,
1-cyclopropyl-9-(2-cyclopropyl-ethyl)-2-((S)-1-[1,4]dioxan-2-ylmethoxy)-6,7-dihydro-pyrido[2,1-a]isoquinolin-4-one,
trifluoro-methanesulfonic acid 1-cyclopropyl-2-((S)-1-[1,4]dioxan-2-ylmethoxy)-4-oxo-6,7-dihydro-4H-pyrido[2,1-a]isoquinolin-9-yl ester,
trifluoro-methanesulfonic acid 2-((S)-1-[1,4]dioxan-2-ylmethoxy)-1-isopropyl-4-oxo-6,7-dihydro-4H-pyrido[2,1-a]isoquinolin-9-yl ester,
1-cyclopropyl-9-cyclopropylethynyl-2-((S)-1-[1,4]dioxan-2-ylmethoxy)-6,7-dihydro-pyrido[2,1-a]isoquinolin-4-one,
2-((S)-1-[1,4]dioxan-2-ylmethoxy)-1-isopropyl-9-(3-methyl-butyl)-6,7-dihydro-pyrido[2,1-a]isoquinolin-4-one,
2-((S)-1-[1,4]dioxan-2-ylmethoxy)-9-hydroxy-8-methoxy-1-methyl-6,7-dihydro-pyrido[2,1-a]isoquinolin-4-one,
9-(2-cyclopropyl-ethyl)-2-((S)-1-[1,4]dioxan-2-ylmethoxy)-1-isopropyl-6,7-dihydro-pyrido[2,1-a]isoquinolin-4-one,
1-cyclopropyl-2-((S)-1-[1,4]dioxan-2-ylmethoxy)-9-(3-methyl-but-1-ynyl)-6,7-dihydro-pyrido[2,1-a]isoquinolin-4-one,
2-([1,4]dioxan-2-ylmethoxy)-9-(2-methoxy-ethoxy)-1-methyl-6,7-dihydro-pyrido[2,1-a]isoquinolin-4-one,
2-((S)-1-[1,4]dioxan-2-ylmethoxy)-1-isopropyl-9-(3-methyl-but-1-ynyl)-6,7-dihydro-pyrido[2,1-a]isoquinolin-4-one,
9-cyclopropylethynyl-2-((S)-1-[11,4]dioxan-2-ylmethoxy)-1-isopropyl-6,7-dihydro-pyrido[2,1-a]isoquinolin-4-one,
2-((S)-1-[1,4]dioxan-2-ylmethoxy)-9-hydroxy-1-methyl-6,7-dihydro-pyrido [2,1-a] isoquinolin-4-one, trifluoro-methanesulfonic acid 2-((S)-1-[1,4]dioxan-2-ylmethoxy)-1-methyl-4-oxo-6,7-dihydro-4H-pyrido[2,1-a]isoquinolin-9-yl ester,
2-((S)-1-[1,4]dioxan-2-ylmethoxy)-1-ethyl-9-hydroxy-6,7-dihydro-pyrido [2,1-a] isoquinolin-4-one,
9-methoxy-2-[(tetrahydro-furan-2-ylmethyl)-amino]-6,7-dihydro-pyrido[2,1-a]isoquinolin-4-one,
10-hydroxy-9-methoxy-1-methyl-2-[(tetrahydro-pyran-2-ylmethyl)-amino]-6,7-dihydro-pyrido[2,1-a]isoquinolin-4-one,
2-[([1,4]dioxan-2-ylmethyl)-amino]-10-hydroxy-9-methoxy-1-methyl-6,7-dihydro-pyrido[2,1-a]isoquinolin-4-one,
10-hydroxy-9-methoxy-1-methyl-2-(tetrahydro-pyran-2-ylmethoxy)-6, 7-dihydro-pyrido [2,1-a]isoquinolin-4-one, and
10-benzyloxy-2-((S)-1-[1,4]dioxan-2-ylmethoxy)-9-methoxy-1-methyl-6,7-dihydro-pyrido[2,1-a]isoquinolin-4-one.

In a further embodiment, the compound for use is selected from
4-[[(2S)-1,4-dioxan-2-yl]methoxy]-9, 10-dimethoxy-1-methyl-6,7-dihydrobenzo[a]quinolizin-2-one,
9,10-dimethoxy-1-methyl-4-(tetrahydrofuran-2-ylmethylamino)-6,7-dihydrobenzo[a]quinolizin-2-one,
1-ethyl-9-hydroxy-4-(tetrahydrofuran-2-ylmethylamino)-6,7-dihydrobenzo[a]quinolizin-2-one,
9,10-Dimethoxy-4-[(tetrahydro-furan-2-ylmethyl)-amino]-6,7-dihydro-pyrido[2,1-a]isoquinolin-2-one,
4-[([1,4]Dioxan-2-ylmethyl)-amino]-9,10-dimethoxy-6,7-dihydro-pyrido[2,1-a]isoquinolin-2-one,
4-[[(2R)-1,4-dioxan-2-yl]methylamino]-9,10-dimethoxy-1-methyl-6,7-dihydrobenzo[a]quinolizin-2-one,
9-(2,2-Difluoro-cyclopropylmethoxy)-1-methyl-4-[(tetrahydro-furan-2-ylmethyl)-amino]-6,7-dihydro-pyrido[2,1-a]isoquinolin-2-one,
1-methyl-4-(tetrahydrofuran-2-ylmethylamino)-9-(2,2,2-trifluoroethoxy)-6,7-dihydrobenzo[a]quinolizin-2-one,
-Methyl-4-[(tetrahydro-furan-2-ylmethyl)-amino]-9-(tetrahydro-furan-2-yloxy)-6,7-dihydro-pyrido[2,1-a]isoquinolin-2-one,
8,9-dimethoxy-1-methyl-4-(tetrahydrofuran-2-ylmethylamino)-6,7-dihydrobenzo[a]quinolizin-2-one,
4-[[(2S)-1,4-dioxan-2-yl]methoxy]-8,9-dimethoxy-1-methyl-6,7-dihydrobenzo[a]quinolizin-2-one,
9-(2,2-difluoroethoxy)-1-methyl-4-(tetrahydropyran-2-ylmethylamino)-6,7-dihydrobenzo[a]quinolizin-2-one,
1-methyl-4-(tetrahydropyran-2-ylmethylamino)-9-(2,2,2-trifluoroethoxy)-6,7-dihydrobenzo[a]quinolizin-2-one,
8,9-dimethoxy-1-methyl-4-(tetrahydropyran-2-ylmethylamino)-6,7-dihydrobenzo[a]quinolizin-2-one,
4-(1,4-dioxan-2-ylmethylamino)-8,9-dimethoxy-1-methyl-6,7-dihydrobenzo[a]quinolizin-2-one,
4-[[(2S)-1,4-dioxan-2-yl]methoxy]-1-methyl-9-(2,2,2-trifluoroethoxy)-6,7-dihydrobenzo[a]quinolizin-2-one,
4-(1,4-dioxan-2-ylmethylamino)-8,9-dimethoxy-6,7-dihydrobenzo[a]quinolizin-2-one,
8,9-dimethoxy-4-(tetrahydrofuran-2-ylmethylamino)-6,7-dihydrobenzo[a]quinolizin-2-one,
4-(1,4-dioxan-2-ylmethylamino)-9-hydroxy-8-methoxy-6,7-dihydrobenzo[a]quinolizin-2-one,
4-(1,4-dioxan-2-ylmethylamino)-8-hydroxy-9-methoxy-6,7-dihydrobenzo[a]quinolizin-2-one,
9-(2,2-Difluoro-ethoxy)-4-[([1,4]dioxan-2-ylmethyl)-amino]-8-methoxy-1-methyl-6,7-dihydro-pyrido[2,1-a] isoquinolin-2-one,
9-(2,2-Difluoro-ethoxy)-8-methoxy-1-methyl-4-[(tetrahydro-furan-2-ylmethyl)-amino]-6,7-dihydro-pyrido[2,1-a]isoquinolin-2-one,
4-[[(2S)-1,4-dioxan-2-yl]methoxy]-1-methyl-2-oxo-6,7-dihydrobenzo[a]quinolizine-9-carbonitrile,
9-(2,2-difluoroethoxy)-1-ethyl-4-(tetrahydrofuran-2-ylmethylamino)-6,7-dihydrobenzo[a]quinolizin-2-one,
1-ethyl-4-(tetrahydrofuran-2-ylmethylamino)-9-(2,2,2-trifluoroethoxy)-6,7-dihydrobenzo[a]quinolizin-2-one,
8,9-dimethoxy-1-methyl-4-(tetrahydrofuran-2-ylmethoxy)-6,7-dihydrobenzo[a]quinolizin-2-one,
8,9-dimethoxy-1-methyl-4-(tetrahydropyran-2-ylmethoxy)-6,7-dihydrobenzo[a]quinolizin-2-one,
4-[[(2S)-1,4-dioxan-2-yl]methoxy]-1-methyl-9-(tetrahydropyran-4-ylmethoxy)-6,7-dihydrobenzo[a]quinolizin-2-one,
4-[[(2S)-1,4-dioxan-2-yl]methoxy]-1-methyl-9-(2-pyridylmethoxy)-6,7-dihydrobenzo[a]quinolizin-2-one,
4-[[(2S)-1,4-dioxan-2-yl]methoxy]-1-methyl-9-[[3-(trifluoromethoxy)phenyl]methoxy]-6,7-dihydrobenzo[a]quinolizin-2-one,
9-benzyloxy-4-[[(2S)-1,4-dioxan-2-yl]methoxy]-1-methyl-6,7-dihydrobenzo[a]quinolizin-2-one,
9-benzyloxy-4-[[(2R)-1,4-dioxan-2-yl]methylamino]-1-methyl-6,7-dihydrobenzo[a]quinolizin-2-one,
9-(2,2-difluoroethoxy)-4-[[(2R)-1,4-dioxan-2-yl]methylamino]-1-methyl-6,7-dihydrobenzo[a]quinolizin-2-one,
4-[[(2R)-1,4-dioxan-2-yl]methylamino]-1-methyl-9-(2,2,2-trifluoroethoxy)-6,7-dihydrobenzo[a]quinolizin-2-one,
[4-[[(2S)-1,4-dioxan-2-yl]methoxy]-1-methyl-2-oxo-6,7-dihydrobenzo[a]quinolizin-9-yl] trifluoromethanesulfonate,
4-[[(2S)-1,4-dioxan-2-yl]methoxy]-1-methyl-9-[(1-methylpyrazol-4-yl)methoxy]-6,7-dihydrobenzo[a]quinolizin-2-one,
9-(3,6-dihydro-2H-pyran-4-yl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-9-(1-ethylpyrazol-4-yl)-1-methyl-6,7-dihydrobenzo[a]quinolizin-2-one,
4-[[(2S)-1,4-dioxan-2-yl]methoxy]-1-methyl-9-tetrahydropyran-4-yl-6,7-dihydrobenzo[a]quinolizin-2-one,
1-methyl-4-[[(2S)-tetrahydrofuran-2-yl]methylamino]-9-(2,2,2-trifluoroethoxy)-6,7-dihydrobenzo[a]quinolizin-2-one,
1-methyl-9-[(3-methyl-1,2,4-oxadiazol-5-yl)methoxy]-4-(tetrahydrofuran-2-ylmethoxy)-6,7-dihydrobenzo[a]quinolizin-2-one,
1-methyl-9-[(3-methyl-1,2,4-oxadiazol-5-yl)methoxy]-4-(tetrahydropyran-2-ylmethoxy)-6,7-dihydrobenzo[a]quinolizin-2-one,
1-methyl-9-(2-pyridylmethoxy)-4-(tetrahydrofuran-2-ylmethoxy)-6,7-dihydrobenzo[a]quinolizin-2-one,
1-methyl-9-(2-pyridylmethoxy)-4-(tetrahydropyran-2-ylmethoxy)-6,7-dihydrobenzo[a]quinolizin-2-one,
1-methyl-4-(tetrahydrofuran-2-ylmethoxy)-9-(tetrahydropyran-3 -ylmethoxy)-6, 7-dihydrobenzo[a]quinolizin-2-one,
1-methyl-4-(tetrahydropyran-2-ylmethoxy)-9-(tetrahydropyran-3-ylmethoxy)-6,7-dihydrobenzo[a]quinolizin-2-one,
4-[[(2S)-1,4-dioxan-2-yl]methoxy]-1-methyl-9-(tetrahydropyran-3-ylmethoxy)-6,7-dihydrobenzo[a]quinolizin-2-one,
1-methyl-9-[(6-methyl-3-pyridyl)methoxy]-4-(tetrahydrofuran-2-ylmethoxy)-6,7-dihydrobenzo[a]quinolizin-2-one,
1-methyl-9-[(6-methyl-3-pyridyl)methoxy]-4-(tetrahydropyran-2-ylmethoxy)-6,7-dihydrobenzo[a]quinolizin-2-one,
4-[[(2S)-1,4-dioxan-2-yl]methoxy]-1-methyl-9-[(6-methyl-3-pyridyl)methoxy]-6,7-dihydrobenzo[a]quinolizin-2-one,
9-(2-dimethylaminoethyloxy)-1-methyl-4-(tetrahydrofuran-2-ylmethoxy)-6,7-dihydrobenzo[a]quinolizin-2-one,
9-(2-dimethylaminoethyloxy)-1-methyl-4-(tetrahydropyran-2-ylmethoxy)-6,7-dihydrobenzo[a]quinolizin-2-one,
9-(2-dimethylaminoethyloxy)-4-[[(2S)-1,4-dioxan-2-yl]methoxy]-1-methyl-6,7-dihydrobenzo[a]quinolizin-2-one,
[4-[[(2S)-1,4-dioxan-2-yl]methoxy]-1-methyl-2-oxo-6,7-dihydrobenzo[a]quinolizin-9-yl] methanesulfonate,
1- methyl-9-(2-pyridylmethoxy)-4-[[(2S)-tetrahydrofuran-2-yl]methoxy]-6,7-dihydrobenzo[a]quinolizin-2-one,
4-[[(2S)-1,4-dioxan-2-yl]methoxy]-1-methyl-9-[(3-methyl-1,2,4-oxadiazol-5-yl)methoxy]-6,7-dihydrobenzo[a]quinolizin-2-one,
9-(difluoromethoxy)-4-[[(2S)-1,4-dioxan-2-yl]methoxy]-1-methyl-6,7-dihydrobenzo[a]quinolizin-2-one, tert-butyl 4-[4-[[(2S)-1,4-dioxan-2-yl]methoxy]-1-methyl-2-oxo-6,7-dihydrobenzo[a]quinolizin-9-yl]piperazine-1 -carboxylate,
9-(2,2-difluoroethoxy)-4-[[(2S)-1,4-dioxan-2-yl]methoxy]-1-methyl-6,7-dihydrobenzo[a]quinolizin-2-one,
4,9-bis[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-morpholino-6,7-dihydrobenzo[a]quinolizin-2-one,
4-[[(2S)-1,4-dioxan-2-yl]methoxy]-1-methyl-9-phenylsulfanyl-6,7-dihydrobenzo[a]quinolizin-2-one,
9-(4,4-difluoro-1-piperidyl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-piperazin-1-yl-6,7-dihydrobenzo[a]quinolizin-2-one,
9-(benzenesulfonyl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-(4-methylsulfonylpiperazin-1-yl)-6,7-dihydrobenzo[a]quinolizin-2-one,
9-[4-(cyclopropanecarbonyl)piperazin-1-yl]-4-[[(2S)-1,4-dioxan-2-yl]methoxy]-1-methyl-6,7-dihydrobenzo[a]quinolizin-2-one,
N-[4-[[(2S)-1,4-dioxan-2-yl]methoxy]-1-methyl-2-oxo-6,7-dihydrobenzo[a]quinolizin-9-yl]cyclopropanecarboxamide,
tert-butyl 3-[4-[[(2S)-1,4-dioxan-2-yl]methoxy]-1-methyl-2-oxo-6,7-dihydrobenzo[a]quinolizin-9-yl]azetidine-1 -carboxylate,
4-[[(2S)-1,4-dioxan-2-yl]methoxy]-1-methyl-9-[2-(trifluoromethoxy)ethoxy]-6,7-dihydrobenzo[a]quinolizin-2-one,
N-[4-[[(2S)-1,4-dioxan-2-yl]methoxy]-1-methyl-2-oxo-6,7-dihydrobenzo[a]quinolizin-9-yl]-N-methyl-cyclopropanecarboxamide,
tert-butyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1 -carboxylate,
tert-butyl 4-[4-[[(2S)-1,4-dioxan-2-yl]methoxy]-1-methyl-2-oxo-6,7-dihydrobenzo[a]quinolizin-9-yl]piperidine-1 -carboxylate,
methyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1 -carboxylate,
ethyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1 -carboxylate,
isopropyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1 -carboxylate,
2,2,2-trifluoroethyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1-carboxylate,
methyl 4-[4-[[(2S)-1,4-dioxan-2-yl]methoxy]-1-methyl-2-oxo-6,7-dihydrobenzo[a]quinolizin-9-yl]piperidine-1 -carboxylate,
ethyl 4-[4-[[(2S)-1,4-dioxan-2-yl]methoxy]-1-methyl-2-oxo-6,7-dihydrobenzo[a]quinolizin-9-yl]piperidine-1 -carboxylate,
isopropyl 4-[4-[[(2S)-1,4-dioxan-2-yl]methoxy]-1-methyl-2-oxo-6,7-dihydrobenzo[a]quinolizin-9-yl]piperidine-1 -carboxylate,
2,2,2-trifluoroethyl 4-[4-[[(2S)-1,4-dioxan-2-yl]methoxy]-1-methyl-2-oxo-6,7-dihydrobenzo[a]quinolizin-9-yl]piperidine-1 -carboxylate,
N-cyclopropyl-4-[[(2S)-1,4-dioxan-2-yl]methoxy]-1-methyl-2-oxo-6,7-dihydrobenzo[a]quinolizine-9-carboxamide,
4-[[(2S)-1,4-dioxan-2-yl]methoxy]-9-hydroxy-1-methyl-6,7-dihydrobenzo[a]quinolizin-2-one,
9-(3,3-difluoroazetidin-1-yl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-(6-oxa-2-azaspiro[3.3]heptan-2-yl)-6,7-dihydrobenzo[a]quinolizin-2-one,
N-cyclopropyl-4-[[(2S)-1,4-dioxan-2-yl]methoxy]-N,1-dimethyl-2-oxo-6,7-dihydrobenzo[a]quinolizine-9-carboxamide,
4-[[(2S)-1,4-dioxan-2-yl]methoxy]-9-methoxy-1-methyl-6,7-dihydrobenzo[a]quinolizin-2-one,
4-(1,4-dioxan-2-ylmethoxy)-1-methyl-9-(2,2,2-trifluoroethoxy)-6,7-dihydrobenzo[a]quinolizin-2-one,
4-[[(2S)-1,4-dioxan-2-yl]methoxy]-9-(3-fluoroazetidin-1-yl)-1-methyl-6,7-dihydrobenzo[a]quinolizin-2-one,
4-[[(2S)-1,4-dioxan-2-yl]methoxy]-9-[3-(1-hydroxy-1-methyl-ethyl)azetidin-1-yl]-1-methyl-6,7-dihydrobenzo[a]quinolizin-2-one,
9-(azetidin-1-yl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-(3-methylsulfonylazetidin-1-yl)-6,7-dihydrobenzo[a]quinolizin-2-one,
4-[[(2S)-1,4-dioxan-2-yl]methoxy]-1-methyl-9-(3-pyrazol-1-ylazetidin-1-yl)-6,7-dihydrobenzo[a]quinolizin-2-one,
9-(3,3-dimethylazetidin-1-yl)-4-[[(2S)-1,4-dioxan-2-yl]methoxy]-1-methyl-6,7-dihydrobenzo[a]quinolizin-2-one,
methyl 1-[4-[[(2S)-1,4-dioxan-2-yl]methoxy]-1-methyl-2-oxo-6,7-dihydrobenzo[a]quinolizin-9-yl]azetidine-3-carboxylate,
4-[[(2S)-1,4-dioxan-2-yl]methoxy]-1-methyl-9-(3-pyridyl)-6,7-dihydrobenzo[a]quinolizin-2-one,
4-[[(2S)-1,4-dioxan-2-yl]methoxy]-9-(3-fluorophenyl)-1-methyl-6,7-dihydrobenzo[a]quinolizin-2-one,
4-[[(2S)-1,4-dioxan-2-yl]methoxy]-1-methyl-9-(4-pyridyl)-6,7-dihydrobenzo[a]quinolizin-2-one,
4-[[(2S)-1,4-dioxan-2-yl]methoxy]-1-methyl-9-(2-pyridyl)-6,7-dihydrobenzo[a]quinolizin-2-one,
4-[[(2S)-1,4-dioxan-2-yl]methoxy]-1-methyl-9-(3-methyl-2-pyridyl)-6,7-dihydrobenzo[a]quinolizin-2-one,
4-[[(2S)-1,4-dioxan-2-yl]methoxy]-1-methyl-9-(4-methyl-2-pyridyl)-6,7-dihydrobenzo[a]quinolizin-2-one,
tert-butyl 3-[[4-[[(2S)-1,4-dioxan-2-yl]methoxy]-1-methyl-2-oxo-6,7-dihydrobenzo[a]quinolizin-9-yl]oxy]azetidine-1-carboxylate,
3-deuterio-9-(1-deuterio-2,2-difluoro-vinyloxy)-4-[[(2S)-1,4-dioxan-2-yl]methoxy]-1-methyl-6,7-dihydrobenzo[a]quinolizin-2-one,
9-(1,1-dideuterio-2,2,2-trifluoro-ethoxy)-4-[[(2S)-1,4-dioxan-2-yl]methoxy]-1-methyl-6,7-dihydrobenzo[a]quinolizin-2-one,
9-benzyloxy-1-methyl-4-(oxetan-2-ylmethoxy)-6,7-dihydrobenzo[a]quinolizin-2-one,
9-benzyloxy-1-methyl-4-[[(2S)-tetrahydrofuran-2-yl]methoxy]-6,7-dihydrobenzo[a]quinolizin-2-one,
9-benzyloxy-1-methyl-4-(tetrahydropyran-2-ylmethoxy)-6,7-dihydrobenzo[a]quinolizin-2-one,
4-[[(2S)-1,4-dioxan-2-yl]methoxy]-9-(3-methoxyazetidin-1-yl)-1-methyl-6,7-dihydrobenzo[a]quinolizin-2-one,
4-[[(2S)-1,4-dioxan-2-yl]methoxy]-9-(4-methoxy-1-piperidyl)-1-methyl-6,7-dihydrobenzo[a]quinolizin-2-one,
4-[[(2S)-1,4-dioxan-2-yl]methoxy]-1-methyl-9-[4-(piperidine-1-carbonyl)-1-piperidyl]-6,7-dihydrobenzo[a]quinolizin-2-one,
4-[[(2S)-1,4-dioxan-2-yl]methoxy]-1-methyl-9-(4-phenyl-1-piperidyl)-6,7-dihydrobenzo[a]quinolizin-2-one,
methyl 1-[4-[[(2S)-1,4-dioxan-2-yl]methoxy]-1-methyl-2-oxo-6,7-dihydrobenzo[a]quinolizin-9-yl]piperidine-4-carboxylate,
4-[[(2S)-1,4-dioxan-2-yl]methoxy]-9-[4-(ethoxymethyl)-1-piperidyl]-1-methyl-6,7-dihydrobenzo[a]quinolizin-2-one,
4-[[(2S)-1,4-dioxan-2-yl]methoxy]-1-methyl-9-(1-piperidyl)-6,7-dihydrobenzo[a]quinolizin-2-one,
4-[[(2S)-1,4-dioxan-2-yl]methoxy]-1-methyl-9-(3-methyl-1-piperidyl)-6,7-dihydrobenzo[a]quinolizin-2-one,
4-[[(2S)-1,4-dioxan-2-yl]methoxy]-9-[4-(4-fluorophenyl)-1-piperidyl]-1-methyl-6,7-dihydrobenzo[a]quinolizin-2-one,
9-[1-(cyclopropanecarbonyl)azetidin-3-yl]oxy-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-[1-(2,2,2-trifluoroacetyl)azetidin-3-yl]oxy-6,7-dihydrobenzo[a]quinolizin-2-one,
ethyl 3-[[4-[[(2S)-1,4-dioxan-2-yl]methoxy]-1-methyl-2-oxo-6,7-dihydrobenzo[a]quinolizin-9-yl]oxy]azetidine-1 -carboxylate,
4-[[(2S)-1,4-dioxan-2-yl]methoxy]-1-methyl-9-[4-(3-pyridyloxy)-1-piperidyl]-6,7-dihydrobenzo[a]quinolizin-2-one,
1-[4-[[(2S)-1,4-dioxan-2-yl]methoxy]-1-methyl-2-oxo-6,7-dihydrobenzo[a]quinolizin-9-yl]piperidine-4-carbonitrile,
9-(3,3-difluoro-1-piperidyl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-9-isopropyl-1-methyl-6,7-dihydrobenzo[a]quinolizin-2-one, 3-deuterio-4-[[(2S)-1,4-dioxan-2-yl]methoxy]-1-methyl-9-(2,2,2-trifluoroethoxy)-6,7-dihydrobenzo[a]quinolizin-2-one,
3-deuterio-4-[[(2S)-1,4-dioxan-2-yl]methoxy]-1-methyl-9-(1,1,2,2-tetradeuterio-2-fluoro-ethoxy)-6,7-dihydrobenzo[a]quinolizin-2-one,
tert-butyl 3-[[4-[[(2S)-1,4-dioxan-2-yl]methoxy]-1-methyl-2-oxo-6,7-dihydrobenzo[a]quinolizin-9-yl]oxy]pyrrolidine-1 -carboxylate,
tert-butyl 4-[[4-[[(2S)-1,4-dioxan-2-yl]methoxy]-1-methyl-2-oxo-6,7-dihydrobenzo[a]quinolizin-9-yl]oxy]piperidine-1 -carboxylate,
4-[[(2S)-1,4-dioxan-2-yl]methoxy]-1-methyl-9-[methyl(3,3,3-trifluoropropyl)amino]-6,7-dihydrobenzo[a]quinolizin-2-one,
4-[[(2S)-1,4-dioxan-2-yl]methoxy]-1-methyl-9-pyrrolidin-1-yl-6,7-dihydrobenzo[a]quinolizin-2-one,
9-(3,3-difluoropyrrolidin-1-yl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-[3-(trifluoromethyl)azetidin-1-yl]-6,7-dihydrobenzo[a]quinolizin-2-one,
4-[[(2S)-1,4-dioxan-2-yl]methoxy]-1-methyl-9-[4-(trifluoromethyl)-3,6-dihydro-2H-pyridin-1-yl]-6,7-dihydrobenzo[a]quinolizin-2-one,
4-[[(2S)-1,4-dioxan-2-yl]methoxy]-1-methyl-9-[(2R)-2-methylpyrrolidin-1-yl]-6,7-dihydrobenzo[a]quinolizin-2-one,
4-[[(2S)-1,4-dioxan-2-yl]methoxy]-9-(3-fluoro-1-piperidyl)-1-methyl-6,7-dihydrobenzo[a]quinolizin-2-one,
9-carbazol-9-yl-4-[[(2S)-1,4-dioxan-2-yl]methoxy]-1-methyl-6,7-dihydrobenzo[a]quinolizin-2-one,
9-(3,5-dimethyl-1-piperidyl)-4-[[(2S)-1,4-dioxan-2-yl]methoxy]-1-methyl-6,7-dihydrobenzo[a]quinolizin-2-one,
9-(3,3-dimethylpyrrolidin-1-yl)-4-[[(2S)-1,4-dioxan-2-yl]methoxy]-1-methyl-6,7-dihydrobenzo[a]quinolizin-2-one,
9-(4,4-dimethyl-1-piperidyl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-[4-(trifluoromethyl)-1-piperidyl]-6,7-dihydrobenzo[a]quinolizin-2-one,
4-[[(2S)-1,4-dioxan-2-yl]methoxy]-1-methyl-9-(4-methyl-1-piperidyl)-6,7-dihydrobenzo[a]quinolizin-2-one,
4-[[(2S)-1,4-dioxan-2-yl]methoxy]-1-methyl-9-(2,2,2-trifluoroethylamino)-6,7-dihydrobenzo[a]quinolizin-2-one,
4-[[(2S)-1,4-dioxan-2-yl]methoxy]-1-methyl-9-(2-methyl-1-piperidyl)-6,7-dihydrobenzo[a]quinolizin-2-one,
9-[1-(cyclopropanecarbonyl)pyrrolidin-3-yl]oxy-4-[[(2S)-1,4-dioxan-2-yl]methoxy]-1-methyl-6,7-dihydrobenzo[a]quinolizin-2-one,
ethyl 3-[[4-[[(2S)-1,4-dioxan-2-yl]methoxy]-1-methyl-2-oxo-6,7-dihydrobenzo[a]quinolizin-9-yl]oxy]pyrrolidine-1 -carboxylate,
9-[1-(cyclopropanecarbonyl)azetidin-3-yl]-4-[[(2S)-1,4-dioxan-2-yl]methoxy]-1-methyl-6,7-dihydrobenzo[a]quinolizin-2-one,
ethyl 3-[4-[[(2S)-1,4-dioxan-2-yl]methoxy]-1-methyl-2-oxo-6,7-dihydrobenzo[a]quinolizin-9-yl]azetidine-1 -carboxylate,
3-[4-[[(2S)-1,4-dioxan-2-yl]methoxy]-1-methyl-2-oxo-6,7-dihydrobenzo[a]quinolizin-9-yl]-N,N-dimethyl-azetidine-1 -carboxamide,
3-[4-[[(2S)-1,4-dioxan-2-yl]methoxy]-1-methyl-2-oxo-6,7-dihydrobenzo[a]quinolizin-9-yl]-N-isopropyl-azetidine-1-carboxamide,
3-[[4-[[(2S)-1,4-dioxan-2-yl]methoxy]-1-methyl-2-oxo-6,7-dihydrobenzo[a]quinolizin-9-yl]oxy]-N,N-dimethyl-azetidine-1-carboxamide,
3-[4-[[(2S)-1,4-dioxan-2-yl]methoxy]-1-methyl-2-oxo-6,7-dihydrobenzo[a]quinolizin-9-yl]-N-isopropyl-azetidine-1-carboxamide,
9-benzyloxy-4-[(4,4-dimethyloxetan-2-yl)methoxy]-1-methyl-6,7-dihydrobenzo[a]quinolizin-2-one,
9-benzyloxy-1-methyl-4-[(2-methyltetrahydrofuran-2-yl)methoxy]-6,7-dihydrobenzo[a]quinolizin-2-one,
9-benzyloxy-4-[(5,5-dimethyltetrahydrofuran-2-yl)methoxy]-1-methyl-6,7-dihydrobenzo[a]quinolizin-2-one,
4-[[(2S)-1,4-dioxan-2-yl]methoxy]-1-methyl-9-(2,2,3,3,3-pentafluoropropoxy)-6,7-dihydrobenzo[a]quinolizin-2-one,
4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-(2-oxopyrrolidin-1-yl)-6,7-dihydrobenzo[a]quinolizin-2-one, and
3-deuterio-4-[[(2S)-1,4-dioxan-2-yl]methoxy]-1-methyl-9-(1,1,2,2-tetradeuterio-2-fluoro-ethoxy)-6,7-dihydrobenzo[a]quinolizin-2-one.

In one embodiment, the compound for use is 9-Cyclopropylethynyl-2-((S)-1-[1,4]dioxan-2-ylmethoxy)-6,7-dihydro-pyrimido [6,1 -a] isoquinolin-4-one.

In another embodiment, the compound for use is 4-[[(2S)-1,4-dioxan-2-yl]methoxy]-1-methyl-9-(2,2,2-trifluoroethoxy)-6,7-dihydrobenzo[a]quinolizin-2-one.

In one embodiment a compound of the invention is not an isotopic variant.

In one aspect a compound of the invention according to any one of the embodiments herein described is present as the free base.

In one aspect a compound of the invention according to any one of the embodiments herein described is a pharmaceutically acceptable salt.

In one aspect a compound of the invention according to any one of the embodiments herein described is a solvate of the compound.

In one aspect a compound of the invention according to any one of the embodiments herein described is a solvate of a pharmaceutically acceptable salt of a compound.

While specified groups for each embodiment have generally been listed above separately, a compound of the invention includes one in which several or each embodiment in the above Formula, as well as other formulae presented herein, is selected from one or more of particular members or groups designated respectively, for each variable. Therefore, this invention is intended to include all combinations of such embodiments within its scope.

While specified groups for each embodiment have generally been listed above separately, a compound of the invention may be one for which one or more variables (for example, R groups) is selected from one or more embodiments according to any of the Formula(e) listed above. Therefore, the present invention is intended to include all combinations of variables from any of the disclosed embodiments within its scope.

Alternatively, the exclusion of one or more of the specified variables from a group or an embodiment, or combinations thereof is also contemplated by the present invention.

In certain aspects, the present invention provides prodrugs and derivatives of the compounds according to the formulae above. Prodrugs are derivatives of the compounds of the invention, which have metabolically cleavable groups and become by solvolysis or under physiological conditions the compounds of the invention, which are pharmaceutically active, *in vivo.* Such examples include, but are not limited to, choline ester derivatives and the like, N-alkylmorpholine esters and the like.

Other derivatives of the compounds of this invention have activity in both their acid and acid derivative forms, but the acid sensitive form often offers advantages of solubility, tissue compatibility, or delayed release in the mammalian organism (Bundgard, H, 1985). Prodrugs include acid derivatives well known to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are preferred prodrugs. In some cases it is desirable to prepare double ester type prodrugs such as (acyloxy)alkyl esters or ((alkoxycarbonyl)oxy)alkylesters. Particularly useful are the C₁ to C₈ alkyl, C₂-C₈ alkenyl, aryl, C₇-C₁₂ substituted aryl, and C₇-C₁₂ arylalkyl esters of the compounds of the invention.

### PHARMACEUTICAL COMPOSITIONS

When employed as a pharmaceutical, a compound of the invention is typically administered in the form of a pharmaceutical composition. Such compositions can be prepared in a manner well known in the pharmaceutical art and comprise at least one active compound of the invention according to Formula I. Generally, a compound of the invention is administered in a pharmaceutically effective amount. The amount of compound of the invention actually administered will typically be determined by a physician, in the light of the relevant circumstances, including the condition to be treated, the chosen route of administration, the actual compound of the invention administered, the age, weight, and response of the individual patient, the severity of the patient's symptoms, and the like.

The pharmaceutical compositions of this invention can be administered by a variety of routes including oral, rectal, transdermal, subcutaneous, intra-articular, intravenous, intramuscular, and intranasal. Depending on the intended route of delivery, a compound of the invention is preferably formulated as either injectable or oral compositions or as salves, as lotions or as patches all for transdermal administration.

The compositions for oral administration can take the form of bulk liquid solutions or suspensions, or bulk powders. More commonly, however, the compositions are presented in unit dosage forms to facilitate accurate dosing. The term `unit dosage forms' refers to physically discrete units suitable as unitary dosages for human subjects and other mammals, each unit containing a predetermined quantity of active material calculated to produce the desired therapeutic effect, in association with a suitable pharmaceutical excipient, vehicle or carrier. Typical unit dosage forms include prefilled, premeasured ampules or syringes of the liquid compositions or pills, tablets, capsules or the like in the case of solid compositions. In such compositions, the compound of the invention according to Formula I is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or non-aqueous vehicle with buffers, suspending and dispensing agents, colorants, flavors and the like. Solid forms may include, for example, any of the following ingredients, or compound of the inventions of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint or orange flavoring.

Injectable compositions are typically based upon injectable sterile saline or phosphate-buffered saline or other injectable carriers known in the art. As before, the active compound of the invention according to Formula I in such compositions is typically a minor component, often being from about 0.05 to 10% by weight with the remainder being the injectable carrier and the like.

Transdermal compositions are typically formulated as a topical ointment or cream containing the active ingredient(s), generally in an amount ranging from about 0.01 to about 20% by weight, preferably from about 0.1 to about 20% by weight, preferably from about 0.1 to about 10% by weight, and more preferably from about 0.5 to about 15% by weight. When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or the formulation. All such known transdermal formulations and ingredients are included within the scope of this invention.

A compound of the invention can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17^{th} edition, 1985, Mack Publishing Company, Easton, Pennsylvania, which is incorporated herein by reference.

A compound of the invention can also be administered in sustained release forms or from sustained release drug delivery systems. A description of representative sustained release materials can be found in Remington's Pharmaceutical Sciences.

The following formulation examples illustrate representative pharmaceutical compositions that may be prepared in accordance with this invention. The present invention, however, is not limited to the following pharmaceutical compositions.

### Formulation 1 - Tablets

A compound of the invention according to Formula I may be admixed as a dry powder with a dry gelatin binder in an approximate 1:2 weight ratio. A minor amount of magnesium stearate may be added as a lubricant. The mixture may be formed into 240-270 mg tablets (80-90 mg of active compound of the invention according to Formula I per tablet) in a tablet press.

### Formulation 2 - Capsules

A compound of the invention according to Formula I may be admixed as a dry powder with a starch diluent in an approximate 1: 1 weight ratio. The mixture may be filled into 250 mg capsules (125 mg of active compound of the invention according to Formula I per capsule).

### Formulation 3 - Liquid

A compound of the invention according to Formula I (125 mg), may be admixed with sucrose (1.75 g) and xanthan gum (4 mg) and the resultant mixture may be blended, passed through a No. 10 mesh U.S. sieve, and then mixed with a previously made solution of microcrystalline cellulose and sodium carboxymethyl cellulose (11:89, 50 mg) in water. Sodium benzoate (10 mg), flavor, and color may be diluted with water and added with stirring. Sufficient water may then be added with stirring. Further sufficient water may be then added to produce a total volume of 5 mL.

### Formulation 4 - Tablets

A compound of the invention according to Formula I may be admixed as a dry powder with a dry gelatin binder in an approximate 1:2 weight ratio. A minor amount of magnesium stearate may be added as a lubricant. The mixture may be formed into 450-900 mg tablets (150-300 mg of active compound of the invention according to Formula I) in a tablet press.

### Formulation 5 - Injection

A compound of the invention according to Formula I may be dissolved or suspended in a buffered sterile saline injectable aqueous medium to a concentration of approximately 5 mg/mL.

### Formulation 6 - Topical

Stearyl alcohol (250 g) and a white petrolatum (250 g) may be melted at about 75°C and then a mixture of A compound of the invention according to Formula I (50 g) methylparaben (0.25 g), propylparaben (0.15 g), sodium lauryl sulfate (10 g), and propylene glycol (120 g) dissolved in water (about 370 g) may be added and the resulting mixture may be stirred until it congeals.

### METHODS OF TREATMENT

In one embodiment, the present invention provides compounds of the invention, or pharmaceutical compositions comprising a compound of the invention, for use in the prophylaxis and/or treatment of one or more fibrotic diseases. In a particular embodiment, the fibrotic disease is NASH and/or NAFLD. In a most particular embodiment, the fibrotic disease is NASH. In another most particular embodiment, the fibrotic disease is idiopathic pulmonary fibrosis (IPF).

In another embodiment, the present invention provides compounds of the invention, or pharmaceutical compositions comprising a compound of the invention for use in the manufacture of a medicament for use in the prophylaxis and/or treatment of one or more fibrotic diseases. In a particular embodiment, the fibrotic disease is NASH and/or NAFLD. In a most particular embodiment, the fibrotic disease is NASH. In another most particular embodiment, the fibrotic disease is idiopathic pulmonary fibrosis (IPF).

In additional method of treatment aspects, this invention provides methods of prophylaxis and/or treatment of a mammal afflicted with fibrotic diseases, which methods comprise the administration of an effective amount of a compound of the invention or one or more of the pharmaceutical compositions herein described for the treatment or prophylaxis of said condition. In a particular embodiment, the fibrotic disease is NASH and/or NAFLD. In a most particular embodiment, the fibrotic disease is NASH. In another most particular embodiment, the fibrotic disease is idiopathic pulmonary fibrosis (IPF).

In one embodiment, the present invention provides pharmaceutical compositions comprising a compound of the invention, and another therapeutic agent. In a particular embodiment, the other therapeutic agent is a fibrotic disease treatment agent. In a particular embodiment, the fibrotic disease is NASH and/or NAFLD. In a most particular embodiment, the fibrotic disease is NASH. In another most particular embodiment, the fibrotic disease is idiopathic pulmonary fibrosis (IPF).

In one embodiment, the present invention provides compounds of the invention, or pharmaceutical compositions comprising a compound of the invention, for use in the prophylaxis and/or treatment of a subject presenting a NAS score of at least 3, at least 4, at least 5, at least 6 or at least 7.

In another embodiment, the present invention provides compounds of the invention, or pharmaceutical compositions comprising a compound of the invention for use in the manufacture of a medicament for use in the prophylaxis and/or treatment of a subject presenting a NAS score ≥ 5.

In additional method of treatment aspects, this invention provides methods of prophylaxis and/or treatment of a mammal presenting a NAS score ≥ 5, which methods comprise the administration of an effective amount of a compound of the invention or one or more of the pharmaceutical compositions herein described for the treatment or prophylaxis of said fibrotic diseases, in particular NASH, and/or NAFLD, more particularly NASH.

In further method of treatment embodiments, the methods of prophylaxis and/or treatment of a mammal comprises measuring the forced vital capacity (FVC) in the subject, wherein the FVC does not decrease following treatment. In a particular embodiment, FVC does not decrease over a period of 12, 16, 20 or 26 weeks of treatment.

In another embodiment, the method comprises measuring the FVC in the subject, wherein the FVC increases by at least 1mL, at least 2 mL, at least 3 mL, at least 4 mL, at least 5 mL, at least 6 mL, at least 7 mL or at least 8 mL. In a particular embodiment, the FVC increases by at least 1mL, at least 2 mL, at least 3 mL, at least 4 mL, at least 5 mL, at least 6 mL, at least 7 mL or at least 8 mL over a period of 12, 16, 20 or 26 weeks of treatment.

In one embodiment, the method comprises measuring the airway volume wherein said airway volume decrease is no more than 5 mL/L, no more than 4 mL/l, or no more than 3 mL/L. In a particular embodiment the airway volume decrease is no more than 5 mL/L, no more than 4 mL/l, or no more than 3 mL/L after 12, 16, 20 or 26 weeks of treatment.

Injection dose levels range from about 0.1 mg/kg/h to at least 10 mg/kg/h, all for from about 1 to about 120 h and especially 24 to 96 h. A preloading bolus of from about 0.1 mg/kg to about 10 mg/kg or more may also be administered to achieve adequate steady state levels. The maximum total dose is not expected to exceed about 1 g/day for a 40 to 80 kg human patient.

For the prophylaxis and/or treatment of long-term conditions, such as degenerative conditions, the regimen for treatment usually stretches over many months or years so oral dosing is preferred for patient convenience and tolerance. With oral dosing, one to four (1-4) regular doses daily, especially one to three (1-3) regular doses daily, typically one to two (1-2) regular doses daily, and most typically one (1) regular dose daily are representative regimens. Alternatively for long lasting effect drugs, with oral dosing, once every other week, once weekly, and once a day are representative regimens. In particular, dosage regimen can be every 1-14 days, more particularly 1-10 days, even more particularly 1-7 days, and most particularly 1-3 days.

Using these dosing patterns, each dose provides from about 1 to about 1000 mg of a compound of the invention, with particular doses each providing from about 10 to about 500 mg and especially about 30 to about 250 mg. In a particular embodiment, a compound of the invention is administered in a 30 to 250 mg (such as 100 mg) daily dose for the treatment and/or prevention of fibrotic disease, more in particular the treatment and/or prevention of NASH, or, the treatment of IPF. In a further and most particular embodiment, said compound of the invention is 9-Cyclopropylethynyl-2-((S)-1-[1,4]dioxan-2-ylmethoxy)-6,7-dihydro-pyrimido [6,1 -a] isoquinolin-4-one.

Transdermal doses are generally selected to provide similar or lower blood levels than are achieved using injection doses.

When used to prevent the onset of a condition, a compound of the invention will be administered to a patient at risk for developing the condition, typically on the advice and under the supervision of a physician, at the dosage levels described above. Patients at risk for developing a particular condition generally include those that have a family history of the condition, or those who have been identified by genetic testing or screening to be particularly susceptible to developing the condition.

A compound of the invention can be administered as the sole active agent or it can be administered in combination with other therapeutic agents, including other compound of the inventions that demonstrate the same or a similar therapeutic activity and that are determined to be safe and efficacious for such combined administration. In a specific embodiment, co-administration of two (or more) agents allows for significantly lower doses of each to be used, thereby reducing the side effects seen.

In one embodiment, a compound of the invention or a pharmaceutical composition comprising a compound of the invention is administered as a medicament. In a specific embodiment, said pharmaceutical composition additionally comprises a further active ingredient.

In one embodiment, a compound of the invention is co-administered with one or more further therapeutic agents for the treatment and/or prophylaxis of a fibrotic disease. In a particular embodiment, a compound of the invention is co-administered with one or two further therapeutic agents for the treatment and/or prophylaxis of a fibrotic disease. In a more particular embodiment, a compound of the invention is co-administered with one further therapeutic agent for the treatment and/or prophylaxis of a fibrotic disease.

In one embodiment, the further therapeutic agent for the treatment and/or prophylaxis of a fibrotic disease include, but are not limited to 5-methyl-1-phenyl-2-(1H)-pyridone (pirfenidone); nintedanib (Ofev^{®} or Vargatef^{®}); STX-100 (ClinicalTrials.gov Identifier NCT01371305), FG-3019 (ClinicalTrials.gov Identifier NCT01890265), lebrikizumab (CAS n# 953400-68-5); tralokinumab (CAS n# 1044515-88-9), CC-90001 (ClinicalTrials.gov Identifier NCT03142191), tipelukast (MN-001; ClinicalTrials.gov Identifier NCT02503657), ND-L02-s0201 (ClinicalTrials.gov Identifier NCT03538301), KD025 (ClinicalTrials.gov Identifier NCT02688647), TD139 (ClinicalTrials.gov Identifier NCT02257177), VAY736 (ClinicalTrials.gov Identifier NCT03287414), PRM-151 (ClinicalTrials.gov Identifier NCT02550873) and PBI-4050 (ClinicalTrials.gov Identifier NCT02538536). In a particular embodiment, the further therapeutic agent for the treatment and/or prophylaxis of a fibrotic disease is an autotaxin (or ectonucleotide pyrophosphatase/phosphodiesterase 2 or NPP2 or ENPP2) inhibitor, examples of which are described in WO 2014/139882, such as GLPG1690.

In one embodiment, a compound of the invention is co-administered with another therapeutic agent for the treatment and/or prophylaxis of NASH, particular agents include but are not limited to weight loss treatment agents (for example Sibutramine, or Orlistat), insulin-sensitizing agents (for example Metformin, Thiazolidinedione, Rosiglitazone, or Pioglitazone), lipid-lowering agents (for example Gemfibrozil), Antioxidants (for example Vitamine E, N-acetylcysteine, Betaine, or Pentoxifylline), Angiotensin-converting enzyme inhibitors, Angiotensin-receptor blockers, Monounsaturated fatty acids, or Polyunsaturated fatty acids. FXR agonists (for example Obeticholic acid), LOXL2 antagonists (for example Simtuzumab), ASK1 antagonists (for example Selonsertib), PPAR agonists (for example clofibrate, gemfibrozil, ciprofibrate, bezafibrate, fenofibrate, thiazolidinediones, ibuprofen, GW-9662, aleglitazar, muraglitazar or tesaglitazar), Acetyl CoA-Carboxylase (ACC) antagonists (for example NDI-010976, PF-05221304), CCR2/CCR5 (for example Cenicriviroc), VAP1 antagonist.

In a more particular embodiment, the further therapeutic agent is selected from GLPG1690, or one or more of the compounds of WO 2017/148787.By co-administration is included any means of delivering two or more therapeutic agents to the patient as part of the same treatment regime, as will be apparent to the skilled person. Whilst the two or more agents may be administered simultaneously in a single formulation, *i.e.* as a single pharmaceutical composition, this is not essential. The agents may be administered in different formulations and at different times.

### BIOLOGICAL EXAMPLES

The methods for the preparation of the compounds of the invention have been described in WO 2013/092791, WO 2014/095798, WO 2015/197550, and WO 2016/169911.

### Example 1. In vitro assays.

The in vitro activity on GPR84 and on neutrophil migration of the compounds of the invention have been described in WO 2013/092791, WO 2014/095798, WO 2015/197550, and WO 2016/169911.

### Example 2. Cellular assay

### 2.1. Neutrophil migration assay

Whereas GPR84 agonists (MCFA such as sodiumdecanoate, 3,3' di indolylmethane and Embelin) may induce neutrophil chemotaxis and GPR84 antagonists may block GPR84 agonist-induced chemotaxis but not IL8-induced chemotaxis, it follows that G Protein-Coupled Receptor 84 (GPR84) is linked to neutrophil recruitment.

The effect of agonists or antagonists for GPR84 can therefore be assayed in a neutrophil migration test. In the neutrophil migration assay, neutrophils, freshly isolated from buffy coats from human volunteers, are treated with a test compound for 30 minutes. Subsequently, the neutrophils are transferred to the upper wells of a Corning HTS transwell 96 permeable support system, of which the lower wells are filled with a embelin solution at ECso (concentration which gives 80% of the activity of GPR84). After 1 h of incubation, migration of the neutrophils towards embelin in the lower compartment can be quantified by measuring the ATP-content of the lower wells using the ATPlite luminescence ATP detection assay system (Perkin Elmer, Cat. N°.: 436110).

### 2.1.1. Isolation of neutrophils from human buffy coat

A human buffy coat is diluted with an equal volume of ice cold DPBS. 20 mL of the diluted buffy coat is gently mixed with 4 mL of ACD buffer (140 mM citric acid, 200 mM sodium citrate and 220 mM dextrose). Then, 12 mL of the 6% dextran/0.9% NaCl solution (15 g dextran T2000 and 2.25 g NaCl dissolved in 250 mL H₂O) is added to the mixture and the samples are inverted gently up to 20 times. The total volume is transferred to a new recipient and incubated at room temperature for 1 h for complete separation of the two phases to occur. The supernatant is then transferred to a clean centrifugation tube and centrifuged for 12 minutes at 1300 rpm and 4°C. After centrifugation, the supernatant is discarded and the remaining cell pellet is rapidly resuspended in 12 mL of ice-cold H₂O for red blood cell lysis to occur. After 20 seconds, 4 mL of ice-cold 0.6 M KCl is added. Samples are mixed carefully and centrifuged for 6 minutes at 1300 rpm, 4°C. The supernatant is discarded and the red blood cell lysis procedure is repeated one more time. Subsequently, the cell pellet is resuspended in 4 mL of DPBS and layered over 5 mL of Lymphoprep (Nycomed Pharma, Cat. N°.: 1114545) in a 15 mL centrifuge tube. After centrifugation for 12 min at 1300 rpm, 4°C, the supernatant is removed and the cell pellet, containing the neutrophils, is resuspended in 25 mL chemotaxis buffer (RPMI 1640 medium, supplemented with 10 mM HEPES; freshly made for each experiment)

### 2.1.2. Migration assay

A cell suspension of 8.9x106 cells per milliliter is prepared. 20 µL of compound solution in chemotaxis buffer is added to 180 µL cell suspension. The mixture is incubated at 37°C for 30 minutes with intermediate resuspension of the cells after 15 minutes. Following this, 70 µL cell suspension is transferred to the upper compartment of a Corning HTS transwell 96 permeable support system with 5.0 µm pore size polycarbonate membrane (Corning, Cat. N°.: 3387). The receiver well of the transwell system is then filled with 200 µL chemotaxis buffer containing compound and chemotactic agent (embelin). After incubation at 37°C in 5% CO₂ for 1 h, the upper plate of the transwell system is removed and the cell suspension in the receiver plate is transferred to a 96-well V-bottom plate. 50 µL of DPBS is added to the receiver plate to prevent remaining cells from drying out. The V-bottom plate is centrifuged for 6 minutes at 1500 rpm. The supernatant is removed and the cells are resuspended in 50 µL DPBS. The cells are then transferred back to the receiver plate of the transwell system. After this, 100 µL ATPlite solution (Perkin Elmer, Cat. N°: 436110) is added to the cells. The plate is incubated for 10 minutes in the dark, while shaking. 170 µL of cell lysate is then transferred to a white 96-well plate and luminescence is measured. The detected luminescent signal is considered as linearly related to the number of cells having migrated from the upper well to the receiver well.

### Example 3. In vivo assays

### 3.1. Materials

In the following illustrative examples, the following compounds are tested, the syntheses of which are disclosed in WO 2013/092791 and WO 2016/169911:

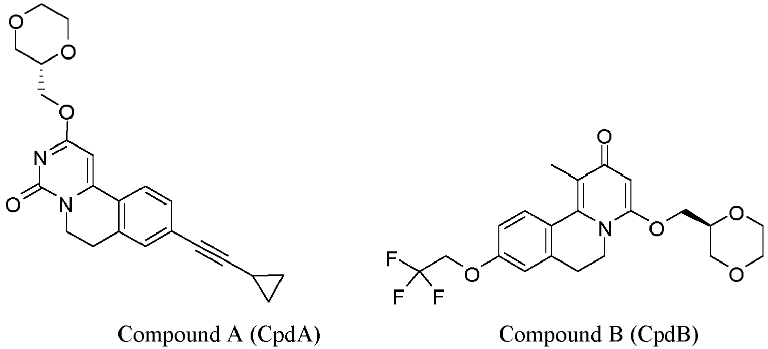

### 3.2. CCl₄ model

A single dose of CCl4 leads to centrizonal necrosis and steatosis, while prolonged administration leads to liver fibrosis, cirrhosis, and hepatocellular carcinoma.(Fujii et al., 2010)

At initiation, 5 weeks-old Male Balb/cJ mice (Janvier Labs, France) maintained at 22°C ± 2°C and humidity at 55% ± 10%, with a 12-hrs dark/light cycle. All mice are fed a standard diet (chow (A04C-10, Safe, France). All animals have access to filtered tap drinking water.

Twice a week, mice are injected intraperitoneally with 0.6 mL/kg of CCl₄ (319961, SIGMA) diluted at ½ in olive oil (01514, SIGMA) for 6 weeks. Control group 1 (C1) animals are injected with olive oil. Control group 2 (C2) animals are also injected with CCl₄ in olive oil.

Three weeks after the initiation, animals are assigned to a test-group or a control-group. Test group animals are dosed with a test compound at 30 mg/kg, i.e. Compound A (CpdA) or Compound B (CpdB), q.d., p.o. formulated in methylcellulose 0.5%, 1 equivalent HCl. The control groups receive a similar volume of vehicle (10 mL/kg).

After sacrifice (week 6), the activity of the test compound on the development of fibrosis is evaluated in liver by histopathological examination of fibrosis (Sirius red), collagen (OH-Pro) dosage and expression of fibrotic and inflammatory genes.

Figure 1 shows the effect of Compound A (CpdA) and Compound B (CpdB) on CCl₄ induced necrosis in the liver. Figure 2 shows the F4/80 glycoprotein stained area fraction [%] observed in liver samples from the animals tested. The F4/80 glycoprotein is a murine macrophage specific cell-surface biomarker. Figure 3 shows the percentage of neutrophils (panel A) and of monocytes (panel B) in total leucocyte cell population of blood samples obtained from the tested animals. Figure 4 shows the percentage of neutrophils (panel A), of monocytes (panel B) and of monocytic macrophages (MoMF, panel C) in total leucocyte cell population of liver samples obtained from the tested animals.

### 3.3. MCD model

The methionine and choline deficient (MCD) diet model is used to produce a severe phenotype of NASH in a time (Santhekadur et al., 2017), and is used to evaluate the compounds of the invention.

At induction, 8 weeks-old male C57BL/6 mice (Janvier Labs, France) maintained at 22°C ± 2°C and humidity at 55% ± 10%, with a 12-hrs dark/light cycle are fed a standard diet (chow (A04C-10, Safe, France) or a Methionine and Choline-deficient (MCD) diet (EFTD.90262, Ssniff, Soest, Germany) for 8 weeks. All animals have access to filtered tap drinking water.

Four weeks after the induction, the animals are either assigned to a control-group or a test-group. Test group animals are dosed with the a test compound, i.e. Compound A (CpdA) or Compound B (CpdB), at 30 mg/kg, q.d., p.o. formulated in methylcellulose 0.5%, 1 equivalent HCl. The control groups receives a similar volume of vehicle (10 mL/kg), i.e. the standard diet for control group 1 (C1) and the MCD diet for control group 2 (C2). Mice are randomly assigned to a treatment group according to their body weight in order to ensure a homogenous repartition.

Figure 5 shows the necrotic area fraction [%] observed in liver samples obtained from the tested animals. Figure 6 shows the F4/80 stained area fraction [%] observed in liver samples. Figure 7 shows Fluorescence-activated cell sorting (FACS) results measured in blood and liver samples obtained from animals in control group 1 (C1), control group 2 (C2), test group dosed with Compound A (CpdA) and test group dosed with Compound B (CpdB) for CD4 cells (Panel A), CD8 cells (Panel B), Blood monocyte (Panel C), Liver neutrophils cells (Panel D), Liver infiltrating monocyte-derived macrophage (MoMF) cells (Panel E), Liver CD19 cells (Panel F), Liver Natural Killer (NK) cells (Panel G), Liver Natural Killer T (NKT) cells (Panel H), and liver Kupfer cells (Panel I). Figure 8 shows gene expression of Col1a1 (panel A) and Timp1 (panel B) in blood samples. Figure 9 shows gene expression of TNFα (panel A) and CCL2 (panel B) in the liver.

After sacrifice (week 8), the activity of the test compound on the development of NASH is assessed by plasma ALT and AST levels and in liver by histopathological examination of fibrosis and steatosis (Sirius red, Oil Red O), collagen (OH-Pro) and triglycerides content and expression of fibrotic and inflammatory genes.

In addition, the NAS score is determined to further evaluate the compounds with respect to their effect on diet induced NASH. The nonalcoholic fatty liver disease activity scoring (NAS) has been proposed and accepted as a tool to measure changes in NAFLD during therapeutic trials (Brunt et al., 2011; Kleiner et al., 2005).

The NAS score (0-8) is defined as the sum of the steatosis score (0-3), lobular inflammation score (0-3), ballooning score (0-2). A NAS score of ≥ 5 is indicative of a NASH diagnostic. Figure 10 shows NAS scores as obtained in tested animals.

**Table I. Colla NRQ gene expression - Fig 8 Panel A**

| **8 weeks chow diet** | **MCD 8wk** | **Compound A (CpdA) (30 mg/kg) 8wk** | **Compound B (CpdB) (30 mg/kg) 8wk** |
|---|---|---|---|
| 0.1 | 1.9 | 2.1 | 0.3 |
| 0.1 | 1.3 | 0.6 | 0.2 |
| 0.1 | 0.6 | 2.4 | 0.3 |
| 0.1 | 1.1 | 0.8 | 0.2 |
| 0.2 | 1.1 | 0.3 | 0.5 |
| 0.1 | 1.5 | 0.8 | 2.0 |
| 0.0 | 0.6 | 1.0 | 1.3 |
| 0.0 | 0.9 | 0.5 | 1.5 |
| 0.1 | 1.0 | 0.1 | 1.5 |
| 0.0 | 0.7 | 0.4 | 0.4 |

**Table II. TIMP1 NRQ gene expression - Fig 8 Panel B**

| **8 weeks chow diet** | **MCD 8wk** | **Compound A (CpdA) (30 mg/kg) 8wk** | **Compound B (CpdB) (30 mg/kg) 8wk** |
|---|---|---|---|
| 0.1 | 1.6 | 2.3 | 0.3 |
| 0.1 | 1.3 | 0.4 | 0.3 |
| 0.3 | 0.6 | 1.0 | 0.3 |
| 0.0 | 1.2 | 1.1 | 0.4 |
| 0.1 | 1.3 | 0.4 | 0.5 |
| 0.1 | 0.7 | 0.8 | 1.7 |
| 0.0 | 0.6 | 0.9 | 1.2 |
| 0.0 | 1.2 | 0.4 | 1.4 |
| 0.1 | 1.1 | 0.1 | 1.7 |
| 0.0 | 0.8 | 0.5 | 0.4 |

**Table III. TNfα NRQ gene expression - Fig 9 Panel A**

| **8 weeks chow diet** | **MCD 8wk** | **Compound A (CpdA) (30 mg/kg) 8wk** | **Compound B (CpdB) (30 mg/kg) 8wk** |
|---|---|---|---|
| 0.1 | 1.3 | 1.5 | 0.3 |
| 0.1 | 0.9 | 0.4 | 0.3 |
| 0.1 | 1.0 | 0.8 | 0.4 |
| 0.1 | 1.1 | 1.4 | 0.4 |
| 0.1 | 1.2 | 0.8 | 0.7 |
| 0.0 | 1.2 | 1.0 | 1.1 |
| 0.1 | 0.8 | 0.7 | 0.8 |
| 0.1 | 1.0 | 0.5 | 1.1 |
| 0.0 | 0.9 | 0.1 | 0.9 |
| 0.0 | 0.9 | 0.8 | 0.6 |

**Table IV. CCl2 NRQ gene expression - Fig 9 Panel B**

| **8 weeks chow diet** | **MCD 8wk** | **Compound A (CpdA) (30 mg/kg) 8wk** | **Compound B (CpdB) (30 mg/kg) 8wk** |
|---|---|---|---|
| 0.0 | 1.4 | 1.0 | 0.5 |
| 0.1 | 1.1 | 0.6 | 0.7 |
| 0.0 | 0.8 | 0.5 | 0.8 |
| 0.1 | 1.1 | 1.2 | 0.3 |
| 0.0 | 1.0 | 0.6 | 0.7 |
| 0.1 | 0.9 | 0.5 | 1.2 |
| 0.1 | 1.3 | 0.7 | 0.9 |
| 0.0 | 0.8 | 0.5 | 1.2 |
| 0.0 | 0.9 | 0.2 | 1.5 |
| 0.0 | 1.0 | 0.5 | 0.9 |

**Table V. NAS scoring data - Fig 10**

| **8 weeks chow diet** | **MCD 8wk** | **Compound A (CpdA) (30 mg/kg) 8wk** | **Compound B (CpdB) (30 mg/kg) 8wk** |
|---|---|---|---|
| 0.0 | 7.0 | 8.0 | 6.0 |
| 1.0 | 8.0 | 6.0 | 6.0 |
| 1.0 | 6.0 | 7.0 | 5.0 |
| 1.0 | 8.0 | 6.0 | 7.0 |
| 0.0 | 7.0 | 7.0 | 6.0 |
| 1.0 | 7.0 | 6.0 | 7.0 |
| 1.0 | 7.0 | 6.0 | 6.0 |
| 1.0 | 8.0 | 7.0 | 6.0 |
| 1.0 | 8.0 | 5.0 | 6.0 |
| 1.0 | 8.0 | 7.0 | 6.0 |

### 3.4. CDAHFD model

The choline-deficient, L-amino acid-defined, high-fat diet (CDAHFD) dietary model is another model that develops steatohepatitis, liver fibrosis and hepatocarcinogenesis similar to MCD diet (Santhekadur et al., 2017) and is used to evaluate the compounds of the invention.

At induction, 8 weeks-old male C57BL/6 mice (Janvier Labs, France) maintained at 22°C ± 2°C and humidity at 55% ± 10%, with a 12-hrs dark/light cycle are fed a standard chow diet (A04C-10, SAFE, France) or choline deficient diet with 0.1% methionine (CDAHF) diet (A06071302, Research Diet, USA) for 8 weeks. All animals have access to filtered tap drinking water.

Four weeks after the induction, the animals are either assigned to a control-group or the test-group. Mice are randomly assigned to a treatment group according to their body weight in order to ensure a homogenous repartition. Test group animals are dosed with the test compound, i.e. Compound A (CpdA), at 30 mg/kg, q.d., p.o. formulated in methylcellulose 0.5%, 1 equivalent HCl. The control groups receives a similar volume of vehicle (10 mL/kg), i.e. the standard diet for control group 1 (C1) and the CDAHF diet for control groups 2 (C2, 8 weeks) and the CDAHF diet + Elafibranor dosed at 30 mg/kg, q.d., p.o. formulated in 0.1% Tween 80 + 1% methyl cellulose + 98.9% water (CpdC, 10 weeks).

After sacrifice (week 8), the activity of the test compound on the development of NASH is assessed by plasma ALT and AST levels and in liver by histopathological examination of fibrosis and steatosis (Sirius red, Oil Red O), collagen (OH-Pro) and triglycerides content and expression of fibrotic and inflammatory genes. Figure 11 reports on the observed effect of the Compound A (CpdA) on CDAHF diet induced formation of fibrotic tissue in the liver. Figure 12, panel A illustrates the effect of Compound A (CpdA) on CDAHF diet gene expression profiles of Col1a1. Figure 12, panel B illustrates the effect of Compound A (CpdA) on CDAHF diet gene expression profiles of TNFα.

**Table VI. Fibrosis (Sirius red area %) - Figure 11**

| **Chow diet 10W - vehicle** | **CDAHFD 8W - vehicle** | **CDAHFD 10W - vehicle** | **CDAHFD 10W - CpdC** | **CDAHFD 10W - Compound A (CpdA)** |
|---|---|---|---|---|
| 0.09 | 0.67 | 1.10 | 0.52 | 0.97 |
| 0.02 | 0.45 | 1.07 | 0.32 | 0.59 |
| 0.04 | 0.40 | 0.68 | 0.45 | 0.53 |
| 0.04 | 0.58 | 0.84 | 0.19 | 0.89 |
| 0.03 | 0.66 | 0.82 | 0.41 | 0.77 |
| 0.05 | 1.01 | 0.55 | 0.75 | 0.46 |
| 0.04 | 0.56 | 1.06 | 0.55 | 0.67 |
| 0.04 | 0.89 | 2.09 | 0.32 | 0.53 |
| 0.06 | 0.42 | 0.96 | 0.26 | 0.80 |
| 0.06 | 0.48 | 1.55 | nd | 0.64 |

| | | | | |
|---|---|---|---|---|
| nd: not determined | | | | |

**Table VII. Col1a1 expression profiles (NRQ-scaled versus Disease-Vehicle) - Fig 12 panel A**

| **Chow diet 10W - vehicle** | **CDAHFD 8W - vehicle** | **CDAHFD 10W - vehicle** | **CDAHFD 10W - CpdC** | **CDAHFD 10W** - **Compound A (CpdA)** |
|---|---|---|---|---|
| 0.01 | 1.01 | 0.82 | 0.61 | 0.12 |
| 0.02 | 0.80 | 1.57 | 0.17 | 0.26 |
| 0.06 | 1.54 | 0.67 | 0.07 | 1.76 |
| 0.11 | 0.70 | 1.79 | 0.06 | 0.36 |
| 0.04 | 0.13 | 2.08 | nd | 0.49 |
| 0.12 | 0.09 | 1.16 | 0.48 | 0.35 |
| 0.13 | 0.63 | 0.19 | 0.15 | 0.43 |
| 0.05 | 0.74 | 0.41 | 0.09 | 0.38 |
| 0.02 | 0.21 | 1.30 | 0.43 | 0.10 |
| 0.02 | 0.78 | 2.72 | nd | 0.18 |

| | | | | |
|---|---|---|---|---|
| nd: not determined | | | | |

**Table VIII. TNFα expression profiles (NRQ-scaled versus Disease-Vehicle) - Figure 12 panel B**

| **Chow diet 10W - vehicle** | **CDAHFD 8W - vehicle** | **CDAHFD 10W - vehicle** | **CDAHFD 10W - CpdC** | **CDAHFD 10W - Compound A (CpdA)** |
|---|---|---|---|---|
| 0.09 | 0.67 | 1.10 | 0.52 | 0.97 |
| 0.02 | 0.45 | 1.07 | 0.32 | 0.59 |
| 0.04 | 0.40 | 0.68 | 0.45 | 0.53 |
| 0.04 | 0.58 | 0.84 | 0.19 | 0.89 |
| 0.03 | 0.66 | 0.82 | 0.41 | 0.77 |
| 0.05 | 1.01 | 0.55 | 0.75 | 0.46 |
| 0.04 | 0.56 | 1.06 | 0.55 | 0.67 |
| 0.04 | 0.89 | 2.09 | 0.32 | 0.53 |
| 0.06 | 0.42 | 0.96 | 0.26 | 0.80 |
| 0.06 | 0.48 | 1.55 | nd | 0.64 |

| | | | | |
|---|---|---|---|---|
| nd: not determined | | | | |

### 3.5. Therapeutic bleomycin induced pulmonary fibrosis 21-day mice model

The aim of the study is to test the efficacy of a test compound at three different doses in a 21-day model of bleomycin induced pulmonary fibrosis in mice.

### 3.5.1. Animals

11 week-old C57BL/6N male mice from Charles River (Italy) are maintained on 12 h light/dark cycle at 22 °C with ad libidum access to tap water and food.

At least one day prior to start of experiment, all animals are allocated randomly into groups as indicated in the table below.

On day 0, all mice in groups 2-4 receive a 1.5 U/kg oropharyngeal administration of bleomycin (BLM) to induce pulmonary fibrosis. Animals in group 1 are not administered bleomycin, but instead receive a single dose of saline via the oropharyngeal route, and are considered sham control mice.

**Table IX. Study groups**

| **Groups** | **Purpose** | **n** | **Dose** | **Treatment schedule Days (Frequency)** | **Route** | **Vehicle** |
|---|---|---|---|---|---|---|
| **1 Sham + vehicle** | control | 10 | - | D7-D21 (QD) | PO | 0.1% Natrosol |
| **2 BLM + vehicle** | control | 15 | - | D7-D21 (QD) | PO | 0.1% Natrosol |
| **3 BLM + nintedanib** | control | 15 | 60 mg/kg | D7-D21 (QD) | PO | 0.1% Natrosol |
| **4 BLM + CpdA** | active | 15 | 30 mg/kg | D7-D21 (BID) | PO | 0.5% methylcellulose |

### 3.5.2. Study

Animals are examined clinically twice daily. List of clinical signs and parameters are indicated in humane endpoints table. Animals are weighed daily starting from D0.

On day 21, lung functionality is determined. After deep anesthesia induced by an intraperitoneal injection (10 mL/kg) of a solution of xylazine (5 mg/kg) and ketamine (75 mg/kg), the trachea is exposed through midline incision and a cannula is inserted. Mice are ventilated with a tidal volume of 10 mL/kg at a frequency of 150 breaths/minute. FlexiVent (SCIREQ^{®}) perturbations are performed three times. Deep Inflation and Pressure-Volume loops (PV loops) with stepwise increasing pressure are performed to measure the Inspiratory Capacity and stepwise pressure-driven perturbation (PVs-P). PV loops between 0 and 30 cm H₂O are generated to obtain Total Lung Capacity (A). A snapshot perturbation maneuver is imposed, which is a threefold sinusoidal wave of in- and expiration controlled by the ventilator, resulting in resistance (Rrs), compliance (Crs), and elastance (Ers) of the whole respiratory system (airways, lung and chest wall). A value for each group is calculated with average FlexiVent-validated measurements recorded in each individual subject.

Then, two hours after the last dose of test substance and nintedanib, mice are sacrificed by anesthetic overdose. The lungs are excised and weighed individually. The lungs are then placed into marked bottles containing 10% buffered formalin for further histopathological evaluation.

### 3.5.3. Sample analysis, data processing and statistical evaluation

Body weight data and lung weight data are processed using MS Excel. Statistical analysis and graphical presentation are performed using GraphPad Prism software (version 5.04).

One-way ANOVA or Mann-Whitney tests are employed for lung weights.

Two-way ANOVA tests are employed for body weight changes.

Differences between groups are considered statistically significant when p<0.05.

For histopathological evaluation, whole lungs are embedded in paraffin and stained with Mallory's trichrome.

Pulmonary histological changes are assessed using Matsuse's modification of Ashcroft score (Ashcroft et al., 1988; Matsuse et al., 1999). Statistical analysis and graphical presentation is performed using GraphPad Prism software (version 5.04). Mann-Whitney test is employed.

Differences between groups are considered statistically significant when p<0.05.

| | **Ashcroft Score** |
|---|---|
| 1 | Normal lungs (no fibrosis) |
| 2 | Minimal fibrotic thickening of alveolar or bronchial walls (network of fine collagen fibrils) |
| 3 | Moderate fibrotic thickening of walls without obvious damage to lung architecture |
| 4 | Fibrosis with damage of pulmonary structure (coarse fibrous bands or small fibrous masses, intra-alveolar collagen fibrils) |
| 5 | Large fibrous area with severe distortion of lung structure |

**Table X. Ashcroft Score - Figure 13**

| **Intact** | **BLM - vehicle** | **BLM - nintedanib** | **BLM - CpdA** |
|---|---|---|---|
| 1.0 | 2.4 | 3.0 | 3.4 |
| 1.0 | 4.0 | 3.5 | 2.3 |
| 1.0 | 3.8 | 2.4 | 2.0 |
| 1.0 | nd | 3.7 | 2.1 |
| 1.0 | 3.9 | 2.0 | nd |
| 1.0 | 3.4 | 2.3 | 2.4 |
| 1.0 | 3.6 | 2.8 | 3.0 |
| 1.0 | 3.1 | 3.2 | 2.5 |
| 1.0 | nd | 3.7 | 3.3 |
| 1.0 | 4.0 | 3.5 | 3.4 |
| | 1.9 | 3.5 | 2.9 |
| | 4.0 | 3.0 | 2.9 |
| | 2.7 | 3.4 | 2.7 |
| | 3.3 | 3.5 | 3.6 |
| | nd | 2.5 | 2.7 |

| | | | |
|---|---|---|---|
| nd: not determined | | | |

**Table XI. Inspiratory Capacity (mL) - Figure 14 panel A**

| **Intact** | **BLM - vehicle** | **BLM** - **nintedanib** | **BLM - CpdA** |
|---|---|---|---|
| 0.74 | nd | 0.76 | 0.84 |
| nd | 0.47 | 0.78 | 0.73 |
| 0.89 | 0.50 | nd | 0.69 |
| 0.84 | nd | 0.33 | 0.71 |
| 0.75 | 0.52 | 0.69 | nd |
| 0.70 | 0.51 | 0.57 | nd |
| 0.73 | 0.49 | 0.62 | 0.58 |
| 0.86 | 0.48 | 0.71 | 0.66 |
| nd | nd | nd | nd |
| nd | nd | 0.48 | nd |
| | nd | nd | 0.65 |
| | 0.28 | 0.58 | 0.59 |
| | 0.70 | 0.68 | 0.75 |
| | 0.50 | 0.53 | 0.58 |
| | nd | 0.56 | 0.69 |

| | | | |
|---|---|---|---|
| nd: not determined | | | |

**Table XII. Compliance of the Respiratory System (mL/cmHzO) - Figure 14 panel B**

| **Intact** | **BLM** - **vehicle** | **BLM** - **nintedanib** | **BLM** - **CpdA** |
|---|---|---|---|
| 0.043 | nd | 0.039 | 0.046 |
| nd | 0.025 | 0.045 | 0.042 |
| 0.046 | 0.026 | nd | 0.041 |
| 0.045 | nd | 0.015 | nd |
| 0.040 | 0.027 | 0.039 | nd |
| 0.038 | 0.027 | 0.031 | 0.034 |
| 0.042 | 0.025 | 0.032 | 0.030 |
| 0.045 | 0.024 | 0.040 | 0.035 |
| nd | nd | nd | 0.037 |
| nd | nd | 0.027 | nd |
| | nd | 0.027 | 0.036 |
| | nd | 0.032 | 0.033 |
| | 0.042 | 0.034 | 0.041 |
| | 0.026 | 0.027 | 0.031 |
| | nd | 0.035 | 0.038 |

| | | | |
|---|---|---|---|
| nd: not determined | | | |

**Table XIII. Elastance of the Respiratory System (cmH₂O/mL) - Figure 14 panel C**

| **Intact** | **BLM - vehicle** | **BLM - nintedanib** | **BLM - CpdA** |
|---|---|---|---|
| 23.18 | nd | 25.68 | 21.92 |
| nd | 40.71 | nd | 23.92 |
| 21.90 | 38.14 | 22.39 | 24.49 |
| 22.34 | nd | 68.28 | nd |
| 25.00 | 36.93 | 25.83 | nd |
| 26.52 | 37.20 | 32.61 | 29.43 |
| 23.84 | 38.89 | 31.04 | 33.18 |
| 22.21 | 42.23 | 25.16 | 28.94 |
| nd | nd | nd | 26.99 |
| nd | nd | 37.06 | nd |
| | nd | 36.60 | 27.50 |
| | nd | 31.06 | 30.18 |
| | 24.02 | 29.07 | 24.40 |
| | 38.70 | 37.31 | 31.90 |
| | nd | 28.26 | 26.35 |

| | | | |
|---|---|---|---|
| nd: not determined | | | |

### 3.6. Radiation induced fibrosis mice model

### 3.6.1. Study overview

Pneumonitis and lung fibrosis are the major radiation-induced complications following thoracic radiotherapy, which is one of the major treatment of lung and breast cancers, lymphomas and hematopoietic transplant conditioning.

The objective of this model is to evaluate the effect of a compound of the invention in lung fibrosis induced by radiation in mice. (Bickelhaupt et al., 2017)

**Table XIV. Study groups**

| **Groups** | **Purpose** | **n** | **Dose** | **Treatment schedule** Days (Frequency) | **Route** | **Vehicle** |
|---|---|---|---|---|---|---|
| **1 Sham + vehicle** | control | 10 | - | D1-D14 (QD) | PO | 0.1% Natrosol |
| **2 Irradiated + vehicle** | control | 12 | - | D1-D14 (QD) | PO | 0.1% Natrosol |
| **3 Irradiated + nintedanib** | control | 12 | 60 mg/kg | D1-D14 (QD) | PO | 0.1% Natrosol |
| **4 BLM + CpdA** | active | 12 | 30 mg/kg | D1-D14 (QD) | PO | 0.5% methylcellulose |

### 3.6.2. Animals

7 weeks old (18/22 gr) female C57BL/6J mice from Charles River (France) are maintained on 12 h light/dark cycle at 22°C with *ad libidum* access to tap water and food.

### 3.6.3. Materials

The test compounds are dissolved/suspended in appropriate vehicle prior to using and then kept light-free, under agitation at room temperature.

An aliquot of the formulation (approx. 200 µL) is frozen at T0 (day of preparation) and all the formulations are checked (daily) for any change in aspect.

The dose volume administered is 10 mL/kg and the volume is adapted following mean body weight (BW) of the group as follows: 200 µL if mean BW < 22.5 g, 250 µL if mean BW ≥ 22.5 g; 300 µL if mean BW > 27.5 g.

### 3.6.4. In vivo experimental procedure

On day 1 of week 1, the animals are exposed at the thorax to a 17 Gray irradiation dose, under isoflurane anesthesia.

At the beginning of week 18 post irradiation (Day 1), animals are randomized into 4 study groups (12 subjects per group, except sham group: 10 subjects): 1) sham (vehicle: methylcellulose 0.5%), 2) diseased (vehicle: methylcellulose 0.5%), 3) positive control (nintedanib 60 mg/kg in 0.1% Natrosol), and 4) test compound (30 mg/kg CpdA in 0.5% methylcellulose), and dosed p.o. q.d until Day 14 (week 21).

Body weight are recorded once a week, and on Day 14, lung function measurement under anesthesia is realized by Flexivent (Devos et al., 2017) for all groups before sacrifice.

### 3.6.5. Histology procedures

### 3.6.5.1. Type I collagen quantification

At sacrifice on Day 14, the lungs are collected and fixed in 4% formaldehyde for 24 h before embedding in paraffin. 4 µm thick sections are immunostained with anti-collagen I antibody (LifeSpan Biosciences, Cat. N°: LS-C343921). The sections are deparaffinized and processed by heat-induced antigen retrieval before incubation one hour with the primary antibody. The anti-collagen I antibody is detected and amplified by ImmPress kit (Vector Laboratories, Cat. N°: MP-7401). The immunostained sections are then scanned (Nanozoomer 2.0HT, Hamamatsu) before quantification by image analysis (CaloPix software, TRIBVN Healthcare). Data are expressed as percentage collagen I area per area of lung tissue.

Values for all mice from the same group are averaged. Data are expressed as mean ± sem and are compared with a one-way ANOVA followed by Dunnett multiple comparison *post hoc* test.

### 3.6.5.2. Oxidative stress: MnSOD quantification

Oxidative stress is a key player in the pathogenesis of IPF (Matsuzawa et al., 2015) and fibrosis (Richter and Kietzmann, 2016).

Manganese superoxide dismutase (MnSOD), located in mitochondria, catalyzes the conversion of superoxide into HzOz, and is a marker of antioxidant response induced by oxidative stress. MnSOD expression is measured immunohistochemically following a protocol adapted from Inghilleri et al. (Inghilleri et al., 2006).

At sacrifice on Day 14, the lungs are collected and fixed in 4% formaldehyde for 24 h before embedding in paraffin. 4 µm thick sections are immunostained with anti-MnSOD antibody (Enzo Life Sciences, Inc., Cat. N°: ADI-SOD110). The sections are deparaffinized and processed by heat-induced antigen retrieval before incubation one hour with the anti-MnSOD primary antibody. The anti-MnSOD antibody is detected by an anti-rabbit biotinylated antibody (Vector Laboratories, Cat. N°: BA-1100) amplified by avidin-biotin peroxidase (Vector Laboratories, Cat. N°: PK-6100). The peroxidase is revealed with DAB substrate (Sigma, Cat. N°: D5905) and 0.025% hydrogen peroxide. The immunostained sections counterstained with Gill's hematoxylin are then scanned (Nanozoomer 2.0HT, Hamamatsu) before quantification by image analysis (CaloPix software, TRIBVN Healthcare). Data are expressed as percentage MnSOD stained area per area of lung tissue.

Values for all mice from the same group are averaged. Data are expressed as mean ± sem and are compared with a one-way ANOVA followed by Dunnett multiple comparison *post hoc* test.

**Table XV. Type I collagen stained area fraction [%] - Figure 15**

| **Sham** | **Irradiated** | **Irradiated nintedanib** | **Irradiated CpdA** |
|---|---|---|---|
| 7.2 | 16.2 | 9.3 | nd |
| 7.7 | 16.2 | 8.9 | 8.9 |
| 7.6 | 16.7 | 13.7 | 9.1 |
| 7.4 | 17.4 | 10.5 | 8.6 |
| 9.9 | 19.1 | 10.5 | 16.2 |
| 8.6 | 11.7 | nd | 14.7 |
| 9.1 | 10.8 | 7.6 | nd |
| 5.2 | 9.3 | 7.1 | 11.0 |
| 6.2 | nd | nd | 13.0 |
| 5.6 | nd | 6.5 | 8.7 |
| | 9.6 | 11.0 | 8.1 |
| | 10.7 | 8.2 | 6.5 |

| | | | |
|---|---|---|---|
| nd: not determined | | | |

**Table XVI. MnSOD stained area fraction [%] - Figure 16**

| **Sham** | **Irradiated** | **Irradiated nintedanib** | **Irradiated CpdA** |
|---|---|---|---|
| 0.45 | 0.84 | 0.69 | nd |
| 0.20 | 1.35 | 0.92 | 0.99 |
| 0.30 | 1.29 | 0.53 | 0.57 |
| 0.43 | 0.84 | 1.11 | 0.26 |
| 0.62 | 1.79 | 1.19 | 0.31 |
| 0.81 | 0.75 | nd | 0.74 |
| 0.43 | 1.05 | 0.75 | nd |
| 0.36 | 0.95 | 0.25 | 0.39 |
| 0.13 | nd | nd | 0.57 |
| 0.31 | nd | 0.50 | 0.57 |
| | 2.14 | 0.79 | 0.71 |
| | 0.79 | 0.62 | 0.66 |

| | | | |
|---|---|---|---|
| nd: not determined | | | |

### CLINICAL EXAMPLES

**Table XVII. List of abbreviations used herein:**

| **Abbreviation** | **Definition** |
|---|---|
| 6MWT | 6-Minute Walk Test |
| AE | adverse event |
| ALAT | Latin American Thoracic Association |
| ALT | alanine aminotransferase |
| AST | aspartate aminotransferase |
| ATS | American Thoracic Society |
| DLCO | diffusing capacity for the lungs for carbon monoxide |
| DNA | deoxyribonucleic acid |
| ECG | electrocardiogram |
| ERS | European Respiratory Society |
| FEV1 | forced expiratory volume in one second |
| FRI | functional respiratory imaging |
| FVC | forced vital capacity |
| HRCT | high-resolution computed tomography |
| IMP | investigational medicinal product |
| IPF | idiopathic pulmonary fibrosis |
| JRS | Japanese Respiratory Society |
| SGRQ | St. George's Respiratory Questionnaire |

### Example 1. Idiopathic pulmonary fibrosis (IPF) clinical study

The study of the current example is a randomized, double-blind, parallel group, placebo-controlled, multicenter, Phase II study to evaluate the efficacy, safety and tolerability of Compound A in subjects with idiopathic pulmonary fibrosis (IPF). The primary objective of this study is to evaluate the efficacy of Compound A treatment in subjects with IPF on pulmonary function as evaluated by FVC compared to placebo over 26 weeks.

### 1.1. Study endpoints

Primary outcome measure is:
- Change from baseline in FVC (mL) over 26 weeks compared to placebo.

Secondary outcome measures are:
- Safety and tolerability changes over time (baseline to 26 weeks).
- Time to any of major events (whichever occurs first) defined as:
   ∘ Respiratory-related death
   ∘ First hospitalization (all-cause and respiratory-related)
   ∘ Need to be placed on a lung transplant list during the study
- Change from baseline 26 weeks in functional exercise capacity, assessed by the 6MWT at Week 26.
- Change from baseline until 26 weeks in quality of life measures, assessed by the St. George's Respiratory Questionnaire (SGRQ) total score and domains and proportion of SGRQ responders.
- Concentrations of Compound A, nintedanib and pirfenidone.

Other outcome measures are:
- Change from baseline in FRI parameters, assessed by chest HRCT.
- Change in target and/or disease specific biomarkers, in relation to genotype subgroups in blood and/or clinical endpoints over time compared to baseline

### 1.2. Study interventions

The study provides for 2 types of interventions:
∘ Drug: Compound A - 100 mg administered orally once daily as 2 capsules of 50 mg with or without food
∘ Drug: Compound A placebo - administered orally once daily as 2 capsules with or without food

### 1.3. Study arms

Study participants are randomized over 2 study arms:
1. Experimental: Compound A: Compound A 100 mg, for 26 weeks
2. Placebo Comparator: Placebo: Compound A placebo for 26 weeks

### 1.4. Eligibility criteria

To be eligible, the subjects must meet the following criteria:
- Males or females of non-child-bearing potential, aged ≥40 years.
- A diagnosis of IPF within 3 years prior to the screening visit as per American Thoracic Society (ATS)/European Respiratory Society (ERS)/Japanese Respiratory Society (JRS)/Latin American Thoracic Association (ALAT) guidelines.
- Meeting all of the following criteria at screening and during the screening period:
   ∘ FVC ≥50% predicted of normal
   ∘ Disease progression in the last 9 months prior to the screening period and at screening, defined as at least one prescreening FVC value and screening value with a decline of FVC (% predicted or mL), at the investigator's discretion
   ∘ Diffusing capacity for the lungs for carbon monoxide (DLCO) ≥30% predicted of normal (corrected for hemoglobin)
   ∘ Ratio of forced expiratory volume in one second (FEV1) to FVC ≥0.70
- In a stable condition and suitable for study participation based on the results of a medical history, physical examination, vital signs, 12-lead ECG, and laboratory evaluation. Stable condition is based on the clinical judgment of the investigator, co-morbidities should be treated according to the local applicable guidelines. Concomitant medication for comorbidities should be stabilized from 4 weeks before screening and during the screening period (stable defined as no change of dose or regimen).
- Estimated minimum life expectancy of 12 months for non-IPF related disease in the opinion of the investigator.
- Male subjects with female partners of child bearing potential are willing to comply with the contraceptive methods described in the protocol (see Section 4.3.4.1) prior to the first dose of the IMP, during the clinical study, and for at least 12 weeks after the last dose of the IMP.
- Able to walk at least 150 meters during the 6MWT at screening; without having a contraindication to perform the 6MWT.
- Able to understand the importance of adherence, and willing to comply to study treatment, study procedures and requirements as per study protocol, including the concomitant medication restrictions.

Key exclusion criteria: subjects meeting one or more of the following criteria cannot be selected for this study:
- Known hypersensitivity to any of the IMP ingredients or a history of a significant allergic reaction to any drug as determined by the investigator (e.g. anaphylaxis requiring hospitalization).
- History of or a current immunosuppressive condition (e.g. human immunodeficiency virus [HIV] infection, congenital, acquired, medication induced).
- Positive serology for hepatitis B (surface antigen and core antibody) or C (antibody), or any history of hepatitis from any cause with the exception of hepatitis A.
- History of malignancy within the past 5 years (except for carcinoma in situ of the uterine cervix, basal cell carcinoma of the skin that has been treated with no evidence of recurrence, and prostate cancer medically managed through active surveillance or watchful waiting, and squamous cell carcinoma of the skin if fully resected).
- Acute IPF exacerbation within 3 months prior to screening and during the screening period.
- Lower respiratory tract infection requiring antibiotics within 4 weeks prior to screening and/or during the screening period.
- Interstitial lung disease associated with known primary diseases (e.g. sarcoidosis, amyloidosis), exposures (e.g. radiation, silica, asbestos, coal dust), and drugs (e.g. amiodarone).
- History of lung volume reduction surgery or lung transplant.
- Unstable cardiovascular, pulmonary (other than IPF) or other disease within 6 months prior to screening or during the screening period (e.g. coronary heart disease, heart failure, stroke).
- Subject participating in a drug, device or biologic investigational research study, concurrently with the current study, or within 5-half-lives of the agent (or within 8 weeks when half-life is unknown) prior to screening, or prior participation in an investigational drug antibody study within 6 months prior to screening.

### CLAUSES

1. A compound having GPR84 antagonist activity for use in the prophylaxis and/or treatment of one or more fibrotic diseases.
2. The compound for use according to clause 1, wherein the compound is according to formula I: 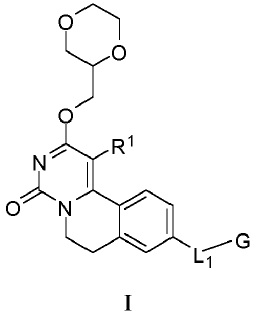 wherein
   R¹ is H, Me, or halo;
   L₁ is absent or is -O-, -S-, or -NR^{4a}-;
   G is
      - R²,
      - -W-L₂-R², or
      - -W-L₃-R³;
   W is C₁₋₄ alkylene, C₂₋₄ alkenylene having one double bond, or C₂₋₄ alkynylene having one triple bond;
   L₂ is absent or is -O-;
   R² is
      - H,
      - C₁₋₈ alkyl, optionally substituted with one to three groups independently selected from
         ∘ OH,
         ∘ halo,
         ∘ CN,
         ∘ C₁₋₆ alkoxy,
         ∘ C₃₋₇ cycloalkyl,
         ∘ 4-6 membered heterocycloalkyl comprising one to three heteroatoms independently selected from S, and O,
         ∘ 5-6 membered heteroaryl comprising one to three heteroatoms independently selected from N, S, and O, and
         ∘ phenyl,
      - C₄₋₇ cycloalkenyl comprising one double bond,
      - 5-7 membered heterocycloalkenyl comprising one double bond, and one to three heteroatoms independently selected from N, O, and S,
      - C₃₋₇ cycloalkyl optionally substituted with one or more independently selected R⁵ groups,
      - 4-10 membered heterocycloalkyl comprising one to two heteroatoms independently selected from S, and O, optionally substituted with one to three independently selected R⁵ groups,
      - 5-10 membered heteroaryl comprising one to three heteroatoms independently selected from N, S, and O, optionally substituted with one to three independently selected R⁶ groups, or
      - C₆₋₁₀ aryl optionally substituted with one or more independently selected R⁶ groups;
   L₃ is -NR^{4b}-;
   R³ is
      - C₁₋₄ alkyl substituted with
         ∘ C₆₋₁₀ aryl optionally substituted with one or more independently selected R⁷ groups, or
         ∘ 5-10 membered heteroaryl comprising one to three heteroatoms independently selected from N, S, and O, optionally substituted with one or more independently selected R⁷ groups,
      - 5-10 membered heteroaryl comprising one to three heteroatoms independently selected from N, S, and O, optionally substituted with one or more independently selected R⁷ groups, or
      - C₆₋₁₀ aryl optionally substituted with one or more independently selected R⁷ groups;
   Each R^{4a} and R^{4b} is independently selected from H, C₁₋₄ alkyl, and C₃₋₇ cycloalkyl;
   R⁵ is oxo or R⁶;
   R⁶ is
      - OH,
      - halo,
      - -NOz,
      - C₁₋₆ alkyl optionally substituted with one to three groups independently selected from halo, and OH,
      - C₁₋₆ alkoxy optionally substituted with one to three groups independently selected from halo, and OH,
      - C₃₋₇ cycloalkyl,
      - -C(=O)OR⁸,
      - -C(=O)NR⁹R¹⁰,
      - -NHC(=O)-C₁₋₄ alkyl,
      - -CN,
      - phenyl,
      - -O-phenyl,
      - 4-7 membered heterocycloalkyl comprising one to three heteroatoms independently selected from N, O, and S, or
      - 5-6 membered heteroaryl comprising one to three heteroatoms independently selected from N, O, and S; optionally substituted with one or more independently selected C₁₋₄ alkyl, C₁₋₄ alkoxy, CN, halo, and -C(=O)OR¹¹;
   R⁷ is C₁₋₄ alkyl, or halo, and
   each of R⁸, R⁹, R¹⁰ and R¹¹ is independently selected from H and C₁₋₄ alkyl;
   or pharmaceutically acceptable salt thereof, or a pharmaceutically acceptable solvate, or the salt of a pharmaceutically acceptable solvate thereof.
3. The compound for use according to clause 2, wherein the compound is according to Formula I.Ia, I.Ib, I.Ic, or I.Id: 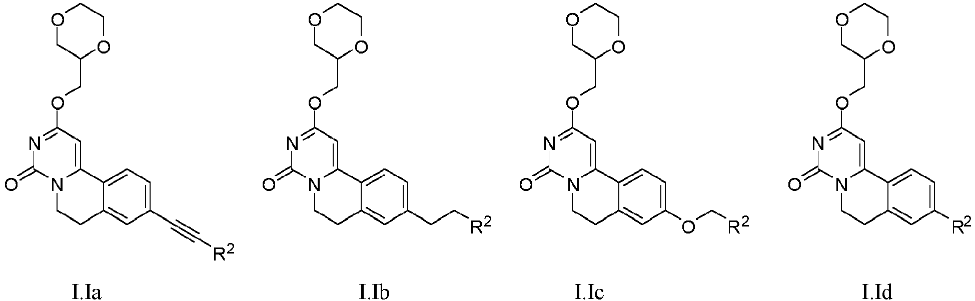
4. The compound for use according to clause 1, wherein the compound is according to Formula II : 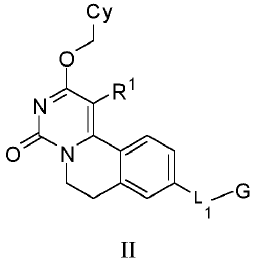 wherein
   Cy is 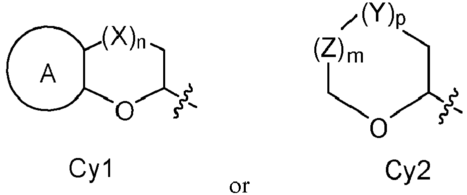
   wherein
   X is O or S;
   Y is -CH₂-, or S;
   Z is -CH₂-;
   each of the subscript n, m, or p is independently selected from 0, and 1; and A is phenyl, or 5-6-membered heteroaryl comprising one or two N-atoms; optionally substituted with one or more independently selected R⁵ groups;
   any one of Cy1 and Cy2 is optionally substituted by one or more independently selected C₁₋₄ alkyl groups; R¹ is H, Me, or halo;
   L₁ is absent or is -O-, -S-, or -NR^{4a}-;
   G is
      - R²,
      - -W-L₂-R², or
      - -W-L₃-R³;
   W is C₁₋₄ alkylene, C₂₋₄ alkenylene having one double bond, or C₂₋₄ alkynylene having one triple bond;
   L₂ is absent or is -O-;
   R² is
      - H,
      - C₁₋₈ alkyl optionally substituted with one to three groups independently selected from
         ∘ OH,
         ∘ halo,
         ∘ CN,
         ∘ C₁₋₆ alkoxy,
         ∘ C₃₋₇ cycloalkyl,
         ∘ 4-6 membered heterocycloalkyl (comprising one to three heteroatoms independently selected from S, and O),
         ∘ 5-6 membered heteroaryl (comprising one to three heteroatoms independently selected from N, S, and O), and
         ∘ phenyl,
      - C₄₋₇ cycloalkenyl comprising one double bond,
      - 5-7 membered heterocycloalkenyl comprising one double bond, and one to three heteroatoms independently selected from O, and S,
      - C₃₋₇ cycloalkyl (optionally substituted with one or more independently selected R⁶ groups),
      - 4-10 membered heterocycloalkyl comprising one to two heteroatoms independently selected from S, and O, (optionally substituted with one to three independently selected R⁶ groups),
      - 5-10 membered heteroaryl comprising one to three heteroatoms independently selected from N, S, and O (optionally substituted with one to three independently selected R⁷ groups), or
      - C₆₋₁₀ aryl (optionally substituted with one or more independently selected R⁷ groups);
   L₃ is -NR^{4b}-;
   R³ is
      - C₁₋₄ alkyl substituted with C₆₋₁₀ aryl (optionally substituted with one or more independently selected R⁸ groups), or 5-10 membered heteroaryl comprising one to three heteroatoms independently selected from N, S, and O, (optionally substituted with one or more independently selected R⁸ groups),
      - 5-10 membered heteroaryl comprising one to three heteroatoms independently selected from N, S, and O, (optionally substituted with one or more independently selected R⁸ groups), or
      - C₆₋₁₀ aryl (optionally substituted with one or more independently selected R⁸ groups);
   each R^{4a} and R^{4b} is independently selected from H, C₁₋₄ alkyl, and C₃₋₇ cycloalkyl;
   R⁵ is halo, C₁₋₄ alkyl or C₁₋₄ alkoxy;
   R⁶ is oxo or R⁷;
   R⁷ is
      - OH,
      - halo,
      - -NOz,
      - C₁₋₆ alkyl (optionally substituted with one to three groups independently selected from halo, and OH),
      - C₁₋₆ alkoxy (optionally substituted with one to three groups independently selected from halo, and OH),
      - C₃₋₇ cycloalkyl,
      - -C(=O)OR⁹,
      - -C(=O)NR¹⁰R¹¹,
      - -NHC(=O)-C₁₋₄ alkyl,
      - -CN,
      - phenyl,
      - -O-phenyl,
      - 4-7 membered heterocycloalkyl comprising one to three heteroatoms independently selected from N, O, and S, or
      - 5-6 membered heteroaryl comprising one to three heteroatoms independently selected from N, O, and S; (optionally substituted with one or more independently selected C₁₋₄ alkyl, C₁₋₄ alkoxy, CN, halo, and -C(=O)OR¹²);
   R⁸ is C₁₋₄ alkyl, or halo; and
   each of R⁹, R¹⁰, R¹¹and R¹², is independently selected from H and C₁₋₄ alkyl;
   or pharmaceutically acceptable salt thereof, or a pharmaceutically acceptable solvate, or the salt of a pharmaceutically acceptable solvate thereof.
5. The compound for use according to clause 1, wherein the compound is according to Formula III : 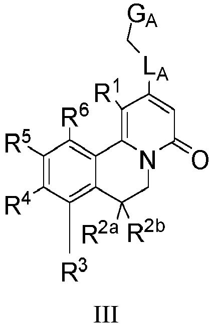 wherein
   R¹ is H, C₁₋₄ alkyl, or cyclopropyl;
   L_{A} is O or NH;
   G_{A} is:
      - 4-6 membered monocyclic heterocycloalkyl containing one or two O,
      - C₃₋₇ monocyclic cycloalkyl, or
      - a bicyclic group of formula Cy: 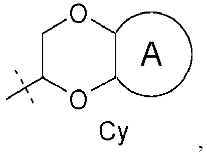
   wherein A is phenyl or 5-6 membered heteroaryl containing one or two heteroatoms independently selected from N, O and S;
   each R^{2a} and R^{2b} are independently H or -CH₃;
   R³ is H, -OH or -OCH₃;
   R⁴ is -CN or -L₁-W₁-G₁,
   wherein
      - L₁ is absent or O,
      - W₁ is absent, or is C₁₋₆ alkylene, C₂₋₄ alkenylene having one double bond or C₂₋₄ alkynylene having one triple bond, each of which is optionally substituted with one or more independently selected halo, -CN or C₁₋₄ alkoxy,
      - Gi is
         ∘ H,
         ∘ -CF₃,
         ∘ -C(=O)-C₁-₄ alkyl,
         ∘ -S(=O)₂-C₁₋₄ alkyl, optionally substituted with one or more independently selected halo,
         ∘ 4-6 membered monocyclic heterocycloalkyl containing one or two O (which heterocycloalkyl is optionally substituted with one or more independently selected R⁷ groups),
         ∘ 6 membered monocyclic heterocycloalkenyl containing one or two O (which heterocycloalkenyl is optionally substituted with one or more independently selected R⁷ groups),
         ∘ C₃₋₇ monocyclic cycloalkyl optionally substituted with one or more independently selected R⁷ groups,
         ∘ phenyl optionally substituted with one or more independently selected R⁷ groups,
         ∘ or 5-6 membered heteroaryl containing one to four heteroatoms independently selected from N, O and S (which heteroaryl is optionally substituted with one or more independently selected R⁷ groups),
   each R⁷ is:
      - halo,
      - -OH,
      - C₁₋₄ alkyl, C₃₋₄ monocyclic cycloalkyl, or C₁₋₄ alkoxy, each of which is optionally substituted with one or more independently selected halo;
   R⁵ is -CN or -L₂-W₂-G₂,
   wherein
      - L₂ is absent, O or S,
      - Wz is absent or C₁₋₄ alkylene, optionally substituted with one or more independently selected halo,
      - Gz is
         ∘ H,
         ∘ -CF₃,
         ∘ C₃₋₇ monocyclic cycloalkyl (which cycloalkyl is optionally substituted with one or more independently selected halo),
         ∘ phenyl,
         ∘ or 5-6 membered heteroaryl containing one to three heteroatoms independently selected from N, O and S; and
   R⁶ is H, -OH or -OCH₃;
   or pharmaceutically acceptable salt thereof, or a pharmaceutically acceptable solvate, or the salt of a pharmaceutically acceptable solvate thereof.
6. The compound for use according to clause 1, wherein the compound is according to Formula IV : 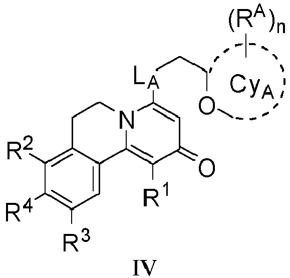 wherein
   L_{A} is O, or NH;
   Cy_{A} is monocyclic 4-6 membered heterocycloalkyl, comprising one or two O atoms;
   each R^{A} is independently selected from halo, and C₁₋₃ alkyl;
   the subscript n is 0, 1 or 2;
   R¹ is H or C₁₋₃ alkyl;
   R² is H, -OH, or C₁₋₃ alkoxy;
   R³ is H or C₁₋₃ alkoxy;
   R⁴ is
      - -CN,
      - -OH,
      - -O-S(=O)₂-C₁₋₄ alkyl optionally substituted with one or more independently selected halo, or
      - -L₁-W₁-G₁;
   L₁ is a direct bond, -O-, -S-, -SO₂-, -C(=O)NR^{5a}-, -NR^{5b}C(=O)-, or -NR^{5c}-;
   R^{5a}, R^{5b} and R^{5c} are independently H or C₁₋₄ alkyl;
   W₁ is a direct bond or C₁₋₂ alkylene optionally substituted with one or more independently selected halo;
   Gi is
      - C₃₋₆ cycloalkyl optionally substituted with one or more independently selected halo,
      - 5-6 membered heteroaryl comprising one to four heteroatoms independently selected from N, O, and S, which heteroaryl is optionally substituted with one or more independently selected C₁₋₄ alkyl,
      - 5-7 membered heterocycloalkenyl comprising one double bond, and one to three heteroatoms independently selected from N, O, and S, which heterocycloalkenyl is optionally substituted with one or more independently selected R⁶,
      - monocyclic or spiro bicyclic 4-8 membered heterocycloalkyl comprising one to three heteroatoms independently selected from N, O, and S, which heterocycloalkyl is optionally substituted with one or more independently selected R⁶,
      - monocyclic 4-6 membered heterocycloalkyl comprising one to three heteroatoms independently selected from N, O, and S, fused to one or two phenyls,
      - C₁₋₄ alkyl optionally substituted with one or more independently selected halo, -NR^{7a}R^{7b}, or C₁₋₄ alkoxy, which alkoxy is optionally substituted with one or more independently selected halo,
      - phenyl optionally substituted with one or more independently selected halo or C₁₋₄ alkoxy, which alkoxy is optionally substituted with one or more independently selected halo;
   R⁶ is
      - halo,
      - =O,
      - -CN,
      - -OH,
      - -C(=O)-C₁₋₄ alkoxy optionally substituted with one or more independently selected halo,
      - -C(=O)-C3-4 cycloalkyl,
      - -S(=O)2-C₁₋₄ alkyl,
      - C₁₋₄ alkyl optionally substituted by one or more independently selected C1-3 alkoxy, halo, or -OH,
      - C₁₋₄ alkoxy,
      - phenyl optionally substituted by one or more independently selected halo,
      - -C(=O)-monocyclic 4-6 membered heterocycloalkyl comprising one to three heteroatoms independently selected from N, O, and S,
      - -C(=O)NR^{8a}R^{8b}, or
      - 5-7 membered heteroaryl comprising one to four heteroatoms independently selected from N, O, and S, which heteroaryl is optionally substituted with one or more independently selected C₁₋₄ alkyl;
   R^{7a} and R^{7b} are independently H or C₁₋₄ alkyl, and
   R^{8a} and R^{8b} are independently H or C₁₋₃ alkyl;
   or pharmaceutically acceptable salt thereof, or a pharmaceutically acceptable solvate, or the salt of a pharmaceutically acceptable solvate thereof.
7. The compound for use according to clause 6, wherein the compound is according to Formula IV.Ia: 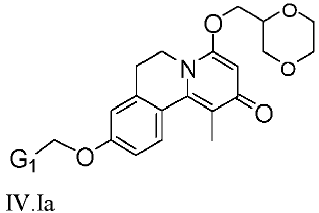
8. The compound or pharmaceutically acceptable salt thereof for use according to clause 1 wherein the compound is selected from:
   9-Allyloxy-2-([1,4]dioxan-2-ylmethoxy)-6,7-dihydro-pyrimido[6,1-a]isoquinolin-4-one,
   2-([1,4]Dioxan-2-ylmethoxy)-9-pyridin-3-yl-6,7-dihydro-pyrimido[6,1-a]isoquinolin-4-one,
   2-([1,4]Dioxan-2-ylmethoxy)-9-pyridin-4-yl-6,7-dihydro-pyrimido[6,1-a]isoquinolin-4-one,
   2-[2-([1,4]Dioxan-2-ylmethoxy)-4-oxo-6,7-dihydro-4H-pyrimido[6,1-a]isoquinolin-9-yl]-benzonitrile,
   3-[2-([1,4]Dioxan-2-ylmethoxy)-4-oxo-6,7-dihydro-4H-pyrimido[6,1-a]isoquinolin-9-yl]-benzonitrile,
   4-[2-([1,4]Dioxan-2-ylmethoxy)-4-oxo-6,7-dihydro-4H-pyrimido[6,1-a]isoquinolin-9-yl]-benzonitrile,
   [2-([1,4]Dioxan-2-ylmethoxy)-4-oxo-6,7-dihydro-4H-pyrimido[6,1-a]isoquinolin-9-yloxy]-acetonitrile,
   2-([1,4]Dioxan-2-ylmethoxy)-9-(oxazol-2-ylmethoxy)-6,7-dihydro-pyrimido[6,1-a]isoquinolin-4-one,
   2-([1,4]Dioxan-2-ylmethoxy)-9-(pyridin-2-ylmethoxy)-6,7-dihydro-pyrimido[6,1-a]isoquinolin-4-one,
   9-(3,5-Dichloro-phenyl)-2-([1,4]dioxan-2-ylmethoxy)-6,7-dihydro-pyrimido[6,1-a]isoquinolin-4-one,
   9-Benzofuran-2-yl-2-([1,4]dioxan-2-ylmethoxy)-6,7-dihydro-pyrimido[6,1-a]isoquinolin-4-one,
   2-[2-([1,4]Dioxan-2-ylmethoxy)-4-oxo-6,7-dihydro-4H-pyrimido[6,1-a]isoquinolin-9-yl]-indole-1-carboxylic acid tert-butyl ester,
   2-([1,4]Dioxan-2-ylmethoxy)-9-(1H-indol-2-yl)-6,7-dihydro-pyrimido[6,1-a]isoquinolin-4-one,
   2-([1,4]Dioxan-2-ylmethoxy)-9-(6-methoxy-pyridin-3-yl)-6,7-dihydro-pyrimido[6,1-a]isoquinolin-4-one,
   2-([1,4]Dioxan-2-ylmethoxy)-9-(6-trifluoromethyl-pyridin-3-yl)-6,7-dihydro-pyrimido[6,1-a]isoquinolin-4-one,
   2-([1,4]Dioxan-2-ylmethoxy)-9-(3-methyl-3H-imidazol-4-ylethynyl)-6,7-dihydro-pyrimido[6,1-a]isoquinolin-4-one,
   9-(5-tert-Butyl-[1,2,4]oxadiazol-3-ylmethoxy)-2-([1,4]dioxan-2-ylmethoxy)-6,7-dihydro-pyrimido [6,1 -a] isoquinolin-4-one,
   5-[2-([1,4]Dioxan-2-ylmethoxy)-4-oxo-6,7-dihydro-4H-pyrimido[6,1-a]isoquinolin-9-yl]-pyridine-2-carboxylic acid methylamide,
   2-([1,4]Dioxan-2-ylmethoxy)-9-pent-1-ynyl-6,7-dihydro-pyrimido[6,1-a]isoquinolin-4-one,
   2-([1,4]Dioxan-2-ylmethoxy)-9-(2-pyridin-2-yl-ethyl)-6,7-dihydro-pyrimido[6,1-a]isoquinolin-4-one,
   2-([1,4]Dioxan-2-ylmethoxy)-9-(2-pyrazin-2-yl-ethyl)-6,7-dihydro-pyrimido[6,1-a]isoquinolin-4-one,
   2-([1,4]Dioxan-2-ylmethoxy)-9-(1H-indol-5-yl)-6,7-dihydro-pyrimido[6,1-a]isoquinolin-4-one,
   2-([1,4]Dioxan-2-ylmethoxy)-9-(2-methoxy-phenyl)-6,7-dihydro-pyrimido[6,1-a]isoquinolin-4-one,
   2-([1,4]Dioxan-2-ylmethoxy)-9-(5-methoxy-pyridin-3-yl)-6,7-dihydro-pyrimido[6,1-a]isoquinolin-4-one,
   2-([1,4]Dioxan-2-ylmethoxy)-9-(1H-indazol-5-yl)-6,7-dihydro-pyrimido[6,1-a]isoquinolin-4-one,
   2-([1,4]Dioxan-2-ylmethoxy)-9-(4-methoxy-phenyl)-6,7-dihydro-pyrimido[6,1-a]isoquinolin-4-one,
   3-[2-([1,4]Dioxan-2-ylmethoxy)-4-oxo-6,7-dihydro-4H-pyrimido[6,1-a]isoquinolin-9-yl]-benzamide,
   5-[2-([1,4]Dioxan-2-ylmethoxy)-4-oxo-6,7-dihydro-4H-pyrimido[6,1-a]isoquinolin-9-yl]-2-fluoro-benzamide,
   N-{3-[2-([1,4]Dioxan-2-ylmethoxy)-4-oxo-6,7-dihydro-4H-pyrimido[6,1-a]isoquinolin-9-yl]-phenyl} -acetamide,
   9-Cyclopropylethynyl-2-([1,4]dioxan-2-ylmethoxy)-6,7-dihydro-pyrimido[6,1-a]isoquinolin-4-one,
   2-([1,4]Dioxan-2-ylmethoxy)-9-(1-hydroxy-cyclopentylethynyl)-6,7-dihydro-pyrimido[6,1-a]isoquinolin-4-one,
   2-([1,4]Dioxan-2-ylmethoxy)-9-pyrimidin-5-yl-6,7-dihydro-pyrimido[6,1-a]isoquinolin-4-one,
   9-Cyclohex-1-enyl-2-([1,4]dioxan-2-ylmethoxy)-6,7-dihydro-pyrimido[6,1-a]isoquinolin-4-one,
   2-([1,4]Dioxan-2-ylmethoxy)-9-(1-methyl-1H-indol-5-yl)-6,7-dihydro-pyrimido[6,1-a]isoquinolin-4-one,
   2-([1,4]Dioxan-2-ylmethoxy)-9-(6-methyl-pyridin-3-yl)-6,7-dihydro-pyrimido[6,1-a]isoquinolin-4-one,
   2-([1,4]Dioxan-2-ylmethoxy)-9-pyridin-2-ylethynyl-6,7-dihydro-pyrimido[6,1-a]isoquinolin-4-one,
   2-([1,4]Dioxan-2-ylmethoxy)-9-(3-methoxy-prop-1-ynyl)-6,7-dihydro-pyrimido[6,1-a]isoquinolin-4-one,
   5-[2-([1,4]Dioxan-2-ylmethoxy)-4-oxo-6,7-dihydro-4H-pyrimido[6,1-a]isoquinolin-9-yl]-pent-4-ynenitrile,
   2-([1,4]Dioxan-2-ylmethoxy)-9-(3-hydroxy-prop-1-ynyl)-6,7-dihydro-pyrimido[6,1-a]isoquinolin-4-one,
   2-([1,4]Dioxan-2-ylmethoxy)-9-(4-methoxy-phenylethynyl)-6,7-dihydro-pyrimido[6,1-a]isoquinolin-4-one,
   2-([1,4]Dioxan-2-ylmethoxy)-9-pyridin-3-ylethynyl-6,7-dihydro-pyrimido[6,1-a]isoquinolin-4-one,
   4-[2-([1,4]Dioxan-2-ylmethoxy)-4-oxo-6,7-dihydro-4H-pyrimido[6,1-a]isoquinolin-9-yl]-N-methyl-benzamide,
   2-([1,4]Dioxan-2-ylmethoxy)-9-(3-methoxy-phenyl)-6,7-dihydro-pyrimido[6,1-a]isoquinolin-4-one,
   9-(2-Chloro-phenyl)-2-([1,4]dioxan-2-ylmethoxy)-6,7-dihydro-pyrimido[6,1-a]isoquinolin-4-one,
   2-([1,4]Dioxan-2-ylmethoxy)-9-(4-hydroxy-but-1-ynyl)-6,7-dihydro-pyrimido[6,1-a]isoquinolin-4-one,
   9-(1,5-Dimethyl-1H-pyrazol-3-ylmethoxy)-2-([1,4]dioxan-2-ylmethoxy)-6,7-dihydro-pyrimido[6,1-a]isoquinolin-4-one,
   2-([1,4]Dioxan-2-ylmethoxy)-9-(1-methyl- 1H-pyrazol-3-ylmethoxy)-6,7-dihydro-pyrimido[6,1-a]isoquinolin-4-one,
   2-([1,4]Dioxan-2-ylmethoxy)-9-(3-methyl-[1,2,4]oxadiazol-5-ylmethoxy)-6,7-dihydro-pyrimido[6,1-a]isoquinolin-4-one,
   2-([1,4]Dioxan-2-ylmethoxy)-9-(4-morpholin-4-yl-phenyl)-6,7-dihydro-pyrimido[6,1-a]isoquinolin-4-one,
   3-[2-([1,4]Dioxan-2-ylmethoxy)-4-oxo-6,7-dihydro-4H-pyrimido[6,1-a]isoquinolin-9-yl]-4-fluoro-benzamide,
   3-[2-([1,4]Dioxan-2-ylmethoxy)-4-oxo-6,7-dihydro-4H-pyrimido[6,1-a]isoquinolin-9-yl]-5-fluoro-benzamide,
   9-(3,3-Dimethyl-but-1-ynyl)-2-([1,4]dioxan-2-ylmethoxy)-6,7-dihydro-pyrimido[6,1-a]isoquinolin-4-one,
   2-([1,4]Dioxan-2-ylmethoxy)-9-pyridin-4-ylethynyl-6,7-dihydro-pyrimido[6,1-a]isoquinolin-4-one,
   2-([1,4]Dioxan-2-ylmethoxy)-9-(3-methyl-isoxazol-5-ylmethoxy)-6,7-dihydro-pyrimido[6,1-a]isoquinolin-4-one,
   2-([1,4]Dioxan-2-ylmethoxy)-9-(3-hydroxy-3-methyl-but-1-ynyl)-6,7-dihydro-pyrimido[6,1-a]isoquinolin-4-one,
   2-([1,4]Dioxan-2-ylmethoxy)-9-(2-methoxy-pyridin-3-yl)-6,7-dihydro-pyrimido[6,1-a]isoquinolin-4-one,
   2-([1,4]Dioxan-2-ylmethoxy)-6,7-dihydro-pyrimido[6,1-a]isoquinolin-4-one,
   9-(3,6-Dihydro-2H-pyran-4-yl)-2-([1,4]dioxan-2-ylmethoxy)-6,7-dihydro-pyrimido[6,1-a]isoquinolin-4-one,
   5-[2-([1,4]Dioxan-2-ylmethoxy)-4-oxo-6,7-dihydro-4H-pyrimido[6,1-a]isoquinolin-9-yl]-pyridine-2-carbonitrile,
   2-([1,4]Dioxan-2-ylmethoxy)-9-(6-isopropoxy-pyridin-3-yl)-6,7-dihydro-pyrimido[6,1-a]isoquinolin-4-one,
   2-([1,4]Dioxan-2-ylmethoxy)-9-(6-ethoxy-pyridin-3-yl)-6,7-dihydro-pyrimido[6,1-a]isoquinolin-4-one,
   2-([1,4]Dioxan-2-ylmethoxy)-9-(6-morpholin-4-yl-pyridin-3-yl)-6,7-dihydro-pyrimido[6,1-a] isoquinolin-4-one,
   9-(2,3-Dimethoxy-phenyl)-2-([1,4]dioxan-2-ylmethoxy)-6,7-dihydro-pyrimido[6,1-a]isoquinolin-4-one,
   9-(3-Chloro-2-methoxy-pyridin-4-yl)-2-([1,4]dioxan-2-ylmethoxy)-6,7-dihydro-pyrimido[6,1-a]isoquinolin-4-one,
   2-([1,4]Dioxan-2-ylmethoxy)-9-(2-methyl-pyridin-4-yl)-6,7-dihydro-pyrimido[6,1-a]isoquinolin-4-one,
   3-[2-([1,4]Dioxan-2-ylmethoxy)-4-oxo-6,7-dihydro-4H-pyrimido[6,1-a]isoquinolin-9-yl]-isonicotinonitrile,
   9-(2,5-Dimethoxy-phenyl)-2-([1,4]dioxan-2-ylmethoxy)-6,7-dihydro-pyrimido[6,1-a]isoquinolin-4-one,
   2-([1,4]Dioxan-2-ylmethoxy)-9-(3,4,5,6-tetrahydro-2H-[1,2']bipyridinyl-5'-yl)-6,7-dihydro-pyrimido[6,1-a]isoquinolin-4-one,
   2-([1,4]Dioxan-2-ylmethoxy)-9-(2-ethoxy-pyridin-3-yl)-6,7-dihydro-pyrimido[6,1-a]isoquinolin-4-one,
   9-(2,6-Dimethoxy-pyridin-3-yl)-2-([1,4]dioxan-2-ylmethoxy)-6,7-dihydro-pyrimido[6,1-a]isoquinolin-4-one,
   4-[2-([1,4]Dioxan-2-ylmethoxy)-4-oxo-6,7-dihydro-4H-pyrimido[6,1-a]isoquinolin-9-yl]-nicotinonitrile,
   9-tert-Butoxymethyl-2-([1,4]dioxan-2-ylmethoxy)-6,7-dihydro-pyrimido[6,1-a]isoquinolin-4-one,
   2-([1,4]Dioxan-2-ylmethoxy)-9-(2-pyrrolidin-1-yl-pyridin-3-yl)-6,7-dihydro-pyrimido[6,1-a]isoquinolin-4-one,
   2-([1,4]Dioxan-2-ylmethoxy)-9-(6-pyrrolidin-1-yl-pyridin-3-yl)-6,7-dihydro-pyrimido[6,1-a]isoquinolin-4-one,
   2-([1,4]Dioxan-2-ylmethoxy)-9-(5-phenyl-oxazol-2-ylmethoxy)-6,7-dihydro-pyrimido[6,1-a]isoquinolin-4-one,
   9-(5-tert-Butyl-oxazol-2-ylmethoxy)-2-([1,4]dioxan-2-ylmethoxy)-6,7-dihydro-pyrimido[6,1-a]isoquinolin-4-one,
   9-(5-Cyclopropyl-[1,2,4]oxadiazol-3-ylmethoxy)-2-([1,4]dioxan-2-ylmethoxy)-6,7-dihydro-pyrimido[6,1-a]isoquinolin-4-one,
   2-([1,4]Dioxan-2-ylmethoxy)-9-(5-ethyl-[1,2,4]oxadiazol-3-ylmethoxy)-6,7-dihydro-pyrimido[6,1-a]isoquinolin-4-one,
   2-([1,4]Dioxan-2-ylmethoxy)-9-(5-methyl-[1,2,4]oxadiazol-3-ylmethoxy)-6,7-dihydro-pyrimido[6,1-a]isoquinolin-4-one,
   2-([1,4]Dioxan-2-ylmethoxy)-9-(5-isopropyl-[1,2,4]oxadiazol-3-ylmethoxy)-6,7-dihydro-pyrimido[6,1-a]isoquinolin-4-one,
   9-Cyclopentylethynyl-2-([1,4]dioxan-2-ylmethoxy)-6,7-dihydro-pyrimido[6,1-a]isoquinolin-4-one,
   9-Cyclohexylethynyl-2-([1,4]dioxan-2-ylmethoxy)-6,7-dihydro-pyrimido[6,1-a]isoquinolin-4-one,
   2-([1,4]Dioxan-2-ylmethoxy)-9-(3-methyl-but-1-ynyl)-6,7-dihydro-pyrimido[6,1-a]isoquinolin-4-one,
   2-([1,4]Dioxan-2-ylmethoxy)-9-hex-1-ynyl-6,7-dihydro-pyrimido[6,1-a]isoquinolin-4-one,
   9-[3-(Benzyl-methyl-amino)-prop-1-ynyl]-2-([1,4]dioxan-2-ylmethoxy)-6,7-dihydro-pyrimido[6,1-a]isoquinolin-4-one,
   2-([1,4]Dioxan-2-ylmethoxy)-9-(3-hydroxy-5-methyl-hex-1-ynyl)-6,7-dihydro-pyrimido[6,1-a]isoquinolin-4-one,
   2-([1,4]Dioxan-2-ylmethoxy)-9-(3-hydroxy-but-1-ynyl)-6,7-dihydro-pyrimido[6,1-a]isoquinolin-4-one,
   9-Cyclopropyl-2-([1,4]dioxan-2-ylmethoxy)-6,7-dihydro-pyrimido[6,1-a]isoquinolin-4-one,
   2-([1,4]Dioxan-2-ylmethoxy)-9-(3-hydroxy-pent-1-ynyl)-6,7-dihydro-pyrimido[6,1-a]isoquinolin-4-one,
   2-([1,4]Dioxan-2-ylmethoxy)-9-(3-hydroxy-4-methyl-pent-1-ynyl)-6,7-dihydro-pyrimido[6,1-a]isoquinolin-4-one,
   2-([1,4]Dioxan-2-ylmethoxy)-9-(3-ethyl-3-hydroxy-pent-1-ynyl)-6,7-dihydro-pyrimido[6,1-a]isoquinolin-4-one,
   2-([1,4]Dioxan-2-ylmethoxy)-9-(3-hydroxy-3-phenyl-but-1-ynyl)-6,7-dihydro-pyrimido[6,1-a]isoquinolin-4-one,
   9-(3-Benzylamino-prop-1-ynyl)-2-([1,4]dioxan-2-ylmethoxy)-6,7-dihydro-pyrimido[6,1-a]isoquinolin-4-one,
   2-([1,4]Dioxan-2-ylmethoxy)-9-[(furan-2-ylmethyl)-amino]-6,7-dihydro-pyrimido[6,1-a]isoquinolin-4-one,
   2-([1,4]Dioxan-2-ylmethoxy)-9-(1-ethyl-1H-pyrazol-4-yl)-6,7-dihydro-pyrimido[6,1-a]isoquinolin-4-one,
   2-([1,4]Dioxan-2-ylmethoxy)-9-[1-(3-methyl-butyl)-1H-pyrazol-4-yl]-6,7-dihydro-pyrimido[6,1-a]isoquinolin-4-one,
   2-([1,4]Dioxan-2-ylmethoxy)-9-(5-methyl-furan-2-yl)-6,7-dihydro-pyrimido[6,1-a]isoquinolin-4-one,
   2-([1,4]Dioxan-2-ylmethoxy)-9-(3-hydroxy-hex-1-ynyl)-6,7-dihydro-pyrimido[6,1-a]isoquinolin-4-one,
   9-(3,5-Dimethyl-1H-pyrazol-4-yl)-2-([1,4]dioxan-2-ylmethoxy)-6,7-dihydro-pyrimido[6,1-a]isoquinolin-4-one,
   2-([1,4]Dioxan-2-ylmethoxy)-9-(1H-pyrazol-4-yl)-6,7-dihydro-pyrimido[6,1-a]isoquinolin-4-one,
   2-([1,4]Dioxan-2-ylmethoxy)-9-(1-propyl-1H-pyrazol-4-yl)-6,7-dihydro-pyrimido[6,1-a]isoquinolin-4-one,
   2-[2-((R)-1-[1,4]Dioxan-2-ylmethoxy)-4-oxo-6,7-dihydro-4H-pyrimido[6,1-a]isoquinolin-9-yl]-benzonitrile,
   2-[2-((S)-1-[1,4]Dioxan-2-ylmethoxy)-4-oxo-6,7-dihydro-4H-pyrimido[6,1-a]isoquinolin-9-yl]-benzonitrile,
   9-(5-Cyclopropyl-[1,2,4]oxadiazol-3-ylmethoxy)-2-((R)-1-[1,4]dioxan-2-ylmethoxy)-6,7-dihydro-pyrimido [6, 1-a] isoquinolin-4-one,
   2-([1,4]Dioxan-2-ylmethoxy)-9-ethynyl-6,7-dihydro-pyrimido[6,1-a]isoquinolin-4-one,
   2-([1,4]Dioxan-2-ylmethoxy)-9-pyrimidin-2-ylethynyl-6,7-dihydro-pyrimido[6,1-a]isoquinolin-4-one,
   2-([1,4]Dioxan-2-ylmethoxy)-9-(3-phenylamino-prop-1-ynyl)-6,7-dihydro-pyrimido[6,1-a]isoquinolin-4-one,
   2-([1,4]Dioxan-2-ylmethoxy)-9-(3-hydroxy-3-pyridin-3-yl-prop-1-ynyl)-6,7-dihydro-pyrimido[6,1-a]isoquinolin-4-one,
   9-Cyclopentyloxymethyl-2-([1,4]dioxan-2-ylmethoxy)-6,7-dihydro-pyrimido[6,1-a]isoquinolin-4-one,
   2-([1,4]Dioxan-2-ylmethoxy)-9-(3-methoxy-4-methyl-pent-1-ynyl)-6,7-dihydro-pyrimido[6,1-a]isoquinolin-4-one,
   9-Cyclopropylethynyl-2-((R)-1-[1,4]dioxan-2-ylmethoxy)-6,7-dihydro-pyrimido[6,1-a]isoquinolin-4-one,
   2-((S)-1-[1,4]Dioxan-2-ylmethoxy)-9-(3-methyl-but-1-ynyl)-6,7-dihydro-pyrimido[6,1-a]isoquinolin-4-one,
   2-([1,4]Dioxan-2-ylmethoxy)-9-(3-imidazol-1-yl-prop-1-ynyl)-6,7-dihydro-pyrimido[6,1-a]isoquinolin-4-one,
   9-(2-Cyclopropyl-ethyl)-2-((R)-1-[1,4]dioxan-2-ylmethoxy)-6,7-dihydro-pyrimido[6,1-a]isoquinolin-4-one,
   9-Cyclopentyloxymethyl-2-((R)-1-[1,4]dioxan-2-ylmethoxy)-6,7-dihydro-pyrimido[6,1-a]isoquinolin-4-one,
   2-([1,4]Dioxan-2-ylmethoxy)-9-(3-hydroxy-3-pyridin-3-yl-propyl)-6,7-dihydro-pyrimido[6,1-a]isoquinolin-4-one,
   9-Allyloxy-2-((R)-1-[1,4]dioxan-2-ylmethoxy)-6,7-dihydro-pyrimido[6,1-a]isoquinolin-4-one,
   9-Allyloxy-2-((S)-1-[1,4]dioxan-2-ylmethoxy)-6,7-dihydro-pyrimido[6,1-a]isoquinolin-4-one,
   2-((R)-1-[1,4]Dioxan-2-ylmethoxy)-9-(tetrahydro-pyran-4-yloxymethyl)-6,7-dihydro-pyrimido[6,1-a]isoquinolin-4-one,
   2-([1,4]Dioxan-2-ylmethoxy)-9-{3-[(pyridin-3-ylmethyl)-amino]-prop-1-ynyl}-6,7-dihydro-pyrimido[6,1-a]isoquinolin-4-one,
   2-((R)-1-[1,4]Dioxan-2-ylmethoxy)-9-pentyl-6,7-dihydro-pyrimido[6,1-a]isoquinolin-4-one,
   9-Cyclopropylethynyl-2-((S)-1-[1,4]dioxan-2-ylmethoxy)-6,7-dihydro-pyrimido[6,1-a]isoquinolin-4-one,
   9-(2-Cyclopropyl-ethyl)-2-((S)-1-[1,4]dioxan-2-ylmethoxy)-6,7-dihydro-pyrimido[6,1-a]isoquinolin-4-one,
   2-((S)-1-[1,4]Dioxan-2-ylmethoxy)-9-(oxetan-3-yloxymethyl)-6,7-dihydro-pyrimido[6,1-a]isoquinolin-4-one,
   2-((S)-1-[1,4]Dioxan-2-ylmethoxy)-9-(3-methyl-oxetan-3-ylmethoxymethyl)-6,7-dihydro-pyrimido[6,1-a]isoquinolin-4-one,
   9-(2,2-Dimethyl-butylamino)-2-((S)-1-[1,4]dioxan-2-ylmethoxy)-6,7-dihydro-pyrimido[6,1-a]isoquinolin-4-one,
   2-((S)-1-[1,4]Dioxan-2-ylmethoxy)-9-(3-hydroxy-4-methyl-pentyl)-6,7-dihydro-pyrimido[6,1-a]isoquinolin-4-one,
   2-((S)-1-[1,4]Dioxan-2-ylmethoxy)-9-(2-ethyl-hexylamino)-6,7-dihydro-pyrimido[6,1-a]isoquinolin-4-one,
   2-((S)-1-[1,4]Dioxan-2-ylmethoxy)-9-(2-methoxy-ethoxy)-6,7-dihydro-pyrimido[6,1-a]isoquinolin-4-one,
   2-((S)-1-[1,4]Dioxan-2-ylmethoxy)-9-(2-ethoxy-ethoxy)-6,7-dihydro-pyrimido[6,1-a]isoquinolin-4-one,
   9-Cyclopropylmethoxy-2-((S)-1-[1,4]dioxan-2-ylmethoxy)-6,7-dihydro-pyrimido[6,1-a]isoquinolin-4-one,
   2-((S)-1-[1,4]Dioxan-2-ylmethoxy)-9-(2-fluoro-ethoxy)-6,7-dihydro-pyrimido[6,1-a]isoquinolin-4-one,
   2-((S)-1-[1,4]Dioxan-2-ylmethoxy)-9-[3-(2-methoxy-ethoxy)-prop-1-ynyl]-6,7-dihydro-pyrimido[6,1-a]isoquinolin-4-one,
   2-((S)-1-[1,4]Dioxan-2-ylmethoxy)-9-[3-(2-ethoxy-ethoxy)-prop-1-ynyl]-6,7-dihydro-pyrimido[6,1-a]isoquinolin-4-one,
   2-((S)-1-[1,4]Dioxan-2-ylmethoxy)-9-[3-(2-fluoro-ethoxy)-prop-1-ynyl]-6,7-dihydro-pyrimido[6,1-a]isoquinolin-4-one,
   9-(2,2-Dimethyl-propoxymethyl)-2-((S)-1-[1,4]dioxan-2-ylmethoxy)-6,7-dihydro-pyrimido [6,1 - a]isoquinolin-4-one,
   9-Cyclohexyloxymethyl-2-((S)-1-[1,4]dioxan-2-ylmethoxy)-6,7-dihydro-pyrimido[6,1-a]isoquinolin-4-one,
   9-Cyclopropylmethoxymethyl-2-((S)-1-[1,4]dioxan-2-ylmethoxy)-6,7-dihydro-pyrimido[6,1-a]isoquinolin-4-one,
   2-((S)-1-[1,4]Dioxan-2-ylmethoxy)-9-(tetrahydro-pyran-2-ylmethoxy)-6,7-dihydro-pyrimido [6,1 - a]isoquinolin-4-one,
   2-((S)-1-[1,4]Dioxan-2-ylmethoxy)-9-(3-hydroxy-butyl)-6,7-dihydro-pyrimido[6,1-a]isoquinolin-4-one,
   9-(4,4-Dimethyl-pentyloxy)-2-((S)-1-[1,4]dioxan-2-ylmethoxy)-6,7-dihydro-pyrimido[6,1-a]isoquinolin-4-one,
   2-((S)-1-[1,4]Dioxan-2-ylmethoxy)-9-(3-methoxy-4-methyl-pentyl)-6,7-dihydro-pyrimido[6,1-a]isoquinolin-4-one,
   9-(3-Cyclopropyl-propoxy)-2-((S)-1-[1,4]dioxan-2-ylmethoxy)-6,7-dihydro-pyrimido[6,1-a]isoquinolin-4-one,
   9-Cyclohexylamino-2-((S)-1-[1,4]dioxan-2-ylmethoxy)-6,7-dihydro-pyrimido[6,1-a]isoquinolin-4-one,
   2-((S)-1-[1,4]Dioxan-2-ylmethoxy)-9-(3-hydroxy-4,4-dimethyl-pentyl)-6,7-dihydro-pyrimido[6,1-a]isoquinolin-4-one,
   9-Cyclopentylmethoxymethyl-2-((S)-1-[1,4]dioxan-2-ylmethoxy)-6,7-dihydro-pyrimido[6,1-a]isoquinolin-4-one,
   2-((S)-1-[1,4]Dioxan-2-ylmethoxy)-9-(3-methoxy-butyl)-6,7-dihydro-pyrimido[6,1-a]isoquinolin-4-one,
   2-((S)-1-[1,4]Dioxan-2-ylmethoxy)-9-(3-phenylamino-propyl)-6,7-dihydro-pyrimido[6,1-a]isoquinolin-4-one,
   2-((S)-1-[1,4]Dioxan-2-ylmethoxy)-9-(4-hydroxy-pentyl)-6,7-dihydro-pyrimido[6,1-a]isoquinolin-4-one,
   2-((S)-1-[1,4]Dioxan-2-ylmethoxy)-9-(4-hydroxy-butyl)-6,7-dihydro-pyrimido[6,1-a]isoquinolin-4-one,
   9-(Cyclohexyl-methyl-amino)-2-((S)-1-[1,4]dioxan-2-ylmethoxy)-6,7-dihydro-pyrimido[6,1-a]isoquinolin-4-one,
   9-(Cyclohexylmethyl-amino)-2-((S)-1-[1,4]dioxan-2-ylmethoxy)-6,7-dihydro-pyrimido[6,1-a]isoquinolin-4-one,
   2-((S)-1-[1,4]Dioxan-2-ylmethoxy)-9-[(tetrahydro-pyran-4-ylmethyl)-amino]-6,7-dihydro-pyrimido[6,1-a]isoquinolin-4-one,
   2-((S)-1-[1,4]Dioxan-2-ylmethoxy)-9-(3-ethyl-3-hydroxy-pentyl)-6,7-dihydro-pyrimido[6,1-a]isoquinolin-4-one,
   2-((S)-1-[1,4]Dioxan-2-ylmethoxy)-9-(3-hydroxy-3-methyl-butyl)-6,7-dihydro-pyrimido[6,1-a]isoquinolin-4-one,
   2-((S)-1-[1,4]Dioxan-2-ylmethoxy)-9-(3-hydroxy-pentyl)-6,7-dihydro-pyrimido[6,1-a]isoquinolin-4-one,
   9-(2,2-Dimethyl-propoxy)-2-((S)-1-[1,4]dioxan-2-ylmethoxy)-6,7-dihydro-pyrimido[6,1-a]isoquinolin-4-one,
   2-((S)-1-[1,4]Dioxan-2-ylmethoxy)-9-(tetrahydro-pyran-4-ylmethoxy)-6,7-dihydro-pyrimido [6,1 - a]isoquinolin-4-one,
   2-((S)-1-[1,4]Dioxan-2-ylmethoxy)-9-(4-hydroxy-4-methyl-pentyl)-6,7-dihydro-pyrimido[6,1-a]isoquinolin-4-one,
   2-((S)-1-[1,4]Dioxan-2-ylmethoxy)-9-(tetrahydro-pyran-4-ylmethoxymethyl)-6,7-dihydro-pyrimido[6,1-a]isoquinolin-4-one,
   2-([1,4]Dioxan-2-ylmethoxy)-9-methoxy-6,7-dihydro-pyrimido[6,1-a]isoquinolin-4-one,
   2-((S)-1-[1,4]Dioxan-2-ylmethoxy)-9-(oxetan-3 -ylmethoxy)-6,7-dihydro-pyrimido[6,1-a]isoquinolin-4-one,
   9-(3-Cyclopropyl-propoxy)-2-((R)-1-[1,4]dioxan-2-ylmethoxy)-6,7-dihydro-pyrimido[6,1-a]isoquinolin-4-one,
   2-((S)-1-[1,4]Dioxan-2-ylmethoxy)-9-(3-methoxy-propyl)-6,7-dihydro-pyrimido[6,1-a]isoquinolin-4-one,
   2-((S)-1-[1,4]Dioxan-2-ylmethoxy)-9-[2-(1-hydroxy-cyclopentyl)-ethyl]-6,7-dihydro-pyrimido[6,1-a]isoquinolin-4-one,
   2-((R)-1-[1,4]Dioxan-2-ylmethoxy)-9-(4-hydroxy-tetrahydro-pyran-4-ylethynyl)-6,7-dihydro-pyrimido[6,1-a]isoquinolin-4-one.
   2-((R)-1-[1,4]Dioxan-2-ylmethoxy)-9-(3-methoxy-propyl)-6,7-dihydro-pyrimido[6,1-a]isoquinolin-4-one,
   2-((R)-1-[1,4]Dioxan-2-ylmethoxy)-9-[2-(1-hydroxy-cyclopentyl)-ethyl]-6,7-dihydro-pyrimido[6,1-a]isoquinolin-4-one,
   2-((S)-1-[1,4]Dioxan-2-ylmethoxy)-9-(2-propoxy-ethoxy)-6,7-dihydro-pyrimido[6,1-a]isoquinolin-4-one,
   2-((S)-1-[1,4]Dioxan-2-ylmethoxy)-9-(2-isopropoxy-ethoxy)-6,7-dihydro-pyrimido[6,1-a]isoquinolin-4-one,
   2-((R)-1-[1,4]Dioxan-2-ylmethoxy)-9-(2-propoxy-ethoxy)-6,7-dihydro-pyrimido[6,1-a]isoquinolin-4-one,
   2-((R)-1-[1,4]Dioxan-2-ylmethoxy)-9-(2-isopropoxy-ethoxy)-6,7-dihydro-pyrimido[6,1-a]isoquinolin-4-one, and
   2-((S)-1-[1,4]Dioxan-2-ylmethoxy)-9-(4-methoxy-butyl)-6,7-dihydro-pyrimido[6,1-a]isoquinolin-4-one.
9. The compound or pharmaceutically acceptable salt thereof for use according to clause 1, wherein said compound is selected from:
   9-Methoxy-2-(tetrahydro-furan-2-ylmethoxy)-6,7-dihydro-pyrimido[6,1-a]isoquinolin-4-one,
   2-(Chroman-2-ylmethoxy)-9-methoxy-6,7-dihydro-pyrimido[6,1-a]isoquinolin-4-one,
   2-(2,3-Dihydro-benzo[1,4]dioxin-2-ylmethoxy)-9-methoxy-6,7-dihydro-pyrimido[6,1-a]isoquinolin-4-one,
   9-Allyloxy-2-(tetrahydro-furan-2-ylmethoxy)-6,7-dihydro-pyrimido[6,1-a]isoquinolin-4-one,
   2-(2,3-Dihydro-benzofuran-2-ylmethoxy)-9-methoxy-6,7-dihydro-pyrimido[6,1-a]isoquinolin-4-one,
   9-Hydroxy-2-(tetrahydro-furan-2-ylmethoxy)-6,7-dihydro-pyrimido[6,1-a]isoquinolin-4-one,
   9-Benzyloxy-2-(tetrahydro-furan-2-ylmethoxy)-6,7-dihydro-pyrimido[6,1-a]isoquinolin-4-one,
   9-(Pyridin-3-ylmethoxy)-2-(tetrahydro-furan-2-ylmethoxy)-6,7-dihydro-pyrimido[6,1-a]isoquinolin-4-one,
   [4-Oxo-2-(tetrahydro-furan-2-ylmethoxy)-6,7-dihydro-4H-pyrimido[6,1-a]isoquinolin-9-yloxy]-acetonitrile,
   9-Butoxy-2-(tetrahydro-furan-2-ylmethoxy)-6,7-dihydro-pyrimido[6,1-a]isoquinolin-4-one,
   9-Cyclopropylmethoxy-2-(tetrahydro-furan-2-ylmethoxy)-6,7-dihydro-pyrimido[6,1-a]isoquinolin-4-one,
   9-Phenethyloxy-2-(tetrahydro-furan-2-ylmethoxy)-6,7-dihydro-pyrimido[6,1-a]isoquinolin-4-one,
   9-(Pyridin-4-ylmethoxy)-2-(tetrahydro-furan-2-ylmethoxy)-6,7-dihydro-pyrimido[6,1-a]isoquinolin-4-one,
   9-(Pyridin-2-ylmethoxy)-2-(tetrahydro-furan-2-ylmethoxy)-6,7-dihydro-pyrimido[6,1-a]isoquinolin-4-one,
   9-(Pyridin-2-ylmethoxy)-2-(tetrahydro-furan-2-ylmethoxy)-6,7-dihydro-pyrimido[6,1-a]isoquinolin-4-one,
   9-(2-Phenoxy-ethoxy)-2-(tetrahydro-furan-2-ylmethoxy)-6,7-dihydro-pyrimido[6,1-a]isoquinolin-4-one,
   9-Methoxy-2-(tetrahydro-pyran-4-ylmethoxy)-6,7-dihydro-pyrimido[6,1-a]isoquinolin-4-one,
   9-Methoxy-2-(tetrahydro-pyran-2-ylmethoxy)-6,7-dihydro-pyrimido[6,1-a]isoquinolin-4-one,
   9-Methoxy-2-(tetrahydro-pyran-2-ylmethoxy)-6,7-dihydro-pyrimido[6,1-a]isoquinolin-4-one,
   4-[4-Oxo-2-(tetrahydro-furan-2-ylmethoxy)-6,7-dihydro-4H-pyrimido[6,1-a]isoquinolin-9-yloxymethyl] -benzonitrile,
   4-[4-Oxo-2-(tetrahydro-furan-2-ylmethoxy)-6,7-dihydro-4H-pyrimido[6,1-a]isoquinolin-9-yloxymethyl] -benzonitrile,
   9-(2,3-Dihydro-benzo[1,4]dioxin-2-ylmethoxy)-2-(tetrahydro-furan-2-ylmethoxy)-6,7-dihydro-pyrimido[6,1-a]isoquinolin-4-one,
   9-(2,3-Dihydro-benzo[1,4]dioxin-2-ylmethoxy)-2-(tetrahydro-furan-2-ylmethoxy)-6,7-dihydro-pyrimido[6,1-a]isoquinolin-4-one,
   3-[4-Oxo-2-(tetrahydro-furan-2-ylmethoxy)-6,7-dihydro-4H-pyrimido[6,1-a]isoquinolin-9-yloxymethyl]-benzonitrile,
   2-[4-Oxo-2-(tetrahydro-furan-2-ylmethoxy)-6,7-dihydro-4H-pyrimido[6,1-a]isoquinolin-9-yloxymethyl] -benzonitrile,
   9-(4-Chloro-benzyloxy)-2-(tetrahydro-furan-2-ylmethoxy)-6,7-dihydro-pyrimido[6,1-a]isoquinolin-4-one,
   9-(3-Chloro-benzyloxy)-2-(tetrahydro-furan-2-ylmethoxy)-6,7-dihydro-pyrimido[6,1-a]isoquinolin-4-one,
   9-(2-Chloro-benzyloxy)-2-(tetrahydro-furan-2-ylmethoxy)-6,7-dihydro-pyrimido[6,1-a] isoquinolin-4-one,
   9-(4-Fluoro-benzyloxy)-2-(tetrahydro-furan-2-ylmethoxy)-6,7-dihydro-pyrimido[6,1-a]isoquinolin-4-one,
   9-(2-Nitro-benzyloxy)-2-(tetrahydro-furan-2-ylmethoxy)-6,7-dihydro-pyrimido[6,1-a]isoquinolin-4-one,
   4-[4-Oxo-2-(tetrahydro-furan-2-ylmethoxy)-6,7-dihydro-4H-pyrimido[6,1-a]isoquinolin-9-yloxymethyl]-benzoic acid methyl ester,
   3-[4-Oxo-2-(tetrahydro-furan-2-ylmethoxy)-6,7-dihydro-4H-pyrimido[6,1-a]isoquinolin-9-yloxymethyl]-benzoic acid methyl ester,
   3-[4-Oxo-2-(tetrahydro-furan-2-ylmethoxy)-6,7-dihydro-4H-pyrimido[6,1-a]isoquinolin-9-yloxymethyl]-benzoic acid methyl ester,
   2-(2,3-Dihydro-benzo[1,4]dioxin-2-ylmethoxy)-9-(pyridin-2-ylmethoxy)-6,7-dihydro-pyrimido [6,1 -a] isoquinolin-4-one,
   [2-(2,3-Dihydro-benzo[1,4]dioxin-2-ylmethoxy)-4-oxo-6,7-dihydro-4H-pyrimido[6,1-a] isoquinolin-9-yloxy]-acetic acid tert-butyl ester,
   2,9-Bis-(2,3-dihydro-benzo[1,4]dioxin-2-ylmethoxy)-6,7-dihydro-pyrimido[6,1-a] isoquinolin-4-one,
   [2-(2,3-Dihydro-benzo[1,4] dioxin-2-ylmethoxy)-4-oxo-6,7-dihydro-4H-pyrimido [6,1-a] isoquinolin-9-yloxy] -acetic acid,
   9-(3-Nitro-benzyloxy)-2-(tetrahydro-furan-2-ylmethoxy)-6,7-dihydro-pyrimido[6,1-a]isoquinolin-4-one,
   9-(3-Nitro-benzyloxy)-2-(tetrahydro-furan-2-ylmethoxy)-6,7-dihydro-pyrimido[6,1-a]isoquinolin-4-one,
   9-(4-Nitro-benzyloxy)-2-(tetrahydro-furan-2-ylmethoxy)-6,7-dihydro-pyrimido[6,1-a]isoquinolin-4-one,
   9-(3-Methyl-benzyloxy)-2-(tetrahydro-furan-2-ylmethoxy)-6,7-dihydro-pyrimido[6,1-a]isoquinolin-4-one,
   9-(4-Methyl-benzyloxy)-2-(tetrahydro-furan-2-ylmethoxy)-6,7-dihydro-pyrimido[6,1-a]isoquinolin-4-one,
   9-(4-Methoxy-benzyloxy)-2-(tetrahydro-furan-2-ylmethoxy)-6,7-dihydro-pyrimido[6,1-a]isoquinolin-4-one,
   9-(Naphthalen-2-ylmethoxy)-2-(tetrahydro-furan-2-ylmethoxy)-6,7-dihydro-pyrimido[6,1-a]isoquinolin-4-one,
   9-(Naphthalen-1-ylmethoxy)-2-(tetrahydro-furan-2-ylmethoxy)-6,7-dihydro-pyrimido[6,1-a]isoquinolin-4-one,
   9-(2-Methyl-benzyloxy)-2-(tetrahydro-furan-2-ylmethoxy)-6,7-dihydro-pyrimido[6,1-a]isoquinolin-4-one,
   9-[2-(2-Methoxy-phenoxy)-ethoxy]-2-(tetrahydro-furan-2-ylmethoxy)-6,7-dihydro-pyrimido[6,1-a]isoquinolin-4-one,
   9-[2-(3-Methoxy-phenoxy)-ethoxy]-2-(tetrahydro-furan-2-ylmethoxy)-6,7-dihydro-pyrimido[6,1-a]isoquinolin-4-one,
   9-[2-(4-Methoxy-phenoxy)-ethoxy]-2-(tetrahydro-furan-2-ylmethoxy)-6,7-dihydro-pyrimido[6,1-a]isoquinolin-4-one,
   9-[2-(2-Chloro-phenoxy)-ethoxy]-2-(tetrahydro-furan-2-ylmethoxy)-6,7-dihydro-pyrimido[6,1-a]isoquinolin-4-one,
   9-[2-(3-Chloro-phenoxy)-ethoxy]-2-(tetrahydro-furan-2-ylmethoxy)-6,7-dihydro-pyrimido[6,1-a]isoquinolin-4-one,
   9-[2-(4-Chloro-phenoxy)-ethoxy]-2-(tetrahydro-furan-2-ylmethoxy)-6,7-dihydro-pyrimido[6,1-a]isoquinolin-4-one,
   2-(2,3-Dihydro-benzo[1,4]dioxin-2-ylmethoxy)-9-(2-morpholin-4-yl-2-oxo-ethoxy)-6,7-dihydro-pyrimido[6,1-a]isoquinolin-4-one,
   2-(2,3-Dihydro-benzo[1,4]dioxin-2-ylmethoxy)-9-(2-morpholin-4-yl-2-oxo-ethoxy)-6,7-dihydro-pyrimido[6,1-a]isoquinolin-4-one,
   2-(2,3-Dihydro-benzo[1,4]dioxin-2-ylmethoxy)-9-(2-morpholin-4-yl-2-oxo-ethoxy)-6,7-dihydro-pyrimido[6,1-a]isoquinolin-4-one,
   2-(2,3-Dihydro-benzo[1,4]dioxin-2-ylmethoxy)-9-(2-morpholin-4-yl-2-oxo-ethoxy)-6,7-dihydro-pyrimido[6,1-a]isoquinolin-4-one,
   2-[2-(2,3-Dihydro-benzo[1,4]dioxin-2-ylmethoxy)-4-oxo-6,7-dihydro-4H-pyrimido[6,1-a]isoquinolin-9-yloxy]-acetamide,
   2-[2-(2,3-Dihydro-benzo[1,4]dioxin-2-ylmethoxy)-4-oxo-6,7-dihydro-4H-pyrimido[6,1-a]isoquinolin-9-yloxy]-N,N-dimethyl-acetamide,
   9-(2,2-Dimethoxy-ethoxy)-2-(tetrahydro-furan-2-ylmethoxy)-6,7-dihydro-pyrimido[6,1-a]isoquinolin-4-one,
   2-[2-(2,3-Dihydro-benzo[1,4]dioxin-2-ylmethoxy)-4-oxo-6,7-dihydro-4H-pyrimido[6,1-a]isoquinolin-9-yloxy]-2-methyl-propionamide,
   2-(2,3-Dihydro-benzo[1,4]dioxin-2-ylmethoxy)-9-(1,1-dimethyl-2-morpholin-4-yl-2-oxo-ethoxy)-6,7-dihydro-pyrimido[6,1-a]isoquinolin-4-one,
   9-(2-Benzyloxy-ethoxy)-2-(tetrahydro-furan-2-ylmethoxy)-6,7-dihydro-pyrimido[6,1-a]isoquinolin-4-one,
   2,9-Bis-(tetrahydro-furan-2-ylmethoxy)-6,7-dihydro-pyrimido[6,1-a]isoquinolin-4-one,
   9-(6-Phenyl-pyridin-2-ylmethoxy)-2-(tetrahydro-furan-2-ylmethoxy)-6,7-dihydro-pyrimido[6,1-a]isoquinolin-4-one,
   2-(2,3-Dihydro-benzo[1,4]dioxin-2-ylmethoxy)-9-(tetrahydro-furan-2-ylmethoxy)-6,7-dihydro-pyrimido[6,1-a]isoquinolin-4-one,
   9-[6-(1-Methyl-1H-pyrazol-4-yl)-pyridin-2-ylmethoxy]-2-(tetrahydro-furan-2-ylmethoxy)-6,7-dihydro-pyrimido [6, 1-a] isoquinolin-4-one,
   9-(6-Furan-3-yl-pyridin-2-ylmethoxy)-2-(tetrahydro-furan-2-ylmethoxy)-6,7-dihydro-pyrimido[6,1-a]isoquinolin-4-one,
   9-(6-Pyrimidin-5-yl-pyridin-2-ylmethoxy)-2-(tetrahydro-furan-2-ylmethoxy)-6,7-dihydro-pyrimido[6,1-a]isoquinolin-4-one,
   9-(1-Cyclopropyl-1H-tetrazol-5-ylmethoxy)-2-(tetrahydro-furan-2-ylmethoxy)-6,7-dihydro-pyrimido[6,1-a]isoquinolin-4-one,
   2-[4-Oxo-2-(tetrahydro-furan-2-ylmethoxy)-6,7-dihydro-4H-pyrimido[6,1-a]isoquinolin-9-yloxy]-acetamide,
   N,N-Diethyl-2-[4-oxo-2-(tetrahydro-furan-2-ylmethoxy)-6,7-dihydro-4H-pyrimido[6,1-a]isoquinolin-9-yloxy]-acetamide,
   N,N-Dimethyl-2-[4-oxo-2-(tetrahydro-furan-2-ylmethoxy)-6,7-dihydro-4H-pyrimido[6,1-a]isoquinolin-9-yloxy]-acetamide,
   N-Isopropyl-N-methyl-2-[4-oxo-2-(tetrahydro-furan-2-ylmethoxy)-6,7-dihydro-4H-pyrimido[6,1-a]isoquinolin-9-yloxy]-acetamide,
   2-[4-Oxo-2-(tetrahydro-furan-2-ylmethoxy)-6,7-dihydro-4H-pyrimido[6,1-a]isoquinolin-9-yloxy]-N-phenyl-acetamide,
   9-(1-Propyl-1H-tetrazol-5-ylmethoxy)-2-(tetrahydro-furan-2-ylmethoxy)-6,7-dihydro-pyrimido[6,1-a]isoquinolin-4-one,
   9-(Oxazol-2-ylmethoxy)-2-(tetrahydro-furan-2-ylmethoxy)-6,7-dihydro-pyrimido[6,1-a]isoquinolin-4-one,
   2-[2-(2,3-Dihydro-benzo[1,4]dioxin-2-ylmethoxy)-4-oxo-6,7-dihydro-4H-pyrimido[6,1-a]isoquinolin-9-yloxy]-N,N-diethyl-acetamide,
   2-[2-(2,3-Dihydro-benzo[1,4]dioxin-2-ylmethoxy)-4-oxo-6,7-dihydro-4H-pyrimido[6,1-a]isoquinolin-9-yloxy]-N-isopropyl-N-methyl-acetamide,
   2-[2-(2,3-Dihydro-benzo[1,4]dioxin-2-ylmethoxy)-4-oxo-6,7-dihydro-4H-pyrimido[6,1-a]isoquinolin-9-yloxy]-N-phenyl-acetamide,
   2-(2,3-Dihydro-benzo[1,4]dioxin-2-ylmethoxy)-9-(1-propyl-1H-tetrazol-5-ylmethoxy)-6,7-dihydro-pyrimido [6, 1-a] isoquinolin-4-one,
   9-(1-Butyl-1H-tetrazol-5-ylmethoxy-ylmethoxy)-2-(2,3-dihydro-benzo[1,4] dioxin-2-ylmethoxy)-6,7-dihydro-pyrimido [6, 1-a] isoquinolin-4-one,
   2-(2,3-Dihydro-benzo[1,4]dioxin-2-ylmethoxy)-9-(2-morpholin-4-yl-ethoxy)-6,7-dihydro-pyrimido[6,1-a]isoquinolin-4-one,
   [2-(2,3-Dihydro-benzo[1,4] dioxin-2-ylmethoxy)-4-oxo-6,7-dihydro-4H-pyrimido [6,1-a]isoquinolin-9-yloxy]-acetonitrile,
   2-(2,3-Dihydro-benzo[1,4]dioxin-2-ylmethoxy)-9-(oxazol-2-ylmethoxy)-6,7-dihydro-pyrimido[6,1-a]isoquinolin-4-one,
   2-(2,3-Dihydro-benzo[1,4]dioxin-2-ylmethoxy)-9-(2-oxo-2-pyrrolidin-1-yl-ethoxy)-6,7-dihydro-pyrimido[6,1-a]isoquinolin-4-one,
   9-(1-Cyclopropyl-1H-tetrazol-5-ylmethoxy)-2-(2,3-dihydro-benzo[1,4] dioxin-2-ylmethoxy)-6,7-dihydro-pyrimido [6, 1-a] isoquinolin-4-one,
   2-(2,3-Dihydro-benzo[1,4]dioxin-2-ylmethoxy)-9-(1H-tetrazol-5-ylmethoxy)-6,7-dihydro-pyrimido[6,1-a]isoquinolin-4-one,
   9-Allyloxy-2-(2,3-dihydro-[1,4]dioxino[2,3-b]pyridin-2-ylmethoxy)-6,7-dihydro-pyrimido[6,1-a]isoquinolin-4-one,
   N-Benzyl-2-[2-(2,3-dihydro-benzo[1,4]dioxin-2-ylmethoxy)-4-oxo-6,7-dihydro-4H-pyrimido [6, 1-a] isoquinolin-9-yloxy] -acetamide,
   2-(2,3-Dihydro-benzo[1,4]dioxin-2-ylmethoxy)-9-(2-pyrrolidin-1-yl-ethoxy)-6,7-dihydro-pyrimido[6,1-a]isoquinolin-4-one,
   2-(2,3-Dihydro-benzo[1,4]dioxin-2-ylmethoxy)-9-(2-piperidin-1-yl-ethoxy)-6,7-dihydro-pyrimido[6,1-a]isoquinolin-4-one,
   2-(2,3-Dihydro-benzo[1,4]dioxin-2-ylmethoxy)-9-(3-piperidin-1-yl-propoxy)-6,7-dihydro-pyrimido[6,1-a]isoquinolin-4-one,
   2-(2,3-Dihydro-benzo[1,4]dioxin-2-ylmethoxy)-9-(3-dimethylamino-propoxy)-6,7-dihydro-pyrimido[6,1-a]isoquinolin-4-one,
   2-(2,3-Dihydro-benzo[1,4]dioxin-2-ylmethoxy)-9-[2-(1-methyl-pyrrolidin-2-yl)-ethoxy]-6,7-dihydro-pyrimido[6,1-a]isoquinolin-4-one,
   2-(2,3-Dihydro-benzo[1,4]dioxin-2-ylmethoxy)-9-[3-(4-methyl-piperazin-1-yl)-propoxy]-6,7-dihydro-pyrimido[6,1-a] isoquinolin-4-one,
   5-[2-(2,3-Dihydro-benzo[1,4]dioxin-2-ylmethoxy)-4-oxo-6,7-dihydro-4H-pyrimido[6,1-a]isoquinolin-9-yloxymethyl]-furan-2-carboxylic acid ethyl ester,
   2-(2,3-Dihydro-[1,4]dioxino[2,3-b]pyridin-2-ylmethoxy)-9-(oxazol-2-ylmethoxy)-6,7-dihydro-pyrimido [6,1 -a] isoquinolin-4-one,
   2-(2,3-Dihydro-[1,4]dioxino[2,3-b]pyridin-2-ylmethoxy)-9-(oxazol-2-ylmethoxy)-6,7-dihydro-pyrimido [6,1 -a] isoquinolin-4-one,
   2-(2,3-Dihydro-[1,4]dioxino[2,3-b]pyridin-2-ylmethoxy)-9-(oxazol-2-ylmethoxy)-6,7-dihydro-pyrimido [6,1 -a] isoquinolin-4-one,
   2-(2,3-Dihydro-[1,4]dioxino[2,3-b]pyridin-2-ylmethoxy)-9-(oxazol-2-ylmethoxy)-6,7-dihydro-pyrimido [6,1 -a] isoquinolin-4-one,
   9-(5-tert-Butyl-[1,2,4]oxadiazol-3-ylmethoxy)-2-(tetrahydro-furan-2-ylmethoxy)-6,7-dihydro-pyrimido [6,1 -a] isoquinolin-4-one,
   9-(5-Phenyl-[1,2,4]oxadiazol-3-ylmethoxy)-2-(tetrahydro-furan-2-ylmethoxy)-6,7-dihydro-pyrimido [6,1 -a] isoquinolin-4-one,
   [2-(2,3-Dihydro-[1,4]dioxino[2,3-b]pyridin-2-ylmethoxy)-4-oxo-6,7-dihydro-4H-pyrimido[6,1-a] isoquinolin-9-yloxy]-acetonitrile,
   [2-(2,3-Dihydro-[1,4]dioxino[2,3-b]pyridin-2-ylmethoxy)-4-oxo-6,7-dihydro-4H-pyrimido[6,1-a] isoquinolin-9-yloxy]-acetonitrile,
   [2-(2,3-Dihydro-[1,4]dioxino[2,3-b]pyridin-2-ylmethoxy)-4-oxo-6,7-dihydro-4H-pyrimido[6,1-a]isoquinolin-9-yloxy]-acetonitrile,
   2-(2,3-Dihydro-[1,4]dioxino[2,3-b]pyridin-2-ylmethoxy)-9-(2-morpholin-4-yl-ethoxy)-6,7-dihydro-pyrimido[6,1-a]isoquinolin-4-one,
   2-(2,3-Dihydro-[1,4]dioxino[2,3-b]pyridin-2-ylmethoxy)-9-(pyridin-2-ylmethoxy)-6,7-dihydro-pyrimido [6,1 -a] isoquinolin-4-one,
   2-(2,3-Dihydro-[1,4]dioxino[2,3-b]pyridin-2-ylmethoxy)-9-[2-(1-methyl-pyrrolidin-2-yl)-ethoxy] -6,7-dihydro-pyrimido [6,1 -a] isoquinolin-4-one,
   2-(2,3-Dihydro-[1,4]dioxino[2,3-b]pyridin-2-ylmethoxy)-9-(1H-tetrazol-5-ylmethoxy)-6,7-dihydro-pyrimido[6,1-a] isoquinolin-4-one,
   9-Pyridin-3-yl-2-(tetrahydro-furan-2-ylmethoxy)-6,7-dihydro-pyrimido[6,1-a]isoquinolin-4-one,
   3-[4-Oxo-2-(tetrahydro-furan-2-ylmethoxy)-6,7-dihydro-4H-pyrimido[6,1-a]isoquinolin-9-yl]-benzonitrile,
   9-Phenyl-2-(tetrahydro-furan-2-ylmethoxy)-6,7-dihydro-pyrimido[6,1-a]isoquinolin-4-one,
   2-(2,3-Dihydro-[1,4]dioxino[2,3-b]pyridin-2-ylmethoxy)-9-pyridin-4-yl-6,7-dihydro-pyrimido[6,1-a]isoquinolin-4-one,
   3-[2-(2,3-Dihydro-[1,4]dioxino[2,3-b]pyridin-2-ylmethoxy)-4-oxo-6,7-dihydro-4H-pyrimido[6,1-a] isoquinolin-9-yl] -benzonitrile,
   9-(2-Methoxy-phenyl)-2-(tetrahydro-furan-2-ylmethoxy)-6,7-dihydro-pyrimido[6,1-a]isoquinolin-4-one,
   9-(3-Methoxy-phenyl)-2-(tetrahydro-furan-2-ylmethoxy)-6,7-dihydro-pyrimido[6,1-a]isoquinolin-4-one,
   9-(4-Methoxy-phenyl)-2-(tetrahydro-furan-2-ylmethoxy)-6,7-dihydro-pyrimido[6,1-a]isoquinolin-4-one,
   4-[4-Oxo-2-(tetrahydro-furan-2-ylmethoxy)-6,7-dihydro-4H-pyrimido[6,1-a]isoquinolin-9-yl]-benzonitrile,
   3-[4-Oxo-2-(tetrahydro-furan-2-ylmethoxy)-6,7-dihydro-4H-pyrimido[6,1-a]isoquinolin-9-yl]-benzoic acid,
   4-[4-Oxo-2-(tetrahydro-furan-2-ylmethoxy)-6,7-dihydro-4H-pyrimido[6,1-a]isoquinolin-9-yl]-benzoic acid,
   9-(4-Dimethylamino-phenyl)-2-(tetrahydro-furan-2-ylmethoxy)-6,7-dihydro-pyrimido[6,1-a]isoquinolin-4-one,
   4-[4-Oxo-2-(tetrahydro-furan-2-ylmethoxy)-6,7-dihydro-4H-pyrimido[6,1-a]isoquinolin-9-yl]-benzamide,
   2-[4-Oxo-2-(tetrahydro-furan-2-ylmethoxy)-6,7-dihydro-4H-pyrimido[6,1-a]isoquinolin-9-yl]-benzonitrile,
   9-(1-Methyl-1H-pyrazol-4-yl)-2-(tetrahydro-furan-2-ylmethoxy)-6,7-dihydro-pyrimido[6,1-a]isoquinolin-4-one,
   N,N-Dimethyl-3-[4-oxo-2-(tetrahydro-furan-2-ylmethoxy)-6,7-dihydro-4H-pyrimido[6,1-a] isoquinolin-9-yl] -benzamide,
   9-(6-Methoxy-pyridin-3-yl)-2-(tetrahydro-furan-2-ylmethoxy)-6,7-dihydro-pyrimido[6,1-a]isoquinolin-4-one,
   9-(2,6-Dimethyl-phenyl)-2-(tetrahydro-furan-2-ylmethoxy)-6,7-dihydro-pyrimido[6,1-a]isoquinolin-4-one,
   9-(3,5-Dimethyl-isoxazol-4-yl)-2-(tetrahydro-furan-2-ylmethoxy)-6,7-dihydro-pyrimido[6,1-a]isoquinolin-4-one,
   9-Naphthalen-2-yl-2-(tetrahydro-furan-2-ylmethoxy)-6,7-dihydro-pyrimido[6,1-a]isoquinolin-4-one,
   9-Naphthalen-1-yl-2-(tetrahydro-furan-2-ylmethoxy)-6,7-dihydro-pyrimido[6,1-a]isoquinolin-4-one,
   9-Pyrimidin-5-yl-2-(tetrahydro-furan-2-ylmethoxy)-6,7-dihydro-pyrimido [6,1-a] isoquinolin-4-one,
   9-(5-Chloro-thiophen-2-yl)-2-(tetrahydro-furan-2-ylmethoxy)-6,7-dihydro-pyrimido[6,1-a]isoquinolin-4-one,
   2-[4-Oxo-2-(tetrahydro-furan-2-ylmethoxy)-6,7-dihydro-4H-pyrimido[6,1-a]isoquinolin-9-yl]-pyrrole-1-carboxylic acid tert-butyl ester,
   Trifluoro-methanesulfonic acid 4-oxo-2-(tetrahydro-furan-2-ylmethoxy)-6,7-dihydro-4H-pyrimido[6,1-a]isoquinolin-9-yl ester,
   2-(2,3-Dihydro-[1,4]dioxino[2,3-b]pyridin-2-ylmethoxy)-9-(6-methoxy-pyridin-3-yl)-6,7-dihydro-pyrimido [6, 1-a] isoquinolin-4-one,
   9-Cyclopropylethynyl-2-(tetrahydro-furan-2-ylmethoxy)-6,7-dihydro-pyrimido[6,1-a]isoquinolin-4-one,
   9-(3,3-Dimethyl-but-1-ynyl)-2-(tetrahydro-furan-2-ylmethoxy)-6,7-dihydro-pyrimido [6,1 - a]isoquinolin-4-one,
   2-(2,3-Dihydro-[1,4]dioxino[2,3-b]pyridin-2-ylmethoxy)-9-(pyridin-4-ylmethoxy)-6,7-dihydro-pyrimido[6,1-a]isoquinolin-4-one,
   2-(2,3-Dihydro-[1,4]dioxino[2,3-b]pyridin-2-ylmethoxy)-9-pyridin-3-yl-6,7-dihydro-pyrimido[6,1-a]isoquinolin-4-one,
   2-[2-(2,3-Dihydro-[1,4]dioxino[2,3-b]pyridin-2-ylmethoxy)-4-oxo-6,7-dihydro-4H-pyrimido[6,1-a] isoquinolin-9-yl] -benzonitrile,
   2-(2,3-Dihydro-[1,4]dioxino[2,3-b]pyridin-2-ylmethoxy)-9-(2-methoxy-phenyl)-6,7-dihydro-pyrimido[6,1-a]isoquinolin-4-one,
   2-(2,3-Dihydro-[1,4]dioxino[2,3-b]pyridin-2-ylmethoxy)-9-(1H-indazol-5-yl)-6,7-dihydro-pyrimido[6,1-a]isoquinolin-4-one,
   2-(2,3-Dihydro-[1,4]dioxino[2,3-b]pyridin-2-ylmethoxy)-9-pyrimidin-5-yl-6,7-dihydro-pyrimido[6,1-a]isoquinolin-4-one,
   3-[2-(2,3-Dihydro-[1,4]dioxino[2,3-b]pyridin-2-ylmethoxy)-4-oxo-6,7-dihydro-4H-pyrimido[6,1-a] isoquinolin-9-yl] -benzamide,
   2-(2,3-Dihydro-[1,4]dioxino[2,3-b]pyridin-2-ylmethoxy)-9-(2-dimethylamino-phenyl)-6,7-dihydro-pyrimido [6, 1-a] isoquinolin-4-one,
   2-(2,3-Dihydro-[1,4]dioxino[2,3-b]pyridin-2-ylmethoxy)-9-pyridin-3-ylethynyl-6,7-dihydro-pyrimido[6,1-a]isoquinolin-4-one,
   2-(2,3-Dihydro-[1,4]dioxino[2,3-b]pyridin-2-ylmethoxy)-9-(3-methoxy-prop-1-ynyl)-6,7-dihydro-pyrimido [6, 1-a] isoquinolin-4-one,
   2-(2,3-Dihydro-[1,4]dioxino[2,3-b]pyridin-2-ylmethoxy)-9-(4-hydroxy-but-1-ynyl)-6,7-dihydro-pyrimido[6,1-a]isoquinolin-4-one,
   2-(2,3-Dihydro-[1,4]dioxino[2,3-b]pyridin-2-ylmethoxy)-9-(1,5-dimethyl-1H-pyrazol-3-ylmethoxy)-6,7-dihydro-pyrimido[6,1-a]isoquinolin-4-one,
   2-(2,3-Dihydro-[1,4]dioxino[2,3-b]pyridin-2-ylmethoxy)-9-(3-methyl-[1,2,4]oxadiazol-5-ylmethoxy)-6,7-dihydro-pyrimido[6,1-a]isoquinolin-4-one,
   2-(2,3-Dihydro-[1,4]dioxino[2,3-b]pyridin-2-ylmethoxy)-9-(3-hydroxy-3-methyl-but-1-ynyl)-6,7-dihydro-pyrimido[6,1-a]isoquinolin-4-one,
   2-(2,3-Dihydro-[1,4]dioxino[2,3-b]pyridin-2-ylmethoxy)-9-pyridin-4-ylethynyl-6,7-dihydro-pyrimido[6,1-a]isoquinolin-4-one,
   2-(2,3-Dihydro-[1,4]dioxino[2,3-b]pyridin-2-ylmethoxy)-9-(3-hydroxy-prop-1-ynyl)-6,7-dihydro-pyrimido[6,1-a]isoquinolin-4-one,
   2-(2,3-Dihydro-[1,4]dioxino[2,3-b]pyridin-2-ylmethoxy)-9-(6-methyl-pyridin-3-yl)-6,7-dihydro-pyrimido[6,1-a]isoquinolin-4-one,
   2-(2,3-Dihydro-[1,4]dioxino[2,3-b]pyridin-2-ylmethoxy)-9-(5-methoxy-pyridin-3-yl)-6,7-dihydro-pyrimido[6,1-a]isoquinolin-4-one,
   9-Cyclopropylethynyl-2-(2,3-dihydro-[1,4]dioxino[2,3-b]pyridin-2-ylmethoxy)-6,7-dihydro-pyrimido[6,1-a]isoquinolin-4-one,
   9-Cyclopropylethynyl-2-(2,3-dihydro-[1,4]dioxino[2,3-b]pyridin-2-ylmethoxy)-6,7-dihydro-pyrimido[6,1-a]isoquinolin-4-one,
   2-(2,3-Dihydro-[1,4]dioxino[2,3-b]pyridin-2-ylmethoxy)-9-(1-hydroxy-cyclopentylethynyl)-6,7-dihydro-pyrimido[6,1-a]isoquinolin-4-one,
   5-[2-(2,3-Dihydro-[1,4]dioxino[2,3-b]pyridin-2-ylmethoxy)-4-oxo-6,7-dihydro-4H-pyrimido[6,1-a] isoquinolin-9-yl] -pent-4-ynenitrile,
   2-(2,3-Dihydro-[1,4]dioxino[2,3-b]pyridin-2-ylmethoxy)-9-(3,3-dimethyl-but-1-ynyl)-6,7-dihydro-pyrimido[6,1-a] isoquinolin-4-one,
   2-(2,3-Dihydro-[1,4]dioxino[2,3-b]pyridin-2-ylmethoxy)-9-(2-methoxy-pyridin-3-yl)-6,7-dihydro-pyrimido[6,1-a] isoquinolin-4-one,
   2-(2,3-Dihydro-[1,4]dioxino[2,3-b]pyridin-2-ylmethoxy)-6,7-dihydro-pyrimido[6,1-a]isoquinolin-4-one,
   5-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-pyridine-2-carboxylic acid methylamide,
   2-(2,3-Dihydro-[1,4]dioxino[2,3-b]pyridin-2-ylmethoxy)-9-furan-3-yl-6,7-dihydro-pyrimido[6,1-a]isoquinolin-4-one,
   2-(2,3-Dihydro-[1,4]dioxino[2,3-b]pyridin-2-ylmethoxy)-9-(1-methyl-1H-pyrazol-4-yl)-6,7-dihydro-pyrimido[6,1-a] isoquinolin-4-one,
   2-(2,3-Dihydro-[1,4]dioxino[2,3-b]pyridin-2-ylmethoxy)-9-morpholin-4-ylmethyl-6,7-dihydro-pyrimido [6,1 -a] isoquinolin-4-one,
   [2-(2,3-Dihydro-[1,4]dioxino[2,3-b]pyridin-2-ylmethoxy)-4-oxo-6,7-dihydro-4H-pyrimido[6,1-a]isoquinolin-9-ylamino]-acetonitrile,
   2-(2,3-Dihydro-[1,4]dioxino[2,3-b]pyridin-2-ylmethoxy)-4-oxo-6,7-dihydro-4H-pyrimido[6,1-a]isoquinoline-9-carbonitrile,
   2-(2,3-Dihydro-[1,4]dioxino[2,3-b]pyridin-2-ylmethoxy)-9-[(oxazol-2-ylmethyl)-amino]-6,7-dihydro-pyrimido [6, 1-a] isoquinolin-4-one,
   2-(2,3-Dihydro-[1,4]dioxino[2,3-b]pyridin-2-ylmethoxy)-9-(5-methyl-[1,2,4]oxadiazol-3-ylmethoxy)-6,7-dihydro-pyrimido[6,1-a]isoquinolin-4-one,
   2-(2,3-Dihydro-[1,4]dioxino[2,3-b]pyridin-2-ylmethoxy)-9-(5-ethyl-[1,2,4]oxadiazol-3-ylmethoxy)-6,7-dihydro-pyrimido[6,1-a]isoquinolin-4-one,
   9-(5-Cyclopropyl-[1,2,4]oxadiazol-3-ylmethoxy)-2-(2,3-dihydro-[1,4]dioxino[2,3-b]pyridin-2-ylmethoxy)-6,7-dihydro-pyrimido[6,1-a]isoquinolin-4-one,
   2-(2,3-Dihydro-[1,4]dioxino[2,3-b]pyridin-2-ylmethoxy)-9-(5-isopropyl-[1,2,4]oxadiazol-3-ylmethoxy)-6,7-dihydro-pyrimido[6,1-a]isoquinolin-4-one,
   9-(5-tert-Butyl-[1,2,4]oxadiazol-3-ylmethoxy)-2-(2,3-dihydro-[1,4]dioxino[2,3-b]pyridin-2-ylmethoxy)-6,7-dihydro-pyrimido[6,1-a]isoquinolin-4-one,
   2-(2,3-Dihydro-[1,4]dioxino[2,3-b]pyridin-2-ylmethoxy)-9-(3-methyl-isoxazol-5-ylmethoxy)-6,7-dihydro-pyrimido [6, 1-a] isoquinolin-4-one,
   9-(3-Chloro-2-methoxy-pyridin-4-yl)-2-(2,3-dihydro-[1,4]dioxino[2,3-b]pyridin-2-ylmethoxy)-6,7-dihydro-pyrimido[6,1-a]isoquinolin-4-one,
   2-(2,3-Dihydro-[1,4]dioxino[2,3-b]pyridin-2-ylmethoxy)-9-(3,6-dihydro-2H-pyran-4-yl)-6,7-dihydro-pyrimido [6, 1-a] isoquinolin-4-one,
   5-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-pyridine-2-carbonitrile,
   2-(2,3-Dihydro-[1,4]dioxino[2,3-b]pyridin-2-ylmethoxy)-9-(2-ethoxy-pyridin-3-yl)-6,7-dihydro-pyrimido[6,1-a]isoquinolin-4-one,
   2-(2,3-Dihydro-[1,4]dioxino[2,3-b]pyridin-2-ylmethoxy)-9-(6-ethoxy-pyridin-3-yl)-6,7-dihydro-pyrimido[6,1-a]isoquinolin-4-one,
   2-(2,3-Dihydro-[1,4]dioxino[2,3-b]pyridin-2-ylmethoxy)-9-(6-morpholin-4-yl-pyridin-3-yl)-6,7-dihydro-pyrimido [6, 1-a] isoquinolin-4-one,
   2-(2,3-Dihydro-[1,4]dioxino[2,3-b]pyridin-2-ylmethoxy)-9-(2-methoxy-pyrimidin-5-yl)-6,7-dihydro-pyrimido [6, 1-a] isoquinolin-4-one,
   2-(2,3-Dihydro-[1,4]dioxino[2,3-b]pyridin-2-ylmethoxy)-9-(2,3-dimethoxy-phenyl)-6,7-dihydro-pyrimido[6,1-a]isoquinolin-4-one,
   2-(2,3-Dihydro-[1,4]dioxino[2,3-b]pyridin-2-ylmethoxy)-9-(2,5-dimethoxy-phenyl)-6,7-dihydro-pyrimido[6,1-a]isoquinolin-4-one,
   2-(2,3-Dihydro-[1,4]dioxino[2,3-b]pyridin-2-ylmethoxy)-9-(3-hydroxy-3-phenyl-prop-1-ynyl)-6,7-dihydro-pyrimido[6,1-a]isoquinolin-4-one,
   3-{3-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-prop-2-ynyloxy}-propionitrile,
   2-(2,3-Dihydro-[1,4]dioxino[2,3-b]pyridin-2-ylmethoxy)-9-(3-methylamino-prop-1-ynyl)-6,7-dihydro-pyrimido [6, 1-a] isoquinolin-4-one,
   2-(2,3-Dihydro-[1,4]dioxino[2,3-b]pyridin-2-ylmethoxy)-9-(3-dimethylamino-prop-1-ynyl)-6,7-dihydro-pyrimido [6, 1-a] isoquinolin-4-one,
   9-[3-(Benzyl-methyl-amino)-prop-1-ynyl]-2-(2,3-diohydro-[1,4]dioxino[2,3-b]pyridin-2-ylmethoxy)-6,7-dihydro-pyrimido[6,1-a]isoquinolin-4-one,
   2-(2,3-Dihydro-[1,4]dioxino[2,3-b]pyridin-2-ylmethoxy)-9-[3-(1,1-dioxo-thiomorpholin-4-yl)-prop-1-ynyl]-6,7-dihydro-pyrimido[6,1-a]isoquinolin-4-one,
   {3-[2-(2,3-Dihydro-[1,4]dioxino[2,3-b]pyridin-2-ylmethoxy)-4-oxo-6,7-dihydro-4H-pyrimido[6,1-a] isoquinolin-9-yl] -prop-2-ynyl} -urea,
   1-{3-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-prop-2-ynyl}-imidazolidine-2,4-dione,
   9-(3-Diethylamino-prop-1-ynyl)-2-(2,3-dihydro-[1,4]dioxino[2,3-b]pyridin-2-ylmethoxy)-6,7-dihydro-pyrimido [6, 1-a] isoquinolin-4-one,
   9-(3-Amino-3-methyl-but-1-ynyl)-2-(2,3-dihydro-[1,4]dioxino[2,3-b]pyridin-2-ylmethoxy)-6,7-dihydro-pyrimido [6, 1-a] isoquinolin-4-one,
   2-(2,3-Dihydro-[1,4]dioxino[2,3-b]pyridin-2-ylmethoxy)-9-(tetrahydro-pyran-2-ylmethoxy)-6,7-dihydro-pyrimido [6, 1-a] isoquinolin-4-one,
   2-(2,3-Dihydro-[1,4]dioxino[2,3-b]pyridin-2-ylmethoxy)-9-(1H-pyrazol-4-yl)-6,7-dihydro-pyrimido[6,1-a]isoquinolin-4-one,
   2-(2,3-Dihydro-[1,4]dioxino[2,3-b]pyridin-2-ylmethoxy)-9-(3-hydroxy-but-1-ynyl)-6,7-dihydro-pyrimido[6,1-a]isoquinolin-4-one,
   2-(2,3-Dihydro-[1,4]dioxino[2,3-b]pyridin-2-ylmethoxy)-9-(3-hydroxy-pent-1-ynyl)-6,7-dihydro-pyrimido[6,1-a]isoquinolin-4-one,
   2-(2,3-Dihydro-[1,4]dioxino[2,3-b]pyridin-2-ylmethoxy)-9-(3-hydroxy-hex-1-ynyl)-6,7-dihydro-pyrimido[6,1-a]isoquinolin-4-one,
   9-(1-Amino-cyclohexylethynyl)-2-(2,3-dihydro-[1,4]dioxino[2,3-b]pyridin-2-ylmethoxy)-6,7-dihydro-pyrimido [6, 1-a] isoquinolin-4-one,
   2-(2,3-Dihydro-[1,4]dioxino[2,3-b]pyridin-2-ylmethoxy)-9-(3-hydroxy-5-methyl-hex-1-ynyl)-6,7-dihydro-pyrimido[6,1-a]isoquinolin-4-one,
   2-(2,3-Dihydro-[1,4]dioxino[2,3-b]pyridin-2-ylmethoxy)-9-(3-ethyl-3-hydroxy-pent-1-ynyl)-6,7-dihydro-pyrimido [6, 1-a] isoquinolin-4-one,
   2-(2,3-Dihydro-[1,4]dioxino [2,3 -b]pyridin-2-ylmethoxy)-9-(3 -hydroxy-3 -phenyl-but-1-ynyl)-6,7-dihydro-pyrimido [6, 1-a] isoquinolin-4-one,
   2-(2,3-Dihydro-[1,4]dioxino[2,3-b]pyridin-2-ylmethoxy)-9-(3-hydroxy-4-methyl-pent-1-ynyl)-6,7-dihydro-pyrimido[6,1-a]isoquinolin-4-one,
   2-[(R)-1-(2,3-Dihydro-[1,4]dioxino[2,3-b]pyridin-2-yl)methoxy]-9-(oxazol-2-ylmethoxy)-6,7-dihydro-pyrimido[6,1-a]isoquinolin-4-one,
   2-[(R)-1-(2,3-Dihydro-[1,4]dioxino[2,3-b]pyridin-2-yl)methoxy]-9-(oxazol-2-ylmethoxy)-6,7-dihydro-pyrimido[6,1-a]isoquinolin-4-one,
   2-[(S)-1-(2,3-Dihydro-[1,4]dioxino[2,3-b]pyridin-2-yl)methoxy]-9-(oxazol-2-ylmethoxy)-6,7-dihydro-pyrimido[6,1-a]isoquinolin-4-one,
   9-(3-Butylamino-prop-1-ynyl)-2-(2,3-dihydro-[1,4]dioxino[2,3-b]pyridin-2-ylmethoxy)-6,7-dihydro-pyrimido[6,1-a]isoquinolin-4-one,
   2-(2,3-Dihydro-[1,4]dioxino[2,3-b]pyridin-2-ylmethoxy)-9-(2-morpholin-4-yl-ethoxymethyl)-6,7-dihydro-pyrimido[6,1-a]isoquinolin-4-one,
   9-(3-Benzylamino-prop-1-ynyl)-2-(2,3-dihydro-[1,4]dioxino[2,3-b]pyridin-2-ylmethoxy)-6,7-dihydro-pyrimido[6,1-a]isoquinolin-4-one,
   2-(2,3-Dihydro-[1,4]dioxino[2,3-b]pyridin-2-ylmethoxy)-9-(3-phenylamino-prop-1-ynyl)-6,7-dihydro-pyrimido[6,1-a]isoquinolin-4-one,
   2-(2,3-Dihydro-[1,4]dioxino[2,3-b]pyridin-2-ylmethoxy)-4-oxo-6,7-dihydro-4H-pyrimido[6,1-a]isoquinoline-9-carboxylic acid (tetrahydro-pyran-4-yl)-amide,
   2-(2,3-Dihydro-[1,4]dioxino[2,3-b]pyridin-2-ylmethoxy)-4-oxo-6,7-dihydro-4H-pyrimido[6,1-a]isoquinoline-9-carboxylic acid (oxetan-3-ylmethyl)-amide,
   2-(2,3-Dihydro-[1,4]dioxino[2,3-b]pyridin-2-ylmethoxy)-9-pyrrolidin-1-ylmethyl-6,7-dihydro-pyrimido [6,1 -a] isoquinolin-4-one,
   9-(tert-Butylamino-methyl)-2-(2,3-dihydro-[1,4]dioxino[2,3-b]pyridin-2-ylmethoxy)-6,7-dihydro-pyrimido[6,1-a]isoquinolin-4-one,
   2-(2,3-Dihydro-[1,4]dioxino[2,3-b]pyridin-2-ylmethoxy)-9-piperidin-1-ylmethyl-6,7-dihydro-pyrimido[6,1-a]isoquinolin-4-one,
   2-(2,3-Dihydro-[1,4]dioxino[2,3-b]pyridin-2-ylmethoxy)-9-(3-methyl-oxetan-3-ylmethoxymethyl)-6,7-dihydro-pyrimido [6,1 -a] isoquinolin-4-one,
   2-(2,3-Dihydro-[1,4]dioxino[2,3-b]pyridin-2-ylmethoxy)-9-(3-methyl-oxetan-3-ylmethoxymethyl)-6,7-dihydro-pyrimido[6,1-a]isoquinolin-4-one,
   2-(2,3-Dihydro-[1,4]dioxino[2,3-b]pyridin-2-ylmethoxy)-9-(oxetan-3-yloxymethyl)-6,7-dihydro-pyrimido[6,1-a]isoquinolin-4-one,
   9-Cyclopropylethynyl-2-(4-isopropyl-oxetan-2-ylmethoxy)-6,7-dihydro-pyrimido[6,1-a]isoquinolin-4-one, and
   9-Cyclopropylethynyl-2-((R)-4-isopropyl-oxetan-2-ylmethoxy)-6,7-dihydro-pyrimido[6,1-a] isoquinolin-4-one.
10. The compound or pharmaceutically acceptable salt thereof for use according to clause 1, wherein said compound is selected from:
   9-methoxy-1-methyl-2-[(tetrahydro-furan-2-ylmethyl)-amino]-10-(2,2,2-trifluoro-ethoxy)-6,7-dihydro-pyrido[2,1-a]isoquinolin-4-one,
   9-cyclopropylethynyl-2-((S)-1-[1,4]dioxan-2-ylmethoxy)-10-methoxy-1-methyl-6,7-dihydro-pyrido[2,1-a]isoquinolin-4-one,
   9-(2,2-difluoro-cyclopropylmethoxy)-2-((S)-1-[1,4]dioxan-2-ylmethoxy)-10-methoxy-1-methyl-6,7-dihydro-pyrido[2,1-a]isoquinolin-4-one,
   10-(2,2-difluoro-ethoxy)-2-[([1,4]dioxan-2-ylmethyl)-amino]-9-methoxy-1-methyl-6,7-dihydro-pyrido[2,1-a]isoquinolin-4-one,
   9-(2,2-difluoro-ethoxy)-10-methoxy-1-methyl-2-[(tetrahydro-furan-2-ylmethyl)-amino]-6,7-dihydro-pyrido [2, 1-a] isoquinolin-4-one,
   10-methoxy-1-methyl-2-[(tetrahydro-furan-2-ylmethyl)-amino]-9-(2,2,2-trifluoro-ethoxy)-6,7-dihydro-pyrido [2, 1-a] isoquinolin-4-one,
   9-(6-cyclopropyl-pyridin-3-yl)-2-((S)-1-[1,4]dioxan-2-ylmethoxy)-10-methoxy-1-methyl-6,7-dihydro-pyrido [2, 1-a] isoquinolin-4-one,
   2-(2,3-dihydro-thieno[3,4-b][1,4]dioxin-2-ylmethoxy)-9,10-dimethoxy-6,7-dihydro-pyrido[2,1-a]isoquinolin-4-one,
   trifluoro-methanesulfonic acid 2-((S)-1-[1,4]dioxan-2-ylmethoxy)-10-methoxy-1-methyl-4-oxo-6,7-dihydro-4H-pyrido[2, 1-a]isoquinolin-9-yl ester,
   2-[([1,4]dioxan-2-ylmethyl)-amino]-9-methoxy-1-methyl-10-(2,2,2-trifluoro-ethoxy)-6,7-dihydro-pyrido [2, 1-a] isoquinolin-4-one,
   10-(2,2-difluoro-ethoxy)-9-methoxy-1-methyl-2-[(tetrahydro-furan-2-ylmethyl)-amino]-6,7-dihydro-pyrido [2, 1-a] isoquinolin-4-one,
   2-((S)-1-[1,4]dioxan-2-ylmethoxy)-10-methoxy-1-methyl-9-[6-(2,2,2-trifluoro-ethoxy)-pyridin-3-yl]-6,7-dihydro-pyrido[2,1-a]isoquinolin-4-one,
   2-((S)-1-[1,4]dioxan-2-ylmethoxy)-9-(6-fluoro-pyridin-3-yl)-10-methoxy-1-methyl-6,7-dihydro-pyrido[2,1-a]isoquinolin-4-one,
   9,10-bis-(2,2-difluoro-cyclopropylmethoxy)-2-((S)-1-[1,4]dioxan-2-ylmethoxy)-1-methyl-6,7-dihydro-pyrido [2, 1-a] isoquinolin-4-one,
   9-(3,3-difluoro-cyclobutylmethoxy)-2-((S)-1-[1,4]dioxan-2-ylmethoxy)-10-methoxy-1-methyl-6,7-dihydro-pyrido[2,1-a]isoquinolin-4-one,
   2-((S)-1-[1,4]dioxan-2-ylmethoxy)-10-methoxy-1-methyl-9-(2,2,2-trifluoro-ethoxy)-6,7-dihydro-pyrido[2,1-a]isoquinolin-4-one,
   10-difluoromethoxy-9-methoxy-1-methyl-2-[(tetrahydro-furan-2-ylmethyl)-amino]-6,7-dihydro-pyrido[2,1-a]isoquinolin-4-one,
   10-(2,2-difluoro-ethoxy)-9-methoxy-1-methyl-2-(tetrahydro-pyran-2-ylmethyl)-amino]-6,7-dihydro-pyrido[2,1-a]isoquinolin-4-one,
   2-((S)-1-[1,4]dioxan-2-ylmethoxy)-10-methoxy-1-methyl-9-(6-oxo-1,6-dihydro-pyridin-3-yl)-6,7-dihydro-pyrido[2,1-a]isoquinolin-4-one,
   2-((S)-1-[1,4]dioxan-2-ylmethoxy)-10-methoxy-1-methyl-9-(3-methyl-oxetan-3-ylmethoxy)-6,7-dihydro-pyrido[2,1-a]isoquinolin-4-one,
   9-methoxy-1-methyl-2-[(tetrahydro-pyran-2-ylmethyl)-amino]-10-(2,2,2-trifluoro-ethoxy)-6,7-dihydro-pyrido[2,1-a]isoquinolin-4-one,
   9-(2,2-difluoro-ethoxy)-2-((S)-1-[1,4]dioxan-2-ylmethoxy)-10-methoxy-1-methyl-6,7-dihydro-pyrido[2,1-a]isoquinolin-4-one,
   2-[([1,4]dioxan-2-ylmethyl)-amino]-9,10-dimethoxy-1-methyl-6,7-dihydro-pyrido[2,1-a]isoquinolin-4-one,
   9-cyclopropylethynyl-2-((S)-1-[1,4]dioxan-2-ylmethoxy)-1-methyl-6,7-dihydro-pyrido[2,1-a]isoquinolin-4-one,
   2-(2,3-dihydro-benzo[1,4]dioxin-2-ylmethoxy)-9-hydroxy-10-methoxy-6,7-dihydro-pyrido[2,1-a]isoquinolin-4-one,
   9-difluoromethoxy-2-((S)-1-[1,4]dioxan-2-ylmethoxy)-10-methoxy-1-methyl-6,7-dihydro-pyrido [2,1 -a] isoquinolin-4-one,
   9-cyclopropylmethoxy-2-((S)-1-[1,4]dioxan-2-ylmethoxy)-10-methoxy-1-methyl-6,7-dihydro-pyrido [2,1 -a] isoquinolin-4-one,
   2-((S)-1-[1,4]dioxan-2-ylmethoxy)-9-(4-ethoxy-3-trifluoromethyl-phenyl)-10-methoxy-1-methyl-6,7-dihydro-pyrido[2,1-a]isoquinolin-4-one,
   9,10-dimethoxy-1-methyl-2-[(tetrahydro-pyran-2-ylmethyl)-amino]-6,7-dihydro-pyrido[2,1-a]isoquinolin-4-one,
   8,9-dimethoxy-2-[(tetrahydro-pyran-2-ylmethyl)-amino]-6,7-dihydro-pyrido[2,1-a]isoquinolin-4-one,
   9,10-dimethoxy-1-methyl-2-[(tetrahydro-furan-2-ylmethyl)-amino]-6,7-dihydro-pyrido[2,1-a]isoquinolin-4-one,
   10-difluoromethylsulfanyl-2-((S)-1-[1,4]dioxan-2-ylmethoxy)-9-methoxy-1-methyl-6,7-dihydro-pyrido[2,1-a]isoquinolin-4-one,
   9-methoxy-1-methyl-2-[(tetrahydro-furan-2-ylmethyl)-amino]-6,7-dihydro-pyrido[2,1-a]isoquinolin-4-one,
   2-((S)-1-[1,4]dioxan-2-ylmethoxy)-10-methoxy-1-methyl-9-(4-trifluoromethoxy-phenyl)-6,7-dihydro-pyrido[2,1-a] isoquinolin-4-one,
   9-(2,2-difluoro-cyclopropylmethoxy)-1-methyl-2-[(tetrahydro-furan-2-ylmethyl)-amino]-6,7-dihydro-pyrido[2,1-a] isoquinolin-4-one,
   8,9-dimethoxy-1-methyl-2-[(tetrahydro-furan-2-ylmethyl)-amino]-6,7-dihydro-pyrido[2,1-a]isoquinolin-4-one,
   2-(2,3-dihydro-benzo[1,4]dioxin-2-ylmethoxy)-9,10-dimethoxy-1-methyl-6,7-dihydro-pyrido[2,1-a]isoquinolin-4-one,
   2-((S)-1-[1,4]dioxan-2-ylmethoxy)-9-methoxy-1,10-dimethyl-6,7-dihydro-pyrido[2,1-a]isoquinolin-4-one,
   8,11-dimethoxy-2-[(tetrahydro-pyran-2-ylmethyl)-amino]-6,7-dihydro-pyrido[2,1-a]isoquinolin-4-one,
   2-[([1,4]dioxan-2-ylmethyl)-amino]-8,11-dimethoxy-6,7-dihydro-pyrido[2,1-a]isoquinolin-4-one,
   8,11-dimethoxy-2-[(tetrahydro-furan-2-ylmethyl)-amino]-6,7-dihydro-pyrido[2,1-a]isoquinolin-4-one,
   2-[(R)-1-(2,3-dihydro-[1,4]dioxino[2,3-b]pyridin-2-yl)methoxy]-9,10-dimethoxy-1-methyl-6,7-dihydro-pyrido[2,1-a] isoquinolin-4-one,
   2-[([1,4]dioxan-2-ylmethyl)-amino]-8,9-dimethoxy-1-methyl-6,7-dihydro-pyrido[2,1-a]isoquinolin-4-one,
   10-(2,2-difluoro-ethoxy)-9-methoxy-1-methyl-2-(tetrahydro-pyran-2-ylmethoxy)-6,7-dihydro-pyrido[2,1-a]isoquinolin-4-one,
   8,9-dimethoxy-2-[(tetrahydro-furan-2-ylmethyl)-amino]-6,7-dihydro-pyrido[2,1-a]isoquinolin-4-one,
   2-((S)-1-[1,4]dioxan-2-ylmethoxy)-8,9-dimethoxy-6,7-dihydro-pyrido[2,1-a]isoquinolin-4-one,
   2-[(S)-1-(2,3-dihydro-[1,4]dioxino[2,3-b]pyridin-2-yl)methoxy]-9,10-dimethoxy-1-methyl-6,7-dihydro-pyrido[2,1-a]isoquinolin-4-one,
   2-((S)-1-[1,4]dioxan-2-ylmethoxy)-1-methyl-9-(pyridin-2-ylmethoxy)-6,7-dihydro-pyrido[2,1-a]isoquinolin-4-one,
   2-[([1,4]dioxan-2-ylmethyl)-amino]-8,9-dimethoxy-6,7-dihydro-pyrido[2,1-a]isoquinolin-4-one,
   2-((S)-1-[1,4]dioxan-2-ylmethoxy)-10-methoxy-1 -methyl-4-oxo-6,7-dihydro-4H-pyrido [2,1-a]isoquinoline-9-carbonitrile,
   9-methoxy-1-methyl-2-(tetrahydro-pyran-2-ylmethoxy)-10-(2,2,2-trifluoro-ethoxy)-6,7-dihydro-pyrido[2,1-a]isoquinolin-4-one,
   9-(2-cyclopropyl-ethyl)-2-((S)-1-[1,4]dioxan-2-ylmethoxy)-1-methyl-6,7-dihydro-pyrido[2,1-a]isoquinolin-4-one,
   9-(2-cyclopropyl-ethyl)-2-((S)-1-[1,4]dioxan-2-ylmethoxy)-1,7,7-trimethyl-6,7-dihydro-pyrido[2,1-a]isoquinolin-4-one,
   2-((S)-1-[1,4]dioxan-2-ylmethoxy)-1,7,7-trimethyl-9-(pyridin-2-ylmethoxy)-6,7-dihydro-pyrido[2,1-a]isoquinolin-4-one,
   9,11-dimethoxy-2-[(tetrahydro-furan-2-ylmethyl)-amino]-6,7-dihydro-pyrido[2,1-a]isoquinolin-4-one,
   2-(2,3-dihydro-benzo[1,4]dioxin-2-ylmethoxy)-9,10-dimethoxy-6,7-dihydro-pyrido[2,1-a]isoquinolin-4-one,
   2-((S)-1-[1,4]dioxan-2-ylmethoxy)-9,10-dimethoxy-1-methyl-6,7-dihydro-pyrido[2,1-a]isoquinolin-4-one,
   methanesulfonic acid 2-((S)-1-[1,4]dioxan-2-ylmethoxy)-10-methoxy-1-methyl-4-oxo-6,7-dihydro-4H-pyrido[2,1-a]isoquinolin-9-yl ester,
   2-((S)-1-[1,4]dioxan-2-ylmethoxy)-1,7,7-trimethyl-9-pentyl-6,7-dihydro-pyrido[2,1-a]isoquinolin-4-one,
   2-((S)-1-[1,4]dioxan-2-ylmethoxy)-1-methyl-9-(3-methyl-butyl)-6,7-dihydro-pyrido[2,1-a]isoquinolin-4-one,
   2-((S)-1-[1,4]dioxan-2-ylmethoxy)-10-methoxy-1 -methyl-6,7-dihydro-pyrido [2,1 -a] isoquinolin-4-one,
   2-([1,4]dioxan-2-ylmethoxy)-9,10-dimethoxy-6,7-dihydro-pyrido[2,1-a]isoquinolin-4-one, 8,11-dimethoxy-2-(tetrahydro-pyran-2-ylmethoxy)-6,7-dihydro-pyrido[2,1-a]isoquinolin-4-one,
   2-((S)-1-[1,4]dioxan-2-ylmethoxy)-9-(5-ethyl-[1,2,4]oxadiazol-3-ylmethoxy)-1-methyl-6,7-dihydro-pyrido[2,1-a]isoquinolin-4-one,
   2-[([1,4]dioxan-2-ylmethyl)-amino]-9,11-dimethoxy-6,7-dihydro-pyrido[2,1-a] isoquinolin-4-one, 8,9-dimethoxy-2-(tetrahydro-pyran-2-ylmethoxy)-6,7-dihydro-pyrido[2,1-a]isoquinolin-4-one,
   2-(2,3-dihydro-benzo[1,4]dioxin-2-ylmethoxy)-9-methoxy-6,7-dihydro-pyrido[2,1-a] isoquinolin-4-one,
   9-methoxy-1-methyl-10-(pyridin-2-ylmethoxy)-2-[(tetrahydro-furan-2-ylmethyl)-amino]-6,7-dihydro-pyrido[2,1-a]isoquinolin-4-one,
   9-(3,5-dimethyl-isoxazol-4-yl)-2-((S)-1-[1,4]dioxan-2-ylmethoxy)-1-methyl-6,7-dihydro-pyrido[2,1-a]isoquinolin-4-one,
   9-benzyloxy-2-((S)-1-[1,4]dioxan-2-ylmethoxy)-1-methyl-6,7-dihydro-pyrido[2,1-a] isoquinolin-4-one,
   9,11-dimethoxy-2-[(tetrahydro-pyran-2-ylmethyl)-amino]-6,7-dihydro-pyrido[2,1-a]isoquinolin-4-one,
   2-((S)-1-[1,4]dioxan-2-ylmethoxy)-9-hydroxy-10-methoxy-1-methyl-6,7-dihydro-pyrido[2,1-a]isoquinolin-4-one,
   8,9-dimethoxy-1-methyl-2-[(tetrahydro-pyran-2-ylmethyl)-amino]-6,7-dihydro-pyrido[2,1-a]isoquinolin-4-one,
   9-benzyloxy-1-cyclopropyl-2-[(tetrahydro-furan-2-ylmethyl)-amino]-6,7-dihydro-pyrido[2,1-a] isoquinolin-4-one,
   2-((S)-1-[1,4]dioxan-2-ylmethoxy)-8,11-dimethoxy-6,7-dihydro-pyrido[2,1-a]isoquinolin-4-one,
   9-(3,6-dihydro-2H-pyran-4-yl)-2-((S)-1-[1,4]dioxan-2-ylmethoxy)-1-methyl-6,7-dihydro-pyrido[2,1-a]isoquinolin-4-one,
   2-((S)-1-[1,4]dioxan-2-ylmethoxy)-1,7,7-trimethyl-9-(oxazol-2-ylmethoxy)-6,7-dihydro-pyrido[2,1-a]isoquinolin-4-one,
   9,11-dimethoxy-2-(tetrahydro-pyran-2-ylmethoxy)-6,7-dihydro-pyrido[2,1-a]isoquinolin-4-one,
   2-((S)-1-[1,4]dioxan-2-ylmethoxy)-1-methyl-9-(tetrahydro-pyran-4-yl)-6,7-dihydro-pyrido[2,1-a]isoquinolin-4-one,
   9-benzyloxy-2-([1,4]dioxan-2-ylmethoxy)-1,7,7-trimethyl-6,7-dihydro-pyrido[2,1-a]isoquinolin-4-one,
   [2-((S)-1-[1,4]dioxan-2-ylmethoxy)-1 -methyl-4-oxo-6,7-dihydro-4H-pyrido [2,1-a] isoquinolin-9-yloxy]-acetonitrile,
   9-cyclopropylethynyl-2-((S)-1-[1,4]dioxan-2-ylmethoxy)-1,7,7-trimethyl-6,7-dihydro-pyrido[2,1-a]isoquinolin-4-one,
   9,10-dimethoxy-1-methyl-2-(tetrahydro-furan-2-ylmethoxy)-6,7-dihydro-pyrido[2,1-a]isoquinolin-4-one,
   2-((S)-1-[1,4]dioxan-2-ylmethoxy)-1-methyl-9-(3-methyl-but-1-ynyl)-6,7-dihydro-pyrido[2,1-a]isoquinolin-4-one,
   9-(5,6-dihydro-[1,4]dioxin-2-yl)-2-((S)-1-[1,4]dioxan-2-ylmethoxy)-1-methyl-6,7-dihydro-pyrido[2,1-a]isoquinolin-4-one,
   2-((S)-1-[1,4]dioxan-2-ylmethoxy)-9-ethoxy-1-methyl-6,7-dihydro-pyrido [2,1-a] isoquinolin-4-one,
   2-((S)-1-[1,4]dioxan-2-ylmethoxy)-1-methyl-9-(1-methyl-1H-pyrazol-4-yl)-6,7-dihydro-pyrido [2,1 -a] isoquinolin-4-one,
   [2-((S)-1-[1,4]dioxan-2-ylmethoxy)-1-ethyl-4-oxo-6,7-dihydro-4H-pyrido [2,1-a] isoquinolin-9-yloxy]-acetonitrile,
   2-((S)-1-[1,4]dioxan-2-ylmethoxy)-1-methyl-9-(1-propyl-1H-tetrazol-5-ylmethoxy)-6,7-dihydro-pyrido [2,1 -a] isoquinolin-4-one,
   2-((S)-1-[1,4]dioxan-2-ylmethoxy)-8,9-dimethoxy-1-methyl-6,7-dihydro-pyrido[2,1-a]isoquinolin-4-one,
   8,11-dimethoxy-2-(tetrahydro-furan-2-ylmethoxy)-6,7-dihydro-pyrido[2,1-a]isoquinolin-4-one,
   9-[1,4]dioxan-2-yl-2-((S)-1-[1,4]dioxan-2-ylmethoxy)-1-methyl-6,7-dihydro-pyrido[2,1-a]isoquinolin-4-one,
   2-((S)-1-[1,4]dioxan-2-ylmethoxy)-9-methoxy-1-methyl-6,7-dihydro-pyrido[2,1-a]isoquinolin-4-one,
   2-((S)-1-[1,4]dioxan-2-ylmethoxy)-8-hydroxy-9-methoxy-1-methyl-6,7-dihydro-pyrido[2,1-a]isoquinolin-4-one,
   9-(1-cyclopropyl-1H-tetrazol-5-ylmethoxy)-2-((S)-1-[1,4]dioxan-2-ylmethoxy)-1-methyl-6,7-dihydro-pyrido[2,1-a] isoquinolin-4-one,
   2-((S)-1-[1,4]dioxan-2-ylmethoxy)-1-methyl-4-oxo-9-(2,2,2-trifluoro-ethoxy)-6,7-dihydro-4H-pyrido[2,1-a] isoquinoline-10-carbonitrile,
   2,9-bis-((S)-1-[1,4]dioxan-2-ylmethoxy)-1-methyl-6,7-dihydro-pyrido[2,1-a]isoquinolin-4-one,
   2-((S)-1-[1,4]dioxan-2-ylmethoxy)-9,1 1-dimethoxy-6,7-dihydro-pyrido[2,1-a]isoquinolin-4-one,
   8,9-dimethoxy-1-methyl-2-(tetrahydro-furan-2-ylmethoxy)-6,7-dihydro-pyrido[2,1-a]isoquinolin-4-one,
   2-((S)-1-[1,4]dioxan-2-ylmethoxy)-1-methyl-9-(3-methyl-oxetan-3-ylmethoxy)-6,7-dihydro-pyrido[2,1-a]isoquinolin-4-one,
   9-cyclopropylethynyl-2-((S)-1-[1,4]dioxan-2-ylmethoxy)-1-ethyl-6,7-dihydro-pyrido[2,1-a]isoquinolin-4-one,
   9,10-dimethoxy-2-(tetrahydro-furan-2-ylmethoxy)-6,7-dihydro-pyrido[2,1-a]isoquinolin-4-one,
   2-((S)-1-[1,4]dioxan-2-ylmethoxy)-1-ethyl-9-(pyridin-2-ylmethoxy)-6,7-dihydro-pyrido[2,1-a]isoquinolin-4-one,
   2-((S)-1-[1,4]dioxan-2-ylmethoxy)-1-ethyl-9-(1H-tetrazol-5ylmethoxy)-6,7-dihydro-pyrido[2,1-a]isoquinolin-4-one,
   2-cyclohexylmethoxy-9,10-dimethoxy-1-methyl-6,7-dihydro-pyrido[2,1-a]isoquinolin-4-one,
   9,10-dimethoxy-1-methyl-2-[(tetrahydro-pyran-3-ylmethyl)-amino]-6,7-dihydro-pyrido[2,1-a]isoquinolin-4-one,
   2-((S)-1-[1,4]dioxan-2-ylmethoxy)-1-ethyl-9-(1-methyl-1H-pyrazol-4-yl)-6,7-dihydro-pyrido[2,1-a]isoquinolin-4-one,
   9,10-dimethoxy-1-methyl-2-(tetrahydro-pyran-4-ylmethoxy)-6,7-dihydro-pyrido[2,1-a]isoquinolin-4-one,
   9-cyclopropylmethoxy-2-((S)-1-[1,4]dioxan-2-ylmethoxy)-1,7,7-trimethyl-6,7-dihydro-pyrido [2,1 -a] isoquinolin-4-one,
   8,9-dimethoxy-2-(tetrahydro-furan-2-ylmethoxy)-6,7-dihydro-pyrido[2,1-a]isoquinolin-4-one,
   2-((S)-1-[1,4]dioxan-2-ylmethoxy)-1-ethyl-9-(3-methoxy-but-1-ynyl)-6,7-dihydro-pyrido[2,1-a]isoquinolin-4-one,
   8,9-dimethoxy-1-methyl-2-(tetrahydro-pyran-2-ylmethoxy)-6,7-dihydro-pyrido[2,1-a]isoquinolin-4-one,
   9-benzyloxy-1-cyclopropyl-2-((S)-1-[1,4]dioxan-2-ylmethoxy)-6,7-dihydro-pyrido[2,1-a]isoquinolin-4-one,
   2-((S)-1-[1,4]dioxan-2-ylmethoxy)-1-ethyl-9-(3-methyl-but-1-ynyl)-6,7-dihydro-pyrido[2,1-a]isoquinolin-4-one,
   trifluoro-methanesulfonic acid 2-((S)-1-[1,4]dioxan-2-ylmethoxy)-1-ethyl-4-oxo-6,7-dihydro-4H-pyrido[2,1-a]isoquinolin-9-yl ester,
   9-(2-cyclopropyl-ethyl)-2-((S)-1-[1,4]dioxan-2-ylmethoxy)-1-ethyl-6,7-dihydro-pyrido[2,1-a]isoquinolin-4-one,
   9-cyclopropylmethoxy-2-([1,4]dioxan-2-ylmethoxy)-1-methyl-6,7-dihydro-pyrido[2,1-a]isoquinolin-4-one,
   2-((S)-1-[1,4]dioxan-2-ylmethoxy)-1-ethyl-9-(3-methyl-butyl)-6,7-dihydro-pyrido[2,1-a] isoquinolin-4-one,
   9,11-dimethoxy-2-(tetrahydro-furan-2-ylmethoxy)-6,7-dihydro-pyrido[2,1-a]isoquinolin-4-one,
   2-((S)-1-[1,4]dioxan-2-ylmethoxy)-9-hydroxy-1,7,7-trimethyl-6,7-dihydro-pyrido[2,1-a]isoquinolin-4-one,
   2-((S)-1-[1,4]dioxan-2-ylmethoxy)-9-methoxy-1-methyl-4-oxo-6,7-dihydro-4H-pyrido[2,1-a] isoquinoline-10-carbonitrile,
   2-((S)-1-[1,4]dioxan-2-ylmethoxy)-1-ethyl-9-pentyl-6,7-dihydro-pyrido[2,1-a]isoquinolin-4-one,
   1-cyclopropyl-2-((S)-1-[1,4]dioxan-2-ylmethoxy)-9-pentyl-6,7-dihydro-pyrido[2,1-a]isoquinolin-4-one,
   9-benzyloxy-2-((S)-1-[1,4]dioxan-2-ylmethoxy)-1-isopropyl-6,7-dihydro-pyrido[2,1-a]isoquinolin-4-one,
   2-((S)-1-[1,4]dioxan-2-ylmethoxy)-1-ethyl-9-(3-methoxy-butyl)-6,7-dihydro-pyrido[2,1-a]isoquinolin-4-one,
   1-cyclopropyl-2-((S)-1-[1,4]dioxan-2-ylmethoxy)-9-(3 -methyl-butyl)-6,7-dihydro-pyrido [2,1-a]isoquinolin-4-one,
   1-cyclopropyl-9-(2-cyclopropyl-ethyl)-2-((S)-1-[1,4]dioxan-2-ylmethoxy)-6,7-dihydro-pyrido[2,1-a]isoquinolin-4-one,
   trifluoro-methanesulfonic acid 1-cyclopropyl-2-((S)-1-[1,4]dioxan-2-ylmethoxy)-4-oxo-6,7-dihydro-4H-pyrido[2,1-a]isoquinolin-9-yl ester,
   trifluoro-methanesulfonic acid 2-((S)-1-[1,4]dioxan-2-ylmethoxy)-1-isopropyl-4-oxo-6,7-dihydro-4H-pyrido[2, 1-a]isoquinolin-9-yl ester,
   1-cyclopropyl-9-cyclopropylethynyl-2-((S)-1-[1,4]dioxan-2-ylmethoxy)-6,7-dihydro-pyrido [2,1-a]isoquinolin-4-one,
   2-((S)-1-[1,4]dioxan-2-ylmethoxy)-1 -isopropyl-9-(3 -methyl-butyl)-6,7-dihydro-pyrido [2,1-a]isoquinolin-4-one,
   2-((S)-1-[1,4]dioxan-2-ylmethoxy)-9-hydroxy-8-methoxy-1-methyl-6,7-dihydro-pyrido[2,1-a]isoquinolin-4-one,
   9-(2-cyclopropyl-ethyl)-2-((S)-1-[1,4]dioxan-2-ylmethoxy)-1 -isopropyl-6,7-dihydro-pyrido [2,1-a]isoquinolin-4-one,
   1-cyclopropyl-2-((S)-1-[1,4]dioxan-2-ylmethoxy)-9-(3-methyl-but-1-ynyl)-6,7-dihydro-pyrido[2,1-a]isoquinolin-4-one,
   2-([1,4]dioxan-2-ylmethoxy)-9-(2-methoxy-ethoxy)-1-methyl-6,7-dihydro-pyrido[2,1-a]isoquinolin-4-one,
   2-((S)-1-[1,4]dioxan-2-ylmethoxy)-1 -isopropyl-9-(3 -methyl-but-1 -ynyl)-6,7-dihydro-pyrido [2,1-a]isoquinolin-4-one,
   9-cyclopropylethynyl-2-((S)-1-[1,4]dioxan-2-ylmethoxy)-1 -isopropyl-6,7-dihydro-pyrido [2,1-a]isoquinolin-4-one,
   2-((S)-1-[1,4]dioxan-2-ylmethoxy)-9-hydroxy-1 -methyl-6,7-dihydro-pyrido [2, 1 -a] isoquinolin-4-one,
   trifluoro-methanesulfonic acid 2-((S)-1-[1,4]dioxan-2-ylmethoxy)-1-methyl-4-oxo-6,7-dihydro-4H-pyrido[2, 1-a]isoquinolin-9-yl ester,
   2-((S)-1-[1,4]dioxan-2-ylmethoxy)-1-ethyl-9-hydroxy-6,7-dihydro-pyrido [2,1-a] isoquinolin-4-one,
   9-methoxy-2-[(tetrahydro-furan-2-ylmethyl)-amino]-6,7-dihydro-pyrido[2,1-a]isoquinolin-4-one,
   10-hydroxy-9-methoxy-1-methyl-2-[(tetrahydro-pyran-2-ylmethyl)-amino] -6, 7-dihydro-pyrido[2,1-a]isoquinolin-4-one,
   2-[([1,4]dioxan-2-ylmethyl)-amino]-10-hydroxy-9-methoxy-1-methyl-6,7-dihydro-pyrido[2,1-a]isoquinolin-4-one,
   10-hydroxy-9-methoxy-1-methyl-2-(tetrahydro-pyran-2-ylmethoxy)-6, 7-dihydro-pyrido [2,1-a]isoquinolin-4-one, and
   10-benzyloxy-2-((S)-1-[1,4]dioxan-2-ylmethoxy)-9-methoxy-1-methyl-6,7-dihydro-pyrido[2,1-a]isoquinolin-4-one.
11. The compound or pharmaceutically acceptable salt thereof for use according to clause 1, wherein said compound is selected from:
   4-[[(2S)-1,4-dioxan-2-yl]methoxy]-9,10-dimethoxy-1-methyl-6,7-dihydrobenzo[a]quinolizin-2-one,
   9,10-dimethoxy-1-methyl-4-(tetrahydrofuran-2-ylmethylamino)-6,7-dihydrobenzo[a]quinolizin-2-one,
   1-ethyl-9-hydroxy-4-(tetrahydrofuran-2-ylmethylamino)-6,7-dihydrobenzo[a]quinolizin-2-one,
   9,10-Dimethoxy-4-[(tetrahydro-furan-2-ylmethyl)-amino]-6,7-dihydro-pyrido[2,1-a]isoquinolin-2-one,
   4-[([1,4]Dioxan-2-ylmethyl)-amino]-9,10-dimethoxy-6,7-dihydro-pyrido[2,1-a]isoquinolin-2-one,
   4-[[(2R)-1,4-dioxan-2-yl]methylamino]-9,10-dimethoxy-1-methyl-6,7-dihydrobenzo[a]quinolizin-2-one,
   9-(2,2-Difluoro-cyclopropylmethoxy)-1-methyl-4-[(tetrahydro-furan-2-ylmethyl)-amino]-6,7-dihydro-pyrido [2,1 -a] isoquinolin-2-one,
   1-methyl-4-(tetrahydrofuran-2-ylmethylamino)-9-(2,2,2-trifluoroethoxy)-6,7-dihydrobenzo[a]quinolizin-2-one,
   -Methyl-4-[(tetrahydro-furan-2-ylmethyl)-amino]-9-(tetrahydro-furan-2-yloxy)-6,7-dihydro-pyrido[2,1-a]isoquinolin-2-one,
   8,9-dimethoxy-1-methyl-4-(tetrahydrofuran-2-ylmethylamino)-6,7-dihydrobenzo[a]quinolizin-2-one,
   4-[[(2S)-1,4-dioxan-2-yl]methoxy]-8,9-dimethoxy-1-methyl-6,7-dihydrobenzo[a]quinolizin-2-one,
   9-(2,2-difluoroethoxy)-1-methyl-4-(tetrahydropyran-2-ylmethylamino)-6,7-dihydrobenzo[a]quinolizin-2-one,
   1-methyl-4-(tetrahydropyran-2-ylmethylamino)-9-(2,2,2-trifluoroethoxy)-6,7-dihydrobenzo[a]quinolizin-2-one,
   8,9-dimethoxy-1-methyl-4-(tetrahydropyran-2-ylmethylamino)-6,7-dihydrobenzo[a]quinolizin-2-one,
   4-(1,4-dioxan-2-ylmethylamino)-8,9-dimethoxy-1-methyl-6,7-dihydrobenzo[a]quinolizin-2-one,
   4-[[(2S)-1,4-dioxan-2-yl]methoxy]-1-methyl-9-(2,2,2-trifluoroethoxy)-6,7-dihydrobenzo[a]quinolizin-2-one,
   4-(1,4-dioxan-2-ylmethylamino)-8,9-dimethoxy-6,7-dihydrobenzo[a]quinolizin-2-one,
   8,9-dimethoxy-4-(tetrahydrofuran-2-ylmethylamino)-6,7-dihydrobenzo[a]quinolizin-2-one,
   4-(1,4-dioxan-2-ylmethylamino)-9-hydroxy-8-methoxy-6,7-dihydrobenzo[a]quinolizin-2-one,
   4-(1,4-dioxan-2-ylmethylamino)-8-hydroxy-9-methoxy-6,7-dihydrobenzo[a]quinolizin-2-one,
   9-(2,2-Difluoro-ethoxy)-4-[([1,4]dioxan-2-ylmethyl)-amino]-8-methoxy-1-methyl-6,7-dihydro-pyrido [2,1 -a] isoquinolin-2-one,
   9-(2,2-Difluoro-ethoxy)-8-methoxy-1-methyl-4-[(tetrahydro-furan-2-ylmethyl)-amino]-6,7-dihydro-pyrido[2,1-a]isoquinolin-2-one,
   4-[[(2S)-1,4-dioxan-2-yl]methoxy]-1-methyl-2-oxo-6,7-dihydrobenzo[a]quinolizine-9-carbonitrile,
   9-(2,2-difluoroethoxy)-1-ethyl-4-(tetrahydrofuran-2-ylmethylamino)-6,7-dihydrobenzo[a]quinolizin-2-one,
   1-ethyl-4-(tetrahydrofuran-2-ylmethylamino)-9-(2,2,2-trifluoroethoxy)-6,7-dihydrobenzo[a]quinolizin-2-one,
   8,9-dimethoxy-1-methyl-4-(tetrahydrofuran-2-ylmethoxy)-6,7-dihydrobenzo[a]quinolizin-2-one,
   8,9-dimethoxy-1-methyl-4-(tetrahydropyran-2-ylmethoxy)-6,7-dihydrobenzo[a]quinolizin-2-one,
   4-[[(2S)-1,4-dioxan-2-yl]methoxy]-1-methyl-9-(tetrahydropyran-4-ylmethoxy)-6,7-dihydrobenzo[a]quinolizin-2-one,
   4-[[(2S)-1,4-dioxan-2-yl]methoxy]-1-methyl-9-(2-pyridylmethoxy)-6,7-dihydrobenzo[a]quinolizin-2-one,
   4-[[(2S)-1,4-dioxan-2-yl]methoxy]-1-methyl-9-[[3-(trifluoromethoxy)phenyl]methoxy]-6,7-dihydrobenzo[a]quinolizin-2-one,
   9-benzyloxy-4-[[(2S)-1,4-dioxan-2-yl]methoxy]-1-methyl-6,7-dihydrobenzo[a]quinolizin-2-one,
   9-benzyloxy-4-[[(2R)-1,4-dioxan-2-yl]methylamino]-1-methyl-6,7-dihydrobenzo[a]quinolizin-2-one,
   9-(2,2-difluoroethoxy)-4-[[(2R)-1,4-dioxan-2-yl]methylamino]-1-methyl-6,7-dihydrobenzo[a]quinolizin-2-one,
   4-[[(2R)-1,4-dioxan-2-yl]methylamino]-1-methyl-9-(2,2,2-trifluoroethoxy)-6,7-dihydrobenzo[a]quinolizin-2-one,
   [4-[[(2S)-1,4-dioxan-2-yl]methoxy]-1-methyl-2-oxo-6,7-dihydrobenzo[a]quinolizin-9-yl] trifluoromethanesulfonate,
   4-[[(2S)-1,4-dioxan-2-yl]methoxy]-1-methyl-9-[(1-methylpyrazol-4-yl)methoxy]-6,7-dihydrobenzo[a]quinolizin-2-one,
   9-(3,6-dihydro-2H-pyran-4-yl)-4-[[(2S)-1,4-dioxan-2-yl]methoxy]-1-methyl-6,7-dihydrobenzo[a]quinolizin-2-one,
   4-[[(2S)-1,4-dioxan-2-yl]methoxy]-9-(1-ethylpyrazol-4-yl)-1-methyl-6,7-dihydrobenzo[a]quinolizin-2-one,
   4-[[(2S)-1,4-dioxan-2-yl]methoxy]-1-methyl-9-tetrahydropyran-4-yl-6,7-dihydrobenzo[a]quinolizin-2-one,
   1-methyl-4-[[(2S)-tetrahydrofuran-2-yl]methylamino]-9-(2,2,2-trifluoroethoxy)-6,7-dihydrobenzo[a]quinolizin-2-one,
   1-methyl-9-[(3-methyl-1,2,4-oxadiazol-5-yl)methoxy]-4-(tetrahydrofuran-2-ylmethoxy)-6,7-dihydrobenzo[a]quinolizin-2-one,
   1-methyl-9-[(3-methyl-1,2,4-oxadiazol-5-yl)methoxy]-4-(tetrahydropyran-2-ylmethoxy)-6,7-dihydrobenzo[a]quinolizin-2-one,
   1-methyl-9-(2-pyridylmethoxy)-4-(tetrahydrofuran-2-ylmethoxy)-6,7-dihydrobenzo[a]quinolizin-2-one,
   1-methyl-9-(2-pyridylmethoxy)-4-(tetrahydropyran-2-ylmethoxy)-6,7-dihydrobenzo[a]quinolizin-2-one,
   1-methyl-4-(tetrahydrofuran-2-ylmethoxy)-9-(tetrahydropyran-3 -ylmethoxy)-6, 7-dihydrobenzo[a]quinolizin-2-one,
   1-methyl-4-(tetrahydropyran-2-ylmethoxy)-9-(tetrahydropyran-3-ylmethoxy)-6,7-dihydrobenzo[a]quinolizin-2-one,
   4-[[(2S)-1,4-dioxan-2-yl]methoxy]-1-methyl-9-(tetrahydropyran-3-ylmethoxy)-6,7-dihydrobenzo[a]quinolizin-2-one,
   1-methyl-9-[(6-methyl-3-pyridyl)methoxy]-4-(tetrahydrofuran-2-ylmethoxy)-6,7-dihydrobenzo[a]quinolizin-2-one,
   1-methyl-9-[(6-methyl-3-pyridyl)methoxy]-4-(tetrahydropyran-2-ylmethoxy)-6,7-dihydrobenzo[a]quinolizin-2-one,
   4-[[(2S)-1,4-dioxan-2-yl]methoxy]-1-methyl-9-[(6-methyl-3-pyridyl)methoxy]-6,7-dihydrobenzo[a]quinolizin-2-one,
   9-(2-dimethylaminoethyloxy)-1-methyl-4-(tetrahydrofuran-2-ylmethoxy)-6,7-dihydrobenzo[a]quinolizin-2-one,
   9-(2-dimethylaminoethyloxy)-1-methyl-4-(tetrahydropyran-2-ylmethoxy)-6,7-dihydrobenzo[a]quinolizin-2-one,
   9-(2-dimethylaminoethyloxy)-4-[[(2S)-1,4-dioxan-2-yl]methoxy]-1-methyl-6,7-dihydrobenzo[a]quinolizin-2-one,
   [4-[[(2S)-1,4-dioxan-2-yl]methoxy]-1-methyl-2-oxo-6,7-dihydrobenzo[a]quinolizin-9-yl] methanesulfonate,
   1-methyl-9-(2-pyridylmethoxy)-4-[[(2S)-tetrahydrofuran-2-yl]methoxy]-6,7-dihydrobenzo[a]quinolizin-2-one,
   4-[[(2S)-1,4-dioxan-2-yl]methoxy]-1-methyl-9-[(3-methyl-1,2,4-oxadiazol-5-yl)methoxy]-6,7-dihydrobenzo[a]quinolizin-2-one,
   9-(difluoromethoxy)-4-[[(2S)-1,4-dioxan-2-yl]methoxy]-1-methyl-6,7-dihydrobenzo[a]quinolizin-2-one,
   tert-butyl 4-[4-[[(2S)-1,4-dioxan-2-yl]methoxy]-1-methyl-2-oxo-6,7-dihydrobenzo[a]quinolizin-9-yl]piperazine-1 -carboxylate,
   9-(2,2-difluoroethoxy)-4-[[(2S)-1,4-dioxan-2-yl]methoxy]-1-methyl-6,7-dihydrobenzo[a]quinolizin-2-one,
   4,9-bis[[(2S)-1,4-dioxan-2-yl]methoxy]-1-methyl-6,7-dihydrobenzo[a]quinolizin-2-one,
   4-[[(2S)-1,4-dioxan-2-yl]methoxy]-1-methyl-9-morpholino-6,7-dihydrobenzo[a]quinolizin-2-one,
   4-[[(2S)-1,4-dioxan-2-yl]methoxy]-1-methyl-9-phenylsulfanyl-6,7-dihydrobenzo[a]quinolizin-2-one,
   9-(4,4-difluoro-1-piperidyl)-4-[[(2S)-1,4-dioxan-2-yl]methoxy]-1-methyl-6,7-dihydrobenzo[a]quinolizin-2-one,
   4-[[(2S)-1,4-dioxan-2-yl]methoxy]-1-methyl-9-piperazin-1-yl-6,7-dihydrobenzo[a]quinolizin-2-one,
   9-(benzenesulfonyl)-4-[[(2S)-1,4-dioxan-2-yl]methoxy]-1-methyl-6,7-dihydrobenzo[a]quinolizin-2-one,
   4-[[(2S)-1,4-dioxan-2-yl]methoxy]-1-methyl-9-(4-methylsulfonylpiperazin-1-yl)-6,7-dihydrobenzo[a]quinolizin-2-one,
   9-[4-(cyclopropanecarbonyl)piperazin-1-yl]-4-[[(2S)-1,4-dioxan-2-yl]methoxy]-1-methyl-6,7-dihydrobenzo[a]quinolizin-2-one,
   N-[4-[[(2S)-1,4-dioxan-2-yl]methoxy]-1-methyl-2-oxo-6,7-dihydrobenzo[a]quinolizin-9-yl]cyclopropanecarboxamide,
   tert-butyl 3-[4-[[(2S)-1,4-dioxan-2-yl]methoxy]-1-methyl-2-oxo-6,7-dihydrobenzo[a]quinolizin-9-yl]azetidine-1-carboxylate,
   4-[[(2S)-1,4-dioxan-2-yl]methoxy]-1-methyl-9-[2-(trifluoromethoxy)ethoxy]-6,7-dihydrobenzo[a]quinolizin-2-one,
   N-[4-[[(2S)-1,4-dioxan-2-yl]methoxy]-1-methyl-2-oxo-6,7-dihydrobenzo[a]quinolizin-9-yl]-N-methyl-cyclopropanecarboxamide,
   tert-butyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1-carboxylate,
   tert-butyl 4-[4-[[(2S)-1,4-dioxan-2-yl]methoxy]-1-methyl-2-oxo-6,7-dihydrobenzo[a]quinolizin-9-yl]piperidine-1-carboxylate,
   methyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1-carboxylate,
   ethyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1-carboxylate,
   isopropyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1-carboxylate,
   2,2,2-trifluoroethyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1-carboxylate,
   methyl 4-[4-[[(2S)-1,4-dioxan-2-yl]methoxy]-1-methyl-2-oxo-6,7-dihydrobenzo[a]quinolizin-9-yl]piperidine-1-carboxylate,
   ethyl 4-[4-[[(2S)-1,4-dioxan-2-yl]methoxy]-1-methyl-2-oxo-6,7-dihydrobenzo[a]quinolizin-9-yl]piperidine-1 -carboxylate,
   isopropyl 4-[4-[[(2S)-1,4-dioxan-2-yl]methoxy]-1-methyl-2-oxo-6,7-dihydrobenzo[a]quinolizin-9-yl]piperidine-1 -carboxylate,
   2,2,2-trifluoroethyl 4-[4-[[(2S)-1,4-dioxan-2-yl]methoxy]-1-methyl-2-oxo-6,7-dihydrobenzo[a]quinolizin-9-yl]piperidine-1-carboxylate,
   N-cyclopropyl-4-[[(2S)-1,4-dioxan-2-yl]methoxy]-1-methyl-2-oxo-6,7-dihydrobenzo[a]quinolizine-9-carboxamide,
   4-[[(2S)-1,4-dioxan-2-yl]methoxy]-9-hydroxy-1-methyl-6,7-dihydrobenzo[a]quinolizin-2-one,
   9-(3,3-difluoroazetidin-1-yl)-4-[[(2S)-1,4-dioxan-2-yl]methoxy]-1-methyl-6,7-dihydrobenzo[a]quinolizin-2-one,
   4-[[(2S)-1,4-dioxan-2-yl]methoxy]-1-methyl-9-(6-oxa-2-azaspiro[3.3]heptan-2-yl)-6,7-dihydrobenzo[a]quinolizin-2-one,
   N-cyclopropyl-4-[[(2S)-1,4-dioxan-2-yl]methoxy]-N,1-dimethyl-2-oxo-6,7-dihydrobenzo[a]quinolizine-9-carboxamide,
   4-[[(2S)-1,4-dioxan-2-yl]methoxy]-9-methoxy-1-methyl-6,7-dihydrobenzo[a]quinolizin-2-one,
   4-(1,4-dioxan-2-ylmethoxy)-1-methyl-9-(2,2,2-trifluoroethoxy)-6,7-dihydrobenzo[a]quinolizin-2-one,
   4-[[(2S)-1,4-dioxan-2-yl]methoxy]-9-(3-fluoroazetidin-1-yl)-1-methyl-6,7-dihydrobenzo[a]quinolizin-2-one,
   4-[[(2S)-1,4-dioxan-2-yl]methoxy]-9-[3-(1-hydroxy-1-methyl-ethyl)azetidin-1-yl]-1-methyl-6,7-dihydrobenzo[a]quinolizin-2-one,
   9-(azetidin-1-yl)-4-[[(2S)-1,4-dioxan-2-yl]methoxy]-1-methyl-6,7-dihydrobenzo[a]quinolizin-2-one,
   4-[[(2S)-1,4-dioxan-2-yl]methoxy]-1-methyl-9-(3-methylsulfonylazetidin-1-yl)-6,7-dihydrobenzo[a]quinolizin-2-one,
   4-[[(2S)-1,4-dioxan-2-yl]methoxy]-1-methyl-9-(3-pyrazol-1-ylazetidin-1-yl)-6,7-dihydrobenzo[a]quinolizin-2-one,
   9-(3,3-dimethylazetidin-1-yl)-4-[[(2S)-1,4-dioxan-2-yl]methoxy]-1-methyl-6,7-dihydrobenzo[a]quinolizin-2-one,
   methyl 1-[4-[[(2S)-1,4-dioxan-2-yl]methoxy]-1-methyl-2-oxo-6,7-dihydrobenzo[a]quinolizin-9-yl]azetidine-3-carboxylate,
   4-[[(2S)-1,4-dioxan-2-yl]methoxy]-1-methyl-9-(3-pyridyl)-6,7-dihydrobenzo[a]quinolizin-2-one,
   4-[[(2S)-1,4-dioxan-2-yl]methoxy]-9-(3-fluorophenyl)-1-methyl-6,7-dihydrobenzo[a]quinolizin-2-one,
   4-[[(2S)-1,4-dioxan-2-yl]methoxy]-1-methyl-9-(4-pyridyl)-6,7-dihydrobenzo[a]quinolizin-2-one,
   4-[[(2S)-1,4-dioxan-2-yl]methoxy]-1-methyl-9-(2-pyridyl)-6,7-dihydrobenzo[a]quinolizin-2-one,
   4-[[(2S)-1,4-dioxan-2-yl]methoxy]-1-methyl-9-(3-methyl-2-pyridyl)-6,7-dihydrobenzo[a]quinolizin-2-one,
   4-[[(2S)-1,4-dioxan-2-yl]methoxy]-1-methyl-9-(4-methyl-2-pyridyl)-6,7-dihydrobenzo[a]quinolizin-2-one,
   tert-butyl 3-[[4-[[(2S)-1,4-dioxan-2-yl]methoxy]-1-methyl-2-oxo-6,7-dihydrobenzo[a]quinolizin-9-yl]oxy]azetidine-1-carboxylate,
   3-deuterio-9-(1-deuterio-2,2-difluoro-vinyloxy)-4-[[(2S)-1,4-dioxan-2-yl]methoxy]-1-methyl-6,7-dihydrobenzo[a]quinolizin-2-one,
   9-(1,1-dideuterio-2,2,2-trifluoro-ethoxy)-4-[[(2S)-1,4-dioxan-2-yl]methoxy]-1-methyl-6,7-dihydrobenzo[a]quinolizin-2-one,
   9-benzyloxy-1-methyl-4-(oxetan-2-ylmethoxy)-6,7-dihydrobenzo[a]quinolizin-2-one,
   9-benzyloxy-1-methyl-4-[[(2S)-tetrahydrofuran-2-yl]methoxy]-6,7-dihydrobenzo[a]quinolizin-2-one,
   9-benzyloxy-1-methyl-4-(tetrahydropyran-2-ylmethoxy)-6,7-dihydrobenzo[a]quinolizin-2-one,
   4-[[(2S)-1,4-dioxan-2-yl]methoxy]-9-(3-methoxyazetidin-1-yl)-1-methyl-6,7-dihydrobenzo[a]quinolizin-2-one,
   4-[[(2S)-1,4-dioxan-2-yl]methoxy]-9-(4-methoxy-1-piperidyl)-1-methyl-6,7-dihydrobenzo[a]quinolizin-2-one,
   4-[[(2S)-1,4-dioxan-2-yl]methoxy]-1-methyl-9-[4-(piperidine-1-carbonyl)-1-piperidyl]-6,7-dihydrobenzo[a]quinolizin-2-one,
   4-[[(2S)-1,4-dioxan-2-yl]methoxy]-1-methyl-9-(4-phenyl-1-piperidyl)-6,7-dihydrobenzo[a]quinolizin-2-one,
   methyl 1-[4-[[(2S)-1,4-dioxan-2-yl]methoxy]-1-methyl-2-oxo-6,7-dihydrobenzo[a]quinolizin-9-yl]piperidine-4-carboxylate,
   4-[[(2S)-1,4-dioxan-2-yl]methoxy]-9-[4-(ethoxymethyl)-1-piperidyl]-1-methyl-6,7-dihydrobenzo[a]quinolizin-2-one,
   4-[[(2S)-1,4-dioxan-2-yl]methoxy]-1-methyl-9-(1-piperidyl)-6,7-dihydrobenzo[a]quinolizin-2-one,
   4-[[(2S)-1,4-dioxan-2-yl]methoxy]-1-methyl-9-(3-methyl-1-piperidyl)-6,7-dihydrobenzo[a]quinolizin-2-one,
   4-[[(2S)-1,4-dioxan-2-yl]methoxy]-9-[4-(4-fluorophenyl)-1-piperidyl]-1-methyl-6,7-dihydrobenzo[a]quinolizin-2-one,
   9-[1-(cyclopropanecarbonyl)azetidin-3-yl]oxy-4-[[(2S)-1,4-dioxan-2-yl]methoxy]-1-methyl-6,7-dihydrobenzo[a]quinolizin-2-one,
   4-[[(2S)-1,4-dioxan-2-yl]methoxy]-1-methyl-9-[1-(2,2,2-trifluoroacetyl)azetidin-3-yl]oxy-6,7-dihydrobenzo[a]quinolizin-2-one,
   ethyl 3-[[4-[[(2S)-1,4-dioxan-2-yl]methoxy]-1-methyl-2-oxo-6,7-dihydrobenzo[a]quinolizin-9-yl]oxy]azetidine-1-carboxylate,
   4-[[(2S)-1,4-dioxan-2-yl]methoxy]-1-methyl-9-[4-(3-pyridyloxy)-1-piperidyl]-6,7-dihydrobenzo[a]quinolizin-2-one,
   1-[4-[[(2S)-1,4-dioxan-2-yl]methoxy]-1-methyl-2-oxo-6,7-dihydrobenzo[a]quinolizin-9-yl]piperidine-4-carbonitrile,
   9-(3,3-difluoro-1-piperidyl)-4-[[(2S)-1,4-dioxan-2-yl]methoxy]-1-methyl-6,7-dihydrobenzo[a]quinolizin-2-one,
   4-[[(2S)-1,4-dioxan-2-yl]methoxy]-9-isopropyl-1-methyl-6,7-dihydrobenzo[a]quinolizin-2-one,
   3-deuterio-4-[[(2S)-1,4-dioxan-2-yl]methoxy]-1-methyl-9-(2,2,2-trifluoroethoxy)-6,7-dihydrobenzo[a]quinolizin-2-one,
   3-deuterio-4-[[(2S)-1,4-dioxan-2-yl]methoxy]-1-methyl-9-(1,1,2,2-tetradeuterio-2-fluoro-ethoxy)-6,7-dihydrobenzo[a]quinolizin-2-one,
   tert-butyl 3-[[4-[[(2S)-1,4-dioxan-2-yl]methoxy]-1-methyl-2-oxo-6,7-dihydrobenzo[a]quinolizin-9-yl]oxy]pyrrolidine-1-carboxylate,
   tert-butyl 4-[[4-[[(2S)-1,4-dioxan-2-yl]methoxy]-1-methyl-2-oxo-6,7-dihydrobenzo[a]quinolizin-9-yl]oxy]piperidine-1-carboxylate,
   4-[[(2S)-1,4-dioxan-2-yl]methoxy]-1-methyl-9-[methyl(3,3,3-trifluoropropyl)amino]-6,7-dihydrobenzo[a]quinolizin-2-one,
   4-[[(2S)-1,4-dioxan-2-yl]methoxy]-1-methyl-9-pyrrolidin-1-yl-6,7-dihydrobenzo[a]quinolizin-2-one,
   9-(3,3-difluoropyrrolidin-1-yl)-4-[[(2S)-1,4-dioxan-2-yl]methoxy]-1-methyl-6,7-dihydrobenzo[a]quinolizin-2-one,
   4-[[(2S)-1,4-dioxan-2-yl]methoxy]-1-methyl-9-[3-(trifluoromethyl)azetidin-1-yl]-6,7-dihydrobenzo[a]quinolizin-2-one,
   4-[[(2S)-1,4-dioxan-2-yl]methoxy]-1-methyl-9-[4-(trifluoromethyl)-3,6-dihydro-2H-pyridin-1-yl]-6,7-dihydrobenzo[a]quinolizin-2-one,
   4-[[(2S)-1,4-dioxan-2-yl]methoxy]-1-methyl-9-[(2R)-2-methylpyrrolidin-1-yl]-6,7-dihydrobenzo[a]quinolizin-2-one,
   4-[[(2S)-1,4-dioxan-2-yl]methoxy]-9-(3-fluoro-1-piperidyl)-1-methyl-6,7-dihydrobenzo[a]quinolizin-2-one,
   9-carbazol-9-yl-4-[[(2S)-1,4-dioxan-2-yl]methoxy]-1-methyl-6,7-dihydrobenzo[a]quinolizin-2-one,
   9-(3,5-dimethyl-1-piperidyl)-4-[[(2S)-1,4-dioxan-2-yl]methoxy]-1-methyl-6,7-dihydrobenzo[a]quinolizin-2-one,
   9-(3,3-dimethylpyrrolidin-1-yl)-4-[[(2S)-1,4-dioxan-2-yl]methoxy]-1-methyl-6,7-dihydrobenzo[a]quinolizin-2-one,
   9-(4,4-dimethyl-1-piperidyl)-4-[[(2S)-1,4-dioxan-2-yl]methoxy]-1-methyl-6,7-dihydrobenzo[a]quinolizin-2-one,
   4-[[(2S)-1,4-dioxan-2-yl]methoxy]-1-methyl-9-[4-(trifluoromethyl)-1-piperidyl]-6,7-dihydrobenzo[a]quinolizin-2-one,
   4-[[(2S)-1,4-dioxan-2-yl]methoxy]-1-methyl-9-(4-methyl-1-piperidyl)-6,7-dihydrobenzo[a]quinolizin-2-one,
   4-[[(2S)-1,4-dioxan-2-yl]methoxy]-1-methyl-9-(2,2,2-trifluoroethylamino)-6,7-dihydrobenzo[a]quinolizin-2-one,
   4-[[(2S)-1,4-dioxan-2-yl]methoxy]-1-methyl-9-(2-methyl-1-piperidyl)-6,7-dihydrobenzo[a]quinolizin-2-one,
   9-[1-(cyclopropanecarbonyl)pyrrolidin-3-yl]oxy-4-[[(2S)-1,4-dioxan-2-yl]methoxy]-1-methyl-6,7-dihydrobenzo[a]quinolizin-2-one,
   ethyl 3-[[4-[[(2S)-1,4-dioxan-2-yl]methoxy]-1-methyl-2-oxo-6,7-dihydrobenzo[a]quinolizin-9-yl]oxy]pyrrolidine-1-carboxylate,
   9-[1-(cyclopropanecarbonyl)azetidin-3-yl]-4-[[(2S)-1,4-dioxan-2-yl]methoxy]-1-methyl-6,7-dihydrobenzo[a]quinolizin-2-one,
   ethyl 3-[4-[[(2S)-1,4-dioxan-2-yl]methoxy]-1-methyl-2-oxo-6,7-dihydrobenzo[a]quinolizin-9-yl]azetidine-1-carboxylate,
   3-[4-[[(2S)-1,4-dioxan-2-yl]methoxy]-1-methyl-2-oxo-6,7-dihydrobenzo[a]quinolizin-9-yl]-N,N-dimethyl-azetidine-1-carboxamide,
   3-[4-[[(2S)-1,4-dioxan-2-yl]methoxy]-1-methyl-2-oxo-6,7-dihydrobenzo[a]quinolizin-9-yl]-N-isopropyl-azetidine-1-carboxamide,
   3-[[4-[[(2S)-1,4-dioxan-2-yl]methoxy]-1-methyl-2-oxo-6,7-dihydrobenzo[a]quinolizin-9-yl]oxy]-N,N-dimethyl-azetidine-1-carboxamide,
   3-[4-[[(2S)-1,4-dioxan-2-yl]methoxy]-1-methyl-2-oxo-6,7-dihydrobenzo[a]quinolizin-9-yl]-N-isopropyl-azetidine-1-carboxamide,
   9-benzyloxy-4-[(4,4-dimethyloxetan-2-yl)methoxy]-1-methyl-6,7-dihydrobenzo[a]quinolizin-2-one,
   9-benzyloxy-1-methyl-4-[(2-methyltetrahydrofuran-2-yl)methoxy]-6,7-dihydrobenzo[a]quinolizin-2-one,
   9-benzyloxy-4-[(5,5-dimethyltetrahydrofuran-2-yl)methoxy]-1-methyl-6,7-dihydrobenzo[a]quinolizin-2-one,
   4-[[(2S)-1,4-dioxan-2-yl]methoxy]-1-methyl-9-(2,2,3,3,3-pentafluoropropoxy)-6,7-dihydrobenzo[a]quinolizin-2-one,
   4-[[(2S)-1,4-dioxan-2-yl]methoxy]-1-methyl-6,7-dihydrobenzo[a]quinolizin-2-one,
   4-[[(2S)-1,4-dioxan-2-yl]methoxy]-1-methyl-9-(2-oxopyrrolidin-1-yl)-6,7-dihydrobenzo[a]quinolizin-2-one, and
   3-deuterio-4-[[(2S)-1,4-dioxan-2-yl]methoxy]-1-methyl-9-(1,1,2,2-tetradeuterio-2-fluoro-ethoxy)-6,7-dihydrobenzo[a]quinolizin-2-one.
12. A pharmaceutical composition for use in the prophylaxis and/or treatment of one or more fibrotic diseases, comprising a compound or pharmaceutically acceptable salt thereof according to anyone of clauses 1-11, and a pharmaceutically acceptable carrier.
13. A pharmaceutical composition for use according to clause 12, comprising a further therapeutic agent.
14. A compound or pharmaceutically acceptable salt thereof for use according to any one of clauses 1-11 or a pharmaceutical composition for use according to clause 12, wherein the fibrotic disease is NASH.
15. A compound or pharmaceutically acceptable salt thereof for use according to any one of clauses 1-11 or a pharmaceutical composition for use according to clause 12, wherein the fibrotic disease is IPF.
16. A pharmaceutical composition for use according to clause 13, wherein the further therapeutic agent is a fibrotic disease treatment agent.

### FINAL REMARKS

It will be appreciated by those skilled in the art that the foregoing descriptions are exemplary and explanatory in nature, and intended to illustrate the invention and its preferred embodiments. Through routine experimentation, an artisan will recognize apparent modifications and variations that may be made without departing from the spirit of the invention. All such modifications coming within the scope of the appended claims are intended to be included therein. Thus, the invention is intended to be defined not by the above description, but by the following claims and their equivalents.

All publications, including but not limited to patents and patent applications, cited in this specification are herein incorporated by reference as if each individual publication are specifically and individually indicated to be incorporated by reference herein as though fully set forth.

It should be understood that factors such as the differential cell penetration capacity of the various compounds can contribute to discrepancies between the activity of the compounds in the *in vitro* biochemical and cellular assays.

At least some of the chemical names of compound of the invention as given and set forth in this application, may have been generated on an automated basis by use of a commercially available chemical naming software program, and have not been independently verified. Representative programs performing this function include the Lexichem naming tool sold by Open Eye Software, Inc. and the Autonom Software tool sold by MDL, Inc. In the instance where the indicated chemical name and the depicted structure differ, the depicted structure will control.

### REFERENCES

Ashcroft, T., Simpson, J.M., Timbrell, V., 1988. Simple method of estimating severity of pulmonary fibrosis on a numerical scale. J. Clin. Pathol. 41, 467-470.
Bickelhaupt, S., Erbel, C., Timke, C., Wirkner, U., Dadrich, M., Flechsig, P., Tietz, A., Pföhler, J., Gross, W., Peschke, P., Hoeltgen, L., Katus, H.A., Gröne, H.-J., Nicolay, N.H., Saffrich, R., Debus, J., Sternlicht, M.D., Seeley, T.W., Lipson, K.E., Huber, P.E., 2017. Effects of CTGF Blockade on Attenuation and Reversal of Radiation-Induced Pulmonary Fibrosis. JNCI J. Natl. Cancer Inst. 109. https://doi.org/10.1093/jnci/djw339
Brunnemer, E., Wälscher, J., Tenenbaum, S., Hausmanns, J., Schulze, K., Setter, M., Heussel, C.P., Warth, A., Herth, F.J.F., Kreuter, M., 2018. Real-World Experience with Nintedanib in Patients with Idiopathic Pulmonary Fibrosis. Respiration 95, 301-309. https://doi.org/10.1159/000485933
Brunt, E.M., Kleiner, D.E., Wilson, L.A., Belt, P., Neuschwander-Tetri, B.A., 2011. The NAS and The Histopathologic Diagnosis in NAFLD: Distinct Clinicopathologic Meanings. Hepatol. Baltim. Md 53, 810-820. https://doi.org/10.1002/hep.24127
Day, C.P., James, O.F.W., 1998. Hepatic steatosis: Innocent bystander or guilty party? Hepatology 27, 1463-1466. https://doi.org/10.1002/hep.510270601
Devos, F.C., Maaske, A., Robichaud, A., Pollaris, L., Seys, S., Lopez, C.A., Verbeken, E., Tenbusch, M., Lories, R., Nemery, B., Hoet, P.H., Vanoirbeek, J.A., 2017. Forced expiration measurements in mouse models of obstructive and restrictive lung diseases. Respir. Res. 18, 123. https://doi.org/10.1186/s12931-017-0610-1
Fujii, T., Fuchs, B.C., Yamada, S., Lauwers, G.Y., Kulu, Y., Goodwin, J.M., Lanuti, M., Tanabe, K.K., 2010. Mouse model of carbon tetrachloride induced liver fibrosis: Histopathological changes and expression of CD133 and epidermal growth factor. BMC Gastroenterol. 10, 79. https://doi.org/10.1186/1471-230X-10-79
Inghilleri, S., Morbini, P., Oggionni, T., Barni, S., Fenoglio, C., 2006. In situ assessment of oxidant and nitrogenic stress in bleomycin pulmonary fibrosis. Histochem. Cell Biol. 125, 661-669. https://doi.org/10.1007/s00418-005-0116-7
Kleiner, D.E., Brunt, E.M., Van Natta, M., Behling, C., Contos, M.J., Cummings, O.W., Ferrell, L.D., Liu, Y.-C., Torbenson, M.S., Unalp-Arida, A., Yeh, M., McCullough, A.J., Sanyal, A.J., Nonalcoholic Steatohepatitis Clinical Research Network, 2005. Design and validation of a histological scoring system for nonalcoholic fatty liver disease. Hepatol. Baltim. Md 41, 1313-1321. https://doi.org/10.1002/hep.20701
Lancaster, L.H., Andrade, J.A. de, Zibrak, J.D., Padilla, M.L., Albera, C., Nathan, S.D., Wijsenbeek, M.S., Stauffer, J.L., Kirchgaessler, K.-U., Costabel, U., 2017. Pirfenidone safety and adverse event management in idiopathic pulmonary fibrosis. Eur. Respir. Rev. 26, 170057. https://doi.org/10.1183/16000617.0057-2017
Marra, F., DeFranco, R., Grappone, C., Milani, S., Pastacaldi, S., Pinzani, M., Romanelli, R.G., Laffi, G., Gentilini, P., 1998. Increased expression of monocyte chemotactic protein-1 during active hepatic fibrogenesis: correlation with monocyte infiltration. Am. J. Pathol. 152, 423-430.
Matsuse, T., Teramoto, S., Katayama, H., Sudo, E., Ekimoto, H., Mitsuhashi, H., Uejima, Y., Fukuchi, Y., Ouchi, Y., 1999. ICAM-1 mediates lung leukocyte recruitment but not pulmonary fibrosis in a murine model of bleomycin-induced lung injury. Eur. Respir. J. 13, 71-77.
Matsuzawa, Y., Kawashima, T., Kuwabara, R., Hayakawa, S., Irie, T., Yoshida, T., Rikitake, H., Wakabayashi, T., Okada, N., Kawashima, K., Suzuki, Y., Shirai, K., 2015. Change in serum marker of oxidative stress in the progression of idiopathic pulmonary fibrosis. Pulm. Pharmacol. Ther. 32, 1-6. https://doi.org/10.1016/j.pupt.2015.03.005
Miyamoto, J., Hasegawa, S., Kasubuchi, M., Ichimura, A., Nakajima, A., Kimura, I., 2016. Nutritional Signaling via Free Fatty Acid Receptors. Int. J. Mol. Sci. 17. https://doi.org/10.3390/ijms17040450
Nanthakumar, C.B., Hatley, R.J.D., Lemma, S., Gauldie, J., Marshall, R.P., Macdonald, S.J.F., 2015. Dissecting fibrosis: therapeutic insights from the small-molecule toolbox. Nat. Rev. Drug Discov. 14, 693-720. https://doi.org/10.1038/nrd4592
Neuschwander-Tetri, B.A., Caldwell, S.H., 2003. Nonalcoholic steatohepatitis: Summary of an AASLD Single Topic Conference. Hepatology 37, 1202-1219. https://doi.org/10.1053/jhep.2003.50193
Richeldi, L., du Bois, R.M., Raghu, G., Azuma, A., Brown, K.K., Costabel, U., Cottin, V., Flaherty, K.R., Hansell, D.M., Inoue, Y., Kim, D.S., Kolb, M., Nicholson, A.G., Noble, P.W., Selman, M., Taniguchi, H., Brun, M., Le Maulf, F., Girard, M., Stowasser, S., Schlenker-Herceg, R., Disse, B., Collard, H.R., INPULSIS Trial Investigators, 2014. Efficacy and safety of nintedanib in idiopathic pulmonary fibrosis. N. Engl. J. Med. 370, 2071-2082. https://doi.org/10.1056/NEJMoa1402584
Richter, K., Kietzmann, T., 2016. Reactive oxygen species and fibrosis: further evidence of a significant liaison. Cell Tissue Res. 365, 591-605. https://doi.org/10.1007/s00441-016-2445-3
Santhekadur, P.K., Kumar, D.P., Sanyal, A.J., 2017. Preclinical Models of Nonalcoholic Fatty Liver Disease. J. Hepatol. https://doi.org/10.1016/j.jhep.2017.10.031
Suzuki, M., Takaishi, S., Nagasaki, M., Onozawa, Y., Iino, I., Maeda, H., Komai, T., Oda, T., 2013. Medium-chain Fatty Acid-sensing Receptor, GPR84, Is a Proinflammatory Receptor. J. Biol. Chem. 288, 10684-10691. https://doi.org/10.1074/jbc.M112.420042
Wang, J., Wu, X., Simonavicius, N., Tian, H., Ling, L., 2006. Medium-chain Fatty Acids as Ligands for Orphan G Protein-coupled Receptor GPR84. J. Biol. Chem. 281, 34457-34464. https://doi.org/10.1074/jbc.M608019200
Wittenberger, T., Schaller, H.C., Hellebrand, S., 2001. An expressed sequence tag (EST) data mining strategy succeeding in the discovery of new G-protein coupled receptors. J. Mol. Biol. 307, 799-813. https://doi.org/10.1006/jmbi.2001.4520
Yousefi, S., Cooper, P.R., Potter, S.L., Mueck, B., Jarai, G., 2001. Cloning and expression analysis of a novel G-protein-coupled receptor selectively expressed on granulocytes. J. Leukoc. Biol. 69, 1045-1052.
Zimmermann, H.W., Seidler, S., Nattermann, J., Gassler, N., Hellerbrand, C., Zernecke, A., Tischendorf, J.J.W., Luedde, T., Weiskirchen, R., Trautwein, C., Tacke, F., 2010. Functional Contribution of Elevated Circulating and Hepatic Non-Classical CD14+CD16+ Monocytes to Inflammation and Human Liver Fibrosis. PLOS ONE 5, e11049. https://doi.org/10.1371/journal.pone.0011049

## Claims

1. A compound having GPR84 antagonist activity for use in the prophylaxis and/or treatment of one or more fibrotic diseases, wherein the compound is according to formula I: 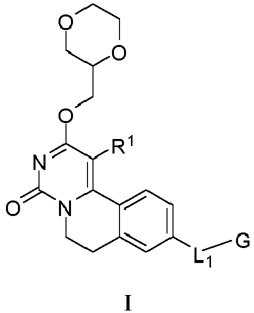 wherein
R¹ is H, Me, or halo;
L₁ is absent or is -O-, -S-, or -NR^{4a}-;
G is
- R²,
- -W-L₂-R², or
- -W-L₃-R³;
W is C₁₋₄ alkylene, C₂₋₄ alkenylene having one double bond, or C₂₋₄ alkynylene having one triple bond;
Lz is absent or is -O-;
R² is
- H,
- C₁₋₈ alkyl, optionally substituted with one to three groups independently selected from
∘ OH,
∘ halo,
∘ CN,
∘ C₁₋₆ alkoxy,
∘ C₃₋₇ cycloalkyl,
∘ 4-6 membered heterocycloalkyl comprising one to three heteroatoms independently selected from S, and O,
∘ 5-6 membered heteroaryl comprising one to three heteroatoms independently selected from N, S, and O, and
∘ phenyl,
- C₄₋₇ cycloalkenyl comprising one double bond,
- 5-7 membered heterocycloalkenyl comprising one double bond, and one to three heteroatoms independently selected from N, O, and S,
- C₃₋₇ cycloalkyl optionally substituted with one or more independently selected R⁵ groups,
- 4-10 membered heterocycloalkyl comprising one to two heteroatoms independently selected from S, and O, optionally substituted with one to three independently selected R⁵ groups,
- 5-10 membered heteroaryl comprising one to three heteroatoms independently selected from N, S, and O, optionally substituted with one to three independently selected R⁶ groups, or
- C₆₋₁₀ aryl optionally substituted with one or more independently selected R⁶ groups;
L₃ is -NR^{4b}-;
R³ is
- C₁₋₄ alkyl substituted with
∘ C₆₋₁₀ aryl optionally substituted with one or more independently selected R⁷ groups, or
∘ 5-10 membered heteroaryl comprising one to three heteroatoms independently selected from N, S, and O, optionally substituted with one or more independently selected R⁷ groups,
- 5-10 membered heteroaryl comprising one to three heteroatoms independently selected from N, S, and O, optionally substituted with one or more independently selected R⁷ groups, or
- C₆₋₁₀ aryl optionally substituted with one or more independently selected R⁷ groups;
Each R^{4a} and R^{4b} is independently selected from H, C₁₋₄ alkyl, and C₃₋₇ cycloalkyl;
R⁵ is oxo or R⁶;
R⁶ is
- OH,
- halo,
- -NOz,
- C₁₋₆ alkyl optionally substituted with one to three groups independently selected from halo, and OH,
- C₁₋₆ alkoxy optionally substituted with one to three groups independently selected from halo, and OH,
- C₃₋₇ cycloalkyl,
- -C(=O)OR⁸,
- -C(=O)NR⁹R¹⁰,
- -NHC(=O)-C₁₋₄ alkyl,
- -CN,
- phenyl,
- -O-phenyl,
- 4-7 membered heterocycloalkyl comprising one to three heteroatoms independently selected from N, O, and S, or
- 5-6 membered heteroaryl comprising one to three heteroatoms independently selected from N, O, and S; optionally substituted with one or more independently selected C₁₋₄ alkyl, C₁₋₄ alkoxy, CN, halo, and -C(=O)OR¹¹;
R⁷ is C₁₋₄ alkyl, or halo, and
each of R⁸, R⁹, R¹⁰ and R¹¹ is independently selected from H and C₁₋₄ alkyl;
or pharmaceutically acceptable salt thereof, or a pharmaceutically acceptable solvate, or the salt of a pharmaceutically acceptable solvate thereof;
or wherein the compound is according to Formula II : 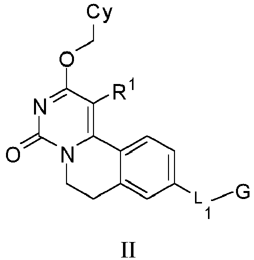 wherein
Cy is 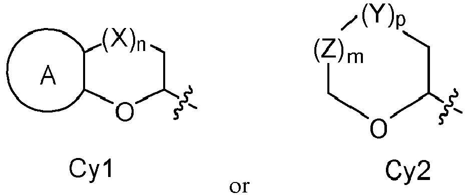
wherein
X is O or S;
Y is -CH₂-, or S;
Z is -CH₂-;
each of the subscript n, m, or p is independently selected from 0, and 1; and A is phenyl, or 5-6-membered heteroaryl comprising one or two N-atoms; optionally substituted with one or more independently selected R⁵ groups;
any one of Cy1 and Cy2 is optionally substituted by one or more independently selected C₁₋₄ alkyl groups;
R¹ is H, Me, or halo;
L₁ is absent or is -O-, -S-, or -NR^{4a}-;
G is
- R²,
- -W-L₂-R², or
- -W-L₃-R³;
W is C₁₋₄ alkylene, C₂₋₄ alkenylene having one double bond, or C₂₋₄ alkynylene having one triple bond;
L₂ is absent or is -O-;
R² is
- H,
- C₁₋₈ alkyl optionally substituted with one to three groups independently selected from
∘ OH,
∘ halo,
∘ CN,
∘ C₁₋₆ alkoxy,
∘ C₃₋₇ cycloalkyl,
∘ 4-6 membered heterocycloalkyl (comprising one to three heteroatoms independently selected from S, and O),
∘ 5-6 membered heteroaryl (comprising one to three heteroatoms independently selected from N, S, and O), and
∘ phenyl,
- C₄₋₇ cycloalkenyl comprising one double bond,
- 5-7 membered heterocycloalkenyl comprising one double bond, and one to three heteroatoms independently selected from O, and S,
- C₃₋₇ cycloalkyl (optionally substituted with one or more independently selected R⁶ groups),
- 4-10 membered heterocycloalkyl comprising one to two heteroatoms independently selected from S, and O, (optionally substituted with one to three independently selected R⁶ groups),
- 5-10 membered heteroaryl comprising one to three heteroatoms independently selected from N, S, and O (optionally substituted with one to three independently selected R⁷ groups), or
- C₆₋₁₀ aryl (optionally substituted with one or more independently selected R⁷ groups);
L₃ is -NR^{4b}-;
R³ is
- C₁₋₄ alkyl substituted with C₆₋₁₀ aryl (optionally substituted with one or more independently selected R⁸ groups), or 5-10 membered heteroaryl comprising one to three heteroatoms independently selected from N, S, and O, (optionally substituted with one or more independently selected R⁸ groups),
- 5-10 membered heteroaryl comprising one to three heteroatoms independently selected from N, S, and O, (optionally substituted with one or more independently selected R⁸ groups), or
- C₆₋₁₀ aryl (optionally substituted with one or more independently selected R⁸ groups);
each R^{4a} and R^{4b} is independently selected from H, C₁₋₄ alkyl, and C₃₋₇ cycloalkyl;
R⁵ is halo, C₁₋₄ alkyl or C₁₋₄ alkoxy;
R⁶ is oxo or R⁷;
R⁷ is
- OH,
- halo,
- -NOz,
- C₁₋₆ alkyl (optionally substituted with one to three groups independently selected from halo, and OH),
- C₁₋₆ alkoxy (optionally substituted with one to three groups independently selected from halo, and OH),
- C₃₋₇ cycloalkyl,
- -C(=O)OR⁹,
- -C(=O)NR¹⁰R¹¹,
- -NHC(=O)-C₁₋₄ alkyl,
- -CN,
- phenyl,
- -O-phenyl,
- 4-7 membered heterocycloalkyl comprising one to three heteroatoms independently selected from N, O, and S, or
- 5-6 membered heteroaryl comprising one to three heteroatoms independently selected from N, O, and S; (optionally substituted with one or more independently selected C₁₋₄ alkyl, C₁₋₄ alkoxy, CN, halo, and -C(=O)OR¹²);
R⁸ is C₁₋₄ alkyl, or halo; and
each of R⁹, R¹⁰, R¹¹and R¹², is independently selected from H and C₁₋₄ alkyl;
or pharmaceutically acceptable salt thereof, or a pharmaceutically acceptable solvate, or the salt of a pharmaceutically acceptable solvate thereof;
or wherein the compound is according to Formula III : 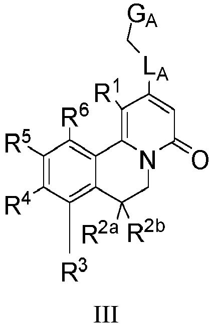
wherein
R¹ is H, C₁₋₄ alkyl, or cyclopropyl;
L_{A} is O or NH;
G_{A} is:
- 4-6 membered monocyclic heterocycloalkyl containing one or two O,
- C₃₋₇ monocyclic cycloalkyl, or
- a bicyclic group of formula Cy: 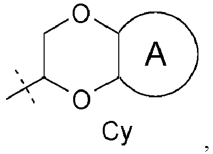
wherein A is phenyl or 5-6 membered heteroaryl containing one or two heteroatoms independently selected from N, O and S;
each R^{2a} and R^{2b} are independently H or -CH₃;
R³ is H, -OH or -OCH₃;
R⁴ is -CN or -Li-Wi-Gi,
wherein
- L₁ is absent or O,
- W₁ is absent, or is C₁₋₆ alkylene, C₂₋₄ alkenylene having one double bond or C₂₋₄ alkynylene having one triple bond, each of which is optionally substituted with one or more independently selected halo, -CN or C₁₋₄ alkoxy,
- Gi is
∘ H,
∘ -CF₃,
∘ -C(=O)-C₁₋₄ alkyl,
∘ -S(=O)₂-C₁₋₄ alkyl, optionally substituted with one or more independently selected halo,
∘ 4-6 membered monocyclic heterocycloalkyl containing one or two O (which heterocycloalkyl is optionally substituted with one or more independently selected R⁷ groups),
∘ 6 membered monocyclic heterocycloalkenyl containing one or two O (which heterocycloalkenyl is optionally substituted with one or more independently selected R⁷ groups),
∘ C₃₋₇ monocyclic cycloalkyl optionally substituted with one or more independently selected R⁷ groups,
∘ phenyl optionally substituted with one or more independently selected R⁷ groups,
∘ or 5-6 membered heteroaryl containing one to four heteroatoms independently selected from N, O and S (which heteroaryl is optionally substituted with one or more independently selected R⁷ groups),
each R⁷ is:
- halo,
- -OH,
- C₁₋₄ alkyl, C₃₋₄ monocyclic cycloalkyl, or C₁₋₄ alkoxy, each of which is optionally substituted with one or more independently selected halo;
R⁵ is -CN or -L₂-W₂-G₂,
wherein
- L₂ is absent, O or S,
- Wz is absent or C₁₋₄ alkylene, optionally substituted with one or more independently selected halo,
- Gz is
∘ H,
∘ -CF₃,
∘ C₃₋₇ monocyclic cycloalkyl (which cycloalkyl is optionally substituted with one or more independently selected halo),
∘ phenyl,
∘ or 5-6 membered heteroaryl containing one to three heteroatoms independently selected from N, O and S; and
R⁶ is H, -OH or -OCH₃;
or pharmaceutically acceptable salt thereof, or a pharmaceutically acceptable solvate, or the salt of a pharmaceutically acceptable solvate thereof;
or wherein the compound is according to Formula IV : 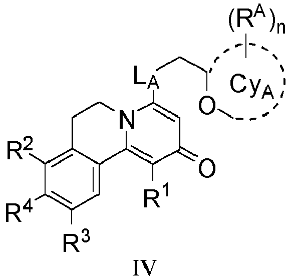
wherein
L_{A} is O, or NH;
Cy_{A} is monocyclic 4-6 membered heterocycloalkyl, comprising one or two O atoms;
each R^{A} is independently selected from halo, and C₁₋₃ alkyl;
the subscript n is 0, 1 or 2;
R¹ is H or C₁₋₃ alkyl;
R² is H, -OH, or C₁₋₃ alkoxy;
R³ is H or C₁₋₃ alkoxy;
R⁴ is
- -CN,
- -OH,
- -O-S(=O)₂-C₁₋₄ alkyl optionally substituted with one or more independently selected halo, or
- -L₁-W₁-G₁;
L₁ is a direct bond, -O-, -S-, -SO₂-, -C(=O)NR^{5a}-, -NR^{5b}C(=O)-, or -NR^{5c}-;
R^{5a}, R^{5b} and R^{5c} are independently H or C₁₋₄ alkyl;
W₁ is a direct bond or C₁₋₂ alkylene optionally substituted with one or more independently selected halo;
Gi is
- C₃₋₆ cycloalkyl optionally substituted with one or more independently selected halo,
- 5-6 membered heteroaryl comprising one to four heteroatoms independently selected from N, O, and S, which heteroaryl is optionally substituted with one or more independently selected C₁₋₄ alkyl,
- 5-7 membered heterocycloalkenyl comprising one double bond, and one to three heteroatoms independently selected from N, O, and S, which heterocycloalkenyl is optionally substituted with one or more independently selected R⁶,
- monocyclic or spiro bicyclic 4-8 membered heterocycloalkyl comprising one to three heteroatoms independently selected from N, O, and S, which heterocycloalkyl is optionally substituted with one or more independently selected R⁶,
- monocyclic 4-6 membered heterocycloalkyl comprising one to three heteroatoms independently selected from N, O, and S, fused to one or two phenyls,
- C₁₋₄ alkyl optionally substituted with one or more independently selected halo, -NR^{7a}R^{7b}, or C₁₋₄ alkoxy, which alkoxy is optionally substituted with one or more independently selected halo,
- phenyl optionally substituted with one or more independently selected halo or C₁₋₄ alkoxy, which alkoxy is optionally substituted with one or more independently selected halo;
R⁶ is
- halo,
- =O,
- -CN,
- -OH,
- -C(=O)-C₁₋₄ alkoxy optionally substituted with one or more independently selected halo,
- -C(=O)-C3-4 cycloalkyl,
- -S(=O)2-C₁₋₄ alkyl,
- C₁₋₄ alkyl optionally substituted by one or more independently selected C1-3 alkoxy, halo, or -OH,
- C₁₋₄ alkoxy,
- phenyl optionally substituted by one or more independently selected halo,
- -C(=O)-monocyclic 4-6 membered heterocycloalkyl comprising one to three heteroatoms independently selected from N, O, and S,
- -C(=O)NR^{8a}R^{8b}, or
- 5-7 membered heteroaryl comprising one to four heteroatoms independently selected from N, O, and S, which heteroaryl is optionally substituted with one or more independently selected C₁₋₄ alkyl;
R^{7a} and R^{7b} are independently H or C₁₋₄ alkyl, and
R^{8a} and R^{8b} are independently H or C₁₋₃ alkyl;
or pharmaceutically acceptable salt thereof, or a pharmaceutically acceptable solvate, or the salt of a pharmaceutically acceptable solvate thereof;
and wherein the fibrotic disease is IPF, non-alcoholic steatohepatitis (NASH), or renal fibrosis.

2. The compound for use according to claim 1, wherein the compound is according to Formula I.Ia, I.Ib, I.Ic, or I.Id:

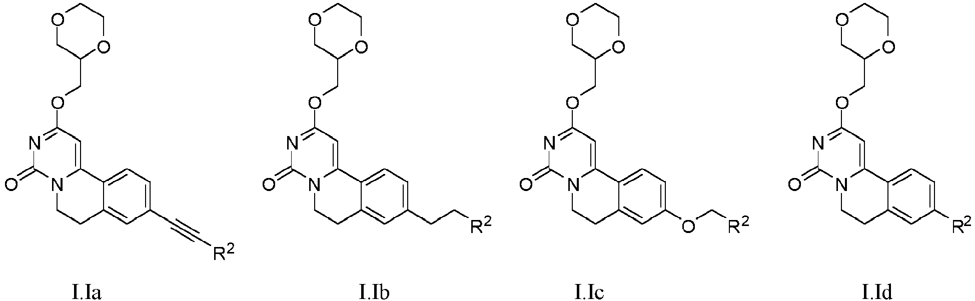

3. The compound for use according to claim 1, wherein the compound is according to Formula IV.la:

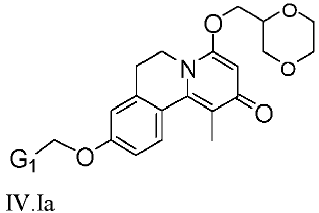

4. The compound or pharmaceutically acceptable salt thereof for use according to claim 1 wherein the compound is selected from:
9-Allyloxy-2-([1,4]dioxan-2-ylmethoxy)-6,7-dihydro-pyrimido[6,1-a]isoquinolin-4-one,
2-([1,4]Dioxan-2-ylmethoxy)-9-pyridin-3-yl-6,7-dihydro-pyrimido[6,1-a]isoquinolin-4-one,
2-([1,4]Dioxan-2-ylmethoxy)-9-pyridin-4-yl-6,7-dihydro-pyrimido[6,1-a]isoquinolin-4-one,
2-[2-([1,4]Dioxan-2-ylmethoxy)-4-oxo-6,7-dihydro-4H-pyrimido[6,1-a]isoquinolin-9-yl]-benzonitrile,
3-[2-([1,4]Dioxan-2-ylmethoxy)-4-oxo-6,7-dihydro-4H-pyrimido[6,1-a]isoquinolin-9-yl]-benzonitrile,
4-[2-([1,4]Dioxan-2-ylmethoxy)-4-oxo-6,7-dihydro-4H-pyrimido[6,1-a]isoquinolin-9-yl]-benzonitrile,
[2-([1,4]Dioxan-2-ylmethoxy)-4-oxo-6,7-dihydro-4H-pyrimido[6,1-a]isoquinolin-9-yloxy]-acetonitrile,
2-([1,4]Dioxan-2-ylmethoxy)-9-(oxazol-2-ylmethoxy)-6,7-dihydro-pyrimido[6,1-a]isoquinolin-4-one,
2-([1,4]Dioxan-2-ylmethoxy)-9-(pyridin-2-ylmethoxy)-6,7-dihydro-pyrimido[6,1-a]isoquinolin-4-one,
9-(3,5-Dichloro-phenyl)-2-([1,4]dioxan-2-ylmethoxy)-6,7-dihydro-pyrimido[6,1-a]isoquinolin-4-one,
9-Benzofuran-2-yl-2-([1,4]dioxan-2-ylmethoxy)-6,7-dihydro-pyrimido[6,1-a]isoquinolin-4-one,
2-[2-([1,4]Dioxan-2-ylmethoxy)-4-oxo-6,7-dihydro-4H-pyrimido[6,1-a]isoquinolin-9-yl]-indole-1-carboxylic acid tert-butyl ester,
2-([1,4]Dioxan-2-ylmethoxy)-9-(1H-indol-2-yl)-6,7-dihydro-pyrimido[6,1-a]isoquinolin-4-one,
2-([1,4]Dioxan-2-ylmethoxy)-9-(6-methoxy-pyridin-3-yl)-6,7-dihydro-pyrimido[6,1-a]isoquinolin-4-one,
2-([1,4]Dioxan-2-ylmethoxy)-9-(6-trifluoromethyl-pyridin-3-yl)-6,7-dihydro-pyrimido[6,1-a]isoquinolin-4-one,
2-([1,4]Dioxan-2-ylmethoxy)-9-(3-methyl-3H-imidazol-4-ylethynyl)-6,7-dihydro-pyrimido[6,1-a]isoquinolin-4-one,
9-(5-tert-Butyl-[1,2,4]oxadiazol-3-ylmethoxy)-2-([1,4]dioxan-2-ylmethoxy)-6,7-dihydro-pyrimido[6,1-a]isoquinolin-4-one,
5-[2-([1,4]Dioxan-2-ylmethoxy)-4-oxo-6,7-dihydro-4H-pyrimido[6,1-a]isoquinolin-9-yl]-pyridine-2-carboxylic acid methylamide,
2-([1,4]Dioxan-2-ylmethoxy)-9-pent-1-ynyl-6,7-dihydro-pyrimido[6,1-a]isoquinolin-4-one,
2-([1,4]Dioxan-2-ylmethoxy)-9-(2-pyridin-2-yl-ethyl)-6,7-dihydro-pyrimido[6,1-a]isoquinolin-4-one,
2-([1,4]Dioxan-2-ylmethoxy)-9-(2-pyrazin-2-yl-ethyl)-6,7-dihydro-pyrimido[6,1-a]isoquinolin-4-one,
2-([1,4]Dioxan-2-ylmethoxy)-9-(1H-indol-5-yl)-6,7-dihydro-pyrimido[6,1-a]isoquinolin-4-one,
2-([1,4]Dioxan-2-ylmethoxy)-9-(2-methoxy-phenyl)-6,7-dihydro-pyrimido[6,1-a]isoquinolin-4-one,
2-([1,4]Dioxan-2-ylmethoxy)-9-(5-methoxy-pyridin-3-yl)-6,7-dihydro-pyrimido[6,1-a]isoquinolin-4-one,
2-([1,4]Dioxan-2-ylmethoxy)-9-(1H-indazol-5-yl)-6,7-dihydro-pyrimido[6,1-a]isoquinolin-4-one,
2-([1,4]Dioxan-2-ylmethoxy)-9-(4-methoxy-phenyl)-6,7-dihydro-pyrimido[6,1-a]isoquinolin-4-one,
3-[2-([1,4]Dioxan-2-ylmethoxy)-4-oxo-6,7-dihydro-4H-pyrimido[6,1-a]isoquinolin-9-yl]-benzamide,
5-[2-([1,4]Dioxan-2-ylmethoxy)-4-oxo-6,7-dihydro-4H-pyrimido[6,1-a]isoquinolin-9-yl]-2-fluoro-benzamide,
N-{3-[2-([1,4]Dioxan-2-ylmethoxy)-4-oxo-6,7-dihydro-4H-pyrimido[6,1-a]isoquinolin-9-yl]-phenyl} -acetamide,
9-Cyclopropylethynyl-2-([1,4]dioxan-2-ylmethoxy)-6,7-dihydro-pyrimido[6,1-a]isoquinolin-4-one,
2-([1,4]Dioxan-2-ylmethoxy)-9-(1-hydroxy-cyclopentylethynyl)-6,7-dihydro-pyrimido[6,1-a]isoquinolin-4-one,
2-([1,4]Dioxan-2-ylmethoxy)-9-pyrimidin-5-yl-6,7-dihydro-pyrimido[6,1-a]isoquinolin-4-one,
9-Cyclohex-1-enyl-2-([1,4]dioxan-2-ylmethoxy)-6,7-dihydro-pyrimido[6,1-a]isoquinolin-4-one,
2-([1,4]Dioxan-2-ylmethoxy)-9-(1-methyl-1H-indol-5-yl)-6,7-dihydro-pyrimido[6,1-a]isoquinolin-4-one,
2-([1,4]Dioxan-2-ylmethoxy)-9-(6-methyl-pyridin-3-yl)-6,7-dihydro-pyrimido[6,1-a]isoquinolin-4-one,
2-([1,4]Dioxan-2-ylmethoxy)-9-pyridin-2-ylethynyl-6,7-dihydro-pyrimido[6,1-a]isoquinolin-4-one,
2-([1,4]Dioxan-2-ylmethoxy)-9-(3-methoxy-prop-1-ynyl)-6,7-dihydro-pyrimido[6,1-a]isoquinolin-4-one,
5-[2-([1,4]Dioxan-2-ylmethoxy)-4-oxo-6,7-dihydro-4H-pyrimido[6,1-a]isoquinolin-9-yl]-pent-4-ynenitrile,
2-([1,4]Dioxan-2-ylmethoxy)-9-(3-hydroxy-prop-1-ynyl)-6,7-dihydro-pyrimido[6,1-a]isoquinolin-4-one,
2-([1,4]Dioxan-2-ylmethoxy)-9-(4-methoxy-phenylethynyl)-6,7-dihydro-pyrimido[6,1-a]isoquinolin-4-one,
2-([1,4]Dioxan-2-ylmethoxy)-9-pyridin-3-ylethynyl-6,7-dihydro-pyrimido[6,1-a]isoquinolin-4-one,
4-[2-([1,4]Dioxan-2-ylmethoxy)-4-oxo-6,7-dihydro-4H-pyrimido[6,1-a]isoquinolin-9-yl]-N-methyl-benzamide,
2-([1,4]Dioxan-2-ylmethoxy)-9-(3-methoxy-phenyl)-6,7-dihydro-pyrimido[6,1-a]isoquinolin-4-one,
9-(2-Chloro-phenyl)-2-([1,4]dioxan-2-ylmethoxy)-6,7-dihydro-pyrimido[6,1-a]isoquinolin-4-one,
2-([1,4]Dioxan-2-ylmethoxy)-9-(4-hydroxy-but-1-ynyl)-6,7-dihydro-pyrimido[6,1-a]isoquinolin-4-one,
9-(1,5-Dimethyl-1H-pyrazol-3-ylmethoxy)-2-([1,4]dioxan-2-ylmethoxy)-6,7-dihydro-pyrimido[6,1-a]isoquinolin-4-one,
2-([1,4]Dioxan-2-ylmethoxy)-9-(1-methyl-1H-pyrazol-3-ylmethoxy)-6,7-dihydro-pyrimido[6,1-a]isoquinolin-4-one,
2-([1,4]Dioxan-2-ylmethoxy)-9-(3-methyl-[1,2,4]oxadiazol-5-ylmethoxy)-6,7-dihydro-pyrimido[6,1-a]isoquinolin-4-one,
2-([1,4]Dioxan-2-ylmethoxy)-9-(4-morpholin-4-yl-phenyl)-6,7-dihydro-pyrimido[6,1-a]isoquinolin-4-one,
3-[2-([1,4]Dioxan-2-ylmethoxy)-4-oxo-6,7-dihydro-4H-pyrimido[6,1-a]isoquinolin-9-yl]-4-fluoro-benzamide,
3-[2-([1,4]Dioxan-2-ylmethoxy)-4-oxo-6,7-dihydro-4H-pyrimido[6,1-a]isoquinolin-9-yl]-5-fluoro-benzamide,
9-(3,3-Dimethyl-but-1-ynyl)-2-([1,4]dioxan-2-ylmethoxy)-6,7-dihydro-pyrimido[6,1-a]isoquinolin-4-one,
2-([1,4]Dioxan-2-ylmethoxy)-9-pyridin-4-ylethynyl-6,7-dihydro-pyrimido[6,1-a]isoquinolin-4-one,
2-([1,4]Dioxan-2-ylmethoxy)-9-(3-methyl-isoxazol-5-ylmethoxy)-6,7-dihydro-pyrimido[6,1-a]isoquinolin-4-one,
2-([1,4]Dioxan-2-ylmethoxy)-9-(3-hydroxy-3-methyl-but-1-ynyl)-6,7-dihydro-pyrimido[6,1-a]isoquinolin-4-one,
2-([1,4]Dioxan-2-ylmethoxy)-9-(2-methoxy-pyridin-3-yl)-6,7-dihydro-pyrimido[6,1-a]isoquinolin-4-one,
2-([1,4]Dioxan-2-ylmethoxy)-6,7-dihydro-pyrimido[6,1-a]isoquinolin-4-one,
9-(3,6-Dihydro-2H-pyran-4-yl)-2-([1,4]dioxan-2-ylmethoxy)-6,7-dihydro-pyrimido[6,1-a]isoquinolin-4-one,
5-[2-([1,4]Dioxan-2-ylmethoxy)-4-oxo-6,7-dihydro-4H-pyrimido[6,1-a]isoquinolin-9-yl]-pyridine-2-carbonitrile,
2-([1,4]Dioxan-2-ylmethoxy)-9-(6-isopropoxy-pyridin-3-yl)-6,7-dihydro-pyrimido[6,1-a]isoquinolin-4-one,
2-([1,4]Dioxan-2-ylmethoxy)-9-(6-ethoxy-pyridin-3-yl)-6,7-dihydro-pyrimido[6,1-a]isoquinolin-4-one,
2-([1,4]Dioxan-2-ylmethoxy)-9-(6-morpholin-4-yl-pyridin-3-yl)-6,7-dihydro-pyrimido[6,1-a]isoquinolin-4-one,
9-(2,3-Dimethoxy-phenyl)-2-([1,4]dioxan-2-ylmethoxy)-6,7-dihydro-pyrimido[6,1-a]isoquinolin-4-one,
9-(3-Chloro-2-methoxy-pyridin-4-yl)-2-([1,4]dioxan-2-ylmethoxy)-6,7-dihydro-pyrimido[6,1-a]isoquinolin-4-one,
2-([1,4]Dioxan-2-ylmethoxy)-9-(2-methyl-pyridin-4-yl)-6,7-dihydro-pyrimido[6,1-a]isoquinolin-4-one,
3-[2-([1,4]Dioxan-2-ylmethoxy)-4-oxo-6,7-dihydro-4H-pyrimido[6,1-a]isoquinolin-9-yl]-isonicotinonitrile,
9-(2,5-Dimethoxy-phenyl)-2-([1,4]dioxan-2-ylmethoxy)-6,7-dihydro-pyrimido[6,1-a]isoquinolin-4-one,
2-([1,4]Dioxan-2-ylmethoxy)-9-(3,4,5,6-tetrahydro-2H-[1,2']bipyridinyl-5'-yl)-6,7-dihydro-pyrimido [6,1 -a] isoquinolin-4-one,
2-([1,4]Dioxan-2-ylmethoxy)-9-(2-ethoxy-pyridin-3-yl)-6,7-dihydro-pyrimido[6,1-a]isoquinolin-4-one,
9-(2,6-Dimethoxy-pyridin-3-yl)-2-([1,4]dioxan-2-ylmethoxy)-6,7-dihydro-pyrimido[6,1-a]isoquinolin-4-one,
4-[2-([1,4]Dioxan-2-ylmethoxy)-4-oxo-6,7-dihydro-4H-pyrimido[6,1-a]isoquinolin-9-yl]-nicotinonitrile,
9-tert-Butoxymethyl-2-([1,4]dioxan-2-ylmethoxy)-6,7-dihydro-pyrimido[6,1-a]isoquinolin-4-one,
2-([1,4]Dioxan-2-ylmethoxy)-9-(2-pyrrolidin-1-yl-pyridin-3-yl)-6,7-dihydro-pyrimido[6,1-a]isoquinolin-4-one,
2-([1,4]Dioxan-2-ylmethoxy)-9-(6-pyrrolidin-1-yl-pyridin-3-yl)-6,7-dihydro-pyrimido[6,1-a]isoquinolin-4-one,
2-([1,4]Dioxan-2-ylmethoxy)-9-(5-phenyl-oxazol-2-ylmethoxy)-6,7-dihydro-pyrimido[6,1-a]isoquinolin-4-one,
9-(5-tert-Butyl-oxazol-2-ylmethoxy)-2-([1,4]dioxan-2-ylmethoxy)-6,7-dihydro-pyrimido[6,1-a]isoquinolin-4-one,
9-(5-Cyclopropyl-[1,2,4]oxadiazol-3-ylmethoxy)-2-([1,4]dioxan-2-ylmethoxy)-6,7-dihydro-pyrimido[6,1-a]isoquinolin-4-one,
2-([1,4]Dioxan-2-ylmethoxy)-9-(5-ethyl-[1,2,4]oxadiazol-3-ylmethoxy)-6,7-dihydro-pyrimido[6,1-a]isoquinolin-4-one,
2-([1,4]Dioxan-2-ylmethoxy)-9-(5-methyl-[1,2,4]oxadiazol-3-ylmethoxy)-6,7-dihydro-pyrimido[6,1-a]isoquinolin-4-one,
2-([1,4]Dioxan-2-ylmethoxy)-9-(5-isopropyl-[1,2,4]oxadiazol-3-ylmethoxy)-6,7-dihydro-pyrimido[6,1-a]isoquinolin-4-one,
9-Cyclopentylethynyl-2-([1,4]dioxan-2-ylmethoxy)-6,7-dihydro-pyrimido[6,1-a]isoquinolin-4-one,
9-Cyclohexylethynyl-2-([1,4]dioxan-2-ylmethoxy)-6,7-dihydro-pyrimido[6,1-a]isoquinolin-4-one,
2-([1,4]Dioxan-2-ylmethoxy)-9-(3-methyl-but-1-ynyl)-6,7-dihydro-pyrimido[6,1-a]isoquinolin-4-one,
2-([1,4]Dioxan-2-ylmethoxy)-9-hex-1-ynyl-6,7-dihydro-pyrimido[6,1-a]isoquinolin-4-one, 9-[3-(Benzyl-methyl-amino)-prop-1-ynyl]-2-([1,4]dioxan-2-ylmethoxy)-6,7-dihydro-pyrimido[6,1-a]isoquinolin-4-one,
2-([1,4]Dioxan-2-ylmethoxy)-9-(3-hydroxy-5-methyl-hex-1-ynyl)-6,7-dihydro-pyrimido[6,1-a]isoquinolin-4-one,
2-([1,4]Dioxan-2-ylmethoxy)-9-(3-hydroxy-but-1-ynyl)-6,7-dihydro-pyrimido[6,1-a]isoquinolin-4-one,
9-Cyclopropyl-2-([1,4]dioxan-2-ylmethoxy)-6,7-dihydro-pyrimido[6,1-a]isoquinolin-4-one,
2-([1,4]Dioxan-2-ylmethoxy)-9-(3-hydroxy-pent-1-ynyl)-6,7-dihydro-pyrimido[6,1-a]isoquinolin-4-one,
2-([1,4]Dioxan-2-ylmethoxy)-9-(3-hydroxy-4-methyl-pent-1-ynyl)-6,7-dihydro-pyrimido[6,1-a]isoquinolin-4-one,
2-([1,4]Dioxan-2-ylmethoxy)-9-(3-ethyl-3-hydroxy-pent-1-ynyl)-6,7-dihydro-pyrimido[6,1-a]isoquinolin-4-one,
2-([1,4]Dioxan-2-ylmethoxy)-9-(3-hydroxy-3-phenyl-but-1-ynyl)-6,7-dihydro-pyrimido[6,1-a]isoquinolin-4-one,
9-(3-Benzylamino-prop-1-ynyl)-2-([1,4]dioxan-2-ylmethoxy)-6,7-dihydro-pyrimido[6,1-a]isoquinolin-4-one,
2-([1,4]Dioxan-2-ylmethoxy)-9-[(furan-2-ylmethyl)-amino]-6,7-dihydro-pyrimido[6,1-a]isoquinolin-4-one,
2-([1,4]Dioxan-2-ylmethoxy)-9-(1-ethyl-1H-pyrazol-4-yl)-6,7-dihydro-pyrimido[6,1-a]isoquinolin-4-one,
2-([1,4]Dioxan-2-ylmethoxy)-9-[1-(3-methyl-butyl)-1H-pyrazol-4-yl]-6,7-dihydro-pyrimido[6,1-a]isoquinolin-4-one,
2-([1,4]Dioxan-2-ylmethoxy)-9-(5-methyl-furan-2-yl)-6,7-dihydro-pyrimido[6,1-a]isoquinolin-4-one,
2-([1,4]Dioxan-2-ylmethoxy)-9-(3-hydroxy-hex-1-ynyl)-6,7-dihydro-pyrimido[6,1-a]isoquinolin-4-one,
9-(3,5-Dimethyl-1H-pyrazol-4-yl)-2-([1,4]dioxan-2-ylmethoxy)-6,7-dihydro-pyrimido[6,1-a]isoquinolin-4-one,
2-([1,4]Dioxan-2-ylmethoxy)-9-(1H-pyrazol-4-yl)-6,7-dihydro-pyrimido[6,1-a]isoquinolin-4-one,
2-([1,4]Dioxan-2-ylmethoxy)-9-(1-propyl-1H-pyrazol-4-yl)-6,7-dihydro-pyrimido[6,1-a]isoquinolin-4-one,
2-[2-((R)-1-[1,4]Dioxan-2-ylmethoxy)-4-oxo-6,7-dihydro-4H-pyrimido[6,1-a]isoquinolin-9-yl]-benzonitrile,
2-[2-((S)-1-[1,4]Dioxan-2-ylmethoxy)-4-oxo-6,7-dihydro-4H-pyrimido[6,1-a]isoquinolin-9-yl]-benzonitrile,
9-(5-Cyclopropyl-[1,2,4]oxadiazol-3-ylmethoxy)-2-((R)-1-[1,4]dioxan-2-ylmethoxy)-6,7-dihydro-pyrimido [6, 1-a] isoquinolin-4-one,
2-([1,4]Dioxan-2-ylmethoxy)-9-ethynyl-6,7-dihydro-pyrimido[6,1-a]isoquinolin-4-one,
2-([1,4]Dioxan-2-ylmethoxy)-9-pyrimidin-2-ylethynyl-6,7-dihydro-pyrimido[6,1-a]isoquinolin-4-one,
2-([1,4]Dioxan-2-ylmethoxy)-9-(3-phenylamino-prop-1-ynyl)-6,7-dihydro-pyrimido[6,1-a]isoquinolin-4-one,
2-([1,4]Dioxan-2-ylmethoxy)-9-(3-hydroxy-3-pyridin-3-yl-prop-1-ynyl)-6,7-dihydro-pyrimido[6,1-a]isoquinolin-4-one,
9-Cyclopentyloxymethyl-2-([1,4]dioxan-2-ylmethoxy)-6,7-dihydro-pyrimido[6,1-a]isoquinolin-4-one,
2-([1,4]Dioxan-2-ylmethoxy)-9-(3-methoxy-4-methyl-pent-1-ynyl)-6,7-dihydro-pyrimido[6,1-a]isoquinolin-4-one,
9-Cyclopropylethynyl-2-((R)-1-[1,4]dioxan-2-ylmethoxy)-6,7-dihydro-pyrimido[6,1-a] isoquinolin-4-one,
2-((S)-1-[1,4]Dioxan-2-ylmethoxy)-9-(3-methyl-but-1-ynyl)-6,7-dihydro-pyrimido[6,1-a]isoquinolin-4-one,
2-([1,4]Dioxan-2-ylmethoxy)-9-(3-imidazol-1-yl-prop-1-ynyl)-6,7-dihydro-pyrimido [6,1-a]isoquinolin-4-one,
9-(2-Cyclopropyl-ethyl)-2-((R)-1-[1,4]dioxan-2-ylmethoxy)-6,7-dihydro-pyrimido[6,1-a]isoquinolin-4-one,
9-Cyclopentyloxymethyl-2-((R)-1-[1,4]dioxan-2-ylmethoxy)-6,7-dihydro-pyrimido[6,1-a]isoquinolin-4-one,
2-([1,4]Dioxan-2-ylmethoxy)-9-(3-hydroxy-3-pyridin-3-yl-propyl)-6,7-dihydro-pyrimido[6,1-a]isoquinolin-4-one,
9-Allyloxy-2-((R)-1-[1,4]dioxan-2-ylmethoxy)-6,7-dihydro-pyrimido[6,1-a]isoquinolin-4-one,
9-Allyloxy-2-((S)-1-[1,4]dioxan-2-ylmethoxy)-6,7-dihydro-pyrimido[6,1-a]isoquinolin-4-one,
2-((R)-1-[1,4]Dioxan-2-ylmethoxy)-9-(tetrahydro-pyran-4-yloxymethyl)-6,7-dihydro-pyrimido[6,1-a]isoquinolin-4-one,
2-([1,4]Dioxan-2-ylmethoxy)-9-{3-[(pyridin-3-ylmethyl)-amino]-prop-1-ynyl}-6,7-dihydro-pyrimido[6,1-a]isoquinolin-4-one,
2-((R)-1-[1,4]Dioxan-2-ylmethoxy)-9-pentyl-6,7-dihydro-pyrimido[6,1-a]isoquinolin-4-one,
9-Cyclopropylethynyl-2-((S)-1-[1,4]dioxan-2-ylmethoxy)-6,7-dihydro-pyrimido[6,1-a]isoquinolin-4-one,
9-(2-Cyclopropyl-ethyl)-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(oxetan-3-yloxymethyl)-6,7-dihydro-pyrimido[6,1-a]isoquinolin-4-one,
2-((S)-1-[1,4]Dioxan-2-ylmethoxy)-9-(3-methyl-oxetan-3-ylmethoxymethyl)-6,7-dihydro-pyrimido[6,1-a]isoquinolin-4-one,
9-(2,2-Dimethyl-butylamino)-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(3-hydroxy-4-methyl-pentyl)-6,7-dihydro-pyrimido[6,1-a]isoquinolin-4-one,
2-((S)-1-[1,4]Dioxan-2-ylmethoxy)-9-(2-ethyl-hexylamino)-6,7-dihydro-pyrimido[6,1-a]isoquinolin-4-one,
2-((S)-1-[1,4]Dioxan-2-ylmethoxy)-9-(2-methoxy-ethoxy)-6,7-dihydro-pyrimido[6,1-a]isoquinolin-4-one,
2-((S)-1-[1,4]Dioxan-2-ylmethoxy)-9-(2-ethoxy-ethoxy)-6,7-dihydro-pyrimido[6,1-a]isoquinolin-4-one,
9-Cyclopropylmethoxy-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(2-fluoro-ethoxy)-6,7-dihydro-pyrimido[6,1-a]isoquinolin-4-one,
2-((S)-1-[1,4]Dioxan-2-ylmethoxy)-9-[3-(2-methoxy-ethoxy)-prop-1-ynyl]-6,7-dihydro-pyrimido[6,1-a]isoquinolin-4-one,
2-((S)-1-[1,4]Dioxan-2-ylmethoxy)-9-[3-(2-ethoxy-ethoxy)-prop-1-ynyl]-6,7-dihydro-pyrimido[6,1-a]isoquinolin-4-one,
2-((S)-1-[1,4]Dioxan-2-ylmethoxy)-9-[3-(2-fluoro-ethoxy)-prop-1-ynyl]-6,7-dihydro-pyrimido[6,1-a]isoquinolin-4-one,
9-(2,2-Dimethyl-propoxymethyl)-2-((S)-1-[1,4]dioxan-2-ylmethoxy)-6,7-dihydro-pyrimido[6,1-a]isoquinolin-4-one,
9-Cyclohexyloxymethyl-2-((S)-1-[1,4]dioxan-2-ylmethoxy)-6,7-dihydro-pyrimido[6,1-a]isoquinolin-4-one,
9-Cyclopropylmethoxymethyl-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(tetrahydro-pyran-2-ylmethoxy)-6,7-dihydro-pyrimido[6,1-a]isoquinolin-4-one,
2-((S)-1-[1,4]Dioxan-2-ylmethoxy)-9-(3-hydroxy-butyl)-6,7-dihydro-pyrimido[6,1-a]isoquinolin-4-one,
9-(4,4-Dimethyl-pentyloxy)-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(3-methoxy-4-methyl-pentyl)-6,7-dihydro-pyrimido[6,1-a]isoquinolin-4-one,
9-(3-Cyclopropyl-propoxy)-2-((S)-1-[1,4]dioxan-2-ylmethoxy)-6,7-dihydro-pyrimido[6,1-a]isoquinolin-4-one,
9-Cyclohexylamino-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(3-hydroxy-4,4-dimethyl-pentyl)-6,7-dihydro-pyrimido[6,1-a]isoquinolin-4-one,
9-Cyclopentylmethoxymethyl-2-((S)-1-[1,4]dioxan-2-ylmethoxy)-6,7-dihydro-pyrimido[6,1-a] isoquinolin-4-one,
2-((S)-1-[1,4]Dioxan-2-ylmethoxy)-9-(3-methoxy-butyl)-6,7-dihydro-pyrimido[6,1-a]isoquinolin-4-one,
2-((S)-1-[1,4]Dioxan-2-ylmethoxy)-9-(3-phenylamino-propyl)-6,7-dihydro-pyrimido[6,1-a]isoquinolin-4-one,
2-((S)-1-[1,4]Dioxan-2-ylmethoxy)-9-(4-hydroxy-pentyl)-6,7-dihydro-pyrimido[6,1-a]isoquinolin-4-one,
2-((S)-1-[1,4]Dioxan-2-ylmethoxy)-9-(4-hydroxy-butyl)-6,7-dihydro-pyrimido[6,1-a]isoquinolin-4-one,
9-(Cyclohexyl-methyl-amino)-2-((S)-1-[1,4]dioxan-2-ylmethoxy)-6,7-dihydro-pyrimido[6,1-a] isoquinolin-4-one,
9-(Cyclohexylmethyl-amino)-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-[(tetrahydro-pyran-4-ylmethyl)-amino]-6,7-dihydro-pyrimido[6,1-a]isoquinolin-4-one,
2-((S)-1-[1,4]Dioxan-2-ylmethoxy)-9-(3-ethyl-3-hydroxy-pentyl)-6,7-dihydro-pyrimido[6,1-a]isoquinolin-4-one,
2-((S)-1-[1,4]Dioxan-2-ylmethoxy)-9-(3-hydroxy-3-methyl-butyl)-6,7-dihydro-pyrimido[6,1-a]isoquinolin-4-one,
2-((S)-1-[1,4]Dioxan-2-ylmethoxy)-9-(3-hydroxy-pentyl)-6,7-dihydro-pyrimido[6,1-a]isoquinolin-4-one,
9-(2,2-Dimethyl-propoxy)-2-((S)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(tetrahydro-pyran-4-ylmethoxy)-6,7-dihydro-pyrimido [6,1 - a]isoquinolin-4-one,
2-((S)-1-[1,4]Dioxan-2-ylmethoxy)-9-(4-hydroxy-4-methyl-pentyl)-6,7-dihydro-pyrimido[6,1-a]isoquinolin-4-one,
2-((S)-1-[1,4]Dioxan-2-ylmethoxy)-9-(tetrahydro-pyran-4-ylmethoxymethyl)-6,7-dihydro-pyrimido[6,1-a]isoquinolin-4-one,
2-([1,4]Dioxan-2-ylmethoxy)-9-methoxy-6,7-dihydro-pyrimido[6,1-a]isoquinolin-4-one,
2-((S)-1-[1,4]Dioxan-2-ylmethoxy)-9-(oxetan-3 -ylmethoxy)-6,7-dihydro-pyrimido[6,1-a]isoquinolin-4-one,
9-(3-Cyclopropyl-propoxy)-2-((R)-1-[1,4]dioxan-2-ylmethoxy)-6,7-dihydro-pyrimido[6,1-a]isoquinolin-4-one,
2-((S)-1-[1,4]Dioxan-2-ylmethoxy)-9-(3-methoxy-propyl)-6,7-dihydro-pyrimido[6,1-a]isoquinolin-4-one,
2-((S)-1-[1,4]Dioxan-2-ylmethoxy)-9-[2-(1-hydroxy-cyclopentyl)-ethyl]-6,7-dihydro-pyrimido[6,1-a]isoquinolin-4-one,
2-((R)-1-[1,4]Dioxan-2-ylmethoxy)-9-(4-hydroxy-tetrahydro-pyran-4-ylethynyl)-6,7-dihydro-pyrimido[6,1-a]isoquinolin-4-one.
2-((R)-1-[1,4]Dioxan-2-ylmethoxy)-9-(3-methoxy-propyl)-6,7-dihydro-pyrimido[6,1-a]isoquinolin-4-one,
2-((R)-1-[1,4]Dioxan-2-ylmethoxy)-9-[2-(1-hydroxy-cyclopentyl)-ethyl]-6,7-dihydro-pyrimido[6,1-a]isoquinolin-4-one,
2-((S)-1-[1,4]Dioxan-2-ylmethoxy)-9-(2-propoxy-ethoxy)-6,7-dihydro-pyrimido[6,1-a]isoquinolin-4-one,
2-((S)-1-[1,4]Dioxan-2-ylmethoxy)-9-(2-isopropoxy-ethoxy)-6,7-dihydro-pyrimido[6,1-a]isoquinolin-4-one,
2-((R)-1-[1,4]Dioxan-2-ylmethoxy)-9-(2-propoxy-ethoxy)-6,7-dihydro-pyrimido[6,1-a]isoquinolin-4-one,
2-((R)-1-[1,4]Dioxan-2-ylmethoxy)-9-(2-isopropoxy-ethoxy)-6,7-dihydro-pyrimido[6,1-a]isoquinolin-4-one, and
2-((S)-1-[1,4]Dioxan-2-ylmethoxy)-9-(4-methoxy-butyl)-6,7-dihydro-pyrimido[6,1-a]isoquinolin-4-one.

5. The compound or pharmaceutically acceptable salt thereof for use according to claim 1, wherein said compound is selected from:
9-Methoxy-2-(tetrahydro-furan-2-ylmethoxy)-6,7-dihydro-pyrimido[6,1-a]isoquinolin-4-one,
2-(Chroman-2-ylmethoxy)-9-methoxy-6,7-dihydro-pyrimido[6,1-a]isoquinolin-4-one,
2-(2,3-Dihydro-benzo[1,4]dioxin-2-ylmethoxy)-9-methoxy-6,7-dihydro-pyrimido[6,1-a]isoquinolin-4-one,
9-Allyloxy-2-(tetrahydro-furan-2-ylmethoxy)-6,7-dihydro-pyrimido[6,1-a]isoquinolin-4-one,
2-(2,3-Dihydro-benzofuran-2-ylmethoxy)-9-methoxy-6,7-dihydro-pyrimido[6,1-a]isoquinolin-4-one,
9-Hydroxy-2-(tetrahydro-furan-2-ylmethoxy)-6,7-dihydro-pyrimido[6,1-a]isoquinolin-4-one,
9-Benzyloxy-2-(tetrahydro-furan-2-ylmethoxy)-6,7-dihydro-pyrimido[6,1-a]isoquinolin-4-one,
9-(Pyridin-3-ylmethoxy)-2-(tetrahydro-furan-2-ylmethoxy)-6,7-dihydro-pyrimido[6,1-a]isoquinolin-4-one,
[4-Oxo-2-(tetrahydro-furan-2-ylmethoxy)-6,7-dihydro-4H-pyrimido[6,1-a]isoquinolin-9-yloxy]-acetonitrile,
9-Butoxy-2-(tetrahydro-furan-2-ylmethoxy)-6,7-dihydro-pyrimido[6,1-a]isoquinolin-4-one,
9-Cyclopropylmethoxy-2-(tetrahydro-furan-2-ylmethoxy)-6,7-dihydro-pyrimido[6,1-a]isoquinolin-4-one,
9-Phenethyloxy-2-(tetrahydro-furan-2-ylmethoxy)-6,7-dihydro-pyrimido[6,1-a]isoquinolin-4-one,
9-(Pyridin-4-ylmethoxy)-2-(tetrahydro-furan-2-ylmethoxy)-6,7-dihydro-pyrimido[6,1-a]isoquinolin-4-one,
9-(Pyridin-2-ylmethoxy)-2-(tetrahydro-furan-2-ylmethoxy)-6,7-dihydro-pyrimido[6,1-a]isoquinolin-4-one,
9-(Pyridin-2-ylmethoxy)-2-(tetrahydro-furan-2-ylmethoxy)-6,7-dihydro-pyrimido[6,1-a]isoquinolin-4-one,
9-(2-Phenoxy-ethoxy)-2-(tetrahydro-furan-2-ylmethoxy)-6,7-dihydro-pyrimido[6,1-a]isoquinolin-4-one,
9-Methoxy-2-(tetrahydro-pyran-4-ylmethoxy)-6,7-dihydro-pyrimido[6,1-a]isoquinolin-4-one,
9-Methoxy-2-(tetrahydro-pyran-2-ylmethoxy)-6,7-dihydro-pyrimido[6,1-a]isoquinolin-4-one,
9-Methoxy-2-(tetrahydro-pyran-2-ylmethoxy)-6,7-dihydro-pyrimido[6,1-a]isoquinolin-4-one,
4-[4-Oxo-2-(tetrahydro-furan-2-ylmethoxy)-6,7-dihydro-4H-pyrimido[6,1-a]isoquinolin-9-yloxymethyl] -benzonitrile,
4-[4-Oxo-2-(tetrahydro-furan-2-ylmethoxy)-6,7-dihydro-4H-pyrimido[6,1-a]isoquinolin-9-yloxymethyl] -benzonitrile,
9-(2,3-Dihydro-benzo[1,4]dioxin-2-ylmethoxy)-2-(tetrahydro-furan-2-ylmethoxy)-6,7-dihydro-pyrimido[6,1-a]isoquinolin-4-one,
9-(2,3-Dihydro-benzo[1,4]dioxin-2-ylmethoxy)-2-(tetrahydro-furan-2-ylmethoxy)-6,7-dihydro-pyrimido[6,1-a]isoquinolin-4-one,
3-[4-Oxo-2-(tetrahydro-furan-2-ylmethoxy)-6,7-dihydro-4H-pyrimido[6,1-a]isoquinolin-9-yloxymethyl] -benzonitrile,
2-[4-Oxo-2-(tetrahydro-furan-2-ylmethoxy)-6,7-dihydro-4H-pyrimido[6,1-a]isoquinolin-9-yloxymethyl] -benzonitrile,
9-(4-Chloro-benzyloxy)-2-(tetrahydro-furan-2-ylmethoxy)-6,7-dihydro-pyrimido[6,1-a]isoquinolin-4-one,
9-(3-Chloro-benzyloxy)-2-(tetrahydro-furan-2-ylmethoxy)-6,7-dihydro-pyrimido[6,1-a]isoquinolin-4-one,
9-(2-Chloro-benzyloxy)-2-(tetrahydro-furan-2-ylmethoxy)-6,7-dihydro-pyrimido[6,1-a]isoquinolin-4-one,
9-(4-Fluoro-benzyloxy)-2-(tetrahydro-furan-2-ylmethoxy)-6,7-dihydro-pyrimido[6,1-a]isoquinolin-4-one,
9-(2-Nitro-benzyloxy)-2-(tetrahydro-furan-2-ylmethoxy)-6,7-dihydro-pyrimido[6,1-a]isoquinolin-4-one,
4-[4-Oxo-2-(tetrahydro-furan-2-ylmethoxy)-6,7-dihydro-4H-pyrimido[6,1-a]isoquinolin-9-yloxymethyl]-benzoic acid methyl ester,
3-[4-Oxo-2-(tetrahydro-furan-2-ylmethoxy)-6,7-dihydro-4H-pyrimido[6,1-a]isoquinolin-9-yloxymethyl]-benzoic acid methyl ester,
3-[4-Oxo-2-(tetrahydro-furan-2-ylmethoxy)-6,7-dihydro-4H-pyrimido[6,1-a]isoquinolin-9-yloxymethyl]-benzoic acid methyl ester,
2-(2,3-Dihydro-benzo[1,4]dioxin-2-ylmethoxy)-9-(pyridin-2-ylmethoxy)-6,7-dihydro-pyrimido[6,1-a]isoquinolin-4-one,
[2-(2,3-Dihydro-benzo[1,4]dioxin-2-ylmethoxy)-4-oxo-6,7-dihydro-4H-pyrimido[6,1-a] isoquinolin-9-yloxy]-acetic acid tert-butyl ester,
2,9-Bis-(2,3-dihydro-benzo[1,4]dioxin-2-ylmethoxy)-6,7-dihydro-pyrimido[6,1-a]isoquinolin-4-one,
[2-(2,3-Dihydro-benzo[1,4] dioxin-2-ylmethoxy)-4-oxo-6,7-dihydro-4H-pyrimido [6,1-a] isoquinolin-9-yloxy]-acetic acid,
9-(3-Nitro-benzyloxy)-2-(tetrahydro-furan-2-ylmethoxy)-6,7-dihydro-pyrimido[6,1-a]isoquinolin-4-one,
9-(3-Nitro-benzyloxy)-2-(tetrahydro-furan-2-ylmethoxy)-6,7-dihydro-pyrimido[6,1-a]isoquinolin-4-one,
9-(4-Nitro-benzyloxy)-2-(tetrahydro-furan-2-ylmethoxy)-6,7-dihydro-pyrimido[6,1-a]isoquinolin-4-one,
9-(3-Methyl-benzyloxy)-2-(tetrahydro-furan-2-ylmethoxy)-6,7-dihydro-pyrimido[6,1-a]isoquinolin-4-one,
9-(4-Methyl-benzyloxy)-2-(tetrahydro-furan-2-ylmethoxy)-6,7-dihydro-pyrimido[6,1-a]isoquinolin-4-one,
9-(4-Methoxy-benzyloxy)-2-(tetrahydro-furan-2-ylmethoxy)-6,7-dihydro-pyrimido[6,1-a]isoquinolin-4-one,
9-(Naphthalen-2-ylmethoxy)-2-(tetrahydro-furan-2-ylmethoxy)-6,7-dihydro-pyrimido[6,1-a]isoquinolin-4-one,
9-(Naphthalen-1-ylmethoxy)-2-(tetrahydro-furan-2-ylmethoxy)-6,7-dihydro-pyrimido[6,1-a]isoquinolin-4-one,
9-(2-Methyl-benzyloxy)-2-(tetrahydro-furan-2-ylmethoxy)-6,7-dihydro-pyrimido[6,1-a]isoquinolin-4-one,
9-[2-(2-Methoxy-phenoxy)-ethoxy]-2-(tetrahydro-furan-2-ylmethoxy)-6,7-dihydro-pyrimido[6,1-a]isoquinolin-4-one,
9-[2-(3-Methoxy-phenoxy)-ethoxy]-2-(tetrahydro-furan-2-ylmethoxy)-6,7-dihydro-pyrimido[6,1-a]isoquinolin-4-one,
9-[2-(4-Methoxy-phenoxy)-ethoxy]-2-(tetrahydro-furan-2-ylmethoxy)-6,7-dihydro-pyrimido[6,1-a]isoquinolin-4-one,
9-[2-(2-Chloro-phenoxy)-ethoxy]-2-(tetrahydro-furan-2-ylmethoxy)-6,7-dihydro-pyrimido[6,1-a]isoquinolin-4-one,
9-[2-(3-Chloro-phenoxy)-ethoxy]-2-(tetrahydro-furan-2-ylmethoxy)-6,7-dihydro-pyrimido[6,1-a]isoquinolin-4-one,
9-[2-(4-Chloro-phenoxy)-ethoxy]-2-(tetrahydro-furan-2-ylmethoxy)-6,7-dihydro-pyrimido[6,1-a]isoquinolin-4-one,
2-(2,3-Dihydro-benzo[1,4]dioxin-2-ylmethoxy)-9-(2-morpholin-4-yl-2-oxo-ethoxy)-6,7-dihydro-pyrimido[6,1-a]isoquinolin-4-one,
2-(2,3-Dihydro-benzo[1,4]dioxin-2-ylmethoxy)-9-(2-morpholin-4-yl-2-oxo-ethoxy)-6,7-dihydro-pyrimido[6,1-a]isoquinolin-4-one,
2-(2,3-Dihydro-benzo[1,4]dioxin-2-ylmethoxy)-9-(2-morpholin-4-yl-2-oxo-ethoxy)-6,7-dihydro-pyrimido[6,1-a]isoquinolin-4-one,
2-(2,3-Dihydro-benzo[1,4]dioxin-2-ylmethoxy)-9-(2-morpholin-4-yl-2-oxo-ethoxy)-6,7-dihydro-pyrimido[6,1-a]isoquinolin-4-one,
2-[2-(2,3-Dihydro-benzo[1,4]dioxin-2-ylmethoxy)-4-oxo-6,7-dihydro-4H-pyrimido[6,1-a]isoquinolin-9-yloxy]-acetamide,
2-[2-(2,3-Dihydro-benzo[1,4]dioxin-2-ylmethoxy)-4-oxo-6,7-dihydro-4H-pyrimido[6,1-a]isoquinolin-9-yloxy]-N,N-dimethyl-acetamide,
9-(2,2-Dimethoxy-ethoxy)-2-(tetrahydro-furan-2-ylmethoxy)-6,7-dihydro-pyrimido[6,1-a]isoquinolin-4-one,
2-[2-(2,3-Dihydro-benzo[1,4]dioxin-2-ylmethoxy)-4-oxo-6,7-dihydro-4H-pyrimido[6,1-a]isoquinolin-9-yloxy]-2-methyl-propionamide,
2-(2,3-Dihydro-benzo[1,4]dioxin-2-ylmethoxy)-9-(1,1-dimethyl-2-morpholin-4-yl-2-oxo-ethoxy)-6,7-dihydro-pyrimido[6,1-a]isoquinolin-4-one,
9-(2-Benzyloxy-ethoxy)-2-(tetrahydro-furan-2-ylmethoxy)-6,7-dihydro-pyrimido[6,1-a]isoquinolin-4-one,
2,9-Bis-(tetrahydro-furan-2-ylmethoxy)-6,7-dihydro-pyrimido[6,1-a]isoquinolin-4-one,
9-(6-Phenyl-pyridin-2-ylmethoxy)-2-(tetrahydro-furan-2-ylmethoxy)-6,7-dihydro-pyrimido[6,1-a]isoquinolin-4-one,
2-(2,3-Dihydro-benzo[1,4]dioxin-2-ylmethoxy)-9-(tetrahydro-furan-2-ylmethoxy)-6,7-dihydro-pyrimido[6,1-a]isoquinolin-4-one,
9-[6-(1-Methyl-1H-pyrazol-4-yl)-pyridin-2-ylmethoxy]-2-(tetrahydro-furan-2-ylmethoxy)-6,7-dihydro-pyrimido[6,1-a]isoquinolin-4-one,
9-(6-Furan-3-yl-pyridin-2-ylmethoxy)-2-(tetrahydro-furan-2-ylmethoxy)-6,7-dihydro-pyrimido[6,1-a]isoquinolin-4-one,
9-(6-Pyrimidin-5-yl-pyridin-2-ylmethoxy)-2-(tetrahydro-furan-2-ylmethoxy)-6,7-dihydro-pyrimido[6,1-a]isoquinolin-4-one,
9-(1-Cyclopropyl-1H-tetrazol-5-ylmethoxy)-2-(tetrahydro-furan-2-ylmethoxy)-6,7-dihydro-pyrimido[6,1-a]isoquinolin-4-one,
2-[4-Oxo-2-(tetrahydro-furan-2-ylmethoxy)-6,7-dihydro-4H-pyrimido[6,1-a]isoquinolin-9-yloxy]-acetamide,
N,N-Diethyl-2-[4-oxo-2-(tetrahydro-furan-2-ylmethoxy)-6,7-dihydro-4H-pyrimido[6,1-a]isoquinolin-9-yloxy]-acetamide,
N,N-Dimethyl-2-[4-oxo-2-(tetrahydro-furan-2-ylmethoxy)-6,7-dihydro-4H-pyrimido[6,1-a]isoquinolin-9-yloxy]-acetamide,
N-Isopropyl-N-methyl-2-[4-oxo-2-(tetrahydro-furan-2-ylmethoxy)-6,7-dihydro-4H-pyrimido[6,1-a]isoquinolin-9-yloxy]-acetamide,
2-[4-Oxo-2-(tetrahydro-furan-2-ylmethoxy)-6,7-dihydro-4H-pyrimido[6,1-a]isoquinolin-9-yloxy]-N-phenyl-acetamide,
9-(1-Propyl-1H-tetrazol-5-ylmethoxy)-2-(tetrahydro-furan-2-ylmethoxy)-6,7-dihydro-pyrimido[6,1-a]isoquinolin-4-one,
9-(Oxazol-2-ylmethoxy)-2-(tetrahydro-furan-2-ylmethoxy)-6,7-dihydro-pyrimido[6,1-a]isoquinolin-4-one,
2-[2-(2,3-Dihydro-benzo[1,4]dioxin-2-ylmethoxy)-4-oxo-6,7-dihydro-4H-pyrimido[6,1-a]isoquinolin-9-yloxy]-N,N-diethyl-acetamide,
2-[2-(2,3-Dihydro-benzo[1,4]dioxin-2-ylmethoxy)-4-oxo-6,7-dihydro-4H-pyrimido[6,1-a]isoquinolin-9-yloxy]-N-isopropyl-N-methyl-acetamide,
2-[2-(2,3-Dihydro-benzo[1,4]dioxin-2-ylmethoxy)-4-oxo-6,7-dihydro-4H-pyrimido[6,1-a]isoquinolin-9-yloxy]-N-phenyl-acetamide,
2-(2,3-Dihydro-benzo[1,4]dioxin-2-ylmethoxy)-9-(1-propyl-1H-tetrazol-5-ylmethoxy)-6,7-dihydro-pyrimido[6,1-a]isoquinolin-4-one,
9-(1-Butyl-1H-tetrazol-5-ylmethoxy)-2-(2,3-dihydro-benzo[1,4]dioxin-2-ylmethoxy)-6,7-dihydro-pyrimido[6,1-a]isoquinolin-4-one,
2-(2,3-Dihydro-benzo[1,4]dioxin-2-ylmethoxy)-9-(2-morpholin-4-yl-ethoxy)-6,7-dihydro-pyrimido[6,1-a]isoquinolin-4-one,
[2-(2,3-Dihydro-benzo[1,4] dioxin-2-ylmethoxy)-4-oxo-6,7-dihydro-4H-pyrimido [6,1-a]isoquinolin-9-yloxy]-acetonitrile,
2-(2,3-Dihydro-benzo[1,4]dioxin-2-ylmethoxy)-9-(oxazol-2-ylmethoxy)-6,7-dihydro-pyrimido[6,1-a]isoquinolin-4-one,
2-(2,3-Dihydro-benzo[1,4]dioxin-2-ylmethoxy)-9-(2-oxo-2-pyrrolidin-1-yl-ethoxy)-6,7-dihydro-pyrimido[6,1-a]isoquinolin-4-one,
9-(1-Cyclopropyl-1H-tetrazol-5-ylmethoxy)-2-(2,3-dihydro-benzo[1,4]dioxin-2-ylmethoxy)-6,7-dihydro-pyrimido[6,1-a]isoquinolin-4-one,
2-(2,3-Dihydro-benzo[1,4]dioxin-2-ylmethoxy)-9-(1H-tetrazol-5-ylmethoxy)-6,7-dihydro-pyrimido[6,1-a]isoquinolin-4-one,
9-Allyloxy-2-(2,3-dihydro-[1,4]dioxino[2,3-b]pyridin-2-ylmethoxy)-6,7-dihydro-pyrimido[6,1-a]isoquinolin-4-one,
N-Benzyl-2-[2-(2,3-dihydro-benzo[1,4]dioxin-2-ylmethoxy)-4-oxo-6,7-dihydro-4H-pyrimido[6,1-a]isoquinolin-9-yloxy]-acetamide,
2-(2,3-Dihydro-benzo[1,4]dioxin-2-ylmethoxy)-9-(2-pyrrolidin-1-yl-ethoxy)-6,7-dihydro-pyrimido[6,1-a]isoquinolin-4-one,
2-(2,3-Dihydro-benzo[1,4]dioxin-2-ylmethoxy)-9-(2-piperidin-1-yl-ethoxy)-6,7-dihydro-pyrimido[6,1-a]isoquinolin-4-one,
2-(2,3-Dihydro-benzo[1,4]dioxin-2-ylmethoxy)-9-(3-piperidin-1-yl-propoxy)-6,7-dihydro-pyrimido[6,1-a]isoquinolin-4-one,
2-(2,3-Dihydro-benzo[1,4]dioxin-2-ylmethoxy)-9-(3-dimethylamino-propoxy)-6,7-dihydro-pyrimido[6,1-a]isoquinolin-4-one,
2-(2,3-Dihydro-benzo[1,4]dioxin-2-ylmethoxy)-9-[2-(1-methyl-pyrrolidin-2-yl)-ethoxy]-6,7-dihydro-pyrimido[6,1-a]isoquinolin-4-one,
2-(2,3-Dihydro-benzo[1,4]dioxin-2-ylmethoxy)-9-[3-(4-methyl-piperazin-1-yl)-propoxy]-6,7-dihydro-pyrimido[6,1-a]isoquinolin-4-one,
5-[2-(2,3-Dihydro-benzo[1,4]dioxin-2-ylmethoxy)-4-oxo-6,7-dihydro-4H-pyrimido[6,1-a]isoquinolin-9-yloxymethyl]-furan-2-carboxylic acid ethyl ester,
2-(2,3-Dihydro-[1,4]dioxino[2,3-b]pyridin-2-ylmethoxy)-9-(oxazol-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(oxazol-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(oxazol-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(oxazol-2-ylmethoxy)-6,7-dihydro-pyrimido[6,1-a]isoquinolin-4-one,
9-(5-tert-Butyl-[1,2,4]oxadiazol-3-ylmethoxy)-2-(tetrahydro-furan-2-ylmethoxy)-6,7-dihydro-pyrimido[6,1-a]isoquinolin-4-one,
9-(5-Phenyl-[I,2,4]oxadiazol-3-ylmethoxy)-2-(tetrahydro-furan-2-ylmethoxy)-6,7-dihydro-pyrimido[6,1-a]isoquinolin-4-one,
[2-(2,3-Dihydro-[1,4]dioxino[2,3-b]pyridin-2-ylmethoxy)-4-oxo-6,7-dihydro-4H-pyrimido[6,1-a] isoquinolin-9-yloxy]-acetonitrile,
[2-(2,3-Dihydro-[1,4]dioxino[2,3-b]pyridin-2-ylmethoxy)-4-oxo-6,7-dihydro-4H-pyrimido[6,1-a] isoquinolin-9-yloxy]-acetonitrile,
[2-(2,3-Dihydro-[1,4]dioxino[2,3-b]pyridin-2-ylmethoxy)-4-oxo-6,7-dihydro-4H-pyrimido[6,1-a] isoquinolin-9-yloxy]-acetonitrile,
2-(2,3-Dihydro-[1,4]dioxino[2,3-b]pyridin-2-ylmethoxy)-9-(2-morpholin-4-yl-ethoxy)-6,7-dihydro-pyrimido[6,1-a]isoquinolin-4-one,
2-(2,3-Dihydro-[1,4]dioxino[2,3-b]pyridin-2-ylmethoxy)-9-(pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-[2-(1-methyl-pyrrolidin-2-yl)-ethoxy]-6,7-dihydro-pyrimido[6,1-a]isoquinolin-4-one,
2-(2,3-Dihydro-[1,4]dioxino[2,3-b]pyridin-2-ylmethoxy)-9-(1H-tetrazol-5-ylmethoxy)-6,7-dihydro-pyrimido[6,1-a]isoquinolin-4-one,
9-Pyridin-3-yl-2-(tetrahydro-furan-2-ylmethoxy)-6,7-dihydro-pyrimido[6,1-a]isoquinolin-4-one,
3-[4-Oxo-2-(tetrahydro-furan-2-ylmethoxy)-6,7-dihydro-4H-pyrimido[6,1-a]isoquinolin-9-yl]-benzonitrile,
9-Phenyl-2-(tetrahydro-furan-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-pyridin-4-yl-6,7-dihydro-pyrimido[6,1-a]isoquinolin-4-one,
3-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-benzonitrile,
9-(2-Methoxy-phenyl)-2-(tetrahydro-furan-2-ylmethoxy)-6,7-dihydro-pyrimido[6,1-a]isoquinolin-4-one,
9-(3-Methoxy-phenyl)-2-(tetrahydro-furan-2-ylmethoxy)-6,7-dihydro-pyrimido[6,1-a]isoquinolin-4-one,
9-(4-Methoxy-phenyl)-2-(tetrahydro-furan-2-ylmethoxy)-6,7-dihydro-pyrimido[6,1-a]isoquinolin-4-one,
4-[4-Oxo-2-(tetrahydro-furan-2-ylmethoxy)-6,7-dihydro-4H-pyrimido[6,1-a]isoquinolin-9-yl]-benzonitrile,
3-[4-Oxo-2-(tetrahydro-furan-2-ylmethoxy)-6,7-dihydro-4H-pyrimido[6,1-a]isoquinolin-9-yl]-benzoic acid,
4-[4-Oxo-2-(tetrahydro-furan-2-ylmethoxy)-6,7-dihydro-4H-pyrimido[6,1-a]isoquinolin-9-yl]-benzoic acid,
9-(4-Dimethylamino-phenyl)-2-(tetrahydro-furan-2-ylmethoxy)-6,7-dihydro-pyrimido[6,1-a]isoquinolin-4-one,
4-[4-Oxo-2-(tetrahydro-furan-2-ylmethoxy)-6,7-dihydro-4H-pyrimido[6,1-a]isoquinolin-9-yl]-benzamide,
2-[4-Oxo-2-(tetrahydro-furan-2-ylmethoxy)-6,7-dihydro-4H-pyrimido[6,1-a]isoquinolin-9-yl]-benzonitrile,
9-(1-Methyl-1H-pyrazol-4-yl)-2-(tetrahydro-furan-2-ylmethoxy)-6,7-dihydro-pyrimido[6,1-a]isoquinolin-4-one,
N,N-Dimethyl-3-[4-oxo-2-(tetrahydro-furan-2-ylmethoxy)-6,7-dihydro-4H-pyrimido[6,1-a] isoquinolin-9-yl] -benzamide,
9-(6-Methoxy-pyridin-3-yl)-2-(tetrahydro-furan-2-ylmethoxy)-6,7-dihydro-pyrimido[6,1-a]isoquinolin-4-one,
9-(2,6-Dimethyl-phenyl)-2-(tetrahydro-furan-2-ylmethoxy)-6,7-dihydro-pyrimido[6,1-a]isoquinolin-4-one,
9-(3,5-Dimethyl-isoxazol-4-yl)-2-(tetrahydro-furan-2-ylmethoxy)-6,7-dihydro-pyrimido[6,1-a]isoquinolin-4-one,
9-Naphthalen-2-yl-2-(tetrahydro-furan-2-ylmethoxy)-6,7-dihydro-pyrimido[6,1-a]isoquinolin-4-one,
9-Naphthalen-1-yl-2-(tetrahydro-furan-2-ylmethoxy)-6,7-dihydro-pyrimido[6,1-a]isoquinolin-4-one,
9-Pyrimidin-5-yl-2-(tetrahydro-furan-2-ylmethoxy)-6,7-dihydro-pyrimido[6,1-a]isoquinolin-4-one,
9-(5-Chloro-thiophen-2-yl)-2-(tetrahydro-furan-2-ylmethoxy)-6,7-dihydro-pyrimido[6,1-a]isoquinolin-4-one,
2-[4-Oxo-2-(tetrahydro-furan-2-ylmethoxy)-6,7-dihydro-4H-pyrimido[6,1-a]isoquinolin-9-yl]-pyrrole-1-carboxylic acid tert-butyl ester,
Trifluoro-methanesulfonic acid 4-oxo-2-(tetrahydro-furan-2-ylmethoxy)-6,7-dihydro-4H-pyrimido[6,1-a]isoquinolin-9-ylester,
2-(2,3-Dihydro-[1,4]dioxino[2,3-b]pyridin-2-ylmethoxy)-9-(6-methoxy-pyridin-3-yl)-6,7-dihydro-pyrimido[6,1-a] isoquinolin-4-one,
9-Cyclopropylethynyl-2-(tetrahydro-furan-2-ylmethoxy)-6,7-dihydro-pyrimido[6,1-a]isoquinolin-4-one,
9-(3,3-Dimethyl-but-1-ynyl)-2-(tetrahydro-furan-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(pyridin-4-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-pyridin-3-yl-6,7-dihydro-pyrimido[6,1-a]isoquinolin-4-one,
2-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-benzonitrile,
2-(2,3-Dihydro-[1,4]dioxino[2,3-b]pyridin-2-ylmethoxy)-9-(2-methoxy-phenyl)-6,7-dihydro-pyrimido[6,1-a]isoquinolin-4-one,
2-(2,3-Dihydro-[1,4]dioxino[2,3-b]pyridin-2-ylmethoxy)-9-(1H-indazol-5-yl)-6,7-dihydro-pyrimido[6,1-a]isoquinolin-4-one,
2-(2,3-Dihydro-[1,4]dioxino[2,3-b]pyridin-2-ylmethoxy)-9-pyrimidin-5-yl-6,7-dihydro-pyrimido[6,1-a]isoquinolin-4-one,
3-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-benzamide,
2-(2,3-Dihydro-[1,4]dioxino[2,3-b]pyridin-2-ylmethoxy)-9-(2-dimethylamino-phenyl)-6,7-dihydro-pyrimido[6,1-a]isoquinolin-4-one,
2-(2,3-Dihydro-[1,4]dioxino[2,3-b]pyridin-2-ylmethoxy)-9-pyridin-3-ylethynyl-6,7-dihydro-pyrimido[6,1-a]isoquinolin-4-one,
2-(2,3-Dihydro-[1,4]dioxino[2,3-b]pyridin-2-ylmethoxy)-9-(3-methoxy-prop-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(4-hydroxy-but-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(1,5-dimethyl-1H-pyrazol-3-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(3-methyl-[1,2,4]oxadiazol-5-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-3-methyl-but-1-ynyl)-6,7-dihydro-pyrimido[6,1-a]isoquinolin-4-one,
2-(2,3-Dihydro-[1,4]dioxino[2,3-b]pyridin-2-ylmethoxy)-9-pyridin-4-ylethynyl-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-prop-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(6-methyl-pyridin-3-yl)-6,7-dihydro-pyrimido[6,1-a]isoquinolin-4-one,
2-(2,3-Dihydro-[1,4]dioxino[2,3-b]pyridin-2-ylmethoxy)-9-(5-methoxy-pyridin-3-yl)-6,7-dihydro-pyrimido[6,1-a]isoquinolin-4-one,
9-Cyclopropylethynyl-2-(2,3-dihydro-[1,4]dioxino[2,3-b]pyridin-2-ylmethoxy)-6,7-dihydro-pyrimido[6,1-a]isoquinolin-4-one,
9-Cyclopropylethynyl-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(1-hydroxy-cyclopentylethynyl)-6,7-dihydro-pyrimido[6,1-a]isoquinolin-4-one,
5-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-pent-4-ynenitrile,
2-(2,3-Dihydro-[1,4]dioxino[2,3-b]pyridin-2-ylmethoxy)-9-(3,3-dimethyl-but-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(2-methoxy-pyridin-3-yl)-6,7-dihydro-pyrimido[6,1-a]isoquinolin-4-one,
2-(2,3-Dihydro-[1,4]dioxino[2,3-b]pyridin-2-ylmethoxy)-6,7-dihydro-pyrimido[6,1-a]isoquinolin-4-one,
5-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-pyridine-2-carboxylic acid methylamide,
2-(2,3-Dihydro-[1,4]dioxino[2,3-b]pyridin-2-ylmethoxy)-9-furan-3-yl-6,7-dihydro-pyrimido[6,1-a]isoquinolin-4-one,
2-(2,3-Dihydro-[1,4]dioxino[2,3-b]pyridin-2-ylmethoxy)-9-(1-methyl-1H-pyrazol-4-yl)-6,7-dihydro-pyrimido[6,1-a]isoquinolin-4-one,
2-(2,3-Dihydro-[1,4]dioxino[2,3-b]pyridin-2-ylmethoxy)-9-morpholin-4-ylmethyl-6,7-dihydro-pyrimido[6,1-a]isoquinolin-4-one,
[2-(2,3-Dihydro-[1,4]dioxino[2,3-b]pyridin-2-ylmethoxy)-4-oxo-6,7-dihydro-4H-pyrimido[6,1-a]isoquinolin-9-ylamino]-acetonitrile,
2-(2,3-Dihydro-[1,4]dioxino[2,3-b]pyridin-2-ylmethoxy)-4-oxo-6,7-dihydro-4H-pyrimido[6,1-a]isoquinoline-9-carbonitrile,
2-(2,3-Dihydro-[1,4]dioxino[2,3-b]pyridin-2-ylmethoxy)-9-[(oxazol-2-ylmethyl)-amino]-6,7-dihydro-pyrimido[6,1-a]isoquinolin-4-one,
2-(2,3-Dihydro-[1,4]dioxino[2,3-b]pyridin-2-ylmethoxy)-9-(5-methyl-[1,2,4]oxadiazol-3-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(5-ethyl-[1,2,4]oxadiazol-3-ylmethoxy)-6,7-dihydro-pyrimido[6,1-a]isoquinolin-4-one,
9-(5-Cyclopropyl-[1,2,4]oxadiazol-3-ylmethoxy)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(5-isopropyl-[1,2,4]oxadiazol-3-ylmethoxy)-6,7-dihydro-pyrimido[6,1-a]isoquinolin-4-one,
9-(5-tert-Butyl-[1,2,4]oxadiazol-3-ylmethoxy)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(3-methyl-isoxazol-5-ylmethoxy)-6,7-dihydro-pyrimido[6,1-a]isoquinolin-4-one,
9-(3-Chloro-2-methoxy-pyridin-4-yl)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(3,6-dihydro-2H-pyran-4-yl)-6,7-dihydro-pyrimido[6,1-a]isoquinolin-4-one,
5-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-pyridine-2-carbonitrile,
2-(2,3-Dihydro-[1,4]dioxino[2,3-b]pyridin-2-ylmethoxy)-9-(2-ethoxy-pyridin-3-yl)-6,7-dihydro-pyrimido[6,1-a]isoquinolin-4-one,
2-(2,3-Dihydro-[1,4]dioxino[2,3-b]pyridin-2-ylmethoxy)-9-(6-ethoxy-pyridin-3-yl)-6,7-dihydro-pyrimido[6,1-a]isoquinolin-4-one,
2-(2,3-Dihydro-[1,4]dioxino[2,3-b]pyridin-2-ylmethoxy)-9-(6-morpholin-4-yl-pyridin-3-yl)-6,7-dihydro-pyrimido[6,1-a]isoquinolin-4-one,
2-(2,3-Dihydro-[1,4]dioxino[2,3-b]pyridin-2-ylmethoxy)-9-(2-methoxy-pyrimidin-5-yl)-6,7-dihydro-pyrimido[6,1-a]isoquinolin-4-one,
2-(2,3-Dihydro-[1,4]dioxino[2,3-b]pyridin-2-ylmethoxy)-9-(2,3-dimethoxy-phenyl)-6,7-dihydro-pyrimido[6,1-a]isoquinolin-4-one,
2-(2,3-Dihydro-[1,4]dioxino[2,3-b]pyridin-2-ylmethoxy)-9-(2,5-dimethoxy-phenyl)-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-3-phenyl-prop-1-ynyl)-6,7-dihydro-pyrimido[6,1-a]isoquinolin-4-one,
3-{3-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-prop-2-ynyloxy}-propionitrile,
2-(2,3-Dihydro-[1,4]dioxino[2,3-b]pyridin-2-ylmethoxy)-9-(3-methylamino-prop-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(3-dimethylamino-prop-1-ynyl)-6,7-dihydro-pyrimido[6,1-a]isoquinolin-4-one,
9-[3-(Benzyl-methyl-amino)-prop-1-ynyl]-2-(2,3-diohydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-[3-(1,1-dioxo-thiomorpholin-4-yl)-prop-1-ynyl]-6,7-dihydro-pyrimido[6,1-a]isoquinolin-4-one,
{3-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-prop-2-ynyl}-urea,
1-{3-[2-(2,3-Dihydro-[1,4]dioxino[2,3-b]pyridin-2-ylmethoxy)-4-oxo-6,7-dihydro-4H-pyrimido[6,1-a]isoquinolin-9-yl]-prop-2-ynyl}-imidazolidine-2,4-dione,
9-(3-Diethylamino-prop-1-ynyl)-2-(2,3-dihydro-[1,4]dioxino[2,3-b]pyridin-2-ylmethoxy)-6,7-dihydro-pyrimido[6,1-a]isoquinolin-4-one,
9-(3-Amino-3-methyl-but-1-ynyl)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(tetrahydro-pyran-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(1H-pyrazol-4-yl)-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-but-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-pent-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-hex-1-ynyl)-6,7-dihydro-pyrimido[6,1-a]isoquinolin-4-one,
9-(1-Amino-cyclohexylethynyl)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-5-methyl-hex-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(3-ethyl-3-hydroxy-pent-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-3-phenyl-but-1-ynyl)-6,7-dihydro-pyrimido[6,1-a]isoquinolin-4-one,
2-(2,3-Dihydro-[1,4]dioxino[2,3-b]pyridin-2-ylmethoxy)-9-(3-hydroxy-4-methyl-pent-1-ynyl)-6,7-dihydro-pyrimido[6,1-a]isoquinolin-4-one,
2-[(R)-1-(2,3-Dihydro-[1,4]dioxino[2,3-b]pyridin-2-yl)methoxy]-9-(oxazol-2-ylmethoxy)-6,7-dihydro-pyrimido[6,1-a]isoquinolin-4-one,
2-[(R)-1-(2,3-Dihydro-[1,4]dioxino[2,3-b]pyridin-2-yl)methoxy]-9-(oxazol-2-ylmethoxy)-6,7-dihydro-pyrimido[6,1-a]isoquinolin-4-one,
2-[(S)-1-(2,3-Dihydro-[1,4]dioxino[2,3-b]pyridin-2-yl)methoxy]-9-(oxazol-2-ylmethoxy)-6,7-dihydro-pyrimido[6,1-a]isoquinolin-4-one,
9-(3-Butylamino-prop-1-ynyl)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(2-morpholin-4-yl-ethoxymethyl)-6,7-dihydro-pyrimido[6,1-a]isoquinolin-4-one,
9-(3-Benzylamino-prop-1-ynyl)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-(3-phenylamino-prop-1-ynyl)-6,7-dihydro-pyrimido[6,1-a]isoquinolin-4-one,
2-(2,3-Dihydro-[1,4]dioxino[2,3-b]pyridin-2-ylmethoxy)-4-oxo-6,7-dihydro-4H-pyrimido[6,1-a]isoquinoline-9-carboxylic acid (tetrahydro-pyran-4-yl)-amide,
2-(2,3-Dihydro-[1,4]dioxino[2,3-b]pyridin-2-ylmethoxy)-4-oxo-6,7-dihydro-4H-pyrimido[6,1-a]isoquinoline-9-carboxylic acid oxetan-3-ylmethyl)-amide,
2-(2,3-Dihydro-[1,4]dioxino[2,3-b]pyridin-2-ylmethoxy)-9-pyrrolidin-1-ylmethyl-6,7-dihydro-pyrimido[6,1-a]isoquinolin-4-one,
9-(tert-Butylamino-methyl)-2-(2,3-dihydro-[1,4]dioxino[2,3-b]pyridin-2-ylmethoxy)-6,7-dihydro-pyrimido[6,1-a]isoquinolin-4-one,
2-(2,3-Dihydro-[1,4]dioxino[2,3-b]pyridin-2-ylmethoxy)-9-piperidin-1-ylmethyl-6,7-dihydro-pyrimido[6,1-a]isoquinolin-4-one,
2-(2,3-Dihydro-[1,4]dioxino[2,3-b]pyridin-2-ylmethoxy)-9-(3-methyl-oxetan-3-ylmethoxymethyl)-6,7-dihydro-pyrimido[6,1-a]isoquinolin-4-one,
2-(2,3-Dihydro-[1,4]dioxino[2,3-b]pyridin-2-ylmethoxy)-9-(3-methyl-oxetan-3-ylmethoxymethyl)-6,7-dihydro-pyrimido[6,1-a]isoquinolin-4-one,
2-(2,3-Dihydro-[1,4]dioxino[2,3-b]pyridin-2-ylmethoxy)-9-(oxetan-3-yloxymethyl)-6,7-dihydro-pyrimido[6,1-a]isoquinolin-4-one,
9-Cyclopropylethynyl-2-(4-isopropyl-oxetan-2-ylmethoxy)-6,7-dihydro-pyrimido[6,1-a]isoquinolin-4-one, and
9-Cyclopropylethynyl-2-((R)-4-isopropyl-oxetan-2-ylmethoxy)-6,7-dihydro-pyrimido[6,1-a]isoquinolin-4-one.

6. The compound or pharmaceutically acceptable salt thereof for use according to claim 1, wherein said compound is selected from:
9-methoxy-1-methyl-2-[(tetrahydro-furan-2-ylmethyl)-amino]-10-(2,2,2-trifluoro-ethoxy)-6,7-dihydro-pyrido[2,1-a]isoquinolin-4-one,
9-cyclopropylethynyl-2-((S)-1-[1,4]dioxan-2-ylmethoxy)-10-methoxy-1-methyl-6,7-dihydro-pyrido[2,1-a]isoquinolin-4-one,
9-(2,2-difluoro-cyclopropylmethoxy)-2-((S)-1-[1,4]dioxan-2-ylmethoxy)-10-methoxy-1-methyl-6,7-dihydro-pyrido[2,1-a]isoquinolin-4-one,
10-(2,2-difluoro-ethoxy)-2-[([1,4]dioxan-2-ylmethyl)-amino]-9-methoxy-1-methyl-6,7-dihydro-pyrido[2,1-a]isoquinolin-4-one,
9-(2,2-difluoro-ethoxy)-10-methoxy-1-methyl-2-[(tetrahydro-furan-2-ylmethyl)-amino]-6,7-dihydro-pyrido[2,1-a]isoquinolin-4-one,
10-methoxy-1-methyl-2-[(tetrahydro-furan-2-ylmethyl)-amino]-9-(2,2,2-trifluoro-ethoxy)-6,7-dihydro-pyrido[2,1-a]isoquinolin-4-one,
9-(6-cyclopropyl-pyridin-3-yl)-2-((S)-1-[1,4]dioxan-2-ylmethoxy)-10-methoxy-1-methyl-6,7-dihydro-pyrido[2,1-a]isoquinolin-4-one,
2-(2,3-dihydro-thieno[3,4-b][1,4]dioxin-2-ylmethoxy)-9,10-dimethoxy-6,7-dihydro-pyrido[2,1-a]isoquinolin-4-one,
trifluoro-methanesulfonic acid 2-((S)-1-[1,4]dioxan-2-ylmethoxy)-10-methoxy-1-methyl-4-oxo-6,7-dihydro-4H-pyrido[2,1-a]isoquinolin-9-yl ester,
2-[([1,4]dioxan-2-ylmethyl)-amino]-9-methoxy-1-methyl-10-(2,2,2-trifluoro-ethoxy)-6,7-dihydro-pyrido[2,1-a]isoquinolin-4-one,
10-(2,2-difluoro-ethoxy)-9-methoxy-1-methyl-2-[(tetrahydro-furan-2-ylmethyl)-amino]-6,7-dihydro-pyrido[2,1-a]isoquinolin-4-one,
2-((S)-1-[1,4]dioxan-2-ylmethoxy)-10-methoxy-1-methyl-9-[6-(2,2,2-trifluoro-ethoxy)-pyridin-3-yl]-6,7-dihydro-pyrido[2,1-a]isoquinolin-4-one,
2-((S)-1-[1,4]dioxan-2-ylmethoxy)-9-(6-fluoro-pyridin-3-yl)-10-methoxy-1-methyl-6,7-dihydro-pyrido[2,1-a]isoquinolin-4-one,
9,10-bis-(2,2-difluoro-cyclopropylmethoxy)-2-((S)-1-[1,4]dioxan-2-ylmethoxy)-1-methyl-6,7-dihydro-pyrido[2,1-a]isoquinolin-4-one,
9-(3,3-difluoro-cyclobutylmethoxy)-2-((S)-1-[1,4]dioxan-2-ylmethoxy)-10-methoxy-1-methyl-6,7-dihydro-pyrido[2,1-a]isoquinolin-4-one,
2-((S)-1-[1,4]dioxan-2-ylmethoxy)-10-methoxy-1-methyl-9-(2,2,2-trifluoro-ethoxy)-6,7-dihydro-pyrido[2,1-a]isoquinolin-4-one,
10-difluoromethoxy-9-methoxy-1-methyl-2-[(tetrahydro-furan-2-ylmethyl)-amino]-6,7-dihydro-pyrido[2,1-a]isoquinolin-4-one,
10-(2,2-difluoro-ethoxy)-9-methoxy-1-methyl-2-(tetrahydro-pyran-2-ylmethyl)-amino]-6,7-dihydro-pyrido[2,1-a]isoquinolin-4-one,
2-((S)-1-[1,4]dioxan-2-ylmethoxy)-10-methoxy-1-methyl-9-(6-oxo-1,6-dihydro-pyridin-3-yl)-6,7-dihydro-pyrido[2,1-a]isoquinolin-4-one,
2-((S)-1-[1,4]dioxan-2-ylmethoxy)-10-methoxy-1-methyl-9-(3-methyl-oxetan-3-ylmethoxy)-6,7-dihydro-pyrido[2,1-a]isoquinolin-4-one,
9-methoxy-1-methyl-2-[(tetrahydro-pyran-2-ylmethyl)-amino]-10-(2,2,2-trifluoro-ethoxy)-6,7-dihydro-pyrido[2,1-a]isoquinolin-4-one,
9-(2,2-difluoro-ethoxy)-2-((S)-1-[1,4]dioxan-2-ylmethoxy)-10-methoxy-1-methyl-6,7-dihydro-pyrido[2,1-a]isoquinolin-4-one,
2-[([1,4]dioxan-2-ylmethyl)-amino]-9,10-dimethoxy-1-methyl-6,7-dihydro-pyrido[2,1-a]isoquinolin-4-one,
9-cyclopropylethynyl-2-((S)-1-[1,4]dioxan-2-ylmethoxy)-1-methyl-6,7-dihydro-pyrido[2,1-a]isoquinolin-4-one,
2-(2,3-dihydro-benzo[1,4]dioxin-2-ylmethoxy)-9-hydroxy-10-methoxy-6,7-dihydro-pyrido[2,1-a]isoquinolin-4-one,
9-difluoromethoxy-2-((S)-1-[1,4]dioxan-2-ylmethoxy)-10-methoxy-1-methyl-6,7-dihydro-pyrido[2,1-a]isoquinolin-4-one,
9-cyclopropylmethoxy-2-((S)-1-[1,4]dioxan-2-ylmethoxy)-10-methoxy-1-methyl-6,7-dihydro-pyrido[2,1-a]isoquinolin-4-one,
2-((S)-1-[1,4]dioxan-2-ylmethoxy)-9-(4-ethoxy-3-trifluoromethyl-phenyl)-10-methoxy-1-methyl-6,7-dihydro-pyrido[2,1-a]isoquinolin-4-one,
9,10-dimethoxy-1-methyl-2-[(tetrahydro-pyran-2-ylmethyl)-amino]-6,7-dihydro-pyrido[2,1-a]isoquinolin-4-one,
8,9-dimethoxy-2-[(tetrahydro-pyran-2-ylmethyl)-amino]-6,7-dihydro-pyrido[2,1-a]isoquinolin-4-one,
9,10-dimethoxy-1-methyl-2-[(tetrahydro-furan-2-ylmethyl)-amino]-6,7-dihydro-pyrido[2,1-a]isoquinolin-4-one,
10-difluoromethylsulfanyl-2-((S)-1-[1,4]dioxan-2-ylmethoxy)-9-methoxy-1-methyl-6,7-dihydro-pyrido[2,I-a]isoquinolin-4-one,
9-methoxy-1-methyl-2-[(tetrahydro-furan-2-ylmethyl)-amino]-6,7-dihydro-pyrido[2,1-a]isoquinolin-4-one,
2-((S)-1-[1,4]dioxan-2-ylmethoxy)-10-methoxy-1-methyl-9-(4-trifluoromethoxy-phenyl)-6,7-dihydro-pyrido[2,1-a] isoquinolin-4-one,
9-(2,2-difluoro-cyclopropylmethoxy)-1-methyl-2-[(tetrahydro-furan-2-ylmethyl)-amino]-6,7-dihydro-pyrido[2,1-a] isoquinolin-4-one,
8,9-dimethoxy-1-methyl-2-[(tetrahydro-furan-2-ylmethyl)-amino]-6,7-dihydro-pyrido[2,1-a]isoquinolin-4-one,
2-(2,3-dihydro-benzo[1,4]dioxin-2-ylmethoxy)-9,10-dimethoxy-1-methyl-6,7-dihydro-pyrido[2,1-a]isoquinolin-4-one,
2-((S)-1-[1,4]dioxan-2-ylmethoxy)-9-methoxy-1,10-dimethyl-6,7-dihydro-pyrido[2,1-a] isoquinolin-4-one,
8,11-dimethoxy-2-[(tetrahydro-pyran-2-ylmethyl)-amino]-6,7-dihydro-pyrido[2,1-a]isoquinolin-4-one,
2-[([1,4]dioxan-2-ylmethyl)-amino]-8,11-dimethoxy-6,7-dihydro-pyrido[2,1-a]isoquinolin-4-one,
8,11-dimethoxy-2-[(tetrahydro-furan-2-ylmethyl)-amino]-6,7-dihydro-pyrido[2,1-a]isoquinolin-4-one,
2-[(R)-1-(2,3-dihydro-[1,4]dioxino[2,3-b]pyridin-2-yl)methoxy]-9,10-dimethoxy-1-methyl-6,7-dihydro-pyrido[2,1-a] isoquinolin-4-one,
2-[([1,4]dioxan-2-ylmethyl)-amino]-8,9-dimethoxy-1-methyl-6,7-dihydro-pyrido[2,1-a]isoquinolin-4-one,
10-(2,2-difluoro-ethoxy)-9-methoxy-1-methyl-2-(tetrahydro-pyran-2-ylmethoxy)-6,7-dihydro-pyrido[2,1-a]isoquinolin-4-one,
8,9-dimethoxy-2-[(tetrahydro-furan-2-ylmethyl)-amino]-6,7-dihydro-pyrido[2,1-a]isoquinolin-4-one,
2-((S)-1-[1,4]dioxan-2-ylmethoxy)-8,9-dimethoxy-6,7-dihydro-pyrido[2,1-a]isoquinolin-4-one,
2-[(S)-1-(2,3-dihydro-[1,4]dioxino[2,3-b]pyridin-2-yl)methoxy]-9,10-dimethoxy-1-methyl-6,7-dihydro-pyrido[2,1-a]isoquinolin-4-one,
2-((S)-1-[1,4]dioxan-2-ylmethoxy)-1-methyl-9-(pyridin-2-ylmethoxy)-6,7-dihydro-pyrido[2,1-a]isoquinolin-4-one,
2-[([1,4]dioxan-2-ylmethyl)-amino]-8,9-dimethoxy-6,7-dihydro-pyrido[2,1-a]isoquinolin-4-one, 2-((S)-1-[1,4]dioxan-2-ylmethoxy)-10-methoxy-1-methyl-4-oxo-6,7-dihydro-4H-pyrido[2,1-a]isoquinoline-9-carbonitrile,
9-methoxy-1-methyl-2-(tetrahydro-pyran-2-ylmethoxy)-10-(2,2,2-trifluoro-ethoxy)-6,7-dihydro-pyrido[2,1-a]isoquinolin-4-one,
9-(2-cyclopropyl-ethyl)-2-((S)-1-[1,4]dioxan-2-ylmethoxy)-1-methyl-6,7-dihydro-pyrido[2,1-a]isoquinolin-4-one,
9-(2-cyclopropyl-ethyl)-2-((S)-1-[1,4]dioxan-2-ylmethoxy)-1,7,7-trimethyl-6,7-dihydro-pyrido[2,1-a]isoquinolin-4-one,
2-((S)-1-[1,4]dioxan-2-ylmethoxy)-1,7,7-trimethyl-9-(pyridin-2-ylmethoxy)-6,7-dihydro-pyrido[2,1-a]isoquinolin-4-one,
9,11-dimethoxy-2-[(tetrahydro-furan-2-ylmethyl)-amino]-6,7-dihydro-pyrido[2,1-a]isoquinolin-4-one,
2-(2,3-dihydro-benzo[1,4]dioxin-2-ylmethoxy)-9,10-dimethoxy-6,7-dihydro-pyrido[2,1-a]isoquinolin-4-one,
2-((S)-1-[1,4]dioxan-2-ylmethoxy)-9,10-dimethoxy-1-methyl-6,7-dihydro-pyrido[2,1-a]isoquinolin-4-one,
methanesulfonic acid 2-((S)-1-[1,4]dioxan-2-ylmethoxy)-10-methoxy-1-methyl-4-oxo-6,7-dihydro-4H-pyrido[2,1-a]isoquinolin-9-yl ester,
2-((S)-1-[1,4]dioxan-2-ylmethoxy)-1,7,7-trimethyl-9-pentyl-6,7-dihydro-pyrido[2,1-a]isoquinolin-4-one,
2-((S)-1-[1,4]dioxan-2-ylmethoxy)-1-methyl-9-(3-methyl-butyl)-6,7-dihydro-pyrido[2,1-a]isoquinolin-4-one,
2-((S)-1-[1,4]dioxan-2-ylmethoxy)-10-methoxy-1-methyl-6,7-dihydro-pyrido[2,1-a]isoquinolin-4-one,
2-([1,4]dioxan-2-ylmethoxy)-9,10-dimethoxy-6,7-dihydro-pyrido[2,1-a]isoquinolin-4-one, 8,11-dimethoxy-2-(tetrahydro-pyran-2-ylmethoxy)-6,7-dihydro-pyrido[2,1-a]isoquinolin-4-one,
2-((S)-1-[1,4]dioxan-2-ylmethoxy)-9-(5-ethyl-[1,2,4]oxadiazol-3-ylmethoxy)-1-methyl-6,7-dihydro-pyrido[2,1-a]isoquinolin-4-one,
2-[([1,4]dioxan-2-ylmethyl)-amino]-9,11-dimethoxy-6,7-dihydro-pyrido[2,1-a]isoquinolin-4-one, 8,9-dimethoxy-2-(tetrahydro-pyran-2-ylmethoxy)-6,7-dihydro-pyrido[2,1-a]isoquinolin-4-one,
2-(2,3-dihydro-benzo[1,4]dioxin-2-ylmethoxy)-9-methoxy-6,7-dihydro-pyrido[2,1-a]isoquinolin-4-one,
9-methoxy-1-methyl-10-(pyridin-2-ylmethoxy)-2-[(tetrahydro-furan-2-ylmethyl)-amino]-6,7-dihydro-pyrido[2,1-a]isoquinolin-4-one,
9-(3,5-dimethyl-isoxazol-4-yl)-2-((S)-1-[1,4]dioxan-2-ylmethoxy)-1-methyl-6,7-dihydro-pyrido[2,1-a]isoquinolin-4-one,
9-benzyloxy-2-((S)-1-[1,4]dioxan-2-ylmethoxy)-1-methyl-6,7-dihydro-pyrido[2,1-a] isoquinolin-4-one,
9,11-dimethoxy-2-[(tetrahydro-pyran-2-ylmethyl)-amino]-6,7-dihydro-pyrido[2,1-a]isoquinolin-4-one,
2-((S)-1-[1,4]dioxan-2-ylmethoxy)-9-hydroxy-10-methoxy-1 -methyl-6,7-dihydro-pyrido[2,1-a]isoquinolin-4-one,
8,9-dimethoxy-1-methyl-2-[(tetrahydro-pyran-2-ylmethyl)-amino]-6,7-dihydro-pyrido[2,1-a]isoquinolin-4-one,
9-benzyloxy-1-cyclopropyl-2-[(tetrahydro-furan-2-ylmethyl)-amino]-6,7-dihydro-pyrido[2,1-a]isoquinolin-4-one,
2-((S)-1-[1,4]dioxan-2-ylmethoxy)-8,11-dimethoxy-6,7-dihydro-pyrido[2,1-a]isoquinolin-4-one,
9-(3,6-dihydro-2H-pyran-4-yl)-2-((S)-1-[1,4]dioxan-2-ylmethoxy)-1-methyl-6,7-dihydro-pyrido[2,1-a]isoquinolin-4-one,
2-((S)-1-[1,4]dioxan-2-ylmethoxy)-1,7,7-trimethyl-9-(oxazol-2-ylmethoxy)-6,7-dihydro-pyrido[2,1-a]isoquinolin-4-one,
9,11-dimethoxy-2-(tetrahydro-pyran-2-ylmethoxy)-6,7-dihydro-pyrido[2,1-a]isoquinolin-4-one,
2-((S)-1-[1,4]dioxan-2-ylmethoxy)-1-methyl-9-(tetrahydro-pyran-4-yl)-6,7-dihydro-pyrido[2,1-a]isoquinolin-4-one,
9-benzyloxy-2-([1,4]dioxan-2-ylmethoxy)-1,7,7-trimethyl-6,7-dihydro-pyrido[2,1-a]isoquinolin-4-one,
[2-((S)-1-[1,4]dioxan-2-ylmethoxy)-1-methyl-4-oxo-6,7-dihydro-4H-pyrido[2,1-a] isoquinolin-9-yloxy]-acetonitrile,
9-cyclopropylethynyl-2-((S)-1-[1,4]dioxan-2-ylmethoxy)-1,7,7-trimethyl-6,7-dihydro-pyrido[2,1-a]isoquinolin-4-one,
9,10-dimethoxy-1-methyl-2-(tetrahydro-furan-2-ylmethoxy)-6,7-dihydro-pyrido[2,1-a]isoquinolin-4-one,
2-((S)-1-[1,4]dioxan-2-ylmethoxy)-1-methyl-9-(3-methyl-but-1-ynyl)-6,7-dihydro-pyrido[2,1-a]isoquinolin-4-one,
9-(5,6-dihydro-[1,4]dioxin-2-yl)-2-((S)-1-[1,4]dioxan-2-ylmethoxy)-1-methyl-6,7-dihydro-pyrido[2,1-a]isoquinolin-4-one,
2-((S)-1-[1,4]dioxan-2-ylmethoxy)-9-ethoxy-1-methyl-6,7-dihydro-pyrido[2,1-a]isoquinolin-4-one,
2-((S)-1-[1,4]dioxan-2-ylmethoxy)-1-methyl-9-(1-methyl-1H-pyrazol-4-yl)-6,7-dihydro-pyrido[2,1-a]isoquinolin-4-one,
[2-((S)-1-[1,4]dioxan-2-ylmethoxy)-1-ethyl-4-oxo-6,7-dihydro-4H-pyrido[2,1-a]isoquinolin-9-yloxy]-acetonitrile,
2-((S)-1-[1,4]dioxan-2-ylmethoxy)-1-methyl-9-(1-propyl-1H-tetrazol-5-ylmethoxy)-6,7-dihydro-pyrido[2,I-a]isoquinolin-4-one,
2-((S)-1-[1,4]dioxan-2-ylmethoxy)-8,9-dimethoxy-1-methyl-6,7-dihydro-pyrido[2,1-a]isoquinolin-4-one,
8,11-dimethoxy-2-(tetrahydro-furan-2-ylmethoxy)-6,7-dihydro-pyrido[2,1-a]isoquinolin-4-one,
9-[1,4]dioxan-2-yl-2-((S)-1-[1,4]dioxan-2-ylmethoxy)-1-methyl-6,7-dihydro-pyrido[2,1-a]isoquinolin-4-one,
2-((S)-1-[1,4]dioxan-2-ylmethoxy)-9-methoxy-1-methyl-6,7-dihydro-pyrido[2,1-a]isoquinolin-4-one,
2-((S)-1-[1,4]dioxan-2-ylmethoxy)-8-hydroxy-9-methoxy-1-methyl-6,7-dihydro-pyrido[2,1-a]isoquinolin-4-one,
9-(1-cyclopropyl-1H-tetrazol-5-ylmethoxy)-2-((S)-1-[1,4]dioxan-2-ylmethoxy)-1-methyl-6,7-dihydro-pyrido[2,1-a]isoquinolin-4-one,
2-((S)-1-[1,4]dioxan-2-ylmethoxy)-1-methyl-4-oxo-9-(2,2,2-trifluoro-ethoxy)-6,7-dihydro-4H-pyrido[2,1-a]isoquinoline-10-carbonitrile,
2,9-bis-((S)-1-[1,4]dioxan-2-ylmethoxy)-1-methyl-6,7-dihydro-pyrido[2,1-a]isoquinolin-4-one,
2-((S)-1-[1,4]dioxan-2-ylmethoxy)-9,11-dimethoxy-6,7-dihydro-pyrido[2,1-a]isoquinolin-4-one,
8,9-dimethoxy-1-methyl-2-(tetrahydro-furan-2-ylmethoxy)-6,7-dihydro-pyrido[2,1-a]isoquinolin-4-one,
2-((S)-1-[1,4]dioxan-2-ylmethoxy)-1-methyl-9-(3-methyl-oxetan-3-ylmethoxy)-6,7-dihydro-pyrido[2,1-a]isoquinolin-4-one,
9-cyclopropylethynyl-2-((S)-1-[1,4]dioxan-2-ylmethoxy)-1-ethyl-6,7-dihydro-pyrido[2,1-a]isoquinolin-4-one,
9,10-dimethoxy-2-(tetrahydro-furan-2-ylmethoxy)-6,7-dihydro-pyrido[2,1-a]isoquinolin-4-one,
2-((S)-1-[1,4]dioxan-2-ylmethoxy)-1-ethyl-9-(pyridin-2-ylmethoxy)-6,7-dihydro-pyrido[2,1-a]isoquinolin-4-one,
2-((S)-1-[1,4]dioxan-2-ylmethoxy)-1-ethyl-9-(1H-tetrazol-5ylmethoxy)-6,7-dihydro-pyrido[2,1-a]isoquinolin-4-one,
2-cyclohexylmethoxy-9,10-dimethoxy-1-methyl-6,7-dihydro-pyrido[2,1-a]isoquinolin-4-one,
9,10-dimethoxy-1-methyl-2-[(tetrahydro-pyran-3-ylmethyl)-amino]-6,7-dihydro-pyrido[2,1-a]isoquinolin-4-one,
2-((S)-1-[1,4]dioxan-2-ylmethoxy)-1-ethyl-9-(1-methyl-1H-pyrazol-4-yl)-6,7-dihydro-pyrido[2,1-a]isoquinolin-4-one,
9,10-dimethoxy-1-methyl-2-(tetrahydro-pyran-4-ylmethoxy)-6,7-dihydro-pyrido[2,1-a]isoquinolin-4-one,
9-cyclopropylmethoxy-2-((S)-1-[1,4]dioxan-2-ylmethoxy)-1,7,7-trimethyl-6,7-dihydro-pyrido[2,1-a]isoquinolin-4-one,
8,9-dimethoxy-2-(tetrahydro-furan-2-ylmethoxy)-6,7-dihydro-pyrido[2,1-a]isoquinolin-4-one,
2-((S)-1-[1,4]dioxan-2-ylmethoxy)-1-ethyl-9-(3-methoxy-but-1-ynyl)-6,7-dihydro-pyrido[2,1-a]isoquinolin-4-one,
8,9-dimethoxy-1-methyl-2-(tetrahydro-pyran-2-ylmethoxy)-6,7-dihydro-pyrido[2,1-a]isoquinolin-4-one,
9-benzyloxy-1-cyclopropyl-2-((S)-1-[1,4]dioxan-2-ylmethoxy)-6,7-dihydro-pyrido[2,1-a]isoquinolin-4-one,
2-((S)-1-[1,4]dioxan-2-ylmethoxy)-1-ethyl-9-(3-methyl-but-1-ynyl)-6,7-dihydro-pyrido[2,1-a]isoquinolin-4-one,
trifluoro-methanesulfonic acid 2-((S)-1-[1,4]dioxan-2-ylmethoxy)-1-ethyl-4-oxo-6,7-dihydro-4H-pyrido[2,1-a]isoquinolin-9-yl ester,
9-(2-cyclopropyl-ethyl)-2-((S)-1-[1,4]dioxan-2-ylmethoxy)-1-ethyl-6,7-dihydro-pyrido[2,1-a]isoquinolin-4-one,
9-cyclopropylmethoxy-2-([1,4]dioxan-2-ylmethoxy)-1-methyl-6,7-dihydro-pyrido[2,1-a] isoquinolin-4-one,
2-((S)-1-[1,4]dioxan-2-ylmethoxy)-1-ethyl-9-(3-methyl-butyl)-6,7-dihydro-pyrido[2,1-a]isoquinolin-4-one,
9,11-dimethoxy-2-(tetrahydro-furan-2-ylmethoxy)-6,7-dihydro-pyrido[2,1-a]isoquinolin-4-one,
2-((S)-1-[1,4]dioxan-2-ylmethoxy)-9-hydroxy-1,7,7-trimethyl-6,7-dihydro-pyrido[2,1-a]isoquinolin-4-one,
2-((S)-1-[1,4]dioxan-2-ylmethoxy)-9-methoxy-1-methyl-4-oxo-6,7-dihydro-4H-pyrido[2,1-a]isoquinoline-10-carbonitrile,
2-((S)-1-[1,4]dioxan-2-ylmethoxy)-1-ethyl-9-pentyl-6,7-dihydro-pyrido[2,1-a]isoquinolin-4-one,
1-cyclopropyl-2-((S)-1-[1,4]dioxan-2-ylmethoxy)-9-pentyl-6,7-dihydro-pyrido[2,1-a]isoquinolin-4-one,
9-benzyloxy-2-((S)-1-[1,4]dioxan-2-ylmethoxy)-1-isopropyl-6,7-dihydro-pyrido[2,1-a]isoquinolin-4-one,
2-((S)-1-[1,4]dioxan-2-ylmethoxy)-1-ethyl-9-(3-methoxy-butyl)-6,7-dihydro-pyrido[2,1-a]isoquinolin-4-one,
1-cyclopropyl-2-((S)-1-[1,4]dioxan-2-ylmethoxy)-9-(3-methyl-butyl)-6,7-dihydro-pyrido[2,1-a]isoquinolin-4-one,
1-cyclopropyl-9-(2-cyclopropyl-ethyl)-2-((S)-1-[1,4]dioxan-2-ylmethoxy)-6,7-dihydro-pyrido [2,1 -a] isoquinolin-4-one,
trifluoro-methanesulfonic acid 1-cyclopropyl-2-((S)-1-[1,4]dioxan-2-ylmethoxy)-4-oxo-6,7-dihydro-4H-pyrido[2,1-a]isoquinolin-9-yl ester,
trifluoro-methanesulfonic acid 2-((S)-1-[1,4]dioxan-2-ylmethoxy)-1-isopropyl-4-oxo-6,7-dihydro-4H-pyrido[2,1-a] isoquinolin-9-yl ester,
1-cyclopropyl-9-cyclopropylethynyl-2-((S)-1-[1,4]dioxan-2-ylmethoxy)-6,7-dihydro-pyrido [2,1-a]isoquinolin-4-one,
2-((S)-1-[1,4]dioxan-2-ylmethoxy)-1-isopropyl-9-(3-methyl-butyl)-6,7-dihydro-pyrido[2,1-a]isoquinolin-4-one,
2-((S)-1-[1,4]dioxan-2-ylmethoxy)-9-hydroxy-8-methoxy-1-methyl-6,7-dihydro-pyrido[2,1-a]isoquinolin-4-one,
9-(2-cyclopropyl-ethyl)-2-((S)-1-[1,4]dioxan-2-ylmethoxy)-1-isopropyl-6,7-dihydro-pyrido[2,1-a]isoquinolin-4-one,
1-cyclopropyl-2-((S)-1-[1,4]dioxan-2-ylmethoxy)-9-(3-methyl-but-1-ynyl)-6,7-dihydro-pyrido[2,1-a]isoquinolin-4-one,
2-([1,4]dioxan-2-ylmethoxy)-9-(2-methoxy-ethoxy)-1-methyl-6,7-dihydro-pyrido[2,1-a]isoquinolin-4-one,
2-((S)-1-[1,4]dioxan-2-ylmethoxy)-1-isopropyl-9-(3-methyl-but-1-ynyl)-6,7-dihydro-pyrido[2,1-a]isoquinolin-4-one,
9-cyclopropylethynyl-2-((S)-1-[1,4]dioxan-2-ylmethoxy)-1-isopropyl-6,7-dihydro-pyrido[2,1-a]isoquinolin-4-one,
2-((S)-1-[1,4]dioxan-2-ylmethoxy)-9-hydroxy-1-methyl-6,7-dihydro-pyrido[2,1-a]isoquinolin-4-one,
trifluoro-methanesulfonic acid 2-((S)-1-[1,4]dioxan-2-ylmethoxy)-1-methyl-4-oxo-6,7-dihydro-4H-pyrido[2,1-a]isoquinolin-9-yl ester,
2-((S)-1-[1,4]dioxan-2-ylmethoxy)-1-ethyl-9-hydroxy-6,7-dihydro-pyrido[2,1-a]isoquinolin-4-one,
9-methoxy-2-[(tetrahydro-furan-2-ylmethyl)-amino]-6,7-dihydro-pyrido[2,1-a]isoquinolin-4-one,
10-hydroxy-9-methoxy-1-methyl-2-[(tetrahydro-pyran-2-ylmethyl)-amino]-6,7-dihydro-pyrido[2,1-a]isoquinolin-4-one,
2-[([1,4]dioxan-2-ylmethyl)-amino]-10-hydroxy-9-methoxy-1-methyl-6,7-dihydro-pyrido[2,1-a]isoquinolin-4-one,
10-hydroxy-9-methoxy-1-methyl-2-(tetrahydro-pyran-2-ylmethoxy)-6,7-dihydro-pyrido[2,1-a]isoquinolin-4-one, and
10-benzyloxy-2-((S)-1-[1,4]dioxan-2-ylmethoxy)-9-methoxy-1-methyl-6,7-dihydro-pyrido[2,1-a]isoquinolin-4-one.

7. The compound or pharmaceutically acceptable salt thereof for use according to claim 1, wherein said compound is selected from:
4-[[(2S)-1,4-dioxan-2-yl]methoxy]-9,10-dimethoxy-1-methyl-6,7-dihydrobenzo[a]quinolizin-2-one,
9,10-dimethoxy-1-methyl-4-(tetrahydrofuran-2-ylmethylamino)-6,7-dihydrobenzo[a]quinolizin-2-one,
1-ethyl-9-hydroxy-4-(tetrahydrofuran-2-ylmethylamino)-6,7-dihydrobenzo[a]quinolizin-2-one,
9,10-Dimethoxy-4-[(tetrahydro-furan-2-ylmethyl)-amino]-6,7-dihydro-pyrido[2,1-a]isoquinolin-2-one,
4-[([1,4]Dioxan-2-ylmethyl)-amino]-9,10-dimethoxy-6,7-dihydro-pyrido[2,1-a]isoquinolin-2-one,
4-[[(2R)-1,4-dioxan-2-yl]methylamino]-9,10-dimethoxy-1-methyl-6,7-dihydrobenzo[a]quinolizin-2-one,
9-(2,2-Difluoro-cyclopropylmethoxy)-1-methyl-4-[(tetrahydro-furan-2-ylmethyl)-amino]-6,7-dihydro-pyrido[2,1-a]isoquinolin-2-one,
1-methyl-4-(tetrahydrofuran-2-ylmethylamino)-9-(2,2,2-trifluoroethoxy)-6,7-dihydrobenzo[a]quinolizin-2-one,
-Methyl-4-[(tetrahydro-furan-2-ylmethyl)-amino]-9-(tetrahydro-furan-2-yloxy)-6,7-dihydro-pyrido[2,1-a]isoquinolin-2-one,
8,9-dimethoxy-1-methyl-4-(tetrahydrofuran-2-ylmethylamino)-6,7-dihydrobenzo[a]quinolizin-2-one,
4-[[(2S)-1,4-dioxan-2-yl]methoxy]-8,9-dimethoxy-1-methyl-6,7-dihydrobenzo[a]quinolizin-2-one,
9-(2,2-difluoroethoxy)-1-methyl-4-(tetrahydropyran-2-ylmethylamino)-6,7-dihydrobenzo[a]quinolizin-2-one,
1-methyl-4-(tetrahydropyran-2-ylmethylamino)-9-(2,2,2-trifluoroethoxy)-6,7-dihydrobenzo[a]quinolizin-2-one,
8,9-dimethoxy-1-methyl-4-(tetrahydropyran-2-ylmethylamino)-6,7-dihydrobenzo[a]quinolizin-2-one,
4-(1,4-dioxan-2-ylmethylamino)-8,9-dimethoxy-1-methyl-6,7-dihydrobenzo[a]quinolizin-2-one,
4-[[(2S)-1,4-dioxan-2-yl]methoxy]-1-methyl-9-(2,2,2-trifluoroethoxy)-6,7-dihydrobenzo[a]quinolizin-2-one,
4-(1,4-dioxan-2-ylmethylamino)-8,9-dimethoxy-6,7-dihydrobenzo[a]quinolizin-2-one,
8,9-dimethoxy-4-(tetrahydrofuran-2-ylmethylamino)-6,7-dihydrobenzo[a]quinolizin-2-one,
4-(1,4-dioxan-2-ylmethylamino)-9-hydroxy-8-methoxy-6,7-dihydrobenzo[a]quinolizin-2-one,
4-(1,4-dioxan-2-ylmethylamino)-8-hydroxy-9-methoxy-6,7-dihydrobenzo[a]quinolizin-2-one,
9-(2,2-Difluoro-ethoxy)-4-[([1,4]dioxan-2-ylmethyl)-amino]-8-methoxy-1-methyl-6,7-dihydro-pyrido[2,1-a]isoquinolin-2-one,
9-(2,2-Difluoro-ethoxy)-8-methoxy-1-methyl-4-[(tetrahydro-furan-2-ylmethyl)-amino]-6,7-dihydro-pyrido [2,1 -a] isoquinolin-2-one,
4-[[(2S)-1,4-dioxan-2-yl]methoxy]-1-methyl-2-oxo-6,7-dihydrobenzo[a]quinolizine-9-carbonitrile,
9-(2,2-difluoroethoxy)-1-ethyl-4-(tetrahydrofuran-2-ylmethylamino)-6,7-dihydrobenzo[a]quinolizin-2-one,
1-ethyl-4-(tetrahydrofuran-2-ylmethylamino)-9-(2,2,2-trifluoroethoxy)-6,7-dihydrobenzo[a]quinolizin-2-one,
8,9-dimethoxy-1-methyl-4-(tetrahydrofuran-2-ylmethoxy)-6,7-dihydrobenzo[a]quinolizin-2-one,
8,9-dimethoxy-1-methyl-4-(tetrahydropyran-2-ylmethoxy)-6,7-dihydrobenzo[a]quinolizin-2-one,
4-[[(2S)-1,4-dioxan-2-yl]methoxy]-1-methyl-9-(tetrahydropyran-4-ylmethoxy)-6,7-dihydrobenzo[a]quinolizin-2-one,
4-[[(2S)-1,4-dioxan-2-yl]methoxy]-1-methyl-9-(2-pyridylmethoxy)-6,7-dihydrobenzo[a]quinolizin-2-one,
4-[[(2S)-1,4-dioxan-2-yl]methoxy]-1-methyl-9-[[3-(trifluoromethoxy)phenyl]methoxy]-6,7-dihydrobenzo[a]quinolizin-2-one,
9-benzyloxy-4-[[(2S)-1,4-dioxan-2-yl]methoxy]-1-methyl-6,7-dihydrobenzo[a]quinolizin-2-one,
9-benzyloxy-4-[[(2R)-1,4-dioxan-2-yl]methylamino]-1-methyl-6,7-dihydrobenzo[a]quinolizin-2-one,
9-(2,2-difluoroethoxy)-4-[[(2R)-1,4-dioxan-2-yl]methylamino]-1-methyl-6,7-dihydrobenzo[a]quinolizin-2-one,
4-[[(2R)-1,4-dioxan-2-yl]methylamino]-1-methyl-9-(2,2,2-trifluoroethoxy)-6,7-dihydrobenzo[a]quinolizin-2-one,
[4-[[(2S)-1,4-dioxan-2-yl]methoxy]-1-methyl-2-oxo-6,7-dihydrobenzo[a]quinolizin-9-yl] trifluoromethanesulfonate,
4-[[(2S)-1,4-dioxan-2-yl]methoxy]-1-methyl-9-[(1-methylpyrazol-4-yl)methoxy]-6,7-dihydrobenzo[a]quinolizin-2-one,
9-(3,6-dihydro-2H-pyran-4-yl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-9-(1-ethylpyrazol-4-yl)-1-methyl-6,7-dihydrobenzo[a]quinolizin-2-one,
4-[[(2S)-1,4-dioxan-2-yl]methoxy]-1-methyl-9-tetrahydropyran-4-yl-6,7-dihydrobenzo[a]quinolizin-2-one,
1-methyl-4-[[(2S)-tetrahydrofuran-2-yl]methylamino]-9-(2,2,2-trifluoroethoxy)-6,7-dihydrobenzo[a]quinolizin-2-one,
1-methyl-9-[(3-methyl-1,2,4-oxadiazol-5-yl)methoxy]-4-(tetrahydrofuran-2-ylmethoxy)-6,7-dihydrobenzo[a]quinolizin-2-one,
1-methyl-9-[(3-methyl-1,2,4-oxadiazol-5-yl)methoxy]-4-(tetrahydropyran-2-ylmethoxy)-6,7-dihydrobenzo[a]quinolizin-2-one,
1-methyl-9-(2-pyridylmethoxy)-4-(tetrahydrofuran-2-ylmethoxy)-6,7-dihydrobenzo[a]quinolizin-2-one,
1-methyl-9-(2-pyridylmethoxy)-4-(tetrahydropyran-2-ylmethoxy)-6,7-dihydrobenzo[a]quinolizin-2-one,
1-methyl-4-(tetrahydrofuran-2-ylmethoxy)-9-(tetrahydropyran-3 -ylmethoxy)-6, 7-dihydrobenzo[a]quinolizin-2-one,
1-methyl-4-(tetrahydropyran-2-ylmethoxy)-9-(tetrahydropyran-3 -ylmethoxy)-6, 7-dihydrobenzo[a]quinolizin-2-one,
4-[[(2S)-1,4-dioxan-2-yl]methoxy]-1-methyl-9-(tetrahydropyran-3-ylmethoxy)-6,7-dihydrobenzo[a]quinolizin-2-one,
1-methyl-9-[(6-methyl-3-pyridyl)methoxy]-4-(tetrahydrofuran-2-ylmethoxy)-6,7-dihydrobenzo[a]quinolizin-2-one,
1-methyl-9-[(6-methyl-3-pyridyl)methoxy]-4-(tetrahydropyran-2-ylmethoxy)-6,7-dihydrobenzo[a]quinolizin-2-one,
4-[[(2S)-1,4-dioxan-2-yl]methoxy]-1-methyl-9-[(6-methyl-3-pyridyl)methoxy]-6,7-dihydrobenzo[a]quinolizin-2-one,
9-(2-dimethylaminoethyloxy)-1-methyl-4-(tetrahydrofuran-2-ylmethoxy)-6, 7-dihydrobenzo[a]quinolizin-2-one,
9-(2-dimethylaminoethyloxy)-1-methyl-4-(tetrahydropyran-2-ylmethoxy)-6, 7-dihydrobenzo[a]quinolizin-2-one,
9-(2-dimethylaminoethyloxy)-4-[[(2S)-1,4-dioxan-2-yl]methoxy]-1-methyl-6,7-dihydrobenzo[a]quinolizin-2-one,
[4-[[(2S)-1,4-dioxan-2-yl]methoxy]-1-methyl-2-oxo-6,7-dihydrobenzo[a]quinolizin-9-yl] methanesulfonate,
1-methyl-9-(2-pyridylmethoxy)-4-[[(2S)-tetrahydrofuran-2-yl]methoxy]-6,7-dihydrobenzo[a]quinolizin-2-one,
4-[[(2S)-1,4-dioxan-2-yl]methoxy]-1-methyl-9-[(3-methyl-1,2,4-oxadiazol-5-yl)methoxy]-6,7-dihydrobenzo[a]quinolizin-2-one,
9-(difluoromethoxy)-4-[[(2S)-1,4-dioxan-2-yl]methoxy]-1-methyl-6,7-dihydrobenzo[a]quinolizin-2-one,
tert-butyl 4-[4-[[(2S)-1,4-dioxan-2-yl]methoxy]-1-methyl-2-oxo-6,7-dihydrobenzo[a]quinolizin-9-yl]piperazine-1 -carboxylate,
9-(2,2-difluoroethoxy)-4-[[(2S)-1,4-dioxan-2-yl]methoxy]-1-methyl-6,7-dihydrobenzo[a]quinolizin-2-one,
4,9-bis[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-morpholino-6,7-dihydrobenzo[a]quinolizin-2-one,
4-[[(2S)-1,4-dioxan-2-yl]methoxy]-1-methyl-9-phenylsulfanyl-6,7-dihydrobenzo[a]quinolizin-2-one,
9-(4,4-difluoro-1-piperidyl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1 -methyl-9-piperazin-1 -yl-6,7-dihydrobenzo[a]quinolizin-2-one,
9-(benzenesulfonyl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-(4-methylsulfonylpiperazin-1-yl)-6,7-dihydrobenzo[a]quinolizin-2-one,
9-[4-(cyclopropanecarbonyl)piperazin-1-yl]-4-[[(2S)-1,4-dioxan-2-yl]methoxy]-1-methyl-6,7-dihydrobenzo[a]quinolizin-2-one,
N-[4-[[(2S)-1,4-dioxan-2-yl]methoxy]-1-methyl-2-oxo-6,7-dihydrobenzo[a]quinolizin-9-yl]cyclopropanecarboxamide,
tert-butyl 3-[4-[[(2S)-1,4-dioxan-2-yl]methoxy]-1-methyl-2-oxo-6,7-dihydrobenzo[a]quinolizin-9-yl]azetidine-1-carboxylate,
4-[[(2S)-1,4-dioxan-2-yl]methoxy]-1-methyl-9-[2-(trifluoromethoxy)ethoxy]-6,7-dihydrobenzo[a]quinolizin-2-one,
N-[4-[[(2S)-1,4-dioxan-2-yl]methoxy]-1-methyl-2-oxo-6,7-dihydrobenzo[a]quinolizin-9-yl]-N-methyl-cyclopropanecarboxamide,
tert-butyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1-carboxylate,
tert-butyl 4-[4-[[(2S)-1,4-dioxan-2-yl]methoxy]-1-methyl-2-oxo-6,7-dihydrobenzo[a]quinolizin-9-yl]piperidine-1-carboxylate,
methyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1-carboxylate,
ethyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1-carboxylate,
isopropyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1-carboxylate,
2,2,2-trifluoroethyl 4-[4-[[(2S)-1,4-dioxan-2-yl]methoxy]-1-methyl-2-oxo-6,7-dihydrobenzo[a]quinolizin-9-yl]-3,6-dihydro-2H-pyridine-1-carboxylate,
methyl 4-[4-[[(2S)-1,4-dioxan-2-yl]methoxy]-1-methyl-2-oxo-6,7-dihydrobenzo[a]quinolizin-9-yl]piperidine-1-carboxylate,
ethyl 4-[4-[[(2S)-1,4-dioxan-2-yl]methoxy]-1-methyl-2-oxo-6,7-dihydrobenzo[a]quinolizin-9-yl]piperidine-1-carboxylate,
isopropyl 4-[4-[[(2S)-1,4-dioxan-2-yl]methoxy]-1-methyl-2-oxo-6,7-dihydrobenzo[a]quinolizin-9-yl]piperidine-1-carboxylate,
2,2,2-trifluoroethyl 4-[4-[[(2S)-1,4-dioxan-2-yl]methoxy]-1-methyl-2-oxo-6,7-dihydrobenzo[a]quinolizin-9-yl]piperidine-1-carboxylate,
N-cyclopropyl-4-[[(2S)-1,4-dioxan-2-yl]methoxy]-1-methyl-2-oxo-6,7-dihydrobenzo[a]quinolizine-9-carboxamide,
4-[[(2S)-1,4-dioxan-2-yl]methoxy]-9-hydroxy-1-methyl-6,7-dihydrobenzo[a]quinolizin-2-one,
9-(3,3-difluoroazetidin-1-yl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-(6-oxa-2-azaspiro[3.3]heptan-2-yl)-6,7-dihydrobenzo[a]quinolizin-2-one,
N-cyclopropyl-4-[[(2S)-1,4-dioxan-2-yl]methoxy]-N,1-dimethyl-2-oxo-6,7-dihydrobenzo[a]quinolizine-9-carboxamide,
4-[[(2S)-1,4-dioxan-2-yl]methoxy]-9-methoxy-1-methyl-6,7-dihydrobenzo[a]quinolizin-2-one,
4-(1,4-dioxan-2-ylmethoxy)-1-methyl-9-(2,2,2-trifluoroethoxy)-6,7-dihydrobenzo[a]quinolizin-2-one,
4-[[(2S)-1,4-dioxan-2-yl]methoxy]-9-(3-fluoroazetidin-1-yl)-1-methyl-6,7-dihydrobenzo[a]quinolizin-2-one,
4-[[(2S)-1,4-dioxan-2-yl]methoxy]-9-[3-(1-hydroxy-1-methyl-ethyl)azetidin-1-yl]-1-methyl-6,7-dihydrobenzo[a]quinolizin-2-one,
9-(azetidin-1-yl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-(3-methylsulfonylazetidin-1-yl)-6,7-dihydrobenzo[a]quinolizin-2-one,
4-[[(2S)-1,4-dioxan-2-yl]methoxy]-1-methyl-9-(3-pyrazol-1-ylazetidin-1-yl)-6,7-dihydrobenzo[a]quinolizin-2-one,
9-(3,3-dimethylazetidin-1-yl)-4-[[(2S)-1,4-dioxan-2-yl]methoxy]-1-methyl-6,7-dihydrobenzo[a]quinolizin-2-one,
methyl 1-[4-[[(2S)-1,4-dioxan-2-yl]methoxy]-1-methyl-2-oxo-6,7-dihydrobenzo[a]quinolizin-9-yl]azetidine-3-carboxylate,
4-[[(2S)-1,4-dioxan-2-yl]methoxy]-1-methyl-9-(3-pyridyl)-6,7-dihydrobenzo[a]quinolizin-2-one,
4-[[(2S)-1,4-dioxan-2-yl]methoxy]-9-(3-fluorophenyl)-1-methyl-6,7-dihydrobenzo[a]quinolizin-2-one,
4-[[(2S)-1,4-dioxan-2-yl]methoxy]-1-methyl-9-(4-pyridyl)-6,7-dihydrobenzo[a]quinolizin-2-one,
4-[[(2S)-1,4-dioxan-2-yl]methoxy]-1-methyl-9-(2-pyridyl)-6,7-dihydrobenzo[a]quinolizin-2-one,
4-[[(2S)-1,4-dioxan-2-yl]methoxy]-1-methyl-9-(3-methyl-2-pyridyl)-6,7-dihydrobenzo[a]quinolizin-2-one,
4-[[(2S)-1,4-dioxan-2-yl]methoxy]-1-methyl-9-(4-methyl-2-pyridyl)-6,7-dihydrobenzo[a]quinolizin-2-one,
tert-butyl 3-[[4-[[(2S)-1,4-dioxan-2-yl]methoxy]-1-methyl-2-oxo-6,7-dihydrobenzo[a]quinolizin-9-yl]oxy]azetidine-1-carboxylate,
3-deuterio-9-(1-deuterio-2,2-difluoro-vinyloxy)-4-[[(2S)-1,4-dioxan-2-yl]methoxy]-1-methyl-6,7-dihydrobenzo[a]quinolizin-2-one,
9-(1,1-dideuterio-2,2,2-trifluoro-ethoxy)-4-[[(2S)-1,4-dioxan-2-yl]methoxy]-1-methyl-6,7-dihydrobenzo[a]quinolizin-2-one,
9-benzyloxy-1-methyl-4-(oxetan-2-ylmethoxy)-6,7-dihydrobenzo[a]quinolizin-2-one,
9-benzyloxy-1-methyl-4-[[(2S)-tetrahydrofuran-2-yl]methoxy]-6,7-dihydrobenzo[a]quinolizin-2-one,
9-benzyloxy-1-methyl-4-(tetrahydropyran-2-ylmethoxy)-6,7-dihydrobenzo[a]quinolizin-2-one,
4-[[(2S)-1,4-dioxan-2-yl]methoxy] -9-(3 -methoxyazetidin-1 -yl)-1 -methyl-6,7-dihydrobenzo[a]quinolizin-2-one,
4-[[(2S)-1,4-dioxan-2-yl]methoxy]-9-(4-methoxy-1-piperidyl)-1-methyl-6,7-dihydrobenzo[a]quinolizin-2-one,
4-[[(2S)-1,4-dioxan-2-yl]methoxy]-1-methyl-9-[4-(piperidine-1-carbonyl)-1-piperidyl]-6,7-dihydrobenzo[a]quinolizin-2-one,
4-[[(2S)-1,4-dioxan-2-yl]methoxy]-1-methyl-9-(4-phenyl-1-piperidyl)-6,7-dihydrobenzo[a]quinolizin-2-one,
methyl 1-[4-[[(2S)-1,4-dioxan-2-yl]methoxy]-1-methyl-2-oxo-6,7-dihydrobenzo[a]quinolizin-9-yl]piperidine-4-carboxylate,
4-[[(2S)-1,4-dioxan-2-yl]methoxy]-9-[4-(ethoxymethyl)-1-piperidyl]-1-methyl-6,7-dihydrobenzo[a]quinolizin-2-one,
4-[[(2S)-1,4-dioxan-2-yl]methoxy]-1-methyl-9-(1-piperidyl)-6,7-dihydrobenzo[a]quinolizin-2-one,
4-[[(2S)-1,4-dioxan-2-yl]methoxy]-1-methyl-9-(3-methyl-1-piperidyl)-6,7-dihydrobenzo[a]quinolizin-2-one,
4-[[(2S)-1,4-dioxan-2-yl]methoxy]-9-[4-(4-fluorophenyl)-1-piperidyl]-1-methyl-6,7-dihydrobenzo[a]quinolizin-2-one,
9-[1-(cyclopropanecarbonyl)azetidin-3-yl]oxy-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-[1-(2,2,2-trifluoroacetyl)azetidin-3-yl]oxy-6,7-dihydrobenzo[a]quinolizin-2-one,
ethyl 3-[[4-[[(2S)-1,4-dioxan-2-yl]methoxy]-1-methyl-2-oxo-6,7-dihydrobenzo[a]quinolizin-9-yl]oxy]azetidine-1-carboxylate,
4-[[(2S)-1,4-dioxan-2-yl]methoxy]-1-methyl-9-[4-(3-pyridyloxy)-1-piperidyl]-6,7-dihydrobenzo[a]quinolizin-2-one,
1-[4-[[(2S)-1,4-dioxan-2-yl]methoxy]-1-methyl-2-oxo-6,7-dihydrobenzo[a]quinolizin-9-yl]piperidine-4-carbonitrile,
9-(3,3-difluoro-1-piperidyl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-9-isopropyl-1-methyl-6,7-dihydrobenzo[a]quinolizin-2-one,
3-deuterio-4-[[(2S)-1,4-dioxan-2-yl]methoxy]-1-methyl-9-(2,2,2-trifluoroethoxy)-6,7-dihydrobenzo[a]quinolizin-2-one,
3-deuterio-4-[[(2S)-1,4-dioxan-2-yl]methoxy]-1-methyl-9-(1,1,2,2-tetradeuterio-2-fluoro-ethoxy)-6,7-dihydrobenzo[a]quinolizin-2-one,
tert-butyl 3-[[4-[[(2S)-1,4-dioxan-2-yl]methoxy]-1-methyl-2-oxo-6,7-dihydrobenzo[a]quinolizin-9-yl]oxy]pyrrolidine-1-carboxylate,
tert-butyl 4-[[4-[[(2S)-1,4-dioxan-2-yl]methoxy]-1-methyl-2-oxo-6,7-dihydrobenzo[a]quinolizin-9-yl]oxy]piperidine-1-carboxylate,
4-[[(2S)-1,4-dioxan-2-yl]methoxy]-1-methyl-9-[methyl(3,3,3-trifluoropropyl)amino]-6,7-dihydrobenzo[a]quinolizin-2-one,
4-[[(2S)-1,4-dioxan-2-yl]methoxy]-1-methyl-9-pyrrolidin-1-yl-6,7-dihydrobenzo[a]quinolizin-2-one,
9-(3,3-difluoropyrrolidin-1-yl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-[3-(trifluoromethyl)azetidin-1-yl]-6,7-dihydrobenzo[a]quinolizin-2-one,
4-[[(2S)-1,4-dioxan-2-yl]methoxy]-1-methyl-9-[4-(trifluoromethyl)-3,6-dihydro-2H-pyridin-1-yl]-6,7-dihydrobenzo[a]quinolizin-2-one,
4-[[(2S)-1,4-dioxan-2-yl]methoxy]-1-methyl-9-[(2R)-2-methylpyrrolidin-1-yl]-6,7-dihydrobenzo [a] quinolizin-2-one,
4-[[(2S)-1,4-dioxan-2-yl]methoxy]-9-(3-fluoro-1-piperidyl)-1-methyl-6,7-dihydrobenzo[a]quinolizin-2-one,
9-carbazol-9-yl-4-[[(2S)-1,4-dioxan-2-yl]methoxy]-1-methyl-6,7-dihydrobenzo[a]quinolizin-2-one,
9-(3,5-dimethyl-1-piperidyl)-4-[[(2S)-1,4-dioxan-2-yl]methoxy]-1-methyl-6,7-dihydrobenzo[a]quinolizin-2-one,
9-(3,3-dimethylpyrrolidin-1-yl)-4-[[(2S)-1,4-dioxan-2-yl]methoxy]-1-methyl-6,7-dihydrobenzo[a]quinolizin-2-one,
9-(4,4-dimethyl-1-piperidyl)-4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-[4-(trifluoromethyl)-1-piperidyl]-6,7-dihydrobenzo[a]quinolizin-2-one,
4-[[(2S)-1,4-dioxan-2-yl]methoxy]-1-methyl-9-(4-methyl-1-piperidyl)-6,7-dihydrobenzo[a]quinolizin-2-one,
4-[[(2S)-1,4-dioxan-2-yl]methoxy]-1-methyl-9-(2,2,2-trifluoroethylamino)-6,7-dihydrobenzo[a]quinolizin-2-one,
4-[[(2S)-1,4-dioxan-2-yl]methoxy]-1-methyl-9-(2-methyl-1-piperidyl)-6,7-dihydrobenzo[a]quinolizin-2-one,
9-[1-(cyclopropanecarbonyl)pyrrolidin-3-yl]oxy-4-[[(2S)-1,4-dioxan-2-yl]methoxy]-1-methyl-6,7-dihydrobenzo[a]quinolizin-2-one,
ethyl 3-[[4-[[(2S)-1,4-dioxan-2-yl]methoxy]-1-methyl-2-oxo-6,7-dihydrobenzo[a]quinolizin-9-yl]oxy]pyrrolidine-1-carboxylate,
9-[1-(cyclopropanecarbonyl)azetidin-3-yl]-4-[[(2S)-1,4-dioxan-2-yl]methoxy]-1-methyl-6,7-dihydrobenzo[a]quinolizin-2-one,
ethyl 3-[4-[[(2S)-1,4-dioxan-2-yl]methoxy]-1-methyl-2-oxo-6,7-dihydrobenzo[a]quinolizin-9-yl]azetidine-1-carboxylate,
3-[4-[[(2S)-1,4-dioxan-2-yl]methoxy]-1-methyl-2-oxo-6,7-dihydrobenzo[a]quinolizin-9-yl]-N,N-dimethyl-azetidine-1-carboxamide,
3-[4-[[(2S)-1,4-dioxan-2-yl]methoxy]-1-methyl-2-oxo-6,7-dihydrobenzo[a]quinolizin-9-yl]-N-isopropyl-azetidine-1-carboxamide,
3-[[4-[[(2S)-1,4-dioxan-2-yl]methoxy]-1-methyl-2-oxo-6,7-dihydrobenzo[a]quinolizin-9-yl]oxy]-N,N-dimethyl-azetidine-1-carboxamide,
3-[4-[[(2S)-1,4-dioxan-2-yl]methoxy]-1-methyl-2-oxo-6,7-dihydrobenzo[a]quinolizin-9-yl]-N-isopropyl-azetidine-1-carboxamide,
9-benzyloxy-4-[(4,4-dimethyloxetan-2-yl)methoxy]-1-methyl-6,7-dihydrobenzo[a]quinolizin-2-one,
9-benzyloxy-1-methyl-4-[(2-methyltetrahydrofuran-2-yl)methoxy]-6,7-dihydrobenzo[a]quinolizin-2-one,
9-benzyloxy-4-[(5,5-dimethyltetrahydrofuran-2-yl)methoxy]-1-methyl-6,7-dihydrobenzo[a]quinolizin-2-one,
4-[[(2S)-1,4-dioxan-2-yl]methoxy]-1-methyl-9-(2,2,3,3,3-pentafluoropropoxy)-6,7-dihydrobenzo[a]quinolizin-2-one,
4-[[(2S)-1,4-dioxan-2-yl]methoxy]-1-methyl-6,7-dihydrobenzo[a]quinolizin-2-one,
4-[[(2S)-1,4-dioxan-2-yl]methoxy]-1-methyl-9-(2-oxopyrrolidin-1-yl)-6,7-dihydrobenzo[a]quinolizin-2-one, and
3-deuterio-4-[[(2S)-1,4-dioxan-2-yl]methoxy]-1-methyl-9-(1,1,2,2-tetradeuterio-2-fluoro-ethoxy)-6,7-dihydrobenzo[a]quinolizin-2-one.

8. The compound or pharmaceutically acceptable salt thereof for use according to claim 1, wherein said compound is:

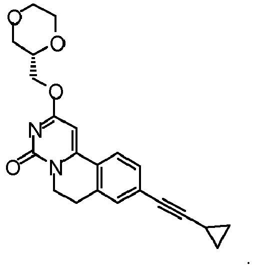

9. A pharmaceutical composition for use in the prophylaxis and/or treatment of one or more fibrotic diseases, wherein the fibrotic disease is IPF, non-alcoholic steatohepatitis (NASH), or renal fibrosis, comprising a compound or pharmaceutically acceptable salt thereof according to anyone of claims 1-8, and a pharmaceutically acceptable carrier.

10. A pharmaceutical composition for use according to claim 9, comprising a further therapeutic agent.

11. A pharmaceutical composition for use according to claim 10, wherein the further therapeutic agent is a fibrotic disease treatment agent.
